# EUROPEAN PATENT SPECIFICATION

(11) **EP 3 665 192 B1**
(45) Date of publication and mention of the grant of the patent: **12.07.2023**
(21) Application number: 18756531.2
(22) Date of filing: 10.08.2018
(51) Int. Cl.: C07K 14/79, C07K 14/705, A61K 38/00, A61K 38/40, A61K 39/00, A61K 47/68, A61P 25/28, C07K 16/00, C07K 16/28, C07K 16/40

(54) **ENGINEERED TRANSFERRIN RECEPTOR BINDING POLYPEPTIDES**
MANIPULIERTE, AN TRANSFERRINREZEPTOR BINDENDE POLYPEPTIDE
POLYPEPTIDES DE LIAISON AU RÉCEPTEUR DE TRANSFERRINE MODIFIÉS

(30) Priority: 10.08.2017 US 201762543658 P; 08.11.2017 US 201762583314 P; 15.02.2018 WO PCT/US2018/018371
(43) Date of publication of application: 17.06.2020
(73) Proprietor: Denali Therapeutics Inc., South San Francisco, California 94080 (US)
(72) Inventor: CHEN, Xiaocheng, South San Francisco California 94080 (US); DENNIS, Mark S., South San Francisco California 94080 (US); KARIOLIS, Mihalis, South San Francisco California 94080 (US); SILVERMAN, Adam P., South San Francisco California 94080 (US); SRIVASTAVA, Ankita, South San Francisco California 94080 (US); WATTS, Ryan J., South San Francisco California 94080 (US); WELLS, Robert C., South San Francisco California 94080 (US); ZUCHERO, Joy Yu, South San Francisco California 94080 (US)
(74) Representative: J A Kemp LLP
(86) International application number: PCT/US2018/046199
(87) International publication number: WO 2019/032955

(56) References cited:
- WO-A1-2014/033074
- WO-A1-2016/207240
- US-A1- 2012 276 125
- PARDRIDGE WILLIAM M: "Blood-brain barrier drug delivery of IgG fusion proteins with a transferrin receptor monoclonal antibody", EXPERT OPINION ON DRUG DELI, INFORMA HEALTHCARE, UK, vol. 12, no. 2, 1 February 2015 (2015-02-01), pages 207-222, XP009183494, ISSN: 1744-7593, DOI: 10.1517/17425247.2014.952627
- ELISABETH LOBNER ET AL: "Engineered IgG1-Fc - one fragment to bind them all", IMMUNOLOGICAL REVIEWS., vol. 270, no. 1, 1 March 2016 (2016-03-01) , pages 113-131, XP055416736, US ISSN: 0105-2896, DOI: 10.1111/imr.12385
- HONGYAN LIU ET AL: "Fc Engineering for Developing Therapeutic Bispecific Antibodies and Novel Scaffolds", FRONTIERS IN IMMUNOLOGY, vol. 8, 26 January 2017 (2017-01-26), XP055396345, DOI: 10.3389/fimmu.2017.00038
- PARK HYE IN ET AL: "The Highly Evolvable Antibody Fc Domain", TRENDS IN BIOTECHNOLOGY, vol. 34, no. 11, November 2016 (2016-11), pages 895-908, XP029778354, ISSN: 0167-7799, DOI: 10.1016/J.TIBTECH.2016.04.005
- WOZNIAK-KNOPP G ET AL: "Introducing antigen-binding sites in structural loops of immunoglobulin constant domains: Fc fragments with engineered HER2/neu-binding sites and antibody properties", PROTEIN ENGINEERING, DESIGN AND SELEC, OXFORD JOURNAL, LONDON, GB, vol. 23, no. 4, 1 April 2010 (2010-04-01), pages 289-297, XP009137665, ISSN: 1741-0126, DOI: 10.1093/PROTEIN/GZQ005 [retrieved on 2010-02-11]

## Description

### CROSS-REFERENCE TO RELATED APPLICATIONS

The present application claims priority to U.S. Provisional Patent Application No. 62/543,658, filed on August 10, 2017, U.S. Provisional Patent Application No. 62/583,314, filed on November 8, 2017, and International Patent Application No. PCT/US2018/018371, filed on February 15, 2018.

### BACKGROUND

Various techniques have been developed that engineer a protein to bind to a target that it does not normally bind. For example, libraries can be generated to screen for engineered proteins with desired binding or enzymatic activity.

Transferrin receptor is a carrier protein for transferrin that, among other functions, is needed for the import of iron into the cell and is regulated in response to intracellular iron concentration. Transferrin receptors are expressed on endothelia, including the endothelium of the blood-brain barrier, and are expressed at increased levels on various cancer cells and inflammatory cells. It is one of the receptors that mediates transcytosis of cognate ligands across the blood-brain barrier. Transferrin receptors can thus be desirable targets for introducing an agent into a cell for either endocytosis in the cell or transcytosis across the cell.

Pardridge et al. Expert Opin Drug Deliv., 12(2): 207-22, 2015 purports to disclose blood-brain barrier drug delivery of IgG fusion proteins with a transferrin receptor monoclonal antibody. Lobner et al. Immunol Rev., 270(1): 113-3 1, 2016 purports to disclose engineered IgG1-Fc fragments as a scaffold for the design of novel therapeutics.

### BRIEF SUMMARY

We have developed polypeptides that include CH3 domains that are capable of binding a transferrin receptor (TfR). These polypeptides have been engineered with substitutions in the CH3 domain that generate a novel TfR binding site. TfR is highly expressed on the blood-brain barrier (BBB), and TfR naturally moves transferrin from the blood into the brain. Because these polypeptides bind TfR, they too can be transported across the BBB and further can be used to transport attached therapeutic agents (*e.g*., therapeutic polypeptides, antibody variable regions such as Fabs, and small molecules) across the BBB. This approach can substantially improve brain uptake of the therapeutic agents and is therefore highly useful for treating disorders and diseases where brain delivery is advantageous.

In one aspect, the disclosure features a polypeptide that specifically binds to a transferrin receptor, comprising a sequence having at least 85%, 90%, or 95% sequence identity to the sequence of any one of SEQ ID NOS:83, 84, 191, 43, and 82, wherein the polypeptide comprises Glu at position 153, Tyr or Phe at position 157, Thr at position 159, Glu at position 160, Trp at position 161, Val, Ser, or Ala at position 162, Asn at position 163, Thr or Ser at position 186, Glu at position 188, Glu at position 189, and Phe at position 194, as numbered with reference to SEQ ID NO: 1.

In some embodiments, the polypeptide further comprises Trp at position 139, as numbered with reference to SEQ ID NO:1. In some embodiments, the polypeptide further comprises Ala at position 7, Ala at position 8, and Trp at position 139, as numbered with reference to SEQ ID NO:1. In some embodiments, the polypeptide further comprises Ala at position 7, Ala at position 8, Trp at position 139, Leu at position 201, and Ser at position 207, as numbered with reference to SEQ ID NO:1. In some embodiments, the polypeptide further comprises Ser at position 139, Ala at position 141, and Val at position 180, as numbered with reference to SEQ ID NO:1. In some embodiments, the polypeptide further comprises Ala at position 7, Ala at position 8, Ser at position 139, Ala at position 141, and Val at position 180, as numbered with reference to SEQ ID NO:1. In some embodiments, the polypeptide further comprises Ala at position 7, Ala at position 8, Ser at position 139, Ala at position 141, Val at position 180, Leu at position 201, and Ser at position 207, as numbered with reference to SEQ ID NO: 1.

In some embodiments, the polypeptide does not comprise the first three amino acids "PCP" at the amino-terminal end of the sequence (*e.g*., the polypeptide comprises a sequence having at least 85%, 90%, or 95% sequence identity to the sequence of any one of SEQ ID NOS:83, 84, 191, 43, and 82 without the first three amino acids "PCP" at the amino-terminal end).

In some embodiments, the polypeptide further comprises an antibody heavy chain variable region.

In another aspect, the disclosure features a polypeptide that specifically binds to a transferrin receptor, comprising a sequence having at least 85%, 90%, or 95% sequence identity to the sequence of SEQ ID NO:83, wherein the polypeptide comprises Glu at position 153, Tyr at position 157, Thr at position 159, Glu at position 160, Trp at position 161, Val at position 162, Asn at position 163, Thr at position 186, Glu at position 188, Glu at position 189, and Phe at position 194, as numbered with reference to SEQ ID NO:1.

In some embodiments, the polypeptide further comprises Trp at position 139, as numbered with reference to SEQ ID NO:1. In certain embodiments, the polypeptide comprises the sequence of SEQ ID NO:133.

In some embodiments, the polypeptide further comprises Ala at position 7, Ala at position 8, and Trp at position 139, as numbered with reference to SEQ ID NO:1. In certain embodiments, the polypeptide comprises the sequence of SEQ ID NO: 134.

In some embodiments, the polypeptide further comprises Ala at position 7, Ala at position 8, Trp at position 139, Leu at position 201, and Ser at position 207, as numbered with reference to SEQ ID NO:1. In certain embodiments, the polypeptide comprises the sequence of SEQ ID NO:260.

In some embodiments, the polypeptide further comprises Ser at position 139, Ala at position 141, and Val at position 180, as numbered with reference to SEQ ID NO:1. In certain embodiments, the polypeptide comprises the sequence of SEQ ID NO:139.

In some embodiments, the polypeptide further comprises Ala at position 7, Ala at position 8, Ser at position 139, Ala at position 141, and Val at position 180, as numbered with reference to SEQ ID NO:1. In certain embodiments, the polypeptide comprises the sequence of SEQ ID NO:140.

In some embodiments, the polypeptide further comprises Ala at position 7, Ala at position 8, Ser at position 139, Ala at position 141, Val at position 180, Leu at position 201, and Ser at position 207, as numbered with reference to SEQ ID NO:1. In certain embodiments, the polypeptide comprises the sequence of SEQ ID NO:263.

In some embodiments, the polypeptide does not comprise the first three amino acids "PCP" at the amino-terminal end of the sequence (*e.g*., the polypeptide comprises a sequence having at least 85%, 90%, or 95% sequence identity to the sequence of any one of SEQ ID NOS:83, 133, 134, 260, 139, 140, and 263 without the first three amino acids "PCP" at the amino-terminal end).

In some embodiments, the polypeptide further comprises an antibody heavy chain variable region.

In another aspect, the disclosure features a polypeptide that specifically binds to a transferrin receptor, comprising a sequence having at least 85%, 90%, or 95% sequence identity to the sequence of SEQ ID NO:84, wherein the polypeptide comprises Glu at position 153, Tyr at position 157, Thr at position 159, Glu at position 160, Trp at position 161, Ser at position 162, Asn at position 163, Ser at position 186, Glu at position 188, Glu at position 189, and Phe at position 194, as numbered with reference to SEQ ID NO: 1.

In some embodiments, the polypeptide further comprises Trp at position 139, as numbered with reference to SEQ ID NO:1. In certain embodiments, the polypeptide comprises the sequence of SEQ ID NO: 145.

In some embodiments, the polypeptide further comprises Ala at position 7, Ala at position 8, and Trp at position 139, as numbered with reference to SEQ ID NO:1. In certain embodiments, the polypeptide comprises the sequence of SEQ ID NO: 146.

In some embodiments, the polypeptide further comprises Ala at position 7, Ala at position 8, Trp at position 139, Leu at position 201, and Ser at position 207, as numbered with reference to SEQ ID NO:1. In certain embodiments, the polypeptide comprises the sequence of SEQ ID NO:267.

In some embodiments, the polypeptide further comprises Ser at position 139, Ala at position 141, and Val at position 180, as numbered with reference to SEQ ID NO:1. In certain embodiments, the polypeptide comprises the sequence of SEQ ID NO:151.

In some embodiments, the polypeptide further comprises Ala at position 7, Ala at position 8, Ser at position 139, Ala at position 141, and Val at position 180, as numbered with reference to SEQ ID NO:1. In certain embodiments, the polypeptide comprises the sequence of SEQ ID NO:152.

In some embodiments, the polypeptide further comprises Ala at position 7, Ala at position 8, Ser at position 139, Ala at position 141, Val at position 180, Leu at position 201, and Ser at position 207, as numbered with reference to SEQ ID NO:1. In certain embodiments, the polypeptide comprises the sequence of SEQ ID NO:270.

In some embodiments, the polypeptide does not comprise the first three amino acids "PCP" at the amino-terminal end of the sequence (e.g., the polypeptide comprises a sequence having at least 85%, 90%, or 95% sequence identity to the sequence of any one of SEQ ID NOS:84, 145, 146, 267, 151, 152, and 270 without the first three amino acids "PCP" at the amino-terminal end).

In some embodiments, the polypeptide further comprises an antibody heavy chain variable region.

In another aspect, the disclosure features a polypeptide that specifically binds to a transferrin receptor, comprising a sequence having at least 85%, 90%, or 95% sequence identity to the sequence of SEQ ID NO: 191, wherein the polypeptide comprises Glu at position 153, Phe at position 157, Thr at position 159, Glu at position 160, Trp at position 161, Ser at position 162, Asn at position 163, Ser at position 186, Glu at position 188, Glu at position 189, and Phe at position 194, as numbered with reference to SEQ ID NO: 1.

In some embodiments, the polypeptide further comprises Trp at position 139, as numbered with reference to SEQ ID NO:1. In certain embodiments, the polypeptide comprises the sequence of SEQ ID NO:232.

In some embodiments, the polypeptide further comprises Ala at position 7, Ala at position 8, and Trp at position 139, as numbered with reference to SEQ ID NO:1. In certain embodiments, the polypeptide comprises the sequence of SEQ ID NO:233.

In some embodiments, the polypeptide further comprises Ala at position 7, Ala at position 8, Trp at position 139, Leu at position 201, and Ser at position 207, as numbered with reference to SEQ ID NO:1. In certain embodiments, the polypeptide comprises the sequence of SEQ ID NO:309.

In some embodiments, the polypeptide further comprises Ser at position 139, Ala at position 141, and Val at position 180, as numbered with reference to SEQ ID NO:1. In certain embodiments, the polypeptide comprises the sequence of SEQ ID NO:238.

In some embodiments, the polypeptide further comprises Ala at position 7, Ala at position 8, Ser at position 139, Ala at position 141, and Val at position 180, as numbered with reference to SEQ ID NO:1. In certain embodiments, the polypeptide comprises the sequence of SEQ ID NO:239.

In some embodiments, the polypeptide further comprises Ala at position 7, Ala at position 8, Ser at position 139, Ala at position 141, Val at position 180, Leu at position 201, and Ser at position 207, as numbered with reference to SEQ ID NO:1. In certain embodiments, the polypeptide comprises the sequence of SEQ ID NO:312.

In some embodiments, the polypeptide does not comprise the first three amino acids "PCP" at the amino-terminal end of the sequence (*e.g.*, the polypeptide comprises a sequence having at least 85%, 90%, or 95% sequence identity to the sequence of any one of SEQ ID NOS:191, 232, 233, 309, 238, 239, and 312 without the first three amino acids "PCP" at the amino-terminal end).

In some embodiments, the polypeptide further comprises an antibody heavy chain variable region.

In another aspect, the disclosure features a polypeptide that specifically binds to a transferrin receptor, comprising a sequence having at least 85%, 90%, or 95% sequence identity to the sequence of SEQ ID NO:43, wherein the polypeptide comprises Glu at position 153, Tyr at position 157, Thr at position 159, Glu at position 160, Trp at position 161, Ser at position 162, Asn at position 163, Thr at position 186, Glu at position 188, Glu at position 189, and Phe at position 194, as numbered with reference to SEQ ID NO:1.

In some embodiments, the polypeptide further comprises Trp at position 139, as numbered with reference to SEQ ID NO:1. In certain embodiments, the polypeptide comprises the sequence of SEQ ID NO: 169.

In some embodiments, the polypeptide further comprises Ala at position 7, Ala at position 8, and Trp at position 139, as numbered with reference to SEQ ID NO:1. In certain embodiments, the polypeptide comprises the sequence of SEQ ID NO:170.

In some embodiments, the polypeptide further comprises Ala at position 7, Ala at position 8, Trp at position 139, Leu at position 201, and Ser at position 207, as numbered with reference to SEQ ID NO:1. In certain embodiments, the polypeptide comprises the sequence of SEQ ID NO:281.

In some embodiments, the polypeptide further comprises Ser at position 139, Ala at position 141, and Val at position 180, as numbered with reference to SEQ ID NO:1. In certain embodiments, the polypeptide comprises the sequence of SEQ ID NO:175.

In some embodiments, the polypeptide further comprises Ala at position 7, Ala at position 8, Ser at position 139, Ala at position 141, and Val at position 180, as numbered with reference to SEQ ID NO:1. In certain embodiments, the polypeptide comprises the sequence of SEQ ID NO:176.

In some embodiments, the polypeptide further comprises Ala at position 7, Ala at position 8, Ser at position 139, Ala at position 141, Val at position 180, Leu at position 201, and Ser at position 207, as numbered with reference to SEQ ID NO:1. In certain embodiments, the polypeptide comprises the sequence of SEQ ID NO:284.

In some embodiments, the polypeptide does not comprise the first three amino acids "PCP" at the amino-terminal end of the sequence (*e.g*., the polypeptide comprises a sequence having at least 85%, 90%, or 95% sequence identity to the sequence of any one of SEQ ID NOS:43, 169, 170, 281, 175, 176, and 284 without the first three amino acids "PCP" at the amino-terminal end).

In some embodiments, the polypeptide further comprises an antibody heavy chain variable region.

In another aspect, the disclosure features a polypeptide that specifically binds to a transferrin receptor, comprising a sequence having at least 85%, 90%, or 95% sequence identity to the sequence of SEQ ID NO:82, wherein the polypeptide comprises Glu at position 153, Tyr at position 157, Thr at position 159, Glu at position 160, Trp at position 161, Ala at position 162, Asn at position 163, Thr at position 186, Glu at position 188, Glu at position 189, and Phe at position 194, as numbered with reference to SEQ ID NO:1.

In some embodiments, the polypeptide further comprises Trp at position 139, as numbered with reference to SEQ ID NO:1. In certain embodiments, the polypeptide comprises the sequence of SEQ ID NO:121.

In some embodiments, the polypeptide further comprises Ala at position 7, Ala at position 8, and Trp at position 139, as numbered with reference to SEQ ID NO:1. In certain embodiments, the polypeptide comprises the sequence of SEQ ID NO:122.

In some embodiments, the polypeptide further comprises Ala at position 7, Ala at position 8, Trp at position 139, Leu at position 201, and Ser at position 207, as numbered with reference to SEQ ID NO:1. In certain embodiments, the polypeptide comprises the sequence of SEQ ID NO:253.

In some embodiments, the polypeptide further comprises Ser at position 139, Ala at position 141, and Val at position 180, as numbered with reference to SEQ ID NO:1. In certain embodiments, the polypeptide comprises the sequence of SEQ ID NO:127.

In some embodiments, the polypeptide further comprises Ala at position 7, Ala at position 8, Ser at position 139, Ala at position 141, and Val at position 180, as numbered with reference to SEQ ID NO:1. In certain embodiments, the polypeptide comprises the sequence of SEQ ID NO:128.

In some embodiments, the polypeptide further comprises Ala at position 7, Ala at position 8, Ser at position 139, Ala at position 141, Val at position 180, Leu at position 201, and Ser at position 207, as numbered with reference to SEQ ID NO:1. In certain embodiments, the polypeptide comprises the sequence of SEQ ID NO:256.

In some embodiments, the polypeptide does not comprise the first three amino acids "PCP" at the amino-terminal end of the sequence (*e.g*., the polypeptide comprises a sequence having at least 85%, 90%, or 95% sequence identity to the sequence of any one of SEQ ID NOS:82, 121, 122, 253, 127, 128, and 256 without the first three amino acids "PCP" at the amino-terminal end).

In some embodiments, the polypeptide further comprises an antibody heavy chain variable region.

In another aspect, the disclosure features a polypeptide that specifically binds to a transferrin receptor, comprising the sequence of SEQ ID NO:347. In another aspect, the disclosure features a polypeptide that specifically binds to a transferrin receptor, comprising the sequence of SEQ ID NO:348. In some embodiments, the polypeptide further comprises an antibody heavy chain variable region.

In another aspect, the disclosure features a polypeptide comprising from N- to C-terminus: an antibody heavy chain variable region, a CH1 domain, a hinge region, and a polypeptide described herein.

In another aspect, the disclosure features an Fc polypeptide dimer, or a dimeric fragment thereof, comprising:
(a) a first Fc polypeptide comprising a polypeptide described herein; and
(b) a second Fc polypeptide capable of dimerizing with the first Fc polypeptide of (a).

In some embodiments, the dimer is monovalent for TfR binding. In some embodiments, the second Fc polypeptide comprises the sequence of any one of SEQ ID NOS:351-356.

In certain embodiments, the first Fc polypeptide comprises the sequence of any one of SEQ ID NOS:133, 134, and 260; and the second Fc polypeptide comprises the sequence of any one of SEQ ID NOS:354-356.

In certain embodiments, the first Fc polypeptide comprises the sequence of any one of SEQ ID NOS:145, 146, and 267; and the second Fc polypeptide comprises the sequence of any one of SEQ ID NOS:354-356.

In certain embodiments, the first Fc polypeptide comprises the sequence of any one of SEQ ID NOS:232, 233, and 309; and the second Fc polypeptide comprises the sequence of any one of SEQ ID NOS:354-356.

In certain embodiments, the first Fc polypeptide comprises the sequence of any one of SEQ ID NOS:169, 170, and 281; and the second Fc polypeptide comprises the sequence of any one of SEQ ID NOS:354-356.

In certain embodiments, the first Fc polypeptide comprises the sequence of any one of SEQ ID NOS:121, 122, and 253; and the second Fc polypeptide comprises the sequence of any one of SEQ ID NOS:354-356.

In certain embodiments, the first Fc polypeptide comprises the sequence of any one of SEQ ID NOS:139, 140, and 263; and the second Fc polypeptide comprises the sequence of any one of SEQ ID NOS:351-353.

In certain embodiments, the first Fc polypeptide comprises the sequence of any one of SEQ ID NOS:151, 152, and 270; and the second Fc polypeptide comprises the sequence of any one of SEQ ID NOS:351-353.

In certain embodiments, the first Fc polypeptide comprises the sequence of any one of SEQ ID NOS:238, 239, and 312; and the second Fc polypeptide comprises the sequence of any one of SEQ ID NOS:351-353.

In certain embodiments, the first Fc polypeptide comprises the sequence of any one of SEQ ID NOS:175, 176, and 284; and the second Fc polypeptide comprises the sequence of any one of SEQ ID NOS:351-353.

In certain embodiments, the first Fc polypeptide comprises the sequence of any one of SEQ ID NOS:127, 128, and 256; and the second Fc polypeptide comprises the sequence of any one of SEQ ID NOS:351-353.

In certain embodiments, the first and second Fc polypeptides in the dimer comprise the following sequences:
(a) the first Fc polypeptide comprises the sequence of SEQ ID NO:134 and the second Fc polypeptide comprises the sequence of SEQ ID NO:355; or
(b) the first Fc polypeptide comprises the sequence of SEQ ID NO:134 and the second Fc polypeptide comprises the sequence of SEQ ID NO:356; or
(c) the first Fc polypeptide comprises the sequence of SEQ ID NO:260 and the second Fc polypeptide comprises the sequence of SEQ ID NO:355; or
(d) the first Fc polypeptide comprises the sequence of SEQ ID NO:260 and the second Fc polypeptide comprises the sequence of SEQ ID NO:356; or
(e) the first Fc polypeptide comprises the sequence of SEQ ID NO:140 and the second Fc polypeptide comprises the sequence of SEQ ID NO:352; or
(f) the first Fc polypeptide comprises the sequence of SEQ ID NO:140 and the second Fc polypeptide comprises the sequence of SEQ ID NO:353; or
(g) the first Fc polypeptide comprises the sequence of SEQ ID NO:263 and the second Fc polypeptide comprises the sequence of SEQ ID NO:352; or
(h) the first Fc polypeptide comprises the sequence of SEQ ID NO:263 and the second Fc polypeptide comprises the sequence of SEQ ID NO:353.

In certain embodiments, the first and second Fc polypeptides in the dimer comprise the following sequences:
(a) the first Fc polypeptide comprises the sequence of SEQ ID NO:146 and the second Fc polypeptide comprises the sequence of SEQ ID NO:355; or
(b) the first Fc polypeptide comprises the sequence of SEQ ID NO:146 and the second Fc polypeptide comprises the sequence of SEQ ID NO:356; or
(c) the first Fc polypeptide comprises the sequence of SEQ ID NO:267 and the second Fc polypeptide comprises the sequence of SEQ ID NO:355; or
(d) the first Fc polypeptide comprises the sequence of SEQ ID NO:267 and the second Fc polypeptide comprises the sequence of SEQ ID NO:356; or
(e) the first Fc polypeptide comprises the sequence of SEQ ID NO:152 and the second Fc polypeptide comprises the sequence of SEQ ID NO:352; or
(f) the first Fc polypeptide comprises the sequence of SEQ ID NO:152 and the second Fc polypeptide comprises the sequence of SEQ ID NO:353; or
(g) the first Fc polypeptide comprises the sequence of SEQ ID NO:270 and the second Fc polypeptide comprises the sequence of SEQ ID NO:352; or
(h) the first Fc polypeptide comprises the sequence of SEQ ID NO:270 and the second Fc polypeptide comprises the sequence of SEQ ID NO:353.

In certain embodiments, the first and second Fc polypeptides in the dimer comprise the following sequences:
(a) the first Fc polypeptide comprises the sequence of SEQ ID NO:233 and the second Fc polypeptide comprises the sequence of SEQ ID NO:355; or
(b) the first Fc polypeptide comprises the sequence of SEQ ID NO:233 and the second Fc polypeptide comprises the sequence of SEQ ID NO:356; or
(c) the first Fc polypeptide comprises the sequence of SEQ ID NO:309 and the second Fc polypeptide comprises the sequence of SEQ ID NO:355; or
(d) the first Fc polypeptide comprises the sequence of SEQ ID NO:309 and the second Fc polypeptide comprises the sequence of SEQ ID NO:356; or
(e) the first Fc polypeptide comprises the sequence of SEQ ID NO:239 and the second Fc polypeptide comprises the sequence of SEQ ID NO:352; or
(f) the first Fc polypeptide comprises the sequence of SEQ ID NO:239 and the second Fc polypeptide comprises the sequence of SEQ ID NO:353; or
(g) the first Fc polypeptide comprises the sequence of SEQ ID NO:312 and the second Fc polypeptide comprises the sequence of SEQ ID NO:352; or
(h) the first Fc polypeptide comprises the sequence of SEQ ID NO:312 and the second Fc polypeptide comprises the sequence of SEQ ID NO:353.

In certain embodiments, the first and second Fc polypeptides in the dimer comprise the following sequences:
(a) the first Fc polypeptide comprises the sequence of SEQ ID NO:170 and the second Fc polypeptide comprises the sequence of SEQ ID NO:355; or
(b) the first Fc polypeptide comprises the sequence of SEQ ID NO:170 and the second Fc polypeptide comprises the sequence of SEQ ID NO:356; or
(c) the first Fc polypeptide comprises the sequence of SEQ ID NO:281 and the second Fc polypeptide comprises the sequence of SEQ ID NO:355; or
(d) the first Fc polypeptide comprises the sequence of SEQ ID NO:281 and the second Fc polypeptide comprises the sequence of SEQ ID NO:356; or
(e) the first Fc polypeptide comprises the sequence of SEQ ID NO:176 and the second Fc polypeptide comprises the sequence of SEQ ID NO:352; or
(f) the first Fc polypeptide comprises the sequence of SEQ ID NO:176 and the second Fc polypeptide comprises the sequence of SEQ ID NO:353; or
(g) the first Fc polypeptide comprises the sequence of SEQ ID NO:284 and the second Fc polypeptide comprises the sequence of SEQ ID NO:352; or
(h) the first Fc polypeptide comprises the sequence of SEQ ID NO:284 and the second Fc polypeptide comprises the sequence of SEQ ID NO:353.

In certain embodiments, the first and second Fc polypeptides in the dimer comprise the following sequences:
(a) the first Fc polypeptide comprises the sequence of SEQ ID NO:122 and the second Fc polypeptide comprises the sequence of SEQ ID NO:355; or
(b) the first Fc polypeptide comprises the sequence of SEQ ID NO:122 and the second Fc polypeptide comprises the sequence of SEQ ID NO:356; or
(c) the first Fc polypeptide comprises the sequence of SEQ ID NO:253 and the second Fc polypeptide comprises the sequence of SEQ ID NO:355; or
(d) the first Fc polypeptide comprises the sequence of SEQ ID NO:253 and the second Fc polypeptide comprises the sequence of SEQ ID NO:356; or
(e) the first Fc polypeptide comprises the sequence of SEQ ID NO:128 and the second Fc polypeptide comprises the sequence of SEQ ID NO:352; or
(f) the first Fc polypeptide comprises the sequence of SEQ ID NO:128 and the second Fc polypeptide comprises the sequence of SEQ ID NO:353; or
(g) the first Fc polypeptide comprises the sequence of SEQ ID NO:256 and the second Fc polypeptide comprises the sequence of SEQ ID NO:352; or
(h) the first Fc polypeptide comprises the sequence of SEQ ID NO:256 and the second Fc polypeptide comprises the sequence of SEQ ID NO:353.

In some embodiments, the first Fc polypeptide does not comprise the first three amino acids "PCP" at the amino-terminal end of the sequence (*e.g*., the first Fc polypeptide comprises the sequence without the first three amino acids "PCP" at the amino-terminal end).

In certain embodiments, the first Fc polypeptide comprises the sequence of SEQ ID NO:347 and the second Fc polypeptide comprises the sequence of SEQ ID NO:350. In certain embodiments, the first Fc polypeptide comprises the sequence of SEQ ID NO:348 and the second Fc polypeptide comprises the sequence of SEQ ID NO:349.

In some embodiments, the dimer is bivalent for TfR binding. In some embodiments, the second Fc polypeptide comprises the sequence of SEQ ID NO:347 or 348. In certain embodiments, the first Fc polypeptide comprises the sequence of SEQ ID NO:347 and the second Fc polypeptide comprises the sequence of SEQ ID NO:348. In some embodiments, the first and second Fc polypeptides comprise the same TfR binding site.

In another aspect, the disclosure features an Fc polypeptide dimer-Fab fusion protein comprising:
(a) an antibody variable region that is capable of binding an antigen, or antigen-binding fragment thereof; and
(b) an Fc polypeptide dimer described herein.

In some embodiments, the antibody variable region forms part of a Fab domain. In certain embodiments, the antibody variable region comprises two antibody heavy chain variable regions and two antibody light chain variable regions, or respective fragments thereof.

In another aspect, the disclosure features a polynucleotide comprising a nucleic acid sequence encoding a polypeptide described herein. In some embodiments, a vector comprising the polynucleotide is provided. In some embodiments, a host cell comprising the polynucleotide is provided. In another aspect, the disclosure features a method for producing a polypeptide that specifically binds to a transferrin receptor, comprising culturing a host cell under conditions in which a polypeptide encoded by the polynucleotide is expressed.

In another aspect, the disclosure features a pharmaceutical composition comprising: a polypeptide, Fc polypeptide dimer, and/or Fc polypeptide dimer-Fab fusion protein described herein; and a pharmaceutically acceptable carrier.

In another aspect, the disclosure features a composition comprising a polypeptide, Fc polypeptide dimer, and/or Fc polypeptide dimer-Fab fusion protein described herein for use in a method for transcytosis of a composition across an endothelium, the method comprising contacting the endothelium with said composition.

In some embodiments, the endothelium is the blood-brain barrier (BBB).

### BRIEF DESCRIPTION OF THE DRAWINGS

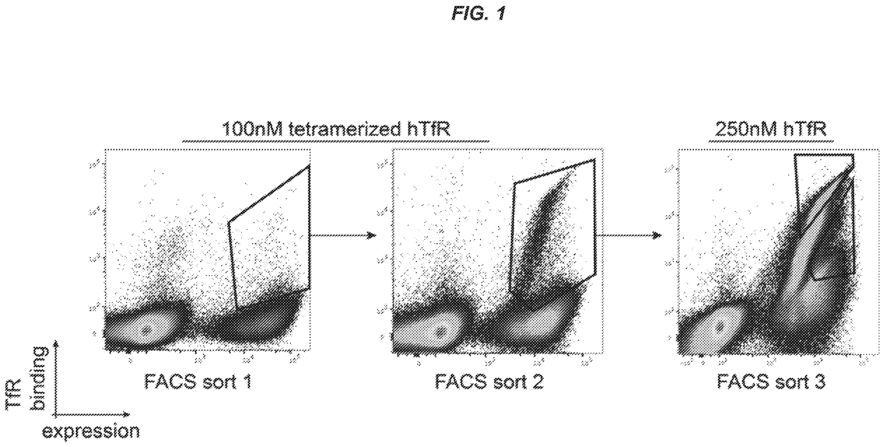

FIG. 1 shows FACS plots for CH3C clone selections on yeast, showing enrichment of binding population after 3 sort rounds. In sort rounds 1 and 2, biotinylated TfR was preloaded on streptavidin-Alexa Fluor^{®} 647 prior to incubating with the yeast. In sort round 3, biotinylated TfR was incubated with the yeast first, and streptavidin-Alexa Fluor^{®} 647 was added for secondary detection. In all sort rounds, expression was monitored using a chicken anti-c-Myc antibody (obtained from Thermo Fisher) against the C-terminal Myc tag on the yeast display construct.

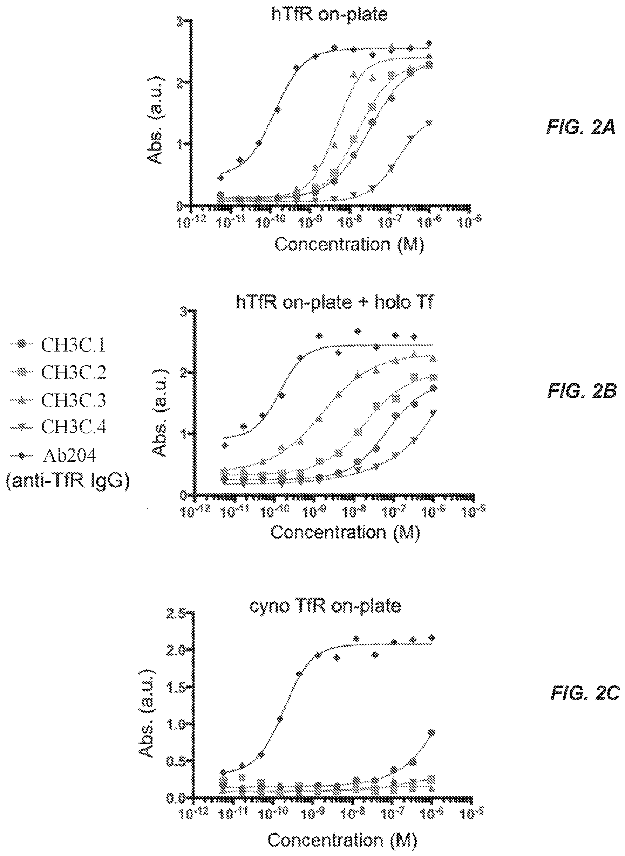

FIGS. 2A-2C show binding of CH3C clones to TfR in the presence or absence of holo-Tf. Clones were assayed in a Fc-Fab fusion format. Ab204, a standard antibody with variable regions that bind to TfR, was used as a positive control in this assay. FIG. 2A shows binding of CH3C variants to human TfR coated on ELISA plates. FIG. 2B shows binding of CH3C variants to human TfR coated on ELISA plates in the presence of 5 µM holo-Tf. FIG. 2C shows binding of CH3C variants to cyno TfR coated on ELISA plates.

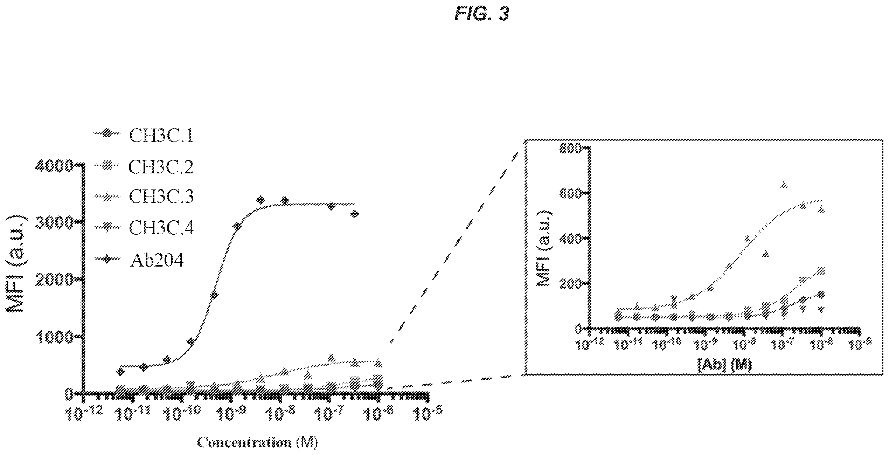

FIG. 3 shows binding of CH3C clones to 293F cells, which endogenously express human TfR. Cells were distributed in 96-well V bottom plates, and varying concentrations of the CH3C clones, formatted as Fc-Fab fusion binding proteins, were added. After 1 hour incubation at 4 °C, the plates were spun and washed, and then incubated with goat-anti-human-IgG-Alexa Fluor^{®} 647 secondary antibody at 4 °C for 30 minutes. After additional washing of the cells, the plates were read on a FACSCanto^{™} II flow cytometer, and median fluorescence values in the APC (647 nm) channel were determined using FlowJo^{®} software.

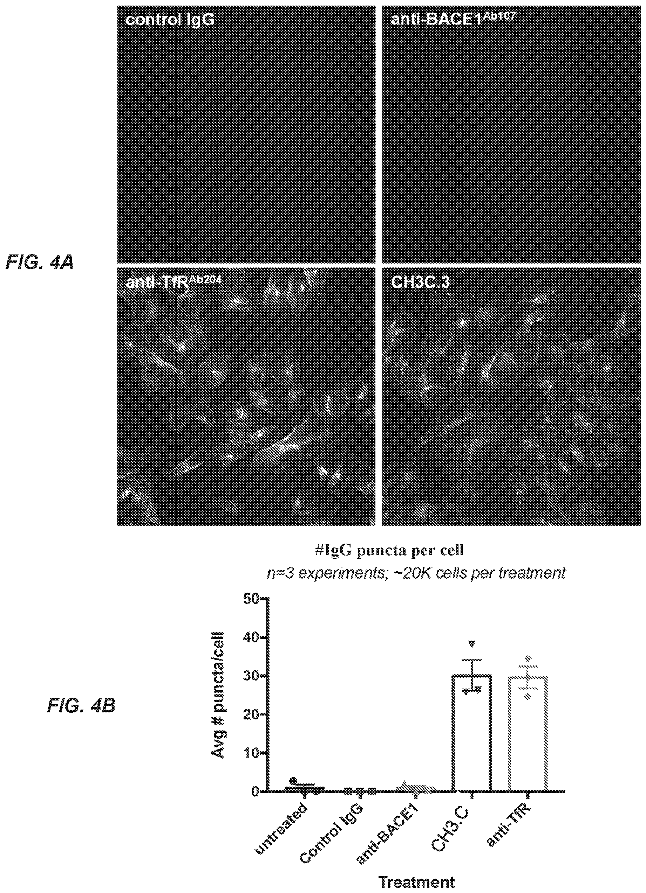

FIGS. 4A and 4B show internalization of CH3C.3 in HEK293 cells, which endogenously express human TfR. CH3C.3 or controls were added at 1 µM concentration at 37 °C and 8% CO₂ concentration for 30 minutes, then the cells were washed, permeabilized, and stained with anti-human-IgG-Alexa Fluor^{®} 488 secondary antibody. After additional washing, the cells were imaged by fluorescence microscopy and the number of puncta was quantified. FIG. 4A shows microscopy data. FIG. 4B shows a graph of the number of puncta per well.

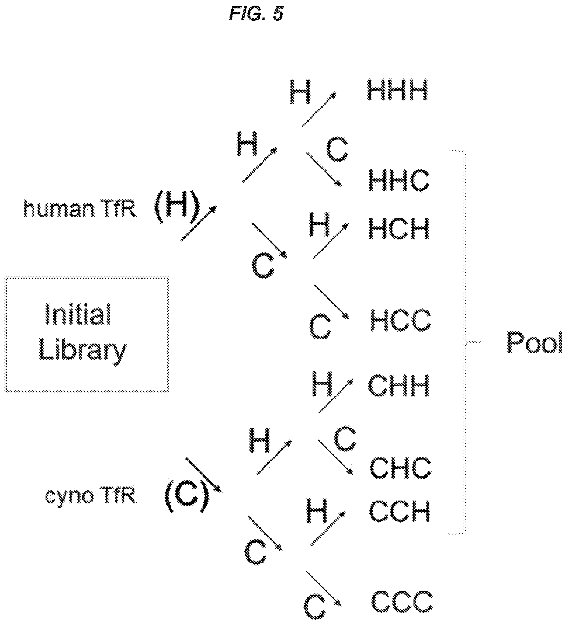

FIG. 5 shows the selection scheme for the CH3C soft library. The initial library was sorted by MACS against either human (H) or cyno (C) TfR. The resulting yeast pools were then split and each sorted against human or cyno TfR as in the first FACS sort round. The resulting pools were split again for another FACS sort round. Finally, the HHH and CCC pools were kept separate and the other pools which had seen both species of target were finally pooled.

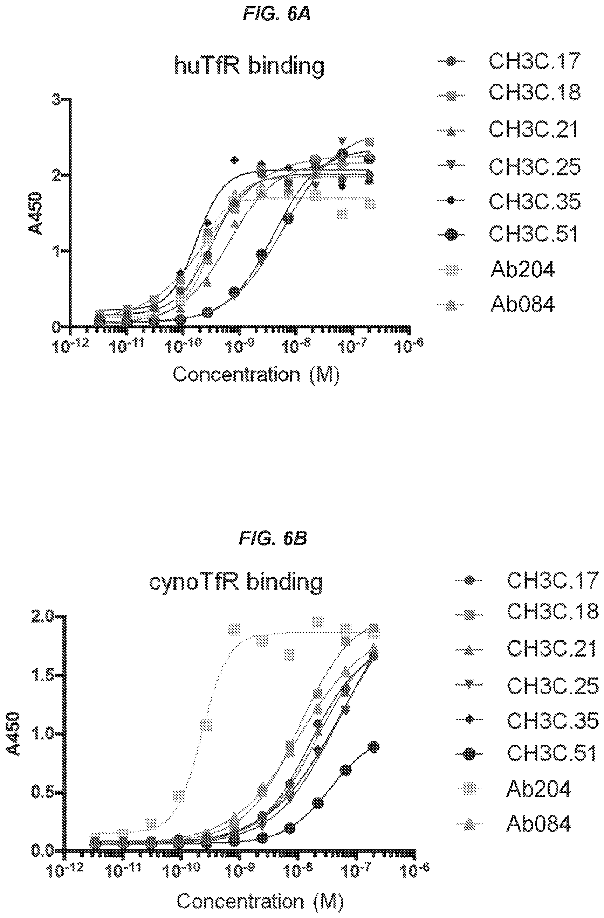

FIGS. 6A and 6B show binding of CH3C clones identified from the first soft randomization library to human and cyno TfR. Positive controls were Ab204, a high affinity anti-TfR antibody, and Ab084, a low-affinity anti-TfR antibody. FIG. 6A shows binding to human TfR. FIG. 6B shows binding to cyno TfR.

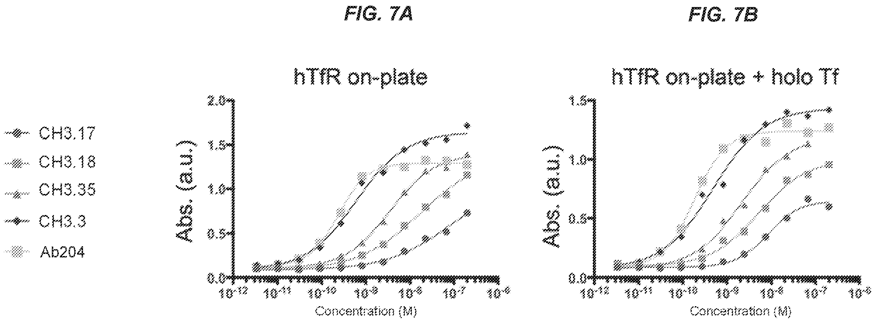

FIGS. 7A and 7B show binding of CH3C clones identified from the first soft randomization library to human TfR in the presence or absence of holo-Tf. Clones were in Fc-Fab fusion format. Ab204, a high affinity anti-TfR antibody, was used as a positive control in this assay. FIG. 7A shows binding of CH3C variants to human TfR coated on ELISA plates. FIG. 7B shows binding of CH3C variants to human TfR coated on ELISA plates in the presence of 5 µM holo-Tf.

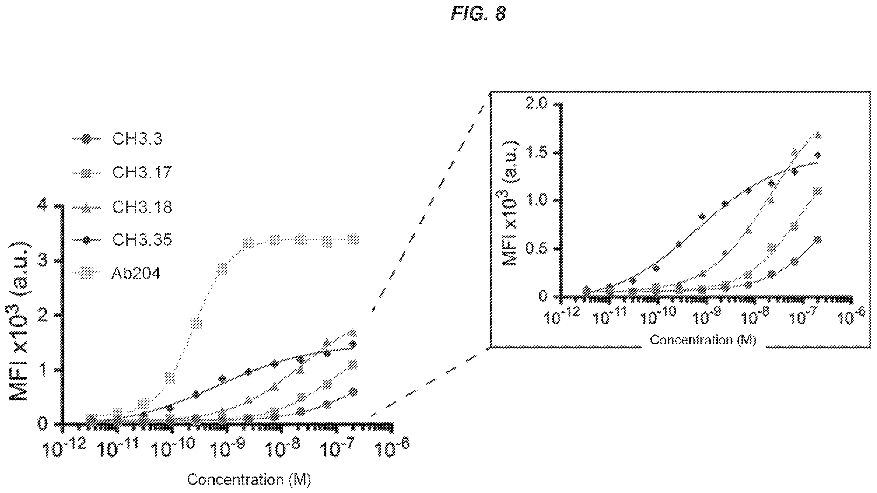

FIG. 8 shows binding of CH3C clones identified from the first soft randomization library to 293F cells. Cells were distributed in 96-well V bottom plates, and varying concentrations of the CH3C clones, formatted as Fc-Fab fusion proteins, were added. After 1 hour incubation at 4 °C, the plates were spun and washed, and then incubated with goat-anti-human-IgG-Alexa Fluor^{®} 647 secondary antibody at 4 °C for 30 minutes. After additional washing of the cells, the plates were read on a FACSCanto^{™} II flow cytometer, and median fluorescence values in the APC (647 nm) channel were determined using FlowJo^{®} software.

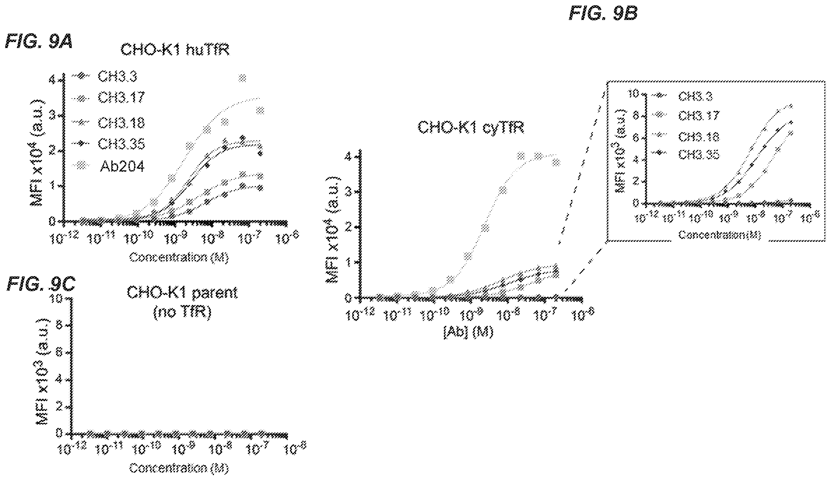

FIGS. 9A-9C show binding of CH3C clones identified from the first soft randomization library to CHO-K1 cells. Cells were distributed in 96-well V bottom plates, and varying concentrations of the CH3C clones, formatted as Fc-Fab fusions, were added. After 1 hour incubation at 4 °C, the plates were spun and washed, and then incubated with goat-anti-human-IgG-Alexa Fluor^{®} 647 secondary antibody at 4 °C for 30 minutes. After additional washing of the cells, the plates were read on a FACSCanto^{™} II flow cytometer, and median fluorescence values in the APC (647 nm) channel were determined using FlowJo^{®} software. FIG. 9A shows CHO-K1 cells that overexpressed human TfR. FIG. 9B shows CHO-K1 cells that overexpressed cyno TfR. FIG. 9C shows CHO-K1 parental cells that did not express human TfR.

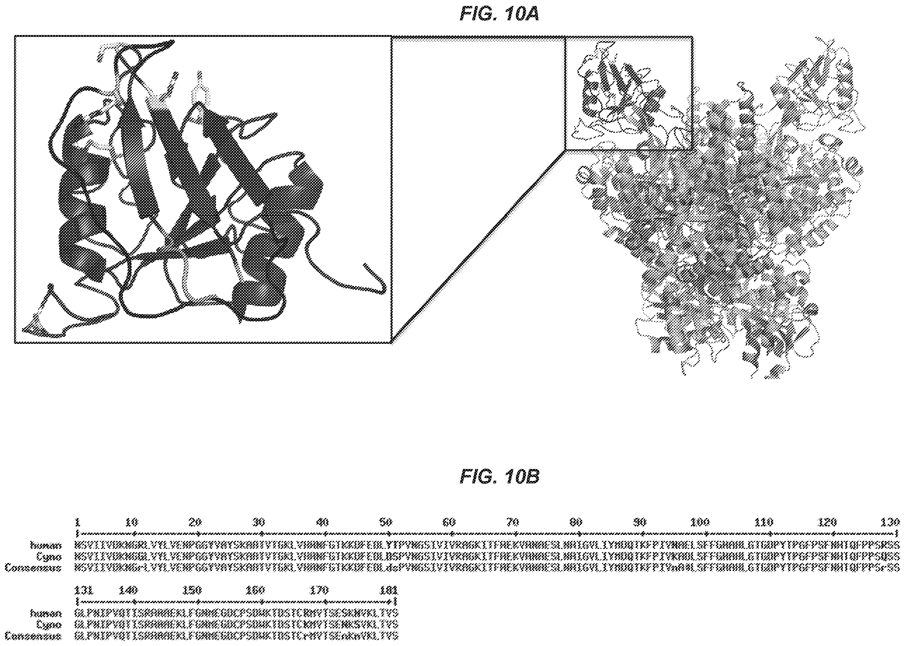

FIGS. 10A and 10B show the TfR apical domain. FIG. 10A shows the location of the apical domain on the human TfR protein. The inset shows a close-up view of the seven residues that differ between human and cyno TfR. FIG. 10B shows a sequence alignment containing the seven residues that differ between human (SEQ ID NO:30) and cyno (SEQ ID NO:31) TfR. The consensus sequence is SEQ ID NO:342.

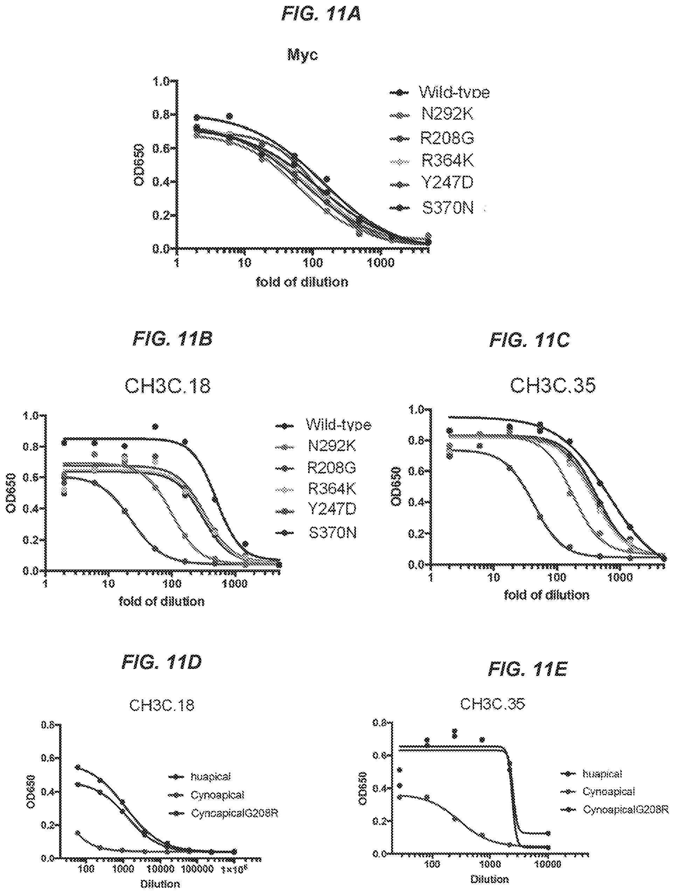

FIGS. 11A-11E show binding of CH3C clones to the apical domain displayed on phage. FIG. 11A shows Myc expression of various TfR apical domain mutants, showing that the expression level of the mutants was similar and normalized. FIG. 11B shows CH3C.18 binding to wild-type and mutant human TfR apical domains, showing reduced binding to the R208G mutant. FIG. 11C shows CH3C.35 binding to wild-type and mutant human TfR apical domains, showing reduced binding to the R208G mutant. FIG. 11D shows CH3C.18 binding to wild-type human and cyno TfR apical domains and the G208R mutant cyno apical domain, showing recovery of binding to the mutant. FIG. 11E shows CH3C.35 binding to wild-type human and cyno TfR apical domains and the G208R mutant cyno apical domain, showing recovery of binding to the mutant.

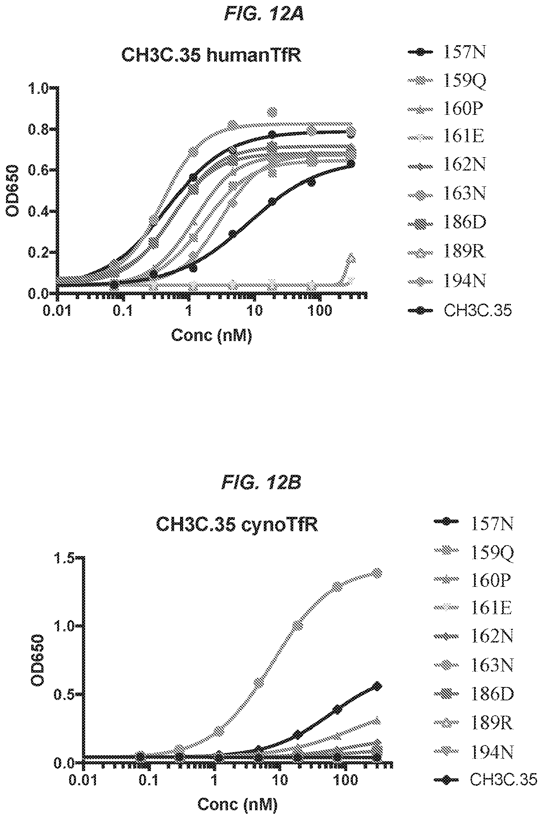

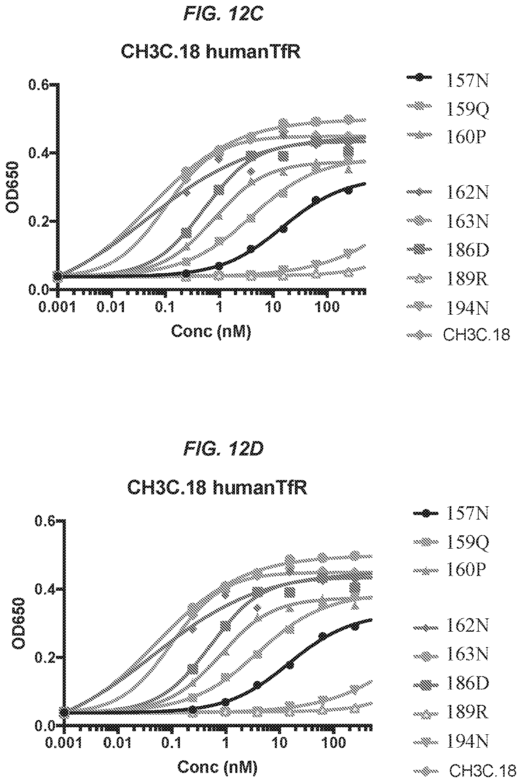

FIGS. 12A-12D show paratope mapping of CH3C variants by reverting mutated positions to wild-type residues. FIG. 12A shows paratope mapping of CH3C.35 by ELISA binding to human TfR for reversion mutants. FIG. 12B shows paratope mapping of CH3C.35 by ELISA binding to cyno TfR for reversion mutants. FIG. 12C shows paratope mapping of CH3C.18 by ELISA binding to human TfR for reversion mutants. FIG. 12D shows paratope mapping of CH3C.18 by ELISA binding to cyno TfR for reversion mutants.

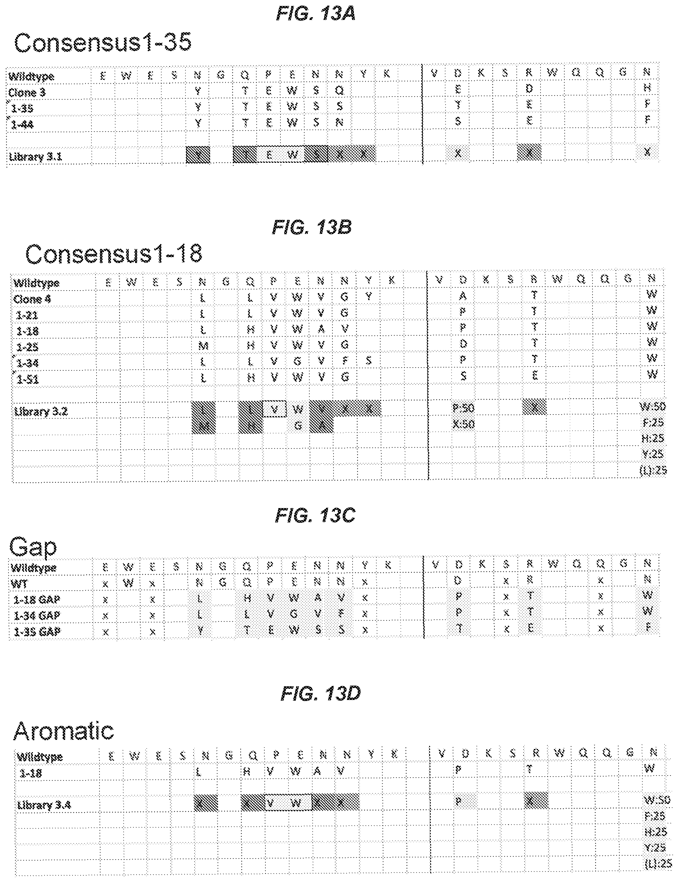

FIGS. 13A-13D show the design of CH3C consensus maturation libraries. FIG. 13A shows the consensus library based on the CH3C.35-like sequences. FIG. 13B shows the consensus library based on the CH3C.18-like sequences. FIG. 13C shows the gap libraries based on CH3C.18 and CH3C.35. FIG. 13D shows the aromatics library based on CH3C. 18.

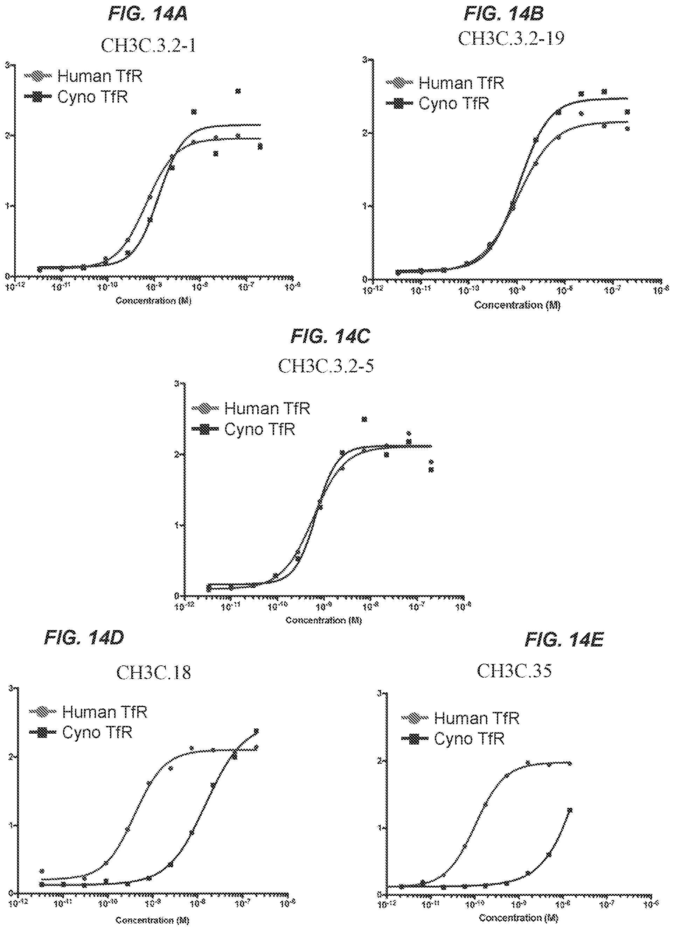

FIGS. 14A-14E show binding ELISAs of CH3C variants from consensus maturation libraries to human or cyno TfR. The new variants (*i.e.,* CH3C.3.2-1, CH3C.3.2-5, and CH3C.3.2-19) had similar binding EC₅₀ values to cyno and human TfR, whereas the parental clones CH3C.18 and CH3C.35 had significantly better EC₅₀ values for human versus cyno TfR. FIG. 14A shows data for CH3C.3.2-1. FIG. 14B shows data for CH3C.3.2-19. FIG. 14C shows data for CH3C.3.2-5. FIG. 14D shows data for CH3C.18. FIG. 14E shows data for CH3C.35.

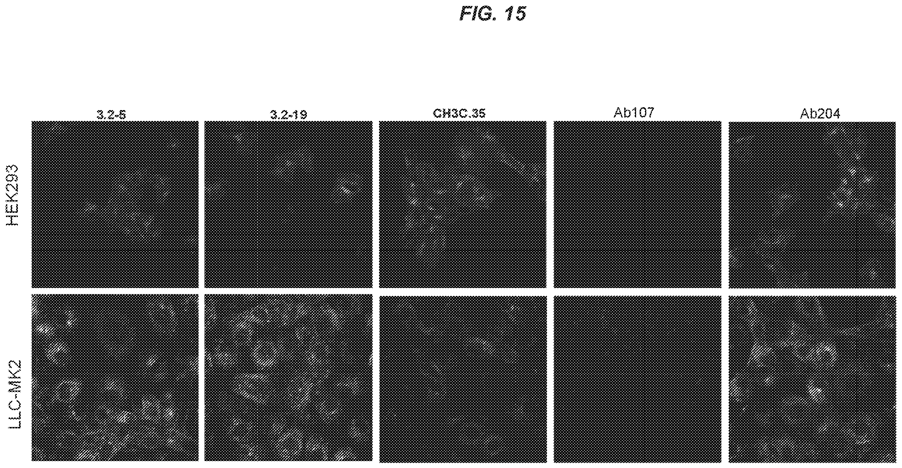

FIG. 15 shows internalization of CH3C variants from consensus maturation libraries in human (HEK293) and monkey (LLC-MK2) cells. Clones CH3C.3.2-5 and CH3C3.2-19, which had similar human and cyno TfR affinities, had significantly improved uptake in monkey cells as compared to clone CH3C.35, which bound better to human TfR. Ab107, an anti-BACE1 antibody, was used as a negative control. (BACE1 is not expressed on HEK293 or MK2 cells). Ab204, an anti-TfR antibody, was used as a positive control.

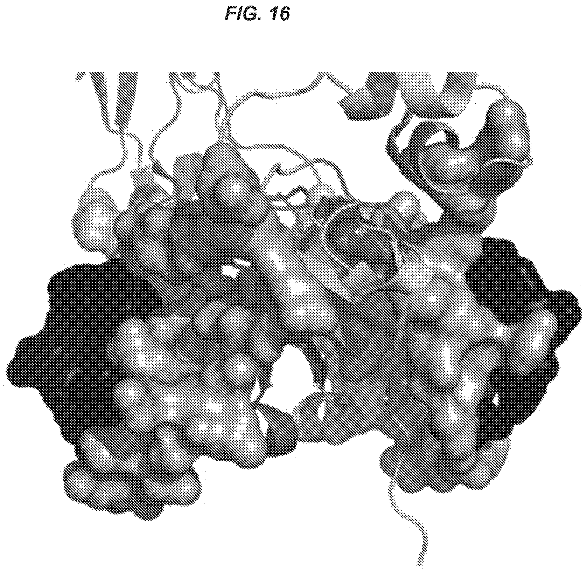

FIG. 16 shows a map of NNK walk residues depicted on the CH3 structure (adapted from PDB 4W4O). Black surfaces show the original CH3C register, grey surfaces show the 44 residues incorporated into the NNK walk structure, and ribbons show the wild-type backbone.

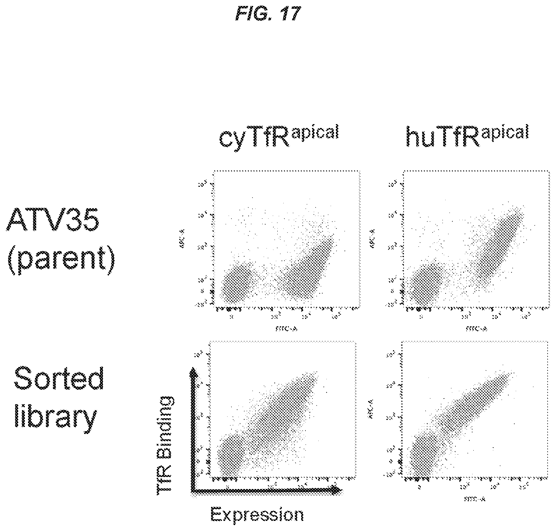

FIG. 17 shows enriched yeast populations after three rounds of sorting the NNK walk library. Yeast were stained with anti- c-Myc to monitor expression (x-axis) and binding to the TfR apical domain (200 nM cyno or 200 nM human) (y-axis). The data presented here clearly show enhanced binding to both TfR apical domain orthologs.

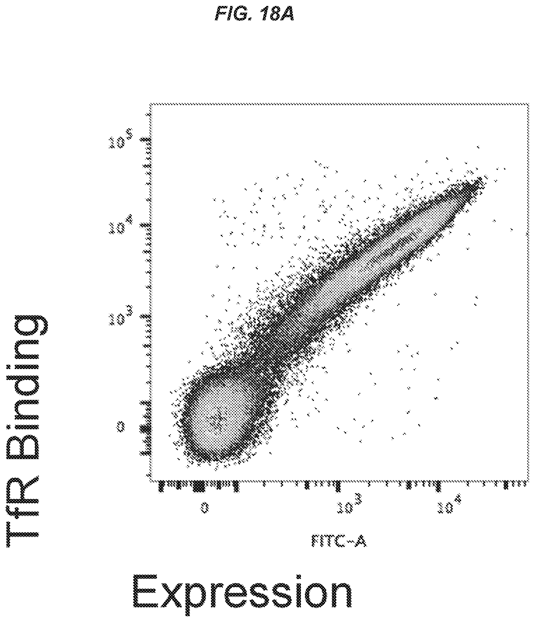

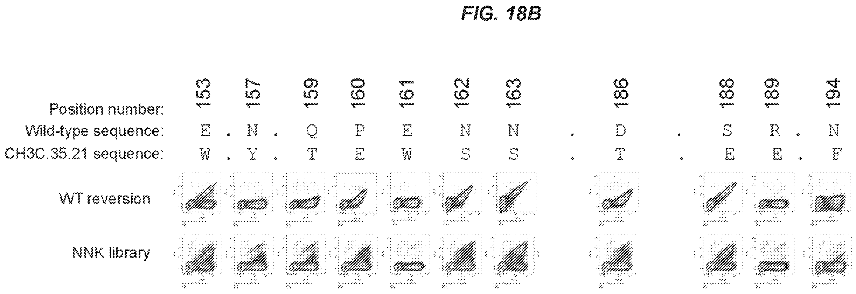

FIGS. 18A and 18B show FACS data for CH3C.35.21 mutants. Yeast were stained with anti-c-Myc to monitor expression (x-axis) and binding to the human TfR apical domain (200 nM) (y-axis). FIG. 18A shows FACS data for clone CH3C.35.21. FIG. 18B shows FACS data for mutants wherein the 11 positions from clone CH3C.35.21 were mutated back to the wild-type (top row of FACS plots) or expressed as an NNK library of all 20 amino acids (bottom row of FACS plots, prior to any sorting).

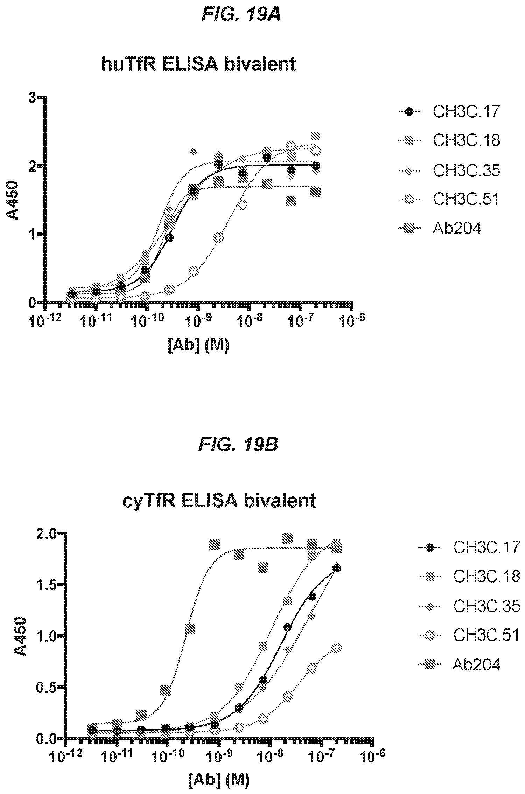

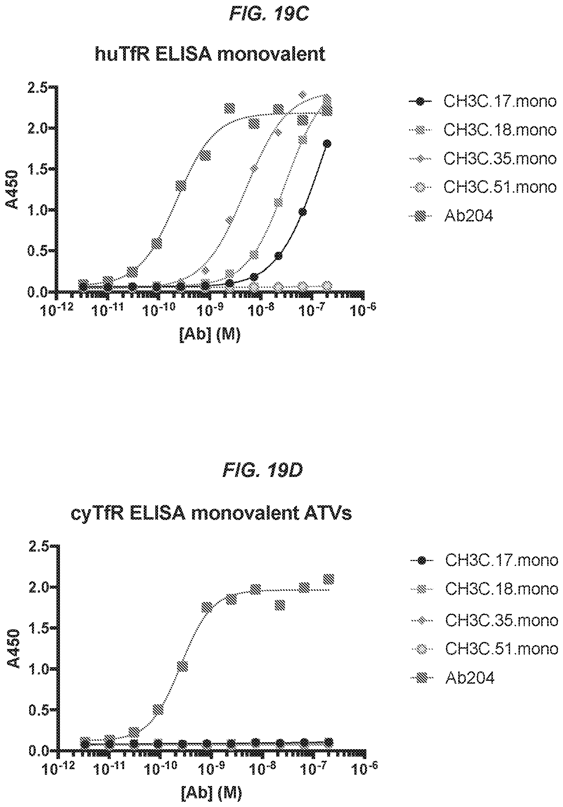

FIGS. 19A-19D show ELISA comparisons of bivalent and monovalent CH3C polypeptide binding to human and cyno TfR. FIG. 19A shows bivalent CH3C polypeptides binding to human TfR. FIG. 19B shows bivalent CH3C polypeptides binding to cyno TfR. FIG. 19C shows monovalent CH3C polypeptides binding to human TfR. FIG. 19D shows monovalent CH3C polypeptides binding to cyno TfR.

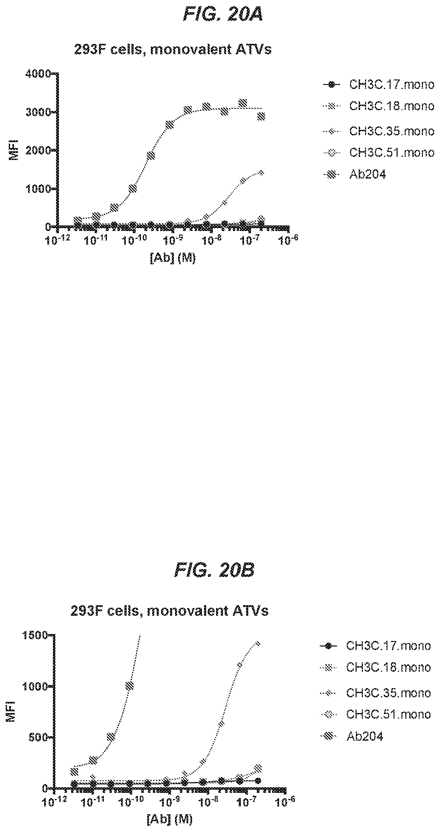

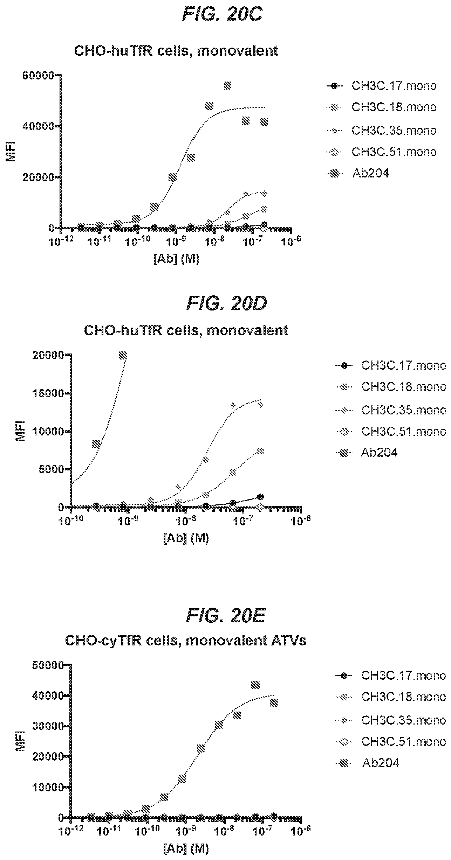

FIGS. 20A-20E show cell binding of monovalent CH3C polypeptides. FIG. 20A shows 293F cells. FIG. 20B shows a zoom-in of the binding to 293F cells depicted in FIG. 20A. FIG. 20C shows CHO-K1 cells stably transfected with human TfR. FIG. 20D shows a zoom-in of the binding to CHO-K1 cells stably transfected with human TfR depicted in FIG. 20C. FIG. 20E shows CHO-K1 cells stably transfected with cyno TfR.

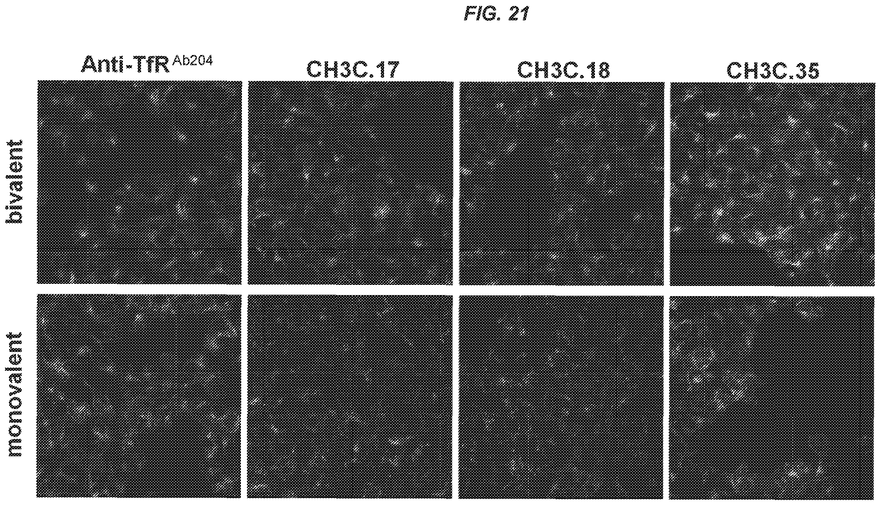

FIG. 21 shows internalization of monovalent and bivalent CH3C polypeptides in HEK293 cells.

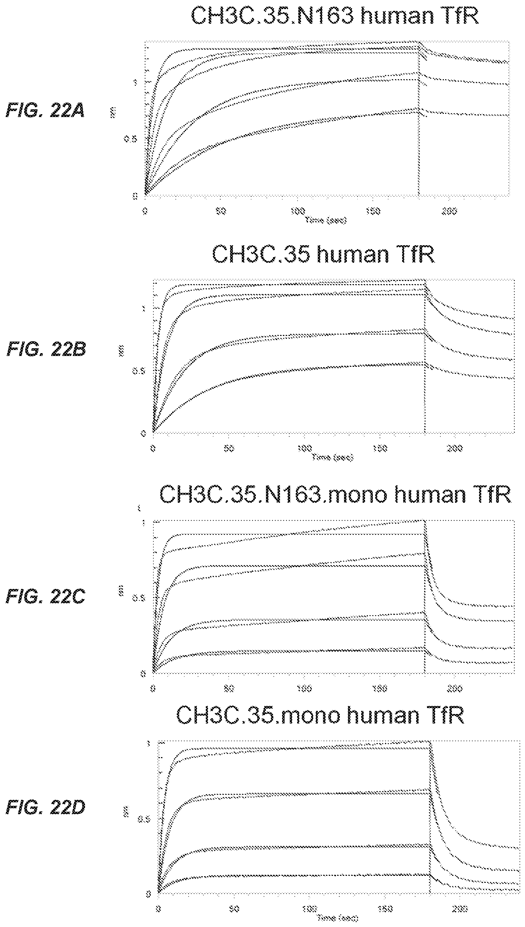

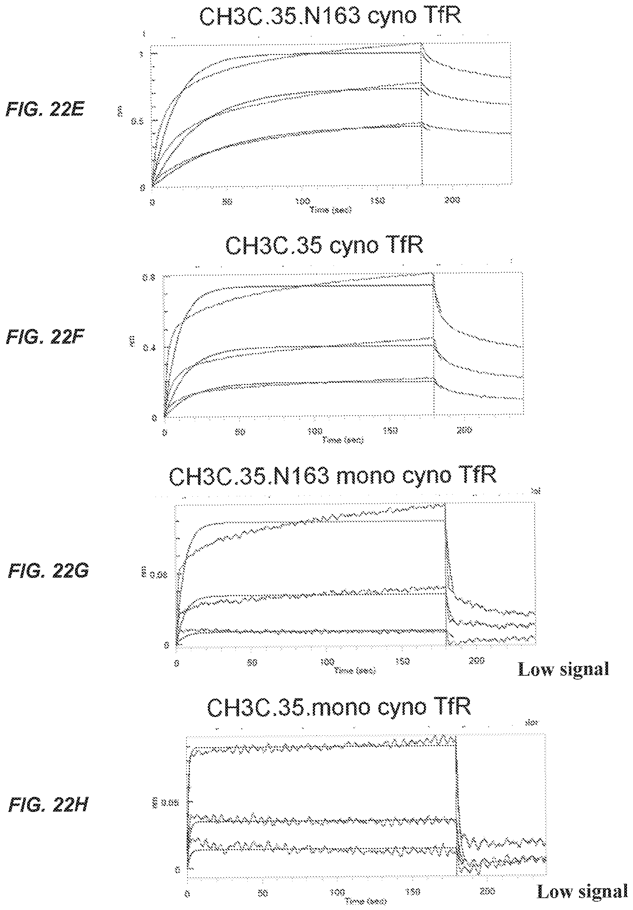

FIGS. 22A-22H show binding kinetics for CH3C polypeptides. FIG. 22A shows data for CH3C.35.N163 binding to human TfR. FIG. 22B shows data for CHC3.35 binding to human TfR. FIG. 22C shows data for CHC3.35.N163 monovalent binding to human TfR. FIG. 22D shows data for CHC3.35 monovalent binding to human TfR. FIG. 22E shows data for CH3C.35.N163 binding to cyno TfR. FIG. 22F shows data for CHC3.35 binding to cyno TfR. FIG. 22G shows data for CHC3.35.N163 monovalent binding to cyno TfR. FIG. 22H shows data for CHC3.35 monovalent binding to cyno TfR.

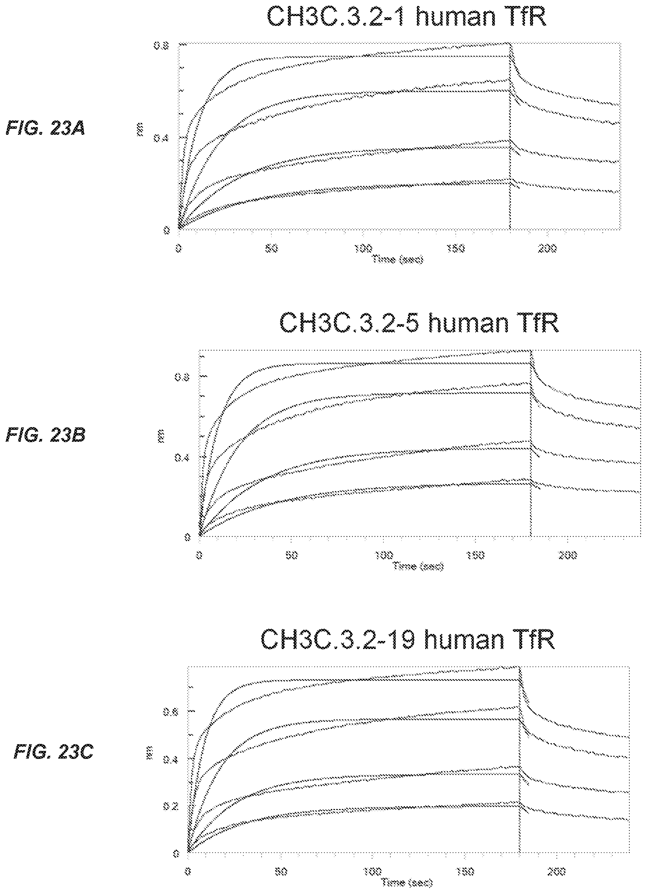

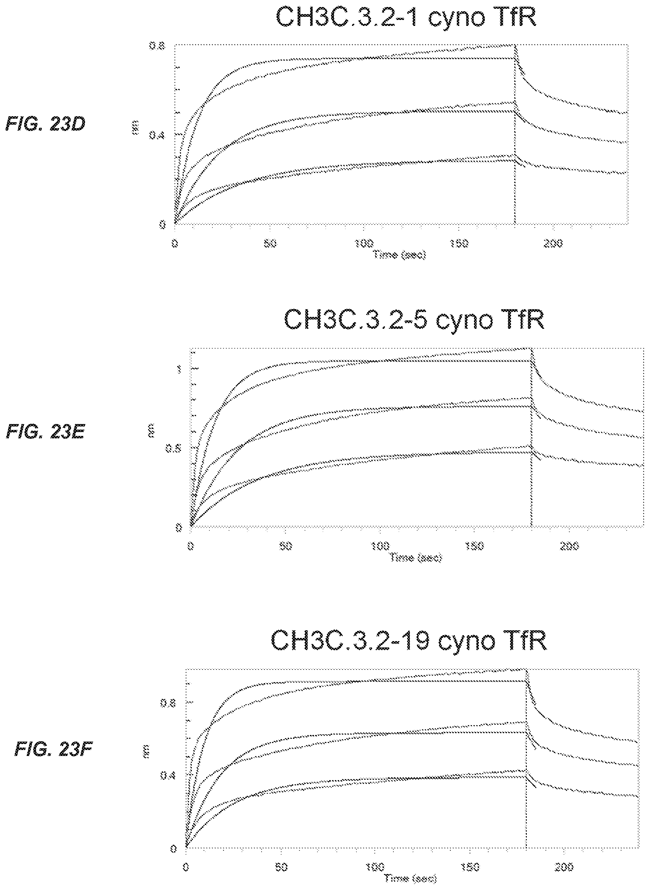

FIGS. 23A-23F show binding kinetics for CH3C polypeptides. FIG. 23A shows data for CH3C.3.2-1 binding to human TfR. FIG. 23B shows data for CH3C.3.2-5 binding to human TfR. FIG. 23C shows data for CH3C.3.2-19 binding to human TfR. FIG. 23D shows data for CH3C.3.2-1 binding to cyno TfR. FIG. 23E shows data for CH3C.3.2-5 binding to cyno TfR. FIG. 23F shows data for CH3C.3.2-19 binding to cyno TfR.

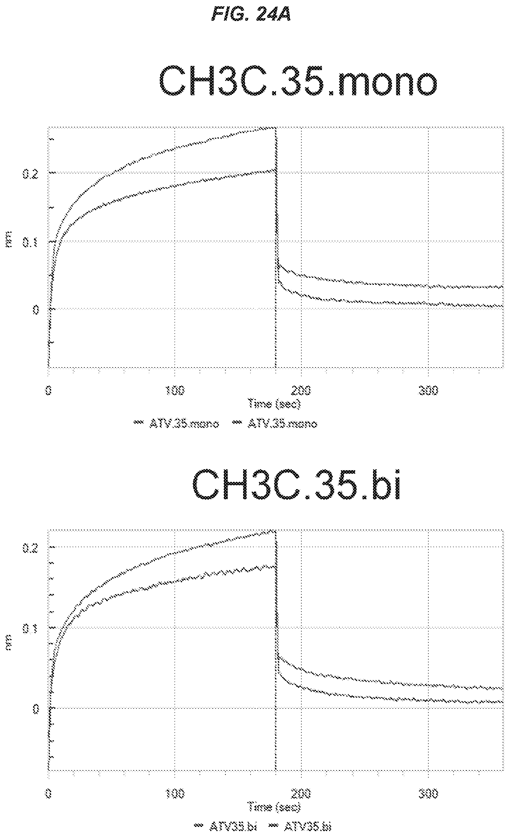

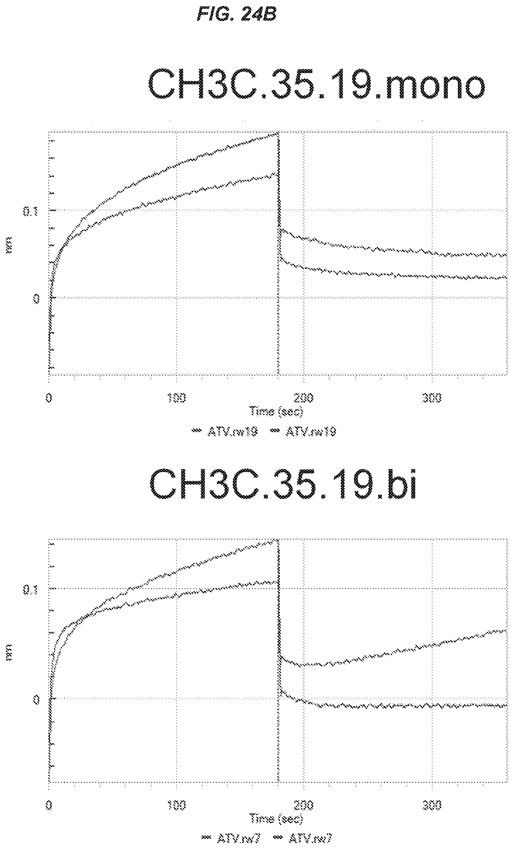

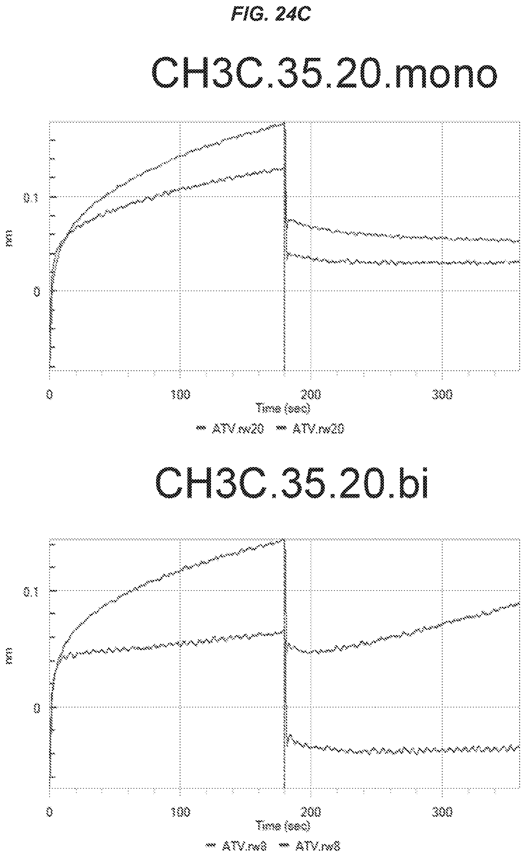

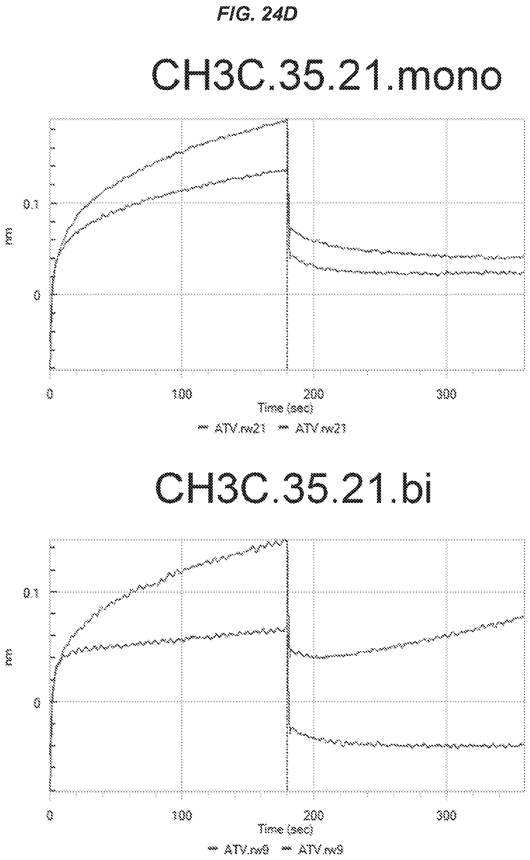

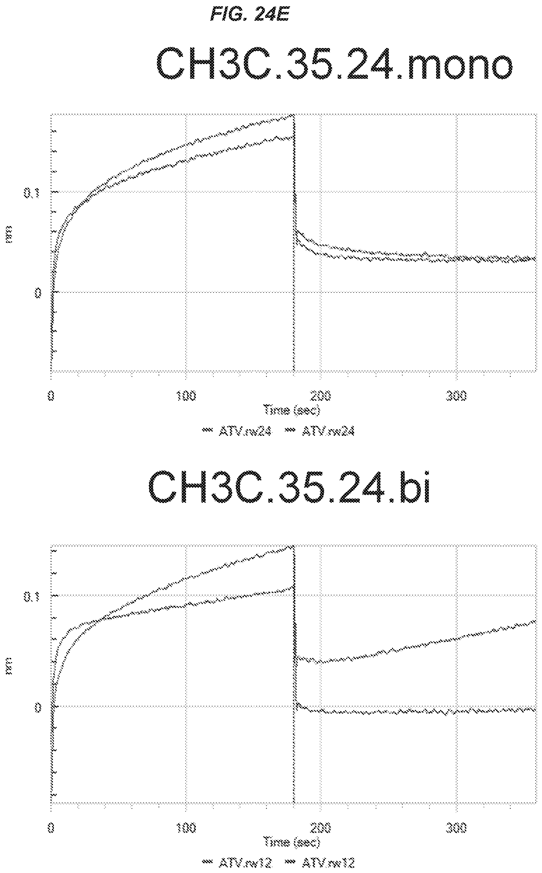

FIGS. 24A-24E show binding of polypeptide-Fab fusions to FcRn at pH 5.5 in the presence (lower traces) or absence (upper traces) of the human TfR extracellular domain. FIG. 24A shows data for clone CH3C.35. FIG. 24B shows data for clone CH3C.35.19. FIG. 24C shows data for clone CH3C.35.20. FIG. 24D shows data for clone CH3C.35.21. FIG. 24E shows data for clone CH3C.35.24.

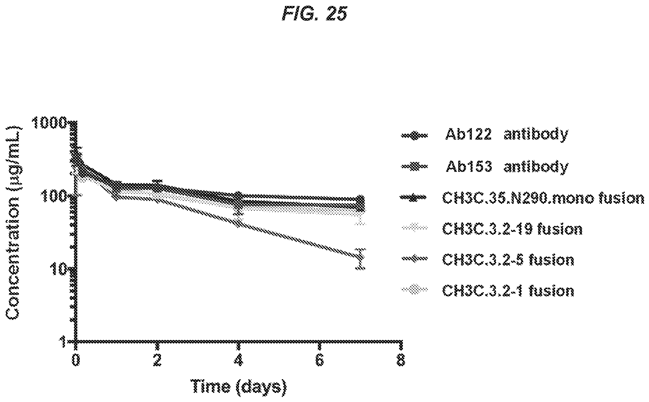

FIG. 25 shows pharmacokinetic (PK) analysis for CH3C polypeptides in wild-type mice. All polypeptide-Fab fusions had comparable clearance to wild-type Fc-Fab fusions (*i.e.,* Ab122, an anti-RSV antibody, and Ab153, an anti-BACE1 antibody) except CH3C.3.2-5, which had faster clearance.

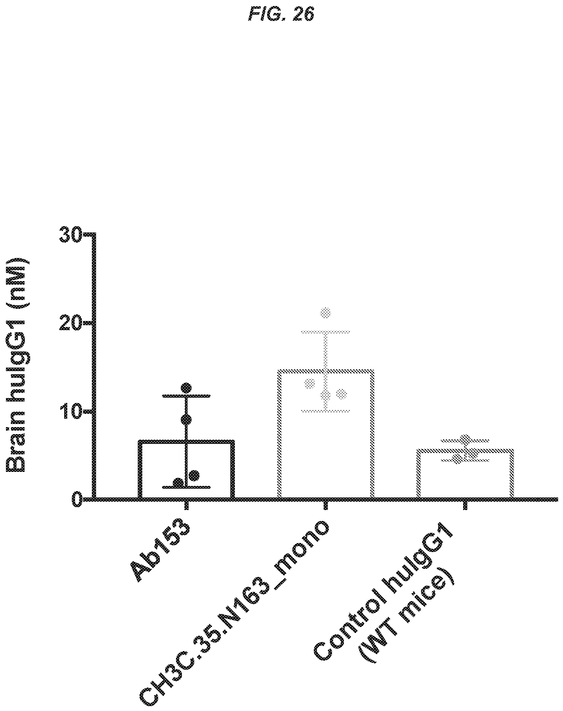

FIG. 26 shows brain pharmacokinetic/pharmacodynamic (PK/PD) data in mouse brain tissue. Chimeric huTfR heterozygous mice (n=4/group) were intravenously dosed with 42 mg/kg of either Ab153 or monovalent CH3C.35.N163 (labeled "CH3C.35.N163_mono"), and wild-type mice (n=3) were dosed intravenously with 50 mg/kg of control human IgG1 (labeled "huIgG1"). Bar graphs represent mean +/- SD.

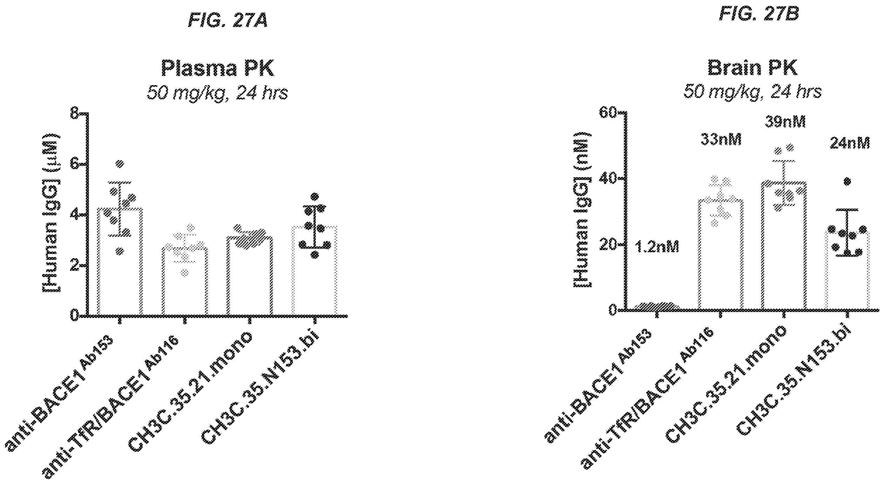

FIGS. 27A and 27B show the concentration of IgG found in hTfR^{apical+/+} mice 24 hours after treatment with polypeptides at 50 mg/kg. FIG. 27A shows the concentration of IgG in plasma. FIG. 27B shows the concentration of IgG in brain tissue.

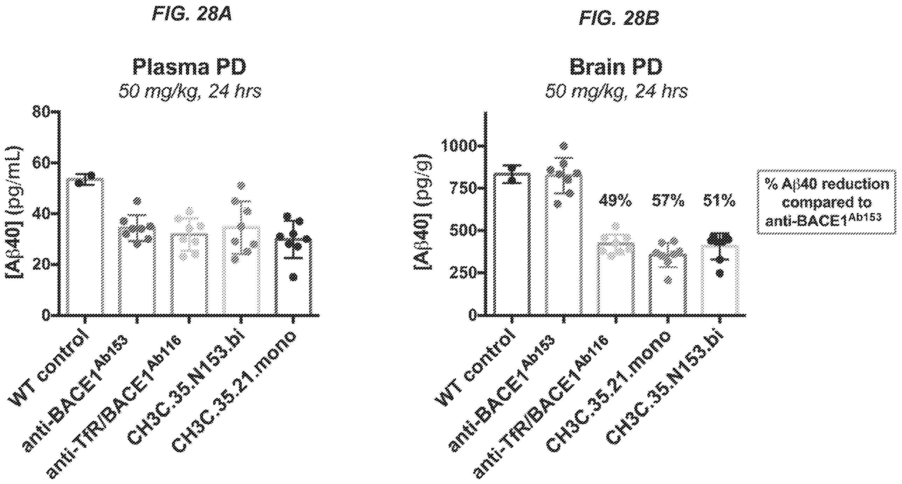

FIGS. 28A and 28B show target engagement of polypeptides dosed in hTfR^{apical+/+} mice after 24 hours, as measured by reductions in amyloid beta-protein 40 (Abeta 40). FIG. 28A shows Abeta 40 concentrations in plasma. FIG. 28B shows Abeta 40 concentrations in brain tissue.

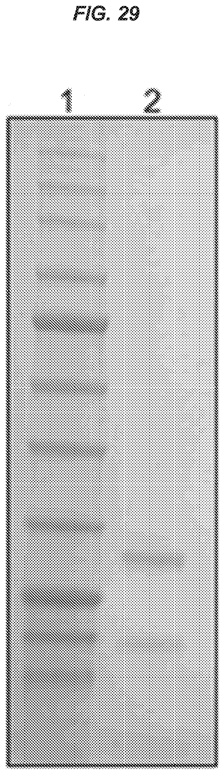

FIG. 29 shows an SDS-PAGE gel of the sizing fraction of the CH3C.18 Fc and the TfR apical domain (AD) complex. Lane 1: Molecular weight marker. Lane 2: Reduced CH3C.18 Fc-AD complex after size-exclusion chromatography.

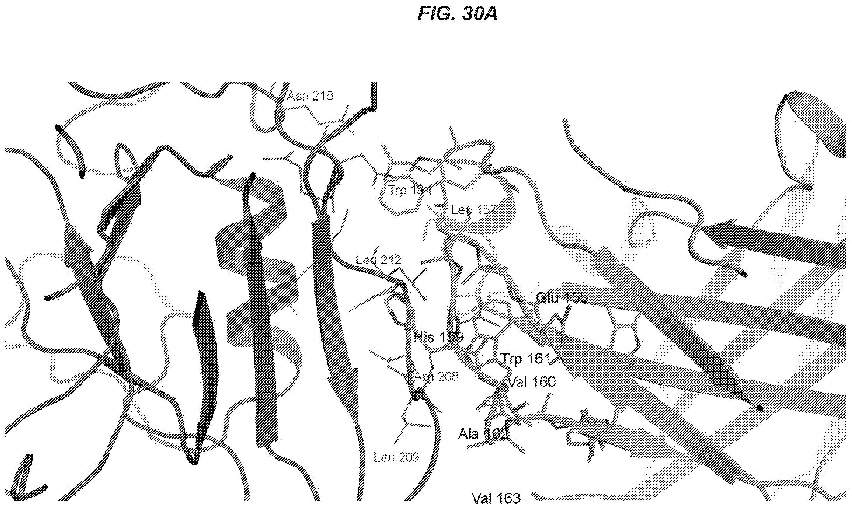

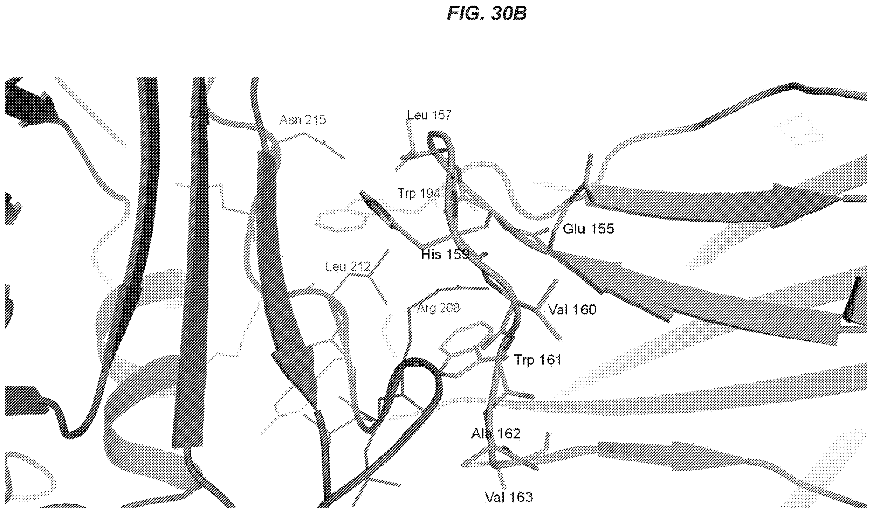

FIGS. 30A and 30B depict binding between polypeptides of the present invention and the transferrin receptor. FIG. 30A depicts the binding interface between clone CH3C.18 and the apical domain of the transferrin receptor. FIG. 30B shows a enlarged view of the binding interface depicted in FIG. 30A.

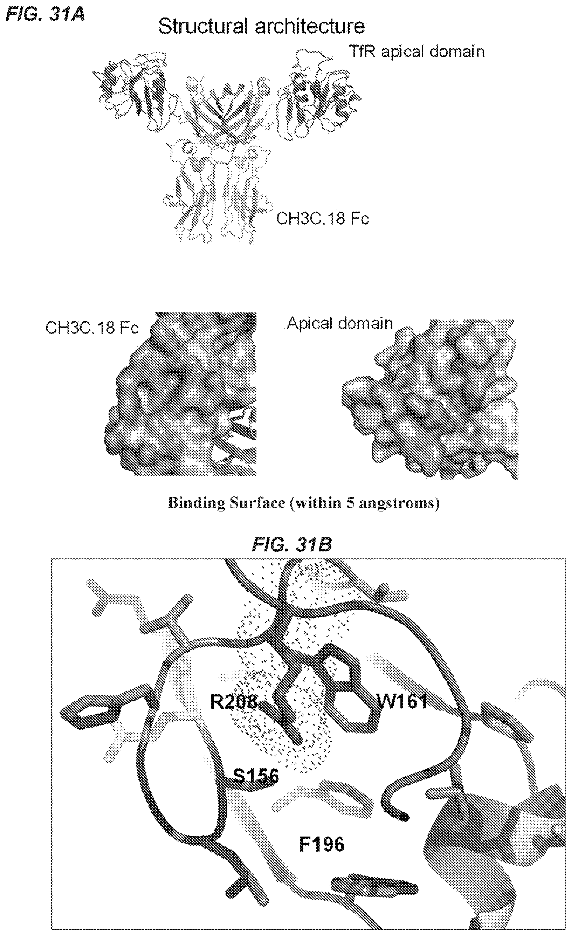

FIGS. 31A and 31B depict interactions between CH3C.18 and the TfR apical domain. FIG. 31A depicts the structural architecture (top) of the TfR apical domain and the CH3C.18 Fc, and the binding surfaces (within 5 angstroms) (bottom) of the TfR apical domain and the CH3C.18 Fc. The co-complex structure was solved at 3.6Å resolution. The structure reveals the epitope on the TfR apical domain bound to CH3C.18. In particular, the N-terminal region of the apical domain is involved in CH3C Fc binding, and the structure is consistent with CH3C.18 Fc and TfR apical domain mutagenesis data. Also, the CH3C.18 library side chains are all contacting the TfR (within 5Å). CH3C.18 library residues: L157, H159, V160, W161, A162, V163, P186, T189, and W194. Non-library residues: F196 and S156. FIG. 31B depicts CH3C.18 Fc and TfR apical domain key interactions. A cation-pi interaction between W161 on the CH3C.18 Fc and R208 on the apical domain is a central binding interaction. Mutation of either CH3C.18 W388 or apical domain R208 disrupts CH3C.18 Fc and apical domain binding. Consistent with this, the R208G mutation from human to cyno explains the reduced cyno affinity. Furthermore, non-conserved residues in the human apical domain (N292 and E294 (K292 and D294 in cyno)) are nearby. Therefore, Q192 in CH3C.18 may be mutated to selectively improve cyno versus human binding.

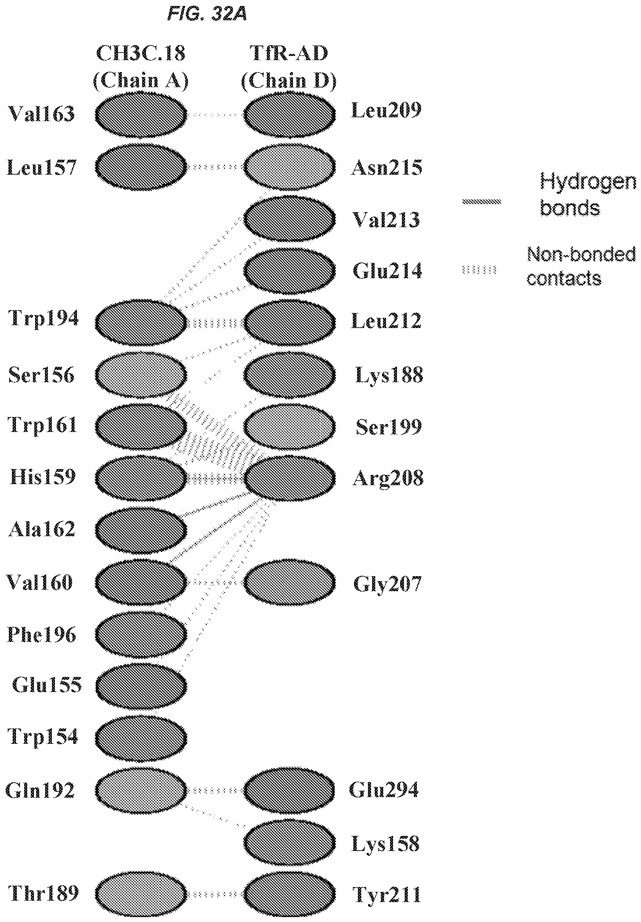

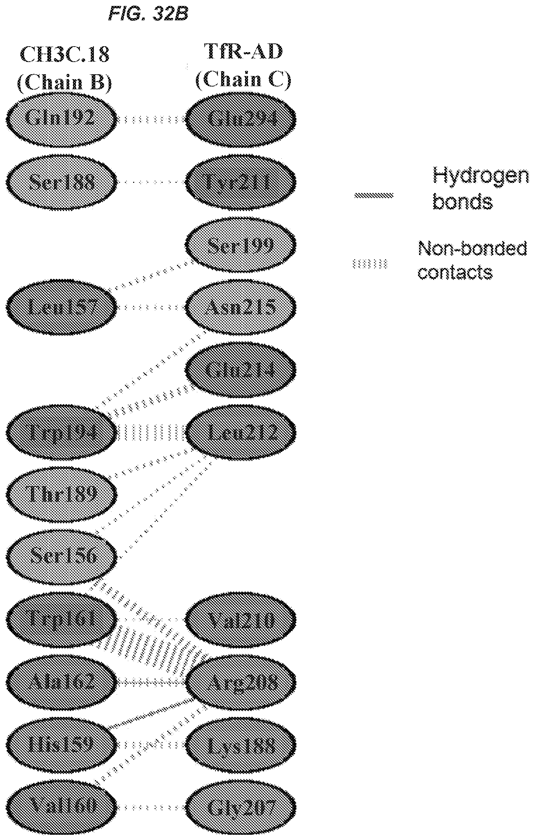

FIGS. 32A and 32B depict binding between polypeptides of the present invention and the transferrin receptor. FIG. 32A depicts hydrogen bonds and non-bonded contacts between residues in clone CH3C.18 (Chain A) and the apical domain of the transferrin receptor (Chain D). FIG. 32B depicts hydrogen bonds and non-bonded contacts between residues in clone CH3C.18 (Chain B) and the apical domain of the transferrin receptor (Chain C).

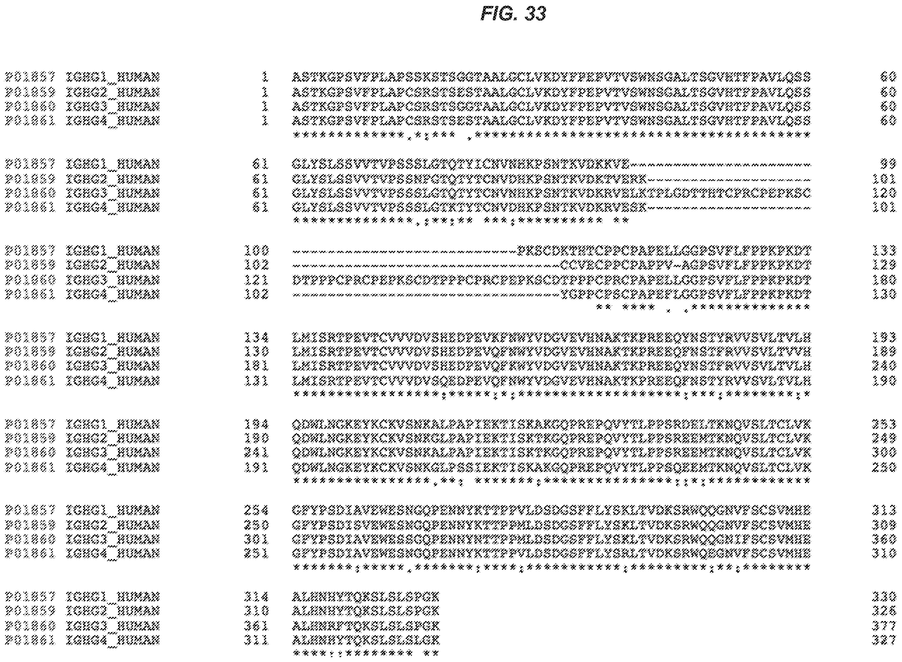

FIG. 33 shows an alignment of human IgG1, IgG2, IgG3, and IgG4 amino acid sequences (SEQ ID NOS:343-346).

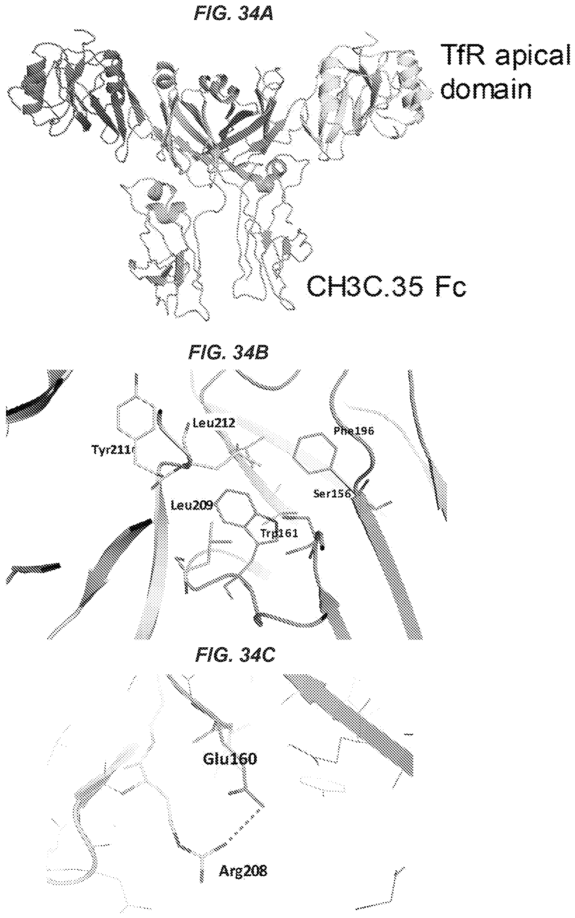

FIGS. 34A-34C depict binding between polypeptides of the present invention and the transferrin receptor. FIG. 34A depicts the structural architecture (top) of the TfR apical domain and the CH3C.35 Fc, and the binding surfaces (within 5 Å) (bottom) of the TfR apical domain and the CH3C.35 Fc. The co-complex structure was solved at 3.4Å resolution. The structure reveals the epitope on the TfR apical domain bound to CH3C.35. The CH3C.35 library side chains are all contacting the TfR (within 5 Å). CH3C.35 library residues: Y157, T159, E160, W161, S162, T186, E189, and W194. Non-library residues: F196, S156, Q192. FIGS. 34B and 34C show enlarged views of the binding interface between clone CH3C.35 and the apical domain of the transferrin receptor depicted in FIG. 34A.

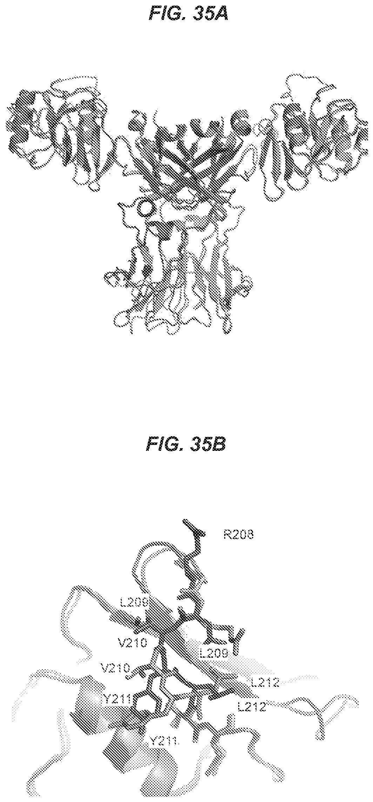

FIG. 35A depicts an overlaid structure between the CH3C.35 Fc and TfR-AD complex and the CH3C.18 Fc and TfR-AD complex.
FIG. 35B depicts an enlarged view of the overlaid structure in FIG. 35A.

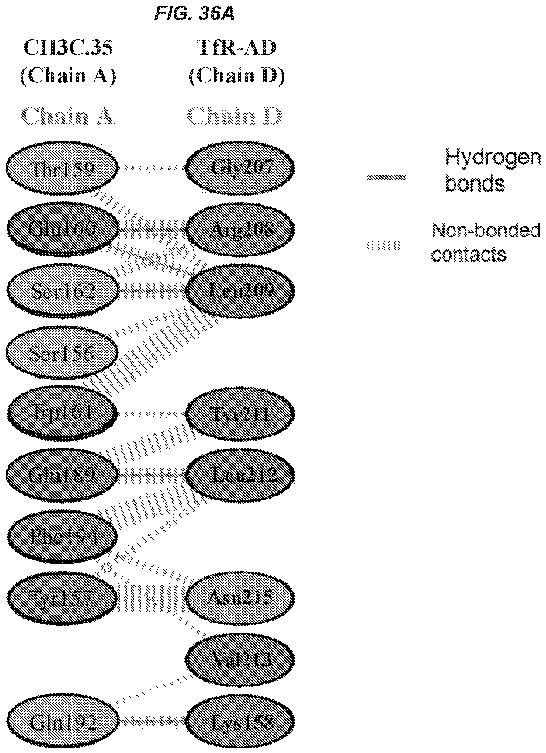

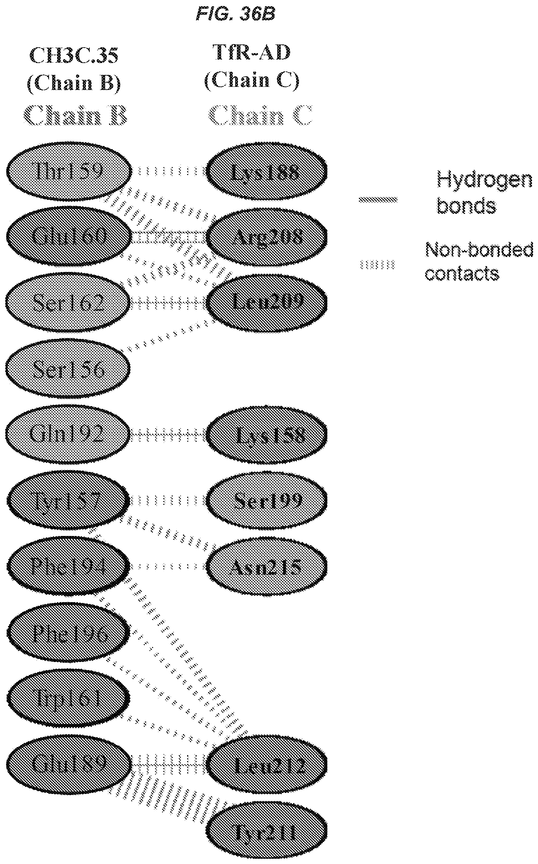

FIGS. 36A and 36B depict binding between polypeptides of the present invention and the transferrin receptor. FIG. 36A depicts hydrogen bonds and non-bonded contacts between residues in clone CH3C.35 (Chain A) and the apical domain of the transferrin receptor (Chain D). FIG. 36B depicts hydrogen bonds and non-bonded contacts between residues in clone CH3C.35 (Chain B) and the apical domain of the transferrin receptor (Chain C).

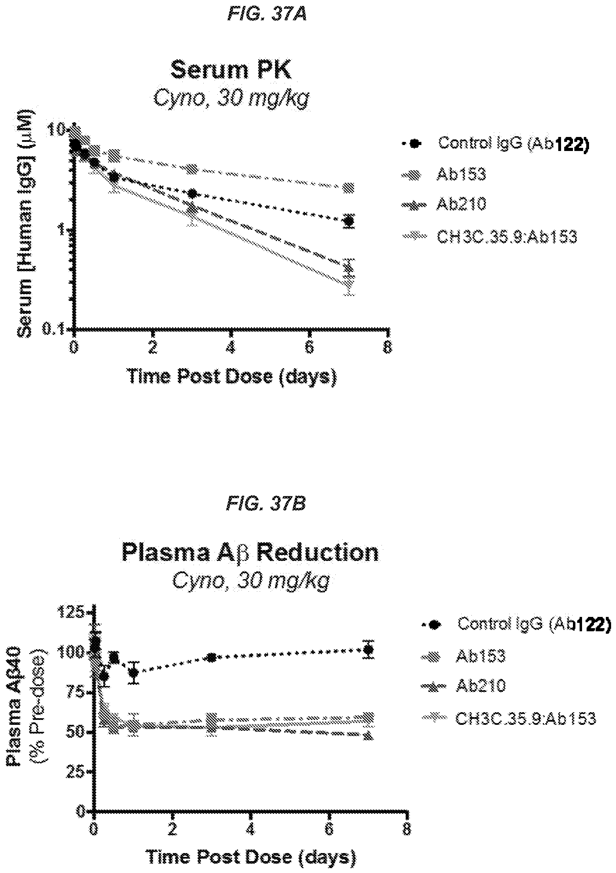

FIGS. 37A and 37B depict plasma PK and Aβ40 reduction for an Fc-Fab fusion polypeptide comprising a CH3C variant fused to the Ab153 Fab domain in cynomolgus monkeys. FIG. 37A shows that Ab210 and CH3C.35.9:Ab153 exhibited faster clearance due to TfR-mediated clearance compared to control IgG (Ab122) and Ab153. FIG. 37B shows that Ab153, Ab210, and CH3C.35.9:Ab153, which all bind to and inhibit BACE1, exhibited significant Aβ40 reduction in plasma.

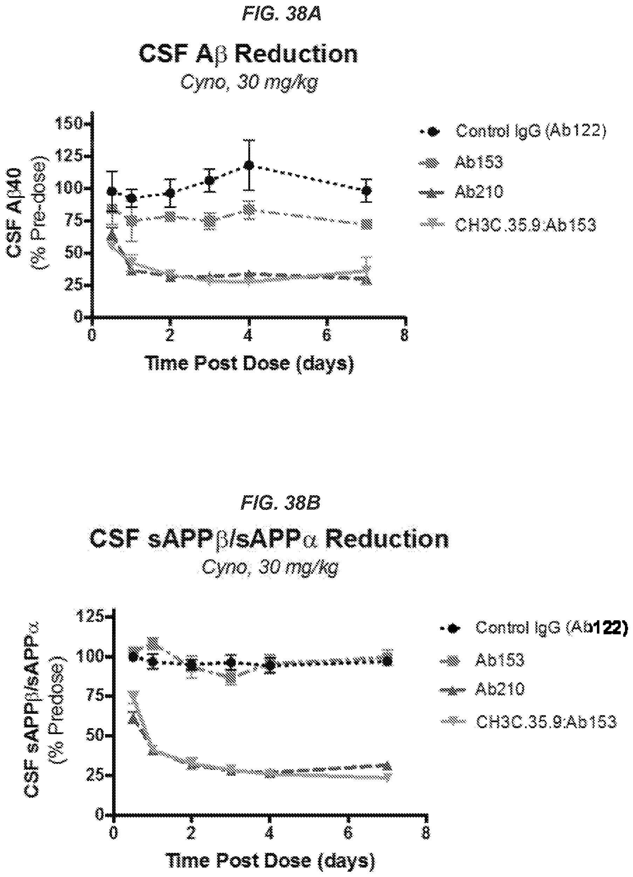

FIGS. 38A and 38B depict significant cerebrospinal fluid (CSF) Aβ and sAPPβ/sAPPα reduction with an Fc-Fab fusion polypeptide comprising a CH3C variant fused to the Ab153 Fab domain in cynomolgus monkeys. FIG. 38A shows that animals dosed with Ab210 and CH3C.35.9:Ab153 showed about 70% reduction in CSF Aβ40 compared to Ab153 and control IgG (Ab122). FIG. 38B shows that animals dosed with Ab210 and CH3C.35.9:Ab153 showed about 75% reduction in sAPPβ/sAPPα ratio compared to Ab153 and control IgG (Ab122). n = 4/group. Line graphs represent mean ± SEM.

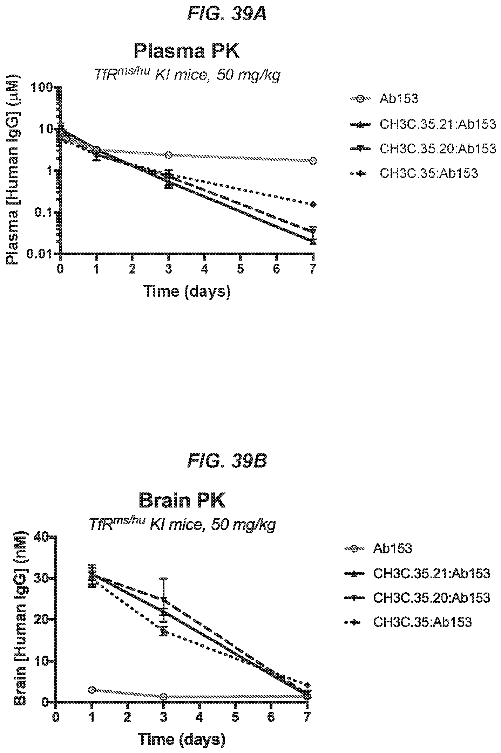

FIGS. 39A and 39B depict hulgG1 concentrations in plasma (FIG. 39A) and brain lysates (FIG. 39B) of hTfRapical^{+/+} knock-in (KI) mice after a single 50 mg/kg systemic injection of anti-BACE1_Ab153, CH3C35.21:Ab153, CH3C35.20:Ab153, or CH3C35:Ab153 polypeptide fusion (mean ± SEM, n=5 per group).

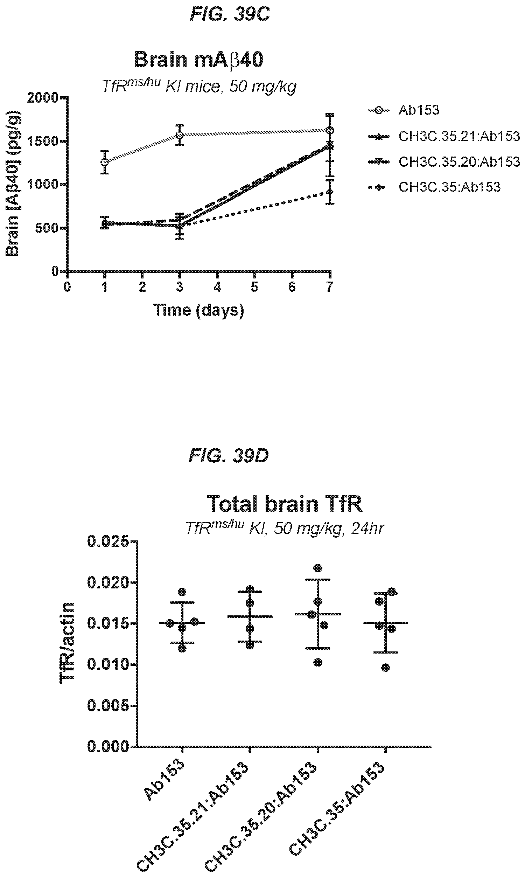

FIG. 39C depicts endogenous mouse Aβ concentration in brain lysate of hTfRapical^{+/+} KI mice after a single 50 mg/kg systemic injection of anti-BACE1_Ab153, CH3C35.21:Ab153, CH3C35.20:Ab153, or CH3C35:Ab153 polypeptide fusion (mean ± SEM, n=5 per group).
FIG. 39D depicts Western blot quantification of brain TfR protein normalized to actin in brain lysate of hTfRapical^{+/+} KI mice after a single 50 mg/kg systemic injection of anti-BACE1_Ab153, CH3C35.21:Ab153, CH3C35.20:Ab153, or CH3C35:Ab153 polypeptide fusion (mean ± SEM, n=5 per group).

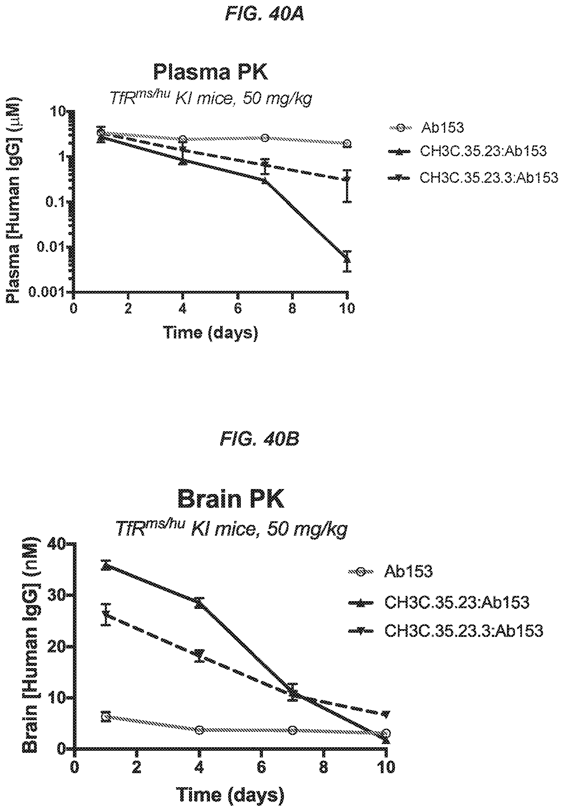

FIGS. 40A and 40B depict huIgG1 concentrations in plasma (FIG. 40A) and brain lysates (FIG. 40B) of hTfR^{apical+/+} KI mice after a single 50 mg/kg systemic injection of anti-BACE1_Ab153, CH3C.35.23:Ab153, or CH3C.35.23.3:Ab153 polypeptide fusion (mean ± SEM, n=5 per group).

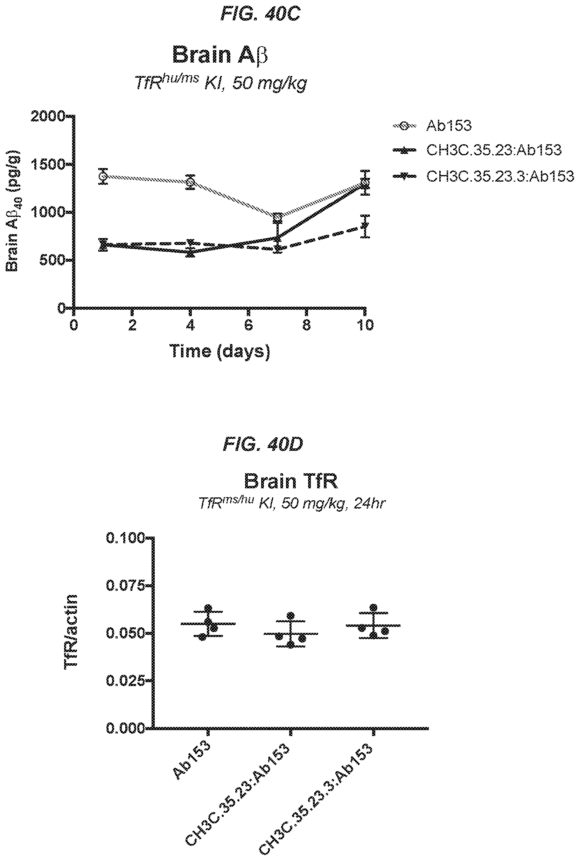

FIG. 40C depicts endogenous mouse Aβ concentration in brain lysate of hTfR^{apical+/+} KI mice after a single 50 mg/kg systemic injection of anti-BACE1_Ab153, CH3C.35.23:Ab153, or CH3C.35.23.3:Ab153 polypeptide fusion (mean ± SEM, n=5 per group).
FIG. 40D depicts Western blot quantification of brain TfR protein normalized to actin in brain lysate of hTfR^{apical+/+} KI mice after a single 50 mg/kg systemic injection of anti-BACE1_Ab153, CH3C.35.23:Ab153, or CH3C.35.23.3:Ab153 polypeptide fusion (mean ± SEM, n=4 per group).

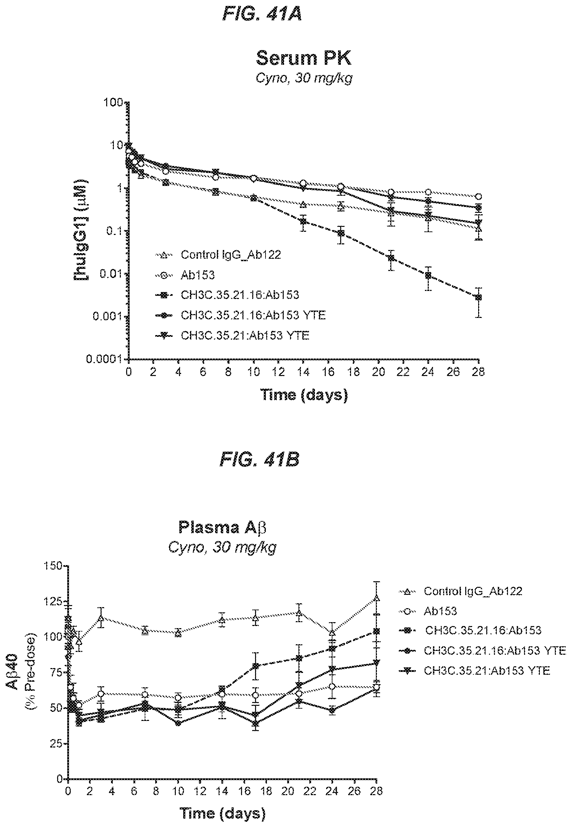

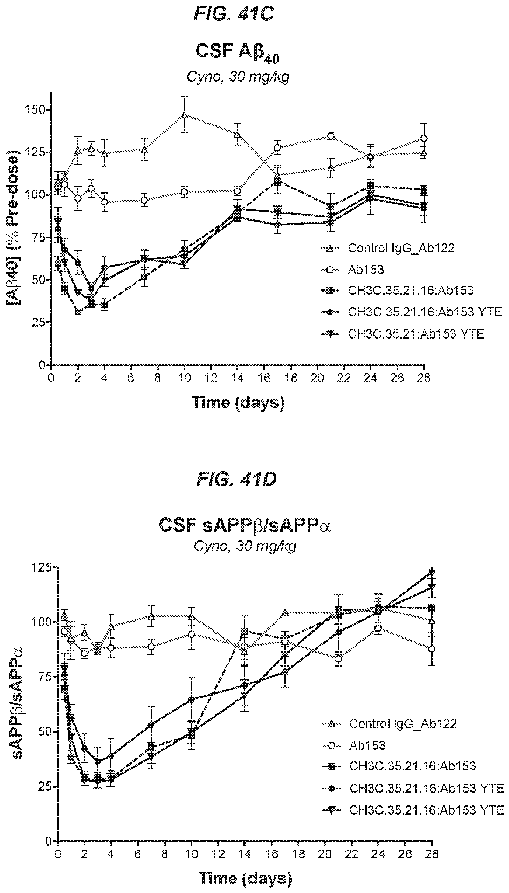

FIGS. 41A-41D depict 28-day PKPD study in cynomolgus monkeys after a single 30 mg/kg dose of the indicated proteins. FIGS. 41A and 41B depict serum huIgG1 in serum and plasma Aβ concentration in plasma, showing peripheral exposure of dosed compounds and resulting effects on plasma Aβ levels over time. FIGS. 41C and 41D depict Aβ and sAPPβ/sAPPα in CSF of cynomolgus monkeys following dosing (mean ± SEM, n=4-5 per group).

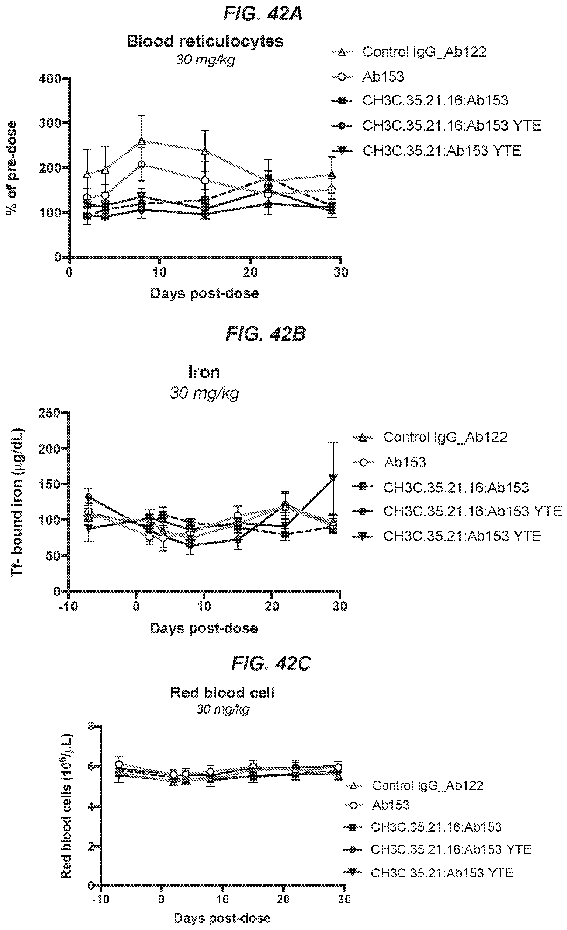

FIGS. 42A-42C depict blood reticulocyte relative to pre-dose levels (FIG. 42A), absolute serum iron levels (FIG. 42B), and absolute red blood cell count (FIG. 42C) in peripheral blood in cynomolgus monkeys after a single 30 mg/kg dose of the indicated proteins (mean ± SEM, n=4-5 per group).

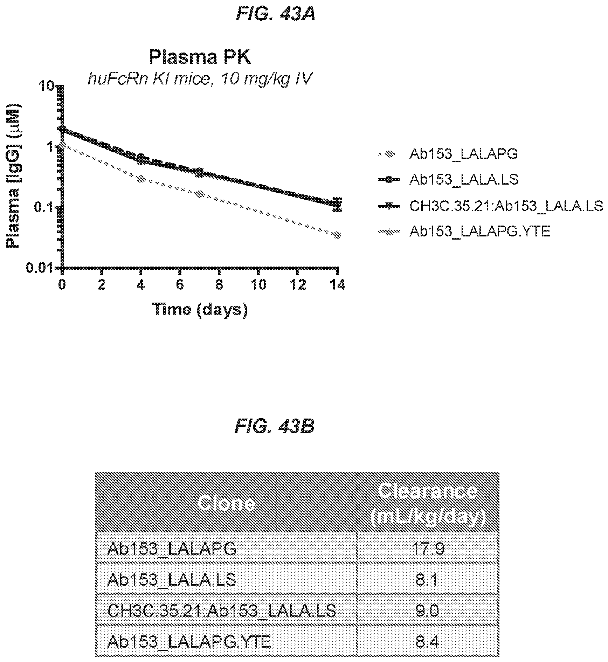

FIGS. 43A and 43B depict peripheral PK analysis (plasma huIgG1 concentrations (FIG. 43A) and clearance values (FIG. 43B)) of indicated proteins in hFcRn knock-in mice after a single 10 mg/kg intravenous injection over 14 days (mean ± SEM, n=3 per group).

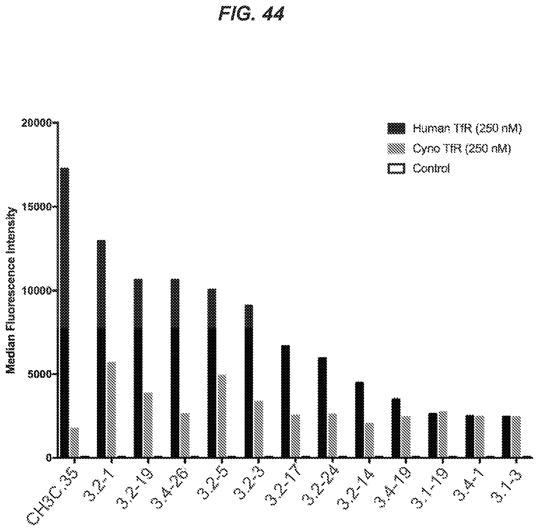

FIG. 44 depicts the median fluorescence intensity of TfR-binding CH3C.18 variants.

### DETAILED DESCRIPTION

### I. INTRODUCTION

Described herein are polypeptides that bind a transferrin receptor (TfR). The invention is based, in part, on the discovery that certain amino acids in an Fc region can be modified to generate a novel binding site specific for TfR in the Fc polypeptide. Taking advantage of the fact that TfR is highly-expressed on the blood-brain barrier (BBB) and that TfR naturally moves transferrin from the blood into the brain, these polypeptides can be used to transport therapeutic agents (*e.g*., therapeutic polypeptides, antibody variable regions such as Fabs, and small molecules) across the BBB. This approach can substantially improve brain uptake of the therapeutic agents and is therefore highly useful for treating disorders and diseases where brain delivery is advantageous.

In one aspect, the invention is based, in part, on the discovery that certain sets of amino acids in a CH3 domain polypeptide can be substituted to generate a polypeptide that binds a transferrin receptor. Thus, in one aspect, provided herein are transferrin receptor-binding polypeptides that have multiple substitutions at a set of amino acids comprising positions 157, 159, 160, 161, 162, 163, 186, 189, and 194, as numbered with reference to SEQ ID NO:1. Anywhere from four to all of the amino acid positions of the set may be substituted. For purposes of this disclosure, a substitution is determined with reference to SEQ ID NO:1. Thus, an amino acid is considered to be a substitution if it differs from the corresponding amino acid in position SEQ ID NO:1 even if the amino acid is present at that position in a naturally occurring CH3 domain polypeptide.

In a further aspect, described herein are treatment methods and methods of using a transferrin receptor-binding polypeptide to target a composition to transferrin receptor-expressing cells, *e.g.,* to deliver the composition to that cell, or to deliver a composition across an endothelium such as the blood-brain barrier.

### II. DEFINITIONS

As used herein, the singular forms "a," "an," and "the" include plural referents unless the content clearly dictates otherwise. Thus, for example, reference to "a polypeptide" may include two or more such molecules, and the like.

As used herein, the terms "about" and "approximately," when used to modify an amount specified in a numeric value or range, indicate that the numeric value as well as reasonable deviations from the value known to the skilled person in the art, for example ± 20%, ± 10%, or ± 5%, are within the intended meaning of the recited value.

A "transferrin receptor" or "TfR" as used in the context of this invention refers to transferrin receptor protein 1. The human transferrin receptor 1 polypeptide sequence is set forth in SEQ ID NO:38. Transferrin receptor protein 1 sequences from other species are also known (*e.g.,* chimpanzee, accession number XP_003310238.1; rhesus monkey, NP_001244232.1; dog, NP_001003111.1; cattle, NP_001193506.1; mouse, NP_035768.1; rat, NP_073203.1; and chicken, NP_990587.1). The term "transferrin receptor" also encompasses allelic variants of exemplary reference sequences, e.g., human sequences, that are encoded by a gene at a transferrin receptor protein 1 chromosomal locus. Full length transferrin receptor protein includes a short N-terminal intracellular region, a transmembrane region, and a large extracellular domain. The extracellular domain is characterized by three domains: a protease-like domain, a helical domain, and an apical domain. The apical domain sequence of human transferrin receptor 1 is set forth in SEQ ID NO:30.

The terms "CH3 domain" and "CH2 domain" as used herein refer to immunoglobulin constant region domain polypeptides. In the context of IgG antibodies, a CH3 domain polypeptide refers to the segment of amino acids from about position 341 to about position 447 as numbered according to the EU numbering scheme, and a CH2 domain polypeptide refers to the segment of amino acids from about position 231 to about position 340 as numbered according to the EU numbering scheme. CH2 and CH3 domain polypeptides may also be numbered by the IMGT (ImMunoGeneTics) numbering scheme in which the CH2 domain numbering is 1-110 and the CH3 domain numbering is 1-107, according to the IMGT Scientific chart numbering (IMGT website). CH2 and CH3 domains are part of the Fc region of an immunoglobulin. In the context of IgG antibodies, an Fc region refers to the segment of amino acids from about position 231 to about position 447 as numbered according to the EU numbering scheme. As used herein, the term "Fc region" may also include at least a part of a hinge region of an antibody. An illustrative hinge region sequence is set forth in SEQ ID NO:37.

The terms "wild-type," "native," and "naturally occurring" with respect to a CH3 or CH2 domain are used herein to refer to a domain that has a sequence that occurs in nature.

In the context of this invention, the term "mutant" with respect to a mutant polypeptide or mutant polynucleotide is used interchangeably with "variant." A variant with respect to a given wild-type CH3 or CH2 domain reference sequence can include naturally occurring allelic variants. A "non-naturally" occurring CH3 or CH2 domain refers to a variant or mutant domain that is not present in a cell in nature and that is produced by genetic modification, e.g., using genetic engineering technology or mutagenesis techniques, of a native CH3 domain or CH2 domain polynucleotide or polypeptide. A "variant" includes any domain comprising at least one amino acid mutation with respect to wild-type. Mutations may include substitutions, insertions, and deletions.

The term "amino acid" refers to naturally occurring and synthetic amino acids, as well as amino acid analogs and amino acid mimetics that function in a manner similar to the naturally occurring amino acids.

Naturally occurring amino acids are those encoded by the genetic code, as well as those amino acids that are later modified, *e.g.,* hydroxyproline, γ-carboxyglutamate and O-phosphoserine. "Amino acid analogs" refers to compounds that have the same basic chemical structure as a naturally occurring amino acid, *i.e.,* an α carbon that is bound to a hydrogen, a carboxyl group, an amino group, and an R group, *e.g*., homoserine, norleucine, methionine sulfoxide, methionine methyl sulfonium. Such analogs have modified R groups (*e.g*., norleucine) or modified peptide backbones, but retain the same basic chemical structure as a naturally occurring amino acid. "Amino acid mimetics" refers to chemical compounds that have a structure that is different from the general chemical structure of an amino acid, but that function in a manner similar to a naturally occurring amino acid.

Naturally occurring α-amino acids include, without limitation, alanine (Ala), cysteine (Cys), aspartic acid (Asp), glutamic acid (Glu), phenylalanine (Phe), glycine (Gly), histidine (His), isoleucine (Ile), arginine (Arg), lysine (Lys), leucine (Leu), methionine (Met), asparagine (Asn), proline (Pro), glutamine (Gln), serine (Ser), threonine (Thr), valine (Val), tryptophan (Trp), tyrosine (Tyr), and combinations thereof. Stereoisomers of a naturally occurring α-amino acids include, without limitation, D-alanine (D-Ala), D-cysteine (D-Cys), D-aspartic acid (D-Asp), D-glutamic acid (D-Glu), D-phenylalanine (D-Phe), D-histidine (D-His), D-isoleucine (D-Ile), D-arginine (D-Arg), D-lysine (D-Lys), D-leucine (D-Leu), D-methionine (D-Met), D-asparagine (D-Asn), D-proline (D-Pro), D-glutamine (D-Gln), D-serine (D-Ser), D-threonine (D-Thr), D-valine (D-Val), D-tryptophan (D-Trp), D-tyrosine (D-Tyr), and combinations thereof.

Amino acids may be referred to herein by either their commonly known three letter symbols or by the one-letter symbols recommended by the IUPAC-IUB Biochemical Nomenclature Commission.

The terms "polypeptide," "peptide," and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymers. Amino acid polymers may comprise entirely L-amino acids, entirely D-amino acids, or a mixture of L and D amino acids.

The term "conservative substitution," "conservative mutation," or "conservatively modified variant" refers to an alteration that results in the substitution of an amino acid with another amino acid that can be categorized as having a similar feature. Examples of categories of conservative amino acid groups defined in this manner can include: a "charged/polar group" including Glu (Glutamic acid or E), Asp (Aspartic acid or D), Asn (Asparagine or N), Gln (Glutamine or Q), Lys (Lysine or K), Arg (Arginine or R), and His (Histidine or H); an "aromatic group" including Phe (Phenylalanine or F), Tyr (Tyrosine or Y), Trp (Tryptophan or W), and (Histidine or H); and an "aliphatic group" including Gly (Glycine or G), Ala (Alanine or A), Val (Valine or V), Leu (Leucine or L), Ile (Isoleucine or I), Met (Methionine or M), Ser (Serine or S), Thr (Threonine or T), and Cys (Cysteine or C). Within each group, subgroups can also be identified. For example, the group of charged or polar amino acids can be sub-divided into sub-groups including: a "positively-charged sub-group" comprising Lys, Arg and His; a "negatively-charged sub-group" comprising Glu and Asp; and a "polar sub-group" comprising Asn and Gln. In another example, the aromatic or cyclic group can be sub-divided into sub-groups including: a "nitrogen ring sub-group" comprising Pro, His and Trp; and a "phenyl sub-group" comprising Phe and Tyr. In another further example, the aliphatic group can be sub-divided into sub-groups, *e.g.,* an "aliphatic non-polar sub-group" comprising Val, Leu, Gly, and Ala; and an "aliphatic slightly-polar sub-group" comprising Met, Ser, Thr, and Cys. Examples of categories of conservative mutations include amino acid substitutions of amino acids within the sub-groups above, such as, but not limited to: Lys for Arg or vice versa, such that a positive charge can be maintained; Glu for Asp or vice versa, such that a negative charge can be maintained; Ser for Thr or vice versa, such that a free -OH can be maintained; and Gln for Asn or vice versa, such that a free -NH₂ can be maintained. In some embodiments, hydrophobic amino acids are substituted for naturally occurring hydrophobic amino acid, *e.g.,* in the active site, to preserve hydrophobicity.

The terms "identical" or percent "identity," in the context of two or more polypeptide sequences, refer to two or more sequences or subsequences that are the same or have a specified percentage of amino acid residues, *e.g.,* at least 60%, at least 65%, at least 70%, at least 75%, at least 80%, at least 85%, at least 90%, or at least 95% or greater, that are identical over a specified region when compared and aligned for maximum correspondence over a comparison window or designated region, as measured using a sequence comparison algorithm or by manual alignment and visual inspection.

For sequence comparison of polypeptides, typically one amino acid sequence acts as a reference sequence, to which a candidate sequence is compared. Alignment can be performed using various methods available to one of skill in the art, *e.g.,* visual alignment or using publicly available software using known algorithms to achieve maximal alignment. Such programs include the BLAST programs, ALIGN, ALIGN-2 (Genentech, South San Francisco, Calif.) or Megalign (DNASTAR). The parameters employed for an alignment to achieve maximal alignment can be determined by one of skill in the art. For sequence comparison of polypeptide sequences for purposes of this application, the BLASTP algorithm standard protein BLAST for aligning two proteins sequence with the default parameters is used.

The terms "corresponding to," "determined with reference to," or "numbered with reference to" when used in the context of the identification of a given amino acid residue in a polypeptide sequence, refers to the position of the residue of a specified reference sequence when the given amino acid sequence is maximally aligned and compared to the reference sequence. Thus, for example, an amino acid residue in a polypeptide "corresponds to" an amino acid in the region of SEQ ID NO:1 from amino acids 114-220 when the residue aligns with the amino acid in SEQ ID NO:1 when optimally aligned to SEQ ID NO:1. The polypeptide that is aligned to the reference sequence need not be the same length as the reference sequence.

A "binding affinity" as used herein refers to the strength of the non-covalent interaction between two molecules, *e.g.,* a single binding site on a polypeptide and a target, e.g., transferrin receptor, to which it binds. Thus, for example, the term may refer to 1:1 interactions between a polypeptide and its target, unless otherwise indicated or clear from context. Binding affinity may be quantified by measuring an equilibrium dissociation constant (K_{D}), which refers to the dissociation rate constant (k_{d}, time⁻¹) divided by the association rate constant (kₐ, time⁻¹ M⁻¹). K_{D} can be determined by measurement of the kinetics of complex formation and dissociation, e.g., using Surface Plasmon Resonance (SPR) methods, *e.g.,* a Biacore^{™} system; kinetic exclusion assays such as KinExA^{®}; and BioLayer interferometry (*e.g*., using the ForteBio^{®} Octet^{®} platform). As used herein, "binding affinity" includes not only formal binding affinities, such as those reflecting 1:1 interactions between a polypeptide and its target, but also apparent affinities for which K_{D}'s are calculated that may reflect avid binding.

As used herein, the term "specifically binds" or "selectively binds" to a target, *e.g.,* TfR, when referring to a polypeptide comprising a modified CH3 domain as described herein, refers to a binding reaction whereby the polypeptide binds to the target with greater affinity, greater avidity, and/or greater duration than it binds to a structurally different target. In typical embodiments, the polypeptide has at least 5-fold, 6-fold, 7-fold, 8-fold, 9-fold, 10-fold, 20-fold, 25-fold, 50-fold, 100-fold, 1000-fold, 10,000-fold, or greater affinity for a specific target, *e.g.,* TfR, compared to an unrelated target when assayed under the same affinity assay conditions. The term "specific binding," "specifically binds to," or "is specific for" a particular target *(e.g.,* TfR), as used herein, can be exhibited, for example, by a molecule having an equilibrium dissociation constant K_{D} for the target to which it binds of, *e.g.,* 10⁻⁴ M or smaller *e.g*., 10⁻⁵ M, 10⁻⁶ M, 10⁻⁷ M, 10⁻⁸ M, 10⁻⁹ M, 10⁻¹⁰ M, 10⁻¹¹ M, or 10⁻¹² M. In some embodiments, a modified CH3 domain polypeptide specifically binds to an epitope on TfR that is conserved among species (*e.g*., structurally conserved among species), *e.g.,* conserved between non-human primate and human species *(e.g.,* structurally conserved between non-human primate and human species). In some embodiments, a polypeptide may bind exclusively to a human TfR.

The term "subject," "individual," and "patient," as used interchangeably herein, refer to a mammal, including but not limited to humans, non-human primates, rodents (*e.g.,* rats, mice, and guinea pigs), rabbits, cows, pigs, horses, and other mammalian species. In one embodiment, the patient is a human.

The terms "treatment," "treating," and the like are used herein to generally mean obtaining a desired pharmacologic and/or physiologic effect. "Treating" or "treatment" may refer to any indicia of success in the treatment or amelioration of an injury, disease, or condition, including any objective or subjective parameter such as abatement, remission, improvement in patient survival, increase in survival time or rate, diminishing of symptoms or making the injury, disease, or condition more tolerable to the patient, slowing in the rate of degeneration or decline, or improving a patient's physical or mental well-being. The treatment or amelioration of symptoms can be based on objective or subjective parameters. The effect of treatment can be compared to an individual or pool of individuals not receiving the treatment, or to the same patient prior to treatment or at a different time during treatment.

The term "pharmaceutically acceptable excipient" refers to a non-active pharmaceutical ingredient that is biologically or pharmacologically compatible for use in humans or animals, such as but not limited to a buffer, carrier, or preservative.

As used herein, a "therapeutic amount" or "therapeutically effective amount" of an agent is an amount of the agent that treats, alleviates, abates, or reduces the severity of symptoms of a disease in a subject. A "therapeutic amount" or "therapeutically effective amount" of an agent may improve patient survival, increase survival time or rate, diminish symptoms, make an injury, disease, or condition more tolerable, slow the rate of degeneration or decline, or improve a patient's physical or mental well-being.

The term "administer" refers to a method of delivering agents, compounds, or compositions to the desired site of biological action. These methods include, but are not limited to, topical delivery, parenteral delivery, intravenous delivery, intradermal delivery, intramuscular delivery, intrathecal delivery, colonic delivery, rectal delivery, or intraperitoneal delivery. In one embodiment, the polypeptides described herein are administered intravenously.

### III. TRANSFERRIN RECEPTOR-BINDING POLYPEPTIDES

This section describes generation of polypeptides in accordance with the invention that bind to a transferrin receptor and are capable of being transported across the blood-brain barrier (BBB).

In one aspect, polypeptides are provided that comprise CH3 domains that have modifications that allow the polypeptides to specifically bind to a transferrin receptor. The modifications are introduced into a specified set of amino acids that are present at the surface of the CH3 domain. In some embodiments, polypeptides comprising modified CH3 domains specifically bind to an epitope in the apical domain of the transferrin receptor.

One of skill understands that CH3 domains of other immunoglobulin isotypes, *e.g.,* IgM, IgA, IgE, IgD, *etc.* may be similarly modified by identifying the amino acids in those domains that correspond to the specified set of amino acids described herein. Modifications may also be made to corresponding domains from immunoglobulins from other species, *e.g.,* non-human primates, monkey, mouse, rat, rabbit, dog, pig, chicken, and the like.

### CH3 transferrin receptor-binding polypeptides

In some embodiments, the domain that is modified is a human Ig CH3 domain, such as an IgG CH3 domain. The CH3 domain can be of any IgG subtype, *i.e.,* from IgG1, IgG2, IgG3, or IgG4. In the context of IgG antibodies, a CH3 domain refers to the segment of amino acids from about position 341 to about position 447 as numbered according to the EU numbering scheme. The positions in the CH3 domain for purposes of identifying the corresponding set of amino acid positions for transferrin receptor binding are determined with reference to SEQ ID NO:3 or determined with reference to amino acids 114-220 of SEQ ID NO:1 unless otherwise specified. Substitutions are also determined with reference to SEQ ID NO:1, *i.e.,* an amino acid is considered to be a substitution relative to the amino acid at the corresponding position in SEQ ID NO:1. SEQ ID NO:1 includes a partial hinge region sequence, PCP, as amino acids 1-3. The numbering of the positions in the CH3 domain with reference to SEQ ID NO:1 includes the first three amino acids.

As indicated above, a set of residues of a CH3 domain that can be modified in accordance with the invention is numbered herein with reference to SEQ ID NO:1. Any CH3 domain, *e.g.,* an IgG1, IgG2, IgG3, or IgG4 CH3 domain, may have modifications, *e.g.,* amino acid substitutions, in a set of residues that correspond to residues at the noted positions in SEQ ID NO:1. An alignment of the human IgG1 amino acid sequence of SEQ ID NO:1 with human IgG2, IgG3, and IgG4 is shown in FIG. 33. The positions of each of the IgG2, IgG3, and IgG4 sequences that correspond to any given position of SEQ ID NO:1 can be readily determined.

In one embodiment, a modified CH3 domain polypeptide that specifically binds transferrin receptor binds to the apical domain of the transferrin receptor at an epitope that comprises position 208 of the full length human transferrin receptor sequence (SEQ ID NO:38), which corresponds to position 11 of the human transferrin receptor apical domain sequence set forth in SEQ ID NO:30. SEQ ID NO:30 corresponds to amino acids 198-378 of the human transferrin receptor-1 uniprotein sequence P02786 (SEQ ID NO:38). In some embodiments, the modified CH3 domain polypeptide binds to the apical domain of the transferrin receptor at an epitope that comprises positions 158, 188, 199, 207, 208, 209, 210, 211, 212, 213, 214, 215, and/or 294 of the full length human transferrin receptor sequence (SEQ ID NO:38). The modified CH3 domain polypeptide may bind to the transferrin receptor without blocking or otherwise inhibiting binding of transferrin to the receptor. In some embodiments, binding of transferrin to TfR is not substantially inhibited. In some embodiments, binding of transferrin to TfR is inhibited by less than about 50% *(e.g.,* less than about 45%, 40%, 35%, 30%, 25%, 20%, 15%, 10%, or 5%). In some embodiments, binding of transferrin to TfR is inhibited by less than about 20% *(e.g.,* less than about 19%, 18%, 17%, 16%, 15%, 14%, 13%, 12%, 11%, 10%, 9%, 8%, 7%, 6%, 5%, 4%, 3%, 2%, or 1%). Illustrative CH3 domain polypeptides that exhibit this binding specificity include polypeptides having amino acid substitutions at positions 157, 159, 160, 161, 162, 163, 186, 189, and 194 as determined with reference to amino acids 114-220 of SEQ ID NO:1.

### CH3 transferrin receptor binding set: 157, 159, 160, 161, 162, 163, 186, 189, and 194

In some embodiments, a modified CH3 domain polypeptide in accordance with the invention comprises at least three or at least four, and typically five, six, seven, eight, or nine substitutions in a set of amino acid positions comprising 157, 159, 160, 161, 162, 163, 186, 189, and 194. Illustrative substitutions that may be introduced at these positions are shown in Table 1. In some embodiments, the amino acid at position 161 and/or 194 is an aromatic amino acid, *e.g.,* Trp, Phe, or Tyr. In some embodiments, the amino acid at position 161 is Trp. In some embodiments, the amino acid at position 161 is Gly. In some embodiments, the aromatic amino acid at position 194 is Trp or Phe.

In some embodiments, a modified CH3 domain polypeptide that specifically binds a transferrin receptor comprises at least one position having a substitution, relative to SEQ ID NO:1, as follows: Leu, Tyr, Met, or Val at position 157; Leu, Thr, His, or Pro at position 159; Val, Pro, or an acidic amino acid at position 160; an aromatic amino acid, *e.g.,Trp* or Gly (*e.g*., Trp) at position 161; Val, Ser, or Ala at position 162; an acidic amino acid, Ala, Ser, Leu, Thr, or Pro at position 186; Thr or an acidic amino acid at position 189; or Trp, Tyr, His, or Phe at position 194. In some embodiments, a modified CH3 domain polypeptide may comprise a conservative substitution, *e.g.,* an amino acid in the same charge grouping, hydrophobicity grouping, side chain ring structure grouping (*e.g*., aromatic amino acids), or size grouping, and/or polar or non-polar grouping, of a specified amino acid at one or more of the positions in the set. Thus, for example, Ile may be present at position 157, 159, and/or position 186. In some embodiments, the acidic amino acid at position one, two, or each of positions 160, 186, and 189 is Glu. In other embodiments, the acidic amino acid at one, two or each of positions 160, 186, and 189 is Asp. In some embodiments, two, three, four five, six, seven, or all eight of positions 157, 159, 160, 161, 162, 186, 189, and 194 have an amino acid substitution as specified in this paragraph.

In some embodiments, the modified CH3 domain polypeptide comprises a native Asn at position 163. In some embodiments, the modified CH3 domain polypeptide comprises Gly, His, Gln, Leu, Lys, Val, Phe, Ser, Ala, or Asp at position 163. In some embodiments, the modified CH3 domain polypeptide further comprises one, two, three, or four substitutions at positions comprising 153, 164, 165, and 188. In some embodiments, Trp, Tyr, Leu, or Gln may be present at position 153. In some embodiments, Ser, Thr, Gln, or Phe may be present at position 164. In some embodiments, Gln, Phe, or His may be present at position 165. In some embodiments, Glu may be present at position 188.

In certain embodiments, the modified CH3 domain polypeptide comprises two, three, four, five, six, seven, eight nine, or ten positions selected from the following: Trp, Leu, or Glu at position 153; Tyr or Phe at position 157; Thr at position 159; Glu at position 160; Trp at position 161; Ser, Ala, Val, or Asn at position 162; Ser or Asn at position 163; Thr or Ser at position 186; Glu or Ser at position 188; Glu at position 189; and/or Phe at position 194. In some embodiments, the modified CH3 domain polypeptide comprises all eleven positions as follows: Trp, Leu, or Glu at position 153; Tyr or Phe at position 157; Thr at position 159; Glu at position 160; Trp at position 161; Ser, Ala, Val, or Asn at position 162; Ser or Asn at position 163; Thr or Ser at position 186; Glu or Ser at position 188; Glu at position 189; and/or Phe at position 194.

In certain embodiments, the modified CH3 domain polypeptide comprises Leu or Met at position 157; Leu, His, or Pro at position 159; Val at position 160; Trp at position 161; Val or Ala at position 162; Pro at position 186; Thr at position 189; and/or Trp at position 194. In some embodiments, the modified CH3 domain polypeptide further comprises Ser, Thr, Gln, or Phe at position 164. In some embodiments, a modified CH3 domain polypeptide further comprises Trp, Tyr, Leu, or Gln at position 153 and/or Gln, Phe, or His at position 165. In some embodiments, Trp is present at position 153 and/or Gln is present at position 165. In some embodiments, a modified CH3 domain polypeptide does not have a Trp at position 153.

In other embodiments, a modified CH3 domain polypeptide comprises Tyr at position 157; Thr at position 159; Glu or Val at position 160; Trp at position 161; Ser at position 162; Ser or Thr at position 186; Glu at position 189; and/or Phe at position 194. In some embodiments, the modified CH3 domain polypeptide comprises a native Asn at position 163. In certain embodiments, the modified CH3 domain polypeptide further comprises Trp, Tyr, Leu, or Gln at position 153; and/or Glu at position 188. In some embodiments, the modified CH3 domain polypeptide further comprises Trp at position 153 and/or Glu at position 188.

In some embodiments, the modified CH3 domain polypeptide comprises one or more of the following substitutions: Trp at position 153; Thr at position 159; Trp at position 161; Val at position 162; Ser or Thr at position 186; Glu at position 188; and/or Phe at position 194.

In additional embodiments, the modified CH3 domain polypeptide further comprises one, two, or three positions selected from the following: position 187 is Lys, Arg, Gly, or Pro; position 197 is Ser, Thr, Glu, or Lys; and position 199 is Ser, Trp, or Gly.

In some embodiments, a modified CH3 domain polypeptide that specifically binds transferrin receptor has at least 70% identity, at least 75% identity, at least 80% identity, at least 85% identity, at least 90% identity, or at least 95% identity to amino acids 114-220 of any one of SEQ ID NOS:4-29, 39-102, and 182-195. In some embodiments, such a modified CH3 domain polypeptide comprises amino acids 157-163 and/or 186-194 of any one of SEQ ID NOS:4-29, 39-102, and 182-195. In some embodiments, such a modified CH3 domain polypeptide comprises amino acids 153-163 and/or 186-194 of any one of SEQ ID NOS:4-29, 39-102, and 182-195. In some embodiments, a modified CH3 domain polypeptide comprises amino acids 153-163 and/or 186-199 of any one of SEQ ID NOS:4-29, 39-102, and 182-195.

In some embodiments, a modified CH3 domain polypeptide that specifically binds transferrin receptor has at least 70% identity, at least 75% identity, at least 80% identity, at least 85% identity, at least 90% identity, or at least 95% identity to amino acids 114-220 of SEQ ID NO:1, with the proviso that the percent identity does not include the set of positions 157, 159, 160, 161, 162, 163, 186, 189, and 194. In some embodiments, the modified CH3 domain polypeptide comprises amino acids 157-163 and/or amino acids 186-194 as set forth in any one of SEQ ID NOS:4-29, 39-102, and 182-195.

In some embodiments, a modified CH3 domain polypeptide has at least 70% identity, at least 75% identity, at least 80% identity, at least 85% identity, at least 90% identity, or at least 95% identity to any one of SEQ ID NOS:4-29, 39-102, and 182-195, with the proviso that at least five, six, seven, eight, nine, ten, eleven, twelve, thirteen, fourteen, fifteen, or sixteen of the positions that correspond to positions 153, 157, 159, 160, 161, 162, 163, 164, 165, 186, 187, 188, 189, 194, 197, and 199 of any one of SEQ ID NOS:4-29, 39-102, and 182-195 are not deleted or substituted.

In some embodiments, the modified CH3 domain polypeptide has at least 75% identity, at least 80% identity, at least 85% identity, at least 90% identity, or at least 95% identity to any one of SEQ ID NOS:4-29, 39-102, and 182-195 and also comprises at at least five, six, seven, eight, nine, ten, eleven, twelve, thirteen, fourteen, fifteen or sixteen of the positions as follows: Trp, Tyr, Leu, Gln, or Glu at position 153; Leu, Tyr, Met, or Val at position 157; Leu, Thr, His, or Pro at position 159; Val, Pro, or an acidic amino acid at position 160; an aromatic amino acid, *e.g*., Trp, at position 161; Val, Ser, or Ala at position 162; Ser or Asn at position 163; Ser, Thr, Gln, or Phe at position 164; Gln, Phe, or His at position 165; an acidic amino acid, Ala, Ser, Leu, Thr, or Pro at position 186; Lys, Arg, Gly or Pro at position 187; Glu or Ser at position 188; Thr or an acidic amino acid at position 189; Trp, Tyr, His or Phe at position 194; Ser, Thr, Glu or Lys at position 197; and Ser, Trp, or Gly at position 199.

In some embodiments, a modified CH3 domain polypeptide in accordance with the invention comprises one or more substitutions in a set of amino acid positions comprising 153, 157, 159, 160, 162, 163, 186, 188, 189, 194, 197, and 199; and wherein the substitutions and the positions are determined with reference to the sequence of SEQ ID NO:13. In some embodiments, the modified CH3 domain comprises Glu, Leu, Ser, Val, Trp, or Tyr at position 153; an aromatic amino acid (*e.g*., Tyr, Phe, or Trp), Met, Pro, or Val at position 157; Thr, Asn, or Val at position 159; Glu, Ile, Pro, or Val at position 160; an aliphatic amino acid (*e*.*g.,* Ala, Ile, or Val), Ser, or Thr at position 162; Ser, Asn, Arg, or Thr at position 163; Thr, His, or Ser at position 186; Glu, Ser, Asp, Gly, Thr, Pro, Gln, or Arg at position 188; Glu or Arg at position 189; Phe, His, Lys, Tyr, or Trp at position 194; Ser, Thr, or Trp at position 197; and Ser, Cys, Pro, Met, or Trp at position 199. In certain embodiments, a modified CH3 domain polypeptide may comprise the sequence of SEQ ID NO:316. In certain embodiments, a modified CH3 domain polypeptide may comprise the sequence of SEQ ID NO:314. In certain embodiments, a modified CH3 domain polypeptide may comprise the sequence of SEQ ID NO:315.

In some embodiments, the modified CH3 domain polypeptide comprises Glu, Leu, or Trp at position 153; an aromatic amino acid at position 157; Thr at position 159; Glu at position 160; an aliphatic amino acid or Ser at position 162; Ser or Asn at position 163; Thr or Ser at position 186; Glu or Ser at position 188; Glu at position 189; Phe, His, Tyr, or Trp at position 194; Ser at position 197; and Ser at position 199, wherein the substitutions and the positions are determined with reference to the sequence of SEQ ID NO:13. In particular embodiments, the aromatic amino acid at position 157 is Tyr or Phe and the aliphatic amino acid at position 162 is Ala or Val. In certain embodiments, a modified CH3 domain polypeptide may comprise the sequence of SEQ ID NO:319. In certain embodiments, a modified CH3 domain polypeptide may comprise the sequence of SEQ ID NO:317. In certain embodiments, a modified CH3 domain polypeptide may comprise the sequence of SEQ ID NO:318.

In further embodiments, the modified CH3 domain polypeptide may comprise Glu, Leu, or Trp at position 153; Tyr or Phe at position 157; Thr at position 159; Glu at position 160; Ala, Val, or Ser at position 162; Ser or Asn at position 163; Thr or Ser at position 186; Glu or Ser at position 188; Glu at position 189; Phe at position 194; Ser at position 197; and Ser at position 199, wherein the substitutions and the positions are determined with reference to the sequence of SEQ ID NO:13. In certain embodiments, a modified CH3 domain polypeptide may comprise the sequence of SEQ ID NO:322. In certain embodiments, a modified CH3 domain polypeptide may comprise the sequence of SEQ ID NO:320. In certain embodiments, a modified CH3 domain polypeptide may comprise the sequence of SEQ ID NO:321.

In some embodiments, a modified CH3 domain polypeptide in accordance with the invention comprises only one substitution in a set of amino acid positions comprising 153, 157, 159, 160, 162, 163, 186, 188, 189, 194, 197, and 199; and wherein the substitutions and the positions are determined with reference to the sequence of SEQ ID NO:41. In some embodiments, the modified CH3 domain polypeptide comprises Glu, Leu, Ser, Val, Trp, or Tyr at position 153. The modified CH3 domain polypeptide may comprise Glu at position 153. The modified CH3 domain polypeptide may comprises Leu at position 153. The modified CH3 domain polypeptide may comprises Ser at position 153. The modified CH3 domain polypeptide may comprises Val at position 153. The modified CH3 domain polypeptide may Trp at position 153. The modified CH3 domain polypeptide may comprises Tyr at position 153. In some embodiments, the modified CH3 domain polypeptide comprises Tyr, Phe, Trp, Met, Pro, or Val at position 157. The modified CH3 domain polypeptide may comprise Tyr at position 157. The modified CH3 domain polypeptide may comprise Phe at position 157. The modified CH3 domain polypeptide may comprise Trp at position 157. The modified CH3 domain polypeptide may comprise Met at position 157. The modified CH3 domain polypeptide may comprise Pro at position 157. The modified CH3 domain polypeptide may comprise Val at position 157. In some embodiments, the modified CH3 domain polypeptide comprises Thr, Asn, or Val at position 159. The modified CH3 domain polypeptide may comprise Thr at position 159. The modified CH3 domain polypeptide may comprise Asn at position 159. The modified CH3 domain polypeptide may comprise Val at position 159. In some embodiments, the modified CH3 domain polypeptide comprises Glu, Ile, Pro, or Val at position 160. The modified CH3 domain polypeptide may comprise Glu at position 160. The modified CH3 domain polypeptide may comprise Ile at position 160. The modified CH3 domain polypeptide may comprise Pro at position 160. The modified CH3 domain polypeptide may comprise Val at position 160. In some embodiments, the modified CH3 domain polypeptide comprises Ala, Ile, Val, Ser, or Thr at position 162. The modified CH3 domain polypeptide may comprise Ala at position 162. The modified CH3 domain polypeptide may comprise Ile at position 162. The modified CH3 domain polypeptide may comprise Val at position 162. The modified CH3 domain polypeptide may comprise Ser at position 162. The modified CH3 domain polypeptide may comprise Thr at position 162. In some embodiments, the modified CH3 domain polypeptide comprises Ser, Asn, Arg, or Thr at position 163. The modified CH3 domain polypeptide may comprise Ser at position 163. The modified CH3 domain polypeptide may comprise Asn at position 163. The modified CH3 domain polypeptide may comprise Arg at position 163. The modified CH3 domain polypeptide may comprise Thr at position 163. In some embodiments, the modified CH3 domain polypeptide comprises Thr, His, or Ser at position 186. The modified CH3 domain polypeptide may comprise Thr at position 186. The modified CH3 domain polypeptide may comprise His at position 186. The modified CH3 domain polypeptide may comprise Ser at position 186. In some embodiments, the modified CH3 domain polypeptide comprises Glu, Ser, Asp, Gly, Thr, Pro, Gln, or Arg at position 188. The modified CH3 domain polypeptide may comprise Glu at position 188. The modified CH3 domain polypeptide may comprise Ser at position 188. The modified CH3 domain polypeptide may comprise Asp at position 188. The modified CH3 domain polypeptide may comprise Gly at position 188. The modified CH3 domain polypeptide may comprise Thr at position 188. The modified CH3 domain polypeptide may comprise Pro at position 188. The modified CH3 domain polypeptide may comprise Gln at position 188. The modified CH3 domain polypeptide may comprise Arg at position 188. In some embodiments, the modified CH3 domain polypeptide comprises Glu or Arg at position 189. The modified CH3 domain polypeptide may comprise Glu at position 189. The modified CH3 domain polypeptide may comprise Arg at position 189. In some embodiments, the modified CH3 domain polypeptide comprises Phe, His, Lys, Tyr, or Trp at position 194. The modified CH3 domain polypeptide may comprise Phe at position 194. The modified CH3 domain polypeptide may comprise His at position 194. The modified CH3 domain polypeptide may comprise Lys at position 194. The modified CH3 domain polypeptide may comprise Tyr at position 194. The modified CH3 domain polypeptide may comprise Trp at position 194. In some embodiments, the modified CH3 domain polypeptide comprises Ser, Thr, or Trp at position 197. The modified CH3 domain polypeptide may comprise Ser at position 197. The modified CH3 domain polypeptide may comprise Thr at position 197. The modified CH3 domain polypeptide may comprise Trp at position 197. In some embodiments, the modified CH3 domain polypeptide comprises Ser, Cys, Pro, Met, or Trp at position 199. The modified CH3 domain polypeptide may comprise Ser at position 199. The modified CH3 domain polypeptide may comprise Cys at position 199. The modified CH3 domain polypeptide may comprise Pro at position 199. The modified CH3 domain polypeptide may comprise Met at position 199. The modified CH3 domain polypeptide may comprise Trp at position 199. A modified CH3 domain polypeptide may have the sequence of any one of SEQ ID NOS:323-334.

In some embodiments, a modified CH3 domain polypeptide in accordance with the invention comprises one or more substitutions in a set of amino acid positions comprising 153, 157, 159, 160, 162, 163, 164, 186, 189, and 194; and wherein the substitutions and the positions are determined with reference to the sequence of SEQ ID NO:9. In some embodiments, the modified CH3 domain comprises Glu or Trp at position 153; Val, Trp, Leu, or Tyr at position 157; Leu, Pro, Phe, Thr, or His at position 159; Pro, Val, or Glu at position 160; Ala, Ser, Val, or Gly at position 162; Leu, His, Gln, Gly, Val, Ala, Asn, Asp, Thr, or Glu at position 163; Thr, Phe, Gln, Val, or Tyr at position 164; Leu, Ser, Glu, Ala, or Pro at position 186; Glu, Asp, Thr, or Asn at position 189; and Trp, Tyr, Phe, or His at position 194. In certain embodiments, a modified CH3 domain polypeptide may comprise the sequence of SEQ ID NO:337. In certain embodiments, a modified CH3 domain polypeptide may comprise the sequence of SEQ ID NO:335. In certain embodiments, a modified CH3 domain polypeptide may comprise the sequence of SEQ ID NO:336.

In some embodiments, the modified CH3 domain polypeptide comprises Glu or Trp at position 153; Trp, Leu, or Tyr at position 157; Thr or His at position 159; Val at position 160; Ala, Ser, or Val at position 162; Val, Asn, or Thr at position 163; Gln or Tyr at position 164; Pro at position 186; Thr or Asn at position 189; and Trp, Tyr, Phe, or His at position 194, wherein the substitutions and the positions are determined with reference to the sequence of SEQ ID NO:9. In certain embodiments, a modified CH3 domain polypeptide may comprise the sequence of SEQ ID NO:340. In certain embodiments, a modified CH3 domain polypeptide may comprise the sequence of SEQ ID NO:338. In certain embodiments, a modified CH3 domain polypeptide may comprise the sequence of SEQ ID NO:339.

In additional embodiments, a transferrin receptor-binding polypeptide comprises amino acids 157-194, amino acids 153-194, or amino acids 153-199, of any one of SEQ ID NOS:4-29, 39-102, and 182-195. In further embodiments, the polypeptide comprises an amino acid sequence having at least 75% identity, at least 80% identity, at least 85% identity, at least 90% identity, or at least 95% identity to amino acids 157-194 of any one of SEQ ID NOS:4-29, 39-102, and 182-195, or to amino acids 153-194, or to amino acids 153-199, of any one of SEQ ID NOS:4-29, 39-102, and 182-195.

In some embodiments, the polypeptide comprises any one of SEQ ID NOS:4-29, 39-102, and 182-195. In further embodiments, the polypeptide comprises any one of SEQ ID NOS:4-29, 39-102, and 182-195 without the first three amino acids "PCP" at the amino-terminal end. In further embodiments, the polypeptide may have at least 75% identity, at least 80% identity, at least 85% identity, at least 90% identity, or at least 95% identity to any one of SEQ ID NOS:4-29, 39-102, and 182-195 as determined without the first three amino acids "PCP" at the amino-terminal end.

### Illustrative polypeptides comprising modified CH3 domains

A modified CH3 domain polypeptide of the present invention may be joined to another domain of an Fc region. In some embodiments, a modified CH3 domain polypeptide of the present invention is joined to a CH2 domain, which may be a naturally occurring CH2 domain or a variant CH2 domain, typically at the C-terminal end of the CH2 domain. In some embodiments, the polypeptide comprising a modified CH3 domain joined to a CH2 domain further comprises a partial or full hinge region of an antibody, thus resulting in a format in which the modified CH3 domain polypeptide is part of an Fc region having a partial or full hinge region. The hinge region can be from any immunoglobulin subclass or isotype. An illustrative immunoglobulin hinge is an IgG hinge region, such as an IgG1 hinge region, *e.g.,* human IgG1 hinge amino acid sequence EPKSCDKTHTCPPCP (SEQ ID NO:37). In further embodiments, the polypeptide, which may be in an Fc format containing a hinge or partial hinge region, is further joined to another moiety, for example, a Fab fragment, thus generating a transferrin receptor-binding Fc-Fab fusion. In some embodiments, the transferrin receptor-binding Fc-Fab fusion comprises a modified CH3 domain polypeptide, a hinge region, and a Fab fragment. The Fab fragment may be to any target of interest, *e.g.,* a therapeutic neurological target, where the Fab is delivered to the target by transcytosis across the blood-brain barrier mediated by the binding of the modified CH3 domain polypeptide to the transferrin receptor.

In some embodiments, a Fab fragment joined to a transferrin receptor-binding polypeptide may bind to a Tau protein *(e.g.,* a human Tau protein) or a fragment thereof. In some embodiments, the Fab fragment may bind to a phosphorylated Tau protein, an unphosphorylated Tau protein, a splice isoform of Tau protein, an N-terminal truncated Tau protein, a C-terminal truncated Tau protein, and/or a fragment thereof.

In some embodiments, a Fab fragment joined to a transferrin receptor-binding polypeptide may bind to a beta-secretase 1 (BACE1) protein *(e.g.,* a human BACE1 protein) or a fragment thereof. In some embodiments, the Fab fragment may bind to one or more splice isoforms of BACE1 protein or a fragment thereof.

In some embodiments, a Fab fragment joined to a transferrin receptor-binding polypeptide may bind to a triggering receptor expressed on myeloid cells 2 (TREM2) protein *(e.g.,* a human TREM2 protein) or a fragment thereof.

In some embodiments, a Fab fragment joined to a transferrin receptor-binding polypeptide may bind to an alpha-synuclein protein *(e.g.,* a human alpha-synuclein protein) or a fragment thereof. In some embodiments, the Fab fragment may bind to a monomeric alpha-synuclein, oligomeric alpha-synuclein, alpha-synuclein fibrils, soluble alpha-synuclein, and/or a fragment thereof.

In some embodiments, an Fc-Fab fusion comprising a modified CH3 domain polypeptide of the present invention is a subunit of a dimer. In some embodiments, the dimer is a heterodimer. In some embodiments, the dimer is a homodimer. In some embodiments, the dimer comprises a single polypeptide that binds to the transferrin receptor, *i.e.,* is monovalent for transferrin receptor binding. In some embodiments, the dimer comprises a second polypeptide that binds to the transferrin receptor. The second polypeptide may comprise the same modified CH3 domain polypeptide present in the Fc-Fab fusion to provide a bivalent binding homodimer, or a second modified CH3 domain polypeptide of the present invention may provide a second transferrin receptor binding site. In some embodiments, the dimer comprises a first subunit comprising a modified CH3 domain polypeptide and a second subunit comprising CH2 and CH3 domains where neither binds transferrin receptor.

The transferrin receptor-binding polypeptide may also be fused to a different polypeptide of interest other than a Fab. For example, in some embodiments, the transferrin receptor-binding polypeptide may be fused to a different polypeptide that is desirable to target to a transferrin receptor-expressing cell or to deliver across an endothelium, e.g., the blood-brain barrier, by trancytosis. In some embodiments, the transferrin receptor-binding polypeptide is fused to a soluble protein, *e.g.,* an extracellular domain of a receptor or a growth factor, a cytokine, or an enzyme.

In still other embodiments, the transferrin receptor-binding polypeptide may be fused to a peptide or protein useful in protein purification, *e.g,* polyhistidine, epitope tags, *e.g.,* FLAG, c-Myc, hemagglutinin tags and the like, glutathione S transferase (GST), thioredoxin, protein A, protein G, or maltose binding protein (MBP). In some cases, the peptide or protein to which the transferrin receptor-binding polypeptide is fused may comprise a protease cleavage site, such as a cleavage site for Factor Xa or Thrombin.

Transferrin receptor-binding polypeptides of the present invention may have a broad range of binding affinities, *e.g.,* based on the format of the polypeptide. For example, in some embodiments, a polypeptide comprising a modified CH3 domain has an affinity for transferrin receptor binding ranging anywhere from 1 pM to 10 µM. In some embodiments, affinity may be measured in a monovalent format. In other embodiments, affinity may be measured in a bivalent format, *e.g.,* as a dimer comprising a polypeptide-Fab fusion protein.

Methods for analyzing binding affinity, binding kinetics, and cross-reactivity are known in the art. These methods include, but are not limited to, solid-phase binding assays *(e.g.,* ELISA assay), immunoprecipitation, surface plasmon resonance (*e.g.,* Biacore^{™} (GE Healthcare, Piscataway, NJ)), kinetic exclusion assays (*e*.*g.*, KinExA^{®}), flow cytometry, fluorescence-activated cell sorting (FACS), BioLayer interferometry (*e.g*., Octet^{®} (FortéBio, Inc., Menlo Park, CA)), and Western blot analysis. In some embodiments, ELISA is used to determine binding affinity and/or cross-reactivity. Methods for performing ELISA assays are known in the art and are also described in the Example section below. In some embodiments, surface plasmon resonance (SPR) is used to determine binding affinity, binding kinetics, and/or cross-reactivity. In some embodiments, kinetic exclusion assays are used to determine binding affinity, binding kinetics, and/or cross-reactivity. In some embodiments, BioLayer interferometry assays are used to determine binding affinity, binding kinetics, and/or cross-reactivity.

### Additional mutations in an Fc region that comprises a modified CH3 domain polypeptide

A polypeptide as provided herein that is modified to bind a transferrin receptor and initiate transport across the BBB may also comprise additional mutations, *e.g.,* to increase serum stability, to modulate effector function, to influence glyscosylation, to reduce immunogenicity in humans, and/or to provide for knob and hole heterodimerization of the polypeptide.

In some embodiments, a polypeptide in accordance with the invention has an amino acid sequence identity of at least about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% to a corresponding wild-type Fc region (*e.g*., a human IgG1, IgG2, IgG3, or IgG4 Fc region).

A polypeptide in accordance with the invention may also have other mutations introduced outside of the specified sets of amino acids, *e.g.,* to influence glyscosylation, to increase serum half-life or, for CH3 domains, to provide for knob and hole heterodimerization of polypeptides that comprise the modified CH3 domain. Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (*e.g*., tyrosine or tryptophan). Compensatory cavities of identical or similar size to the protuberances are created in the interface of the second polypeptide by replacing large amino acid side chains with smaller ones (*e.g*., alanine or threonine). Such additional mutations are at a position in the polypeptide that does not have a negative effect on binding of the modified CH3 domain to the transferrin receptor.

In one illustrative embodiment of a knob and hole approach for dimerization, a position corresponding to position 139 of SEQ ID NO:1 of a first Fc polypeptide subunit to be dimerized has a tryptophan in place of a native threonine and a second Fc polypeptide subunit of the dimer has a valine at a position corresponding to position 180 of SEQ ID NO:1 in place of the native tyrosine. The second subunit of the Fc polypeptide may further comprise a substitution in which the native threonine at the position corresponding to position 139 of SEQ ID NO:1 is substituted with a serine and a native leucine at the position corresponding to position 141 of SEQ ID NO:1 is substituted with an alanine.

A polypeptide as described herein may also be engineered to contain other modifications for heterodimerization, *e.g.,* electrostatic engineering of contact resdiues within a CH3-CH3 interface that are naturally charged or hydrophobic patch modifications.

In some embodiments, modifications to enhance serum half-life may be introduced. For example, in some embodiments, an Fc region comprises a CH2 domain comprising a Tyr at a position corresponding to position 25 of SEQ ID NO:1, Thr at a position corresponding to 27 of SEQ ID NO:1, and Glu at a position corresponding to position 29 of SEQ ID NO:1.

In some embodiments, a mutation, *e.g.,* a substitution, is introduced at one or more of positions 17-30, 52-57, 80-90, 156-163, and 201-208 as determined with reference to SEQ ID NO:1. In some embodiments, one or more mutations are introduced at positions 24, 25, 27, 28, 29, 80, 81, 82, 84, 85, 87, 158, 159, 160, 162, 201, 206, 207, or 209 as determined with reference to SEQ ID NO:1. In some embodiments, mutations are introduced into one, two, or three of positions 25, 27, and 29 as determined with reference to SEQ ID NO:1. In some embodiments, the mutations are M25Y, S27T, and T29E as numbered with reference to SEQ ID NO:1. In some embodiments, a polypeptide as described herein further comprises mutations M25Y, S27T, and T29E. In some embodiments, mutations are introduced into one or two of positions 201 and 207 as determined with reference to SEQ ID NO:1. In some embodiments, the mutations are M201L and N207S as numbered with reference to SEQ ID NO:1. In some embodiments, a polypeptide as described herein further comprises mutation N207S with or without M201L. In some embodiments, a polypeptide as described herein comprises a substitution at one, two or all three of positions T80, E153, and N207 as numbered with reference to SEQ ID NO:1. In some embodiments, the mutations are T80Q and N207A. In some embodiments, a polypeptide as described herein comprises mutations T80A, E153A, and N207A. In some embodiments, a polypeptide as described herein comprises substitutions at positions T23 and M201 as numbered with reference to SEQ ID NO:1. In some embodiments, a polypeptide as described herein comprises mutations T23Q and M201L. In some embodiments, a polypeptide as described herein comprises substitutions at positions M201 and N207 as numbered with reference to SEQ ID NO:1. In some embodiments, a polypeptide as described herein comprises substitutions M201L and N207S. In some embodiments, a polypeptide as described herein comprises an N207S or N207A substitution.

### Fc effector functions

In some embodiments, an Fc region comprising a modified CH3 domain has an effector function, *i.e.,* the Fc region has the ability to induce certain biological functions upon binding to an Fc receptor expressed on an effector cell that mediates the effector function. Effector cells include, but are not limited to, monocytes, macrophages, neutrophils, dendritic cells, eosinophils, mast cells, platelets, B cells, large granular lymphocytes, Langerhans' cells, natural killer (NK) cells, and cytotoxic T cells.

Examples of antibody effector functions include, but are not limited to, C1q binding and complement dependent cytotoxicity (CDC), Fc receptor binding, antibody-dependent cell-mediated cytotoxicity (ADCC), antibody-dependent cell-mediated phagocytosis (ADCP), down-regulation of cell surface receptors *(e.g.,* B cell receptor), and B-cell activation. Effector functions may vary with the antibody class. For example, native human IgG1 and IgG3 antibodies can elicit ADCC and CDC activities upon binding to an appropriate Fc receptor present on an immune system cell; and native human IgG1, IgG2, IgG3, and IgG4 can elicit ADCP functions upon binding to the appropriate Fc receptor present on an immune cell.

In some embodiments, a polypeptide as described herein may include additional modifications that reduce effector function. Alternatively, in some embodiments, a polypeptide comprising a modified CH3 domain of the invention may include additional modifications that enhance effector function.

Illustrative Fc polypeptide mutations that modulate an effector function include, but are not limited to, substitutions in a CH2 domain, *e.g.,* at positions corresponding to positions 7 and 8 of SEQ ID NO:1. In some embodiments, the substitutions in a CH2 domain comprise Ala at positions 7 and 8 of SEQ ID NO:1. In some embodiments, the substitutions in a CH2 domain comprise Ala at positions 7 and 8 and Gly at position 102 of SEQ ID NO:1.

Additional Fc polypeptide mutations that modulate an effector function include, but are not limited to, one or more substitutions at positions 238, 265, 269, 270, 297, 327 and 329 (EU numbering scheme, which correspond to positions 11, 38, 42, 43, 70, 100, and 102 as numbered with reference to SEQ ID NO:1). Illustrative substitutions (as numbered with EU numbering scheme), include the following: position 329 may have a mutation in which proline is substituted with a glycine or arginine or an amino acid residue large enough to destroy the Fc/Fcy receptor interface that is formed between proline 329 of the Fc and tryptophan residues Trp 87 and Trp 110 of FcyRIII. Additional illustrative substitutions include S228P, E233P, L235E, N297A, N297D, and P331S. Multiple substitutions may also be present, *e.g*., L234A and L235A of a human IgG1 Fc region; L234A, L235A, and P329G of a human IgG1 Fc region; S228P and L235E of a human IgG4 Fc region; L234A and G237A of a human IgG1 Fc region; L234A, L235A, and G237A of a human IgG1 Fc region; V234A and G237A of a human IgG2 Fc region; L235A, G237A, and E318A of a human IgG4 Fc region; and S228P and L236E of a human IgG4 Fc region. In some embodiments, a polypeptide of the invention may have one or more amino acid substitutions that modulate ADCC, *e.g.,* substitutions at positions 298, 333, and/or 334 of the Fc region, according to the EU numbering scheme.

In some embodiments, a polypeptide as described herein may have one or more amino acid substitutions that increase or decrease ADCC or may have mutations that alter C1q binding and/or CDC.

### Illustrative polypeptides comprising additional mutations

A polypeptide as described herein (e.g., any one of clones CH3C.35.20.1, CH3C.35.23.2, CH3C.35.23.3, CH3C.35.23.4, CH3C.35.21.17.2, CH3C.35.23, CH3C.35.21, CH3C.35.20.1.1, CH3C.23.2.1, and CH3C.35.23.1.1) may comprise additional mutations including a knob mutation (*e.g*., T139W as numbered with reference to SEQ ID NO:1), hole mutations (*e.g.*, T139S, L141A, and Y180V as numbered with reference to SEQ ID NO:1), mutations that modulate effector function (*e.g.,* L7A, L8A, and/or P102G (*e.g.,* L7A and L8A) as numbered with reference to SEQ ID NO:1), and/or mutations that increase serum stability (*e.g*., (i) M25Y, S27T, and T29E as numbered with reference to SEQ ID NO:1, or (ii) N207S with or without M201L as numbered with reference to SEQ ID NO:1).

In some embodiments, a polypeptide as described herein (*e.g*., any one of clones CH3C.35.20.1, CH3C.35.23.2, CH3C.35.23.3, CH3C.35.23.4, CH3C.35.21.17.2, CH3C.35.23, CH3C.35.21, CH3C.35.20.1.1, CH3C.23.2.1, and CH3C.35.23.1.1) may have a knob mutation (*e.g*., T139W as numbered with reference to SEQ ID NO:1) and at least 85% identity, at least 90% identity, or at least 95% identity to the sequence of any one of SEQ ID NOS:4-29, 39-102, and 182-195. In some embodiments, a polypeptide having the sequence of any one of SEQ ID NOS:4-29, 39-102, and 182-195 may be modified to have a knob mutation.

In some embodiments, a polypeptide as described herein (*e.g*., any one of clones CH3C.35.20.1, CH3C.35.23.2, CH3C.35.23.3, CH3C.35.23.4, CH3C.35.21.17.2, CH3C.35.23, CH3C.35.21, CH3C.35.20.1.1, CH3C.23.2.1, and CH3C.35.23.1.1) may have a knob mutation (*e.g*., T139W as numbered with reference to SEQ ID NO:1), mutations that modulate effector function (*e.g.,* L7A, L8A, and/or P102G (*e.g.,* L7A and L8A) as numbered with reference to SEQ ID NO:1), and at least 85% identity, at least 90% identity, or at least 95% identity to the sequence of any one of SEQ ID NOS:4-29, 39-102, and 182-195. In some embodiments, a polypeptide having the sequence of any one of SEQ ID NOS:4-29, 39-102, and 182-195 may be modified to have a knob mutation and mutations that modulate effector function.

In some embodiments, a polypeptide as described herein (*e.g*., any one of clones CH3C.35.20.1, CH3C.35.23.2, CH3C.35.23.3, CH3C.35.23.4, CH3C.35.21.17.2, CH3C.35.23, CH3C.35.21, CH3C.35.20.1.1, CH3C.23.2.1, and CH3C.35.23.1.1) may have a knob mutation (*e.g*., T139W as numbered with reference to SEQ ID NO:1), mutations that increase serum stability (*e.g*., (i) M25Y, S27T, and T29E as numbered with reference to SEQ ID NO:1, or (ii) N207S with or without M201L as numbered with reference to SEQ ID NO:1), and at least 85% identity, at least 90% identity, or at least 95% identity to the sequence of any one of SEQ ID NOS:4-29, 39-102, and 182-195. In some embodiments, a polypeptide having the sequence of any one of SEQ ID NOS:4-29, 39-102, and 182-195 may be modified to have a knob mutation and mutations that increase serum stability.

In some embodiments, a polypeptide as described herein (*e.g*., any one of clones CH3C.35.20.1, CH3C.35.23.2, CH3C.35.23.3, CH3C.35.23.4, CH3C.35.21.17.2, CH3C.35.23, CH3C.35.21, CH3C.35.20.1.1, CH3C.23.2.1, and CH3C.35.23.1.1) may have a knob mutation (*e.g*., T139W as numbered with reference to SEQ ID NO:1), mutations that modulate effector function (*e.g.,* L7A, L8A, and/or P102G (*e.g.,* L7A and L8A) as numbered with reference to SEQ ID NO:1), mutations that increase serum stability (*e.g*., (i) M25Y, S27T, and T29E as numbered with reference to SEQ ID NO:1, or (ii) N207S with or without M201L as numbered with reference to SEQ ID NO:1), and at least 85% identity, at least 90% identity, or at least 95% identity to the sequence of any one of SEQ ID NOS:4-29, 39-102, and 182-195. In some embodiments, a polypeptide having the sequence of any one of SEQ ID NOS:4-29, 39-102, and 182-195 may be modified to have a knob mutation, mutations that modulate effector function, and mutations that increase serum stability.

In some embodiments, a polypeptide as described herein (e.g., any one of clones CH3C.35.20.1, CH3C.35.23.2, CH3C.35.23.3, CH3C.35.23.4, CH3C.35.21.17.2, CH3C.35.23, CH3C.35.21, CH3C.35.20.1.1, CH3C.23.2.1, and CH3C.35.23.1.1) may have hole mutations (*e.g*., T139S, L141A, and Y180V as numbered with reference to SEQ ID NO:1) and at least 85% identity, at least 90% identity, or at least 95% identity to the sequence of any one of SEQ ID NOS:4-29, 39-102, and 182-195. In some embodiments, a polypeptide having the sequence of any one of SEQ ID NOS:4-29, 39-102, and 182-195 may be modified to have hole mutations.

In some embodiments, a polypeptide as described herein (*e.g*., any one of clones CH3C.35.20.1, CH3C.35.23.2, CH3C.35.23.3, CH3C.35.23.4, CH3C.35.21.17.2, CH3C.35.23, CH3C.35.21, CH3C.35.20.1.1, CH3C.23.2.1, and CH3C.35.23.1.1) may have hole mutations (*e.g.*, T139S, L141A, and Y180V as numbered with reference to SEQ ID NO:1), mutations that modulate effector function (*e.g.,* L7A, L8A, and/or P102G (*e.g.,* L7A and L8A) as numbered with reference to SEQ ID NO:1), and at least 85% identity, at least 90% identity, or at least 95% identity to the sequence of any one of SEQ ID NOS:4-29, 39-102, and 182-195. In some embodiments, a polypeptide having the sequence of any one of SEQ ID NOS:4-29, 39-102, and 182-195 may be modified to have hole mutations and mutations that modulate effector function.

In some embodiments, a polypeptide as described herein (e.g., any one of clones CH3C.35.20.1, CH3C.35.23.2, CH3C.35.23.3, CH3C.35.23.4, CH3C.35.21.17.2, CH3C.35.23, CH3C.35.21, CH3C.35.20.1.1, CH3C.23.2.1, and CH3C.35.23.1.1) may have hole mutations (*e.g.*, T139S, L141A, and Y180V as numbered with reference to SEQ ID NO:1), mutations that increase serum stability (*e.g*., (i) M25Y, S27T, and T29E as numbered with reference to SEQ ID NO:1, or (ii) N207S with or without M201L as numbered with reference to SEQ ID NO:1), and at least 85% identity, at least 90% identity, or at least 95% identity to the sequence of any one of SEQ ID NOS:4-29, 39-102, and 182-195. In some embodiments, a polypeptide having the sequence of any one of SEQ ID NOS:4-29, 39-102, and 182-195 may be modified to have hole mutations and mutations that increase serum stability.

In some embodiments, a polypeptide as described herein (e.g., any one of clones CH3C.35.20.1, CH3C.35.23.2, CH3C.35.23.3, CH3C.35.23.4, CH3C.35.21.17.2, CH3C.35.23, CH3C.35.21, CH3C.35.20.1.1, CH3C.23.2.1, and CH3C.35.23.1.1) may have hole mutations (*e.g.*, T139S, L141A, and Y180V as numbered with reference to SEQ ID NO:1), mutations that modulate effector function (*e.g.,* L7A, L8A, and/or P102G *(e.g.,* L7A and L8A) as numbered with reference to SEQ ID NO:1), mutations that increase serum stability (*e.g*., (i) M25Y, S27T, and T29E as numbered with reference to SEQ ID NO:1, or (ii) N207S with or without M201L as numbered with reference to SEQ ID NO:1), and at least 85% identity, at least 90% identity, or at least 95% identity to the sequence of any one of SEQ ID NOS:4-29, 39-102, and 182-195. In some embodiments, a polypeptide having the sequence of any one of SEQ ID NOS:4-29, 39-102, and 182-195 may be modified to have hole mutations, mutations that modulate effector function, and mutations that increase serum stability.

### Clone CH3C.35.20.1

In some embodiments, clone CH3C.35.20.1 may have a knob mutation (*e.g*., T139W as numbered with reference to SEQ ID NO:1) and at least 85% identity, at least 90% identity, or at least 95% identity to the sequence of SEQ ID NO:109. In some embodiments, clone CH3C.35.20.1 with the knob mutation has the sequence of SEQ ID NO:109.

In some embodiments, clone CH3C.35.20.1 may have a knob mutation (*e.g*., T139W as numbered with reference to SEQ ID NO:1), mutations that modulate effector function (*e.g.,* L7A, L8A, and/or P102G (*e.g.,* L7A and L8A) as numbered with reference to SEQ ID NO:1), and at least 85% identity, at least 90% identity, or at least 95% identity to the sequence of SEQ ID NO:110 or 111. In some embodiments, clone CH3C.35.20.1 with the knob mutation and the mutations that modulate effector function has the sequence of SEQ ID NO:110 or 111.

In some embodiments, clone CH3C.35.20.1 may have a knob mutation (*e.g.*, T139W as numbered with reference to SEQ ID NO:1), mutations that increase serum stability *(e.g.,* M25Y, S27T, and T29E as numbered with reference to SEQ ID NO:1), and at least 85% identity, at least 90% identity, or at least 95% identity to the sequence of SEQ ID NO:112. In some embodiments, clone CH3C.35.20.1 with the knob mutation and the mutations that increase serum stability has the sequence of SEQ ID NO:112.

In some embodiments, clone CH3C.35.20.1 may have a knob mutation (e.g., T139W as numbered with reference to SEQ ID NO:1), mutations that increase serum stability *(e.g.,* N207S with or without M201L as numbered with reference to SEQ ID NO:1), and at least 85% identity, at least 90% identity, or at least 95% identity to the sequence of SEQ ID NO:245. In some embodiments, clone CH3C.35.20.1 with the knob mutation and the mutations that increase serum stability has the sequence of SEQ ID NO:245.

In some embodiments, clone CH3C.35.20.1 may have a knob mutation (*e.g*., T139W as numbered with reference to SEQ ID NO:1), mutations that modulate effector function (*e.g.,* L7A, L8A, and/or P102G (*e.g.,* L7A and L8A) as numbered with reference to SEQ ID NO:1), mutations that increase serum stability (*e.g*., M25Y, S27T, and T29E as numbered with reference to SEQ ID NO:1), and at least 85% identity, at least 90% identity, or at least 95% identity to the sequence of SEQ ID NO:113 or 114. In some embodiments, clone CH3C.35.20.1 with the knob mutation, the mutations that modulate effector function, and the mutations that increase serum stability has the sequence of SEQ ID NO:113 or 114.

In some embodiments, clone CH3C.35.20.1 may have a knob mutation (*e.g*., T139W as numbered with reference to SEQ ID NO:1), mutations that modulate effector function (*e.g.,* L7A, L8A, and/or P102G (*e.g.,* L7A and L8A) as numbered with reference to SEQ ID NO:1), mutations that increase serum stability (*e.g.*, N207S with or without M201L as numbered with reference to SEQ ID NO:1), and at least 85% identity, at least 90% identity, or at least 95% identity to the sequence of SEQ ID NO:246 or 247. In some embodiments, clone CH3C.35.20.1 with the knob mutation, the mutations that modulate effector function, and the mutations that increase serum stability has the sequence of SEQ ID NO:246 or 247.

In some embodiments, clone CH3C.35.20.1 may have hole mutations (*e.g*., T139S, L141A, and Y180V as numbered with reference to SEQ ID NO:1) and at least 85% identity, at least 90% identity, or at least 95% identity to the sequence of SEQ ID NO:115. In some embodiments, clone CH3C.35.20.1 with the hole mutations has the sequence of SEQ ID NO:115.

In some embodiments, clone CH3C.35.20.1 may have hole mutations (*e.g.*, T139S, L141A, and Y180V as numbered with reference to SEQ ID NO:1), mutations that modulate effector function (*e.g.,* L7A, L8A, and/or P102G (*e.g.,* L7A and L8A) as numbered with reference to SEQ ID NO:1), and at least 85% identity, at least 90% identity, or at least 95% identity to the sequence of SEQ ID NO:116 or 117. In some embodiments, clone CH3C.35.20.1 with the hole mutations and the mutations that modulate effector function has the sequence of SEQ ID NO:116 or 117.

In some embodiments, clone CH3C.35.20.1 may have hole mutations (*e.g*., T139S, L141A, and Y180V as numbered with reference to SEQ ID NO:1), mutations that increase serum stability (*e.g*., M25Y, S27T, and T29E as numbered with reference to SEQ ID NO:1), and at least 85% identity, at least 90% identity, or at least 95% identity to the sequence of SEQ ID NO:118. In some embodiments, clone CH3C.35.20.1 with the hole mutations and the mutations that increase serum stability has the sequence of SEQ ID NO: 118.

In some embodiments, clone CH3C.35.20.1 may have hole mutations (*e.g*., T139S, L141A, and Y180V as numbered with reference to SEQ ID NO:1), mutations that increase serum stability (*e.g*., N207S with or without M201L as numbered with reference to SEQ ID NO:1), and at least 85% identity, at least 90% identity, or at least 95% identity to the sequence of SEQ ID NO:248. In some embodiments, clone CH3C.35.20.1 with the hole mutations and the mutations that increase serum stability has the sequence of SEQ ID NO:248.

In some embodiments, clone CH3C.35.20.1 may have hole mutations (*e.g.*, T139S, L141A, and Y180V as numbered with reference to SEQ ID NO:1), mutations that modulate effector function (*e.g.*, L7A, L8A, and/or P102G (*e.g.,* L7A and L8A) as numbered with reference to SEQ ID NO:1), mutations that increase serum stability (*e.g*., M25Y, S27T, and T29E as numbered with reference to SEQ ID NO:1), and at least 85% identity, at least 90% identity, or at least 95% identity to the sequence of SEQ ID NO:119 or 120. In some embodiments, clone CH3C.35.20.1 with the hole mutations, the mutations that modulate effector function, and the mutations that increase serum stability has the sequence of SEQ ID NO:119 or 120.

In some embodiments, clone CH3C.35.20.1 may have hole mutations (*e.g*., T139S, L141A, and Y180V as numbered with reference to SEQ ID NO:1), mutations that modulate effector function (*e.g*., L7A, L8A, and/or P102G (*e.g.,* L7A and L8A) as numbered with reference to SEQ ID NO:1), mutations that increase serum stability (*e.g*., N207S with or without M201L as numbered with reference to SEQ ID NO:1), and at least 85% identity, at least 90% identity, or at least 95% identity to the sequence of SEQ ID NO:249 or 250. In some embodiments, clone CH3C.35.20.1 with the hole mutations, the mutations that modulate effector function, and the mutations that increase serum stability has the sequence of SEQ ID NO:249 or 250.

### Clone CH3C.35.23.2

In some embodiments, clone CH3C.35.23.2 may have a knob mutation (*e.g*., T139W as numbered with reference to SEQ ID NO:1) and at least 85% identity, at least 90% identity, or at least 95% identity to the sequence of SEQ ID NO:121. In some embodiments, clone CH3C.35.23.2 with the knob mutation has the sequence of SEQ ID NO:121.

In some embodiments, clone CH3C.35.23.2 may have a knob mutation (*e.g*., T139W as numbered with reference to SEQ ID NO:1), mutations that modulate effector function (*e.g.,* L7A, L8A, and/or P102G (*e.g.,* L7A and L8A) as numbered with reference to SEQ ID NO:1), and at least 85% identity, at least 90% identity, or at least 95% identity to the sequence of SEQ ID NO:122 or 123. In some embodiments, clone CH3C.35.23.2 with the knob mutation and the mutations that modulate effector function has the sequence of SEQ ID NO:122 or 123.

In some embodiments, clone CH3C.35.23.2 may have a knob mutation (*e.g*., T139W as numbered with reference to SEQ ID NO:1), mutations that increase serum stability *(e.g.,* M25Y, S27T, and T29E as numbered with reference to SEQ ID NO:1), and at least 85% identity, at least 90% identity, or at least 95% identity to the sequence of SEQ ID NO:124. In some embodiments, clone CH3C.35.23.2 with the knob mutation and the mutations that increase serum stability has the sequence of SEQ ID NO:124.

In some embodiments, clone CH3C.35.23.2 may have a knob mutation (*e.g*., T139W as numbered with reference to SEQ ID NO:1), mutations that increase serum stability *(e.g.,* N207S with or without M201L as numbered with reference to SEQ ID NO:1), and at least 85% identity, at least 90% identity, or at least 95% identity to the sequence of SEQ ID NO:252. In some embodiments, clone CH3C.35.23.2 with the knob mutation and the mutations that increase serum stability has the sequence of SEQ ID NO:252.

In some embodiments, clone CH3C.35.23.2 may have a knob mutation (*e.g.*, T139W as numbered with reference to SEQ ID NO:1), mutations that modulate effector function (*e.g.,* L7A, L8A, and/or P102G (*e.g.,* L7A and L8A) as numbered with reference to SEQ ID NO:1), mutations that increase serum stability (*e.g*., M25Y, S27T, and T29E as numbered with reference to SEQ ID NO:1), and at least 85% identity, at least 90% identity, or at least 95% identity to the sequence of SEQ ID NO:125 or 126. In some embodiments, clone CH3C.35.23.2 with the knob mutation, the mutations that modulate effector function, and the mutations that increase serum stability has the sequence of SEQ ID NO:125 or 126.

In some embodiments, clone CH3C.35.23.2 may have a knob mutation (*e.g.*, T139W as numbered with reference to SEQ ID NO:1), mutations that modulate effector function (*e.g.,* L7A, L8A, and/or P102G (*e.g.,* L7A and L8A) as numbered with reference to SEQ ID NO:1), mutations that increase serum stability (*e.g*., N207S with or without M201L as numbered with reference to SEQ ID NO:1), and at least 85% identity, at least 90% identity, or at least 95% identity to the sequence of SEQ ID NO:253 or 254. In some embodiments, clone CH3C.35.23.2 with the knob mutation, the mutations that modulate effector function, and the mutations that increase serum stability has the sequence of SEQ ID NO:253 or 254.

In some embodiments, clone CH3C.35.23.2 may have hole mutations (*e.g*., T139S, L141A, and Y180V as numbered with reference to SEQ ID NO: 1) and at least 85% identity, at least 90% identity, or at least 95% identity to the sequence of SEQ ID NO: 127. In some embodiments, clone CH3C.35.23.2 with the hole mutations has the sequence of SEQ ID NO: 127.

In some embodiments, clone CH3C.35.23.2 may have hole mutations (*e.g*., T139S, L141A, and Y180V as numbered with reference to SEQ ID NO:1), mutations that modulate effector function (*e.g*., L7A, L8A, and/or P102G (*e.g.,* L7A and L8A) as numbered with reference to SEQ ID NO:1), and at least 85% identity, at least 90% identity, or at least 95% identity to the sequence of SEQ ID NO: 128 or 129. In some embodiments, clone CH3C.35.23.2 with the hole mutations and the mutations that modulate effector function has the sequence of SEQ ID NO: 128 or 129.

In some embodiments, clone CH3C.35.23.2 may have hole mutations (*e.g*., T139S, L141A, and Y180V as numbered with reference to SEQ ID NO:1), mutations that increase serum stability (*e.g*., M25Y, S27T, and T29E as numbered with reference to SEQ ID NO:1), and at least 85% identity, at least 90% identity, or at least 95% identity to the sequence of SEQ ID NO: 130. In some embodiments, clone CH3C.35.23.2 with the hole mutations and the mutations that increase serum stability has the sequence of SEQ ID NO: 130.

In some embodiments, clone CH3C.35.23.2 may have hole mutations (*e.g*., T139S, L141A, and Y180V as numbered with reference to SEQ ID NO:1), mutations that increase serum stability (*e.g*., N207S with or without M201L as numbered with reference to SEQ ID NO:1), and at least 85% identity, at least 90% identity, or at least 95% identity to the sequence of SEQ ID NO:255. In some embodiments, clone CH3C.35.23.2 with the hole mutations and the mutations that increase serum stability has the sequence of SEQ ID NO:255.

In some embodiments, clone CH3C.35.23.2 may have hole mutations (*e.g.*, T139S, L141A, and Y180V as numbered with reference to SEQ ID NO:1), mutations that modulate effector function (*e.g*., L7A, L8A, and/or P102G (*e.g.,* L7A and L8A) as numbered with reference to SEQ ID NO:1), mutations that increase serum stability (*e.g*., M25Y, S27T, and T29E as numbered with reference to SEQ ID NO:1), and at least 85% identity, at least 90% identity, or at least 95% identity to the sequence of SEQ ID NO:131 or 132. In some embodiments, clone CH3C.35.23.2 with the hole mutations, the mutations that modulate effector function, and the mutations that increase serum stability has the sequence of SEQ ID NO:131 or 132.

In some embodiments, clone CH3C.35.23.2 may have hole mutations (*e.g.*, T139S, L141A, and Y180V as numbered with reference to SEQ ID NO:1), mutations that modulate effector function (*e.g.*, L7A, L8A, and/or P102G (*e.g.,* L7A and L8A) as numbered with reference to SEQ ID NO:1), mutations that increase serum stability (*e.g.*, N207S with or without M201L as numbered with reference to SEQ ID NO:1), and at least 85% identity, at least 90% identity, or at least 95% identity to the sequence of SEQ ID NO:256 or 257. In some embodiments, clone CH3C.35.23.2 with the hole mutations, the mutations that modulate effector function, and the mutations that increase serum stability has the sequence of SEQ ID NO:256 or 257.

### Clone CH3C.35.23.3

In some embodiments, clone CH3C.35.23.3 may have a knob mutation (*e.g*., T139W as numbered with reference to SEQ ID NO:1) and at least 85% identity, at least 90% identity, or at least 95% identity to the sequence of SEQ ID NO: 133. In some embodiments, clone CH3C.35.23.3 with the knob mutation has the sequence of SEQ ID NO:133.

In some embodiments, clone CH3C.35.23.3 may have a knob mutation (*e.g*., T139W as numbered with reference to SEQ ID NO:1), mutations that modulate effector function (*e.g*., L7A, L8A, and/or P102G (*e.g.,* L7A and L8A) as numbered with reference to SEQ ID NO: 1), and at least 85% identity, at least 90% identity, or at least 95% identity to the sequence of SEQ ID NO: 134 or 135. In some embodiments, clone CH3C.35.23.3 with the knob mutation and the mutations that modulate effector function has the sequence of SEQ ID NO:134 or 135.

In some embodiments, clone CH3C.35.23.3 may have a knob mutation (*e.g*., T139W as numbered with reference to SEQ ID NO:1), mutations that increase serum stability *(e.g.,* M25Y, S27T, and T29E as numbered with reference to SEQ ID NO:1), and at least 85% identity, at least 90% identity, or at least 95% identity to the sequence of SEQ ID NO:136. In some embodiments, clone CH3C.35.23.3 with the knob mutation and the mutations that increase serum stability has the sequence of SEQ ID NO:136.

In some embodiments, clone CH3C.35.23.3 may have a knob mutation (*e.g*., T139W as numbered with reference to SEQ ID NO:1), mutations that increase serum stability

(*e.g.*, N207S with or without M201L as numbered with reference to SEQ ID NO:1), and at least 85% identity, at least 90% identity, or at least 95% identity to the sequence of SEQ ID NO:259. In some embodiments, clone CH3C.35.23.3 with the knob mutation and the mutations that increase serum stability has the sequence of SEQ ID NO:259.

In some embodiments, clone CH3C.35.23.3 may have a knob mutation (e.g., T139W as numbered with reference to SEQ ID NO:1), mutations that modulate effector function (*e.g.,* L7A, L8A, and/or P102G (*e.g.,* L7A and L8A) as numbered with reference to SEQ ID NO:1), mutations that increase serum stability (*e*.*g*., M25Y, S27T, and T29E as numbered with reference to SEQ ID NO:1), and at least 85% identity, at least 90% identity, or at least 95% identity to the sequence of SEQ ID NO:137 or 138. In some embodiments, clone CH3C.35.23.3 with the knob mutation, the mutations that modulate effector function, and the mutations that increase serum stability has the sequence of SEQ ID NO: 137 or 138.

In some embodiments, clone CH3C.35.23.3 may have a knob mutation (e.g., T139W as numbered with reference to SEQ ID NO:1), mutations that modulate effector function (*e.g.,* L7A, L8A, and/or P102G (*e.g.,* L7A and L8A) as numbered with reference to SEQ ID NO:1), mutations that increase serum stability (*e*.*g*., N207S with or without M201L as numbered with reference to SEQ ID NO:1), and at least 85% identity, at least 90% identity, or at least 95% identity to the sequence of SEQ ID NO:260 or 261. In some embodiments, clone CH3C.35.23.3 with the knob mutation, the mutations that modulate effector function, and the mutations that increase serum stability has the sequence of SEQ ID NO:260 or 261.

In some embodiments, clone CH3C.35.23.3 may have hole mutations (e.g., T139S, L141A, and Y180V as numbered with reference to SEQ ID NO: 1) and at least 85% identity, at least 90% identity, or at least 95% identity to the sequence of SEQ ID NO:139. In some embodiments, clone CH3C.35.23.3 with the hole mutations and the sequence of SEQ ID NO:139.

In some embodiments, clone CH3C.35.23.3 may have hole mutations (e.g., T139S, L141A, and Y180V as numbered with reference to SEQ ID NO:1), mutations that modulate effector function (*e.g.,* L7A, L8A, and/or P102G (*e.g.,* L7A and L8A) as numbered with reference to SEQ ID NO:1), and at least 85% identity, at least 90% identity, or at least 95% identity to the sequence of SEQ ID NO:140 or 141. In some embodiments, clone CH3C.35.23.3 with the hole mutations and the mutations that modulate effector function has the sequence of SEQ ID NO:140 or 141.

In some embodiments, clone CH3C.35.23.3 may have hole mutations (e.g., T139S, L141A, and Y180V as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:142. In some embodiments, clone CH3C.35.23.3 with the hole mutations and the mutations that increase serum stability has the sequence of SEQ ID NO: 142.

In some embodiments, clone CH3C.35.23.3 may have hole mutations (e.g., T139S, L141A, and Y180V as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:262. In some embodiments, clone CH3C.35.23.3 with the hole mutations and the mutations that increase serum stability has the sequence of SEQ ID NO:262.

In some embodiments, clone CH3C.35.23.3 may have hole mutations (*e*.*g*., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:143 or 144. In some embodiments, clone CH3C.35.23.3 with the hole mutations, the mutations that modulate effector function, and the mutations that increase serum stability has the sequence of SEQ ID NO:143 or 144.

In some embodiments, clone CH3C.35.23.3 may have hole mutations (*e*.*g*., T139S, L141A, and Y180V as numbered with reference to SEQ ID NO:1), mutations that modulate effector function (*e.g.,* L7A, L8A, and/or P102G (*e.g.,* L7A and L8A) as numbered with reference to SEQ ID NO:1), mutations that increase serum stability (*e*.*g*., N207S with or without M201L as numbered with reference to SEQ ID NO:1), and at least 85% identity, at least 90% identity, or at least 95% identity to the sequence of SEQ ID NO:263 or 264. In some embodiments, clone CH3C.35.23.3 with the hole mutations, the mutations that modulate effector function, and the mutations that increase serum stability has the sequence of SEQ ID NO:263 or 264.

### Clone CH3C.35.23.4

In some embodiments, clone CH3C.35.23.4 may have a knob mutation (*e*.*g*., T139W as numbered with reference to SEQ ID NO:1) and at least 85% identity, at least 90% identity, or at least 95% identity to the sequence of SEQ ID NO: 145. In some embodiments, clone CH3C.35.23.4 with the knob mutation has the sequence of SEQ ID NO:145.

In some embodiments, clone CH3C.35.23.4 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), and at least 85% identity, at least 90% identity, or at least 95% identity to the sequence of SEQ ID NO:146 or 147. In some embodiments, clone CH3C.35.23.4 with the knob mutation and the mutations that modulate effector function has the sequence of SEQ ID NO:146 or 147.

In some embodiments, clone CH3C.35.23.4 may have a knob mutation (e.g., T139W as numbered with reference to SEQ ID NO:1), mutations that increase serum stability *(e.g.,* M25Y, S27T, and T29E as numbered with reference to SEQ ID NO:1), and at least 85% identity, at least 90% identity, or at least 95% identity to the sequence of SEQ ID NO:148. In some embodiments, clone CH3C.35.23.4 with the knob mutation and the mutations that increase serum stability has the sequence of SEQ ID NO:148.

In some embodiments, clone CH3C.35.23.4 may have a knob mutation (*e*.*g*., T139W as numbered with reference to SEQ ID NO:1), mutations that increase serum stability *(e.g.,* N207S with or without M201L as numbered with reference to SEQ ID NO:1), and at least 85% identity, at least 90% identity, or at least 95% identity to the sequence of SEQ ID NO:266. In some embodiments, clone CH3C.35.23.4 with the knob mutation and the mutations that increase serum stability has the sequence of SEQ ID NO:266.

In some embodiments, clone CH3C.35.23.4 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:149 or 150. In some embodiments, clone CH3C.35.23.4 with the knob mutation, the mutations that modulate effector function, and the mutations that increase serum stability has the sequence of SEQ ID NO: 149 or 150.

In some embodiments, clone CH3C.35.23.4 may have a knob mutation (*e.g.*, T139W as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:267 or 268. In some embodiments, clone CH3C.35.23.4 with the knob mutation, the mutations that modulate effector function, and the mutations that increase serum stability has the sequence of SEQ ID NO:267 or 268.

In some embodiments, clone CH3C.35.23.4 may have hole mutations (e.g., T139S, L141A, and Y180V as numbered with reference to SEQ ID NO: 1) and at least 85% identity, at least 90% identity, or at least 95% identity to the sequence of SEQ ID NO:151. In some embodiments, clone CH3C.35.23.4 with the hole mutations has the sequence of SEQ ID NO:151.

In some embodiments, clone CH3C.35.23.4 may have hole mutations (e.g., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), and at least 85% identity, at least 90% identity, or at least 95% identity to the sequence of SEQ ID NO:152 or 153. In some embodiments, clone CH3C.35.23.4 with the hole mutations and the mutations that modulate effector function has the sequence of SEQ ID NO:152 or 153.

In some embodiments, clone CH3C.35.23.4 may have hole mutations (e.g., T139S, L141A, and Y180V as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:154. In some embodiments, clone CH3C.35.23.4 with the hole mutations and the mutations that increase serum stability has the sequence of SEQ ID NO: 154.

In some embodiments, clone CH3C.35.23.4 may have hole mutations (e.g., T139S, L141A, and Y180V as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:269. In some embodiments, clone CH3C.35.23.4 with the hole mutations and the mutations that increase serum stability has the sequence of SEQ ID NO:269.

In some embodiments, clone CH3C.35.23.4 may have hole mutations (e.g., T139S, L141A, and Y180V as numbered with reference to SEQ ID NO:1), mutations that modulate effector function (*e*.*g*., L7A, L8A, and/or P102G (*e.g.*, L7A and L8A) as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:155 or 156. In some embodiments, clone CH3C.35.23.4 with the hole mutations, the mutations that modulate effector function, and the mutations that increase serum stability has the sequence of SEQ ID NO:155 or 156.

In some embodiments, clone CH3C.35.23.4 may have hole mutations (e.g., T139S, L141A, and Y180V as numbered with reference to SEQ ID NO:1), mutations that modulate effector function (*e*.*g*., L7A, L8A, and/or P102G (*e.g.*, L7A and L8A) as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:270 or 271. In some embodiments, clone CH3C.35.23.4 with the hole mutations, the mutations that modulate effector function, and the mutations that increase serum stability has the sequence of SEQ ID NO:270 or 271.

### Clone CH3C.35.21.17.2

In some embodiments, clone CH3C.35.21.17.2 may have a knob mutation (e.g., T139W as numbered with reference to SEQ ID NO:1) and at least 85% identity, at least 90% identity, or at least 95% identity to the sequence of SEQ ID NO: 157. In some embodiments, clone CH3C.35.21.17.2 with the knob mutation has the sequence of SEQ ID NO:157.

In some embodiments, clone CH3C.35.21.17.2 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), and at least 85% identity, at least 90% identity, or at least 95% identity to the sequence of SEQ ID NO:158 or 159. In some embodiments, clone CH3C.35.21.17.2 with the knob mutation and the mutations that modulate effector function has the sequence of SEQ ID NO:158 or 159.

In some embodiments, clone CH3C.35.21.17.2 may have a knob mutation (e.g., T139W as numbered with reference to SEQ ID NO:1), mutations that increase serum stability *(e.g.,* M25Y, S27T, and T29E as numbered with reference to SEQ ID NO:1), and at least 85% identity, at least 90% identity, or at least 95% identity to the sequence of SEQ ID NO:160. In some embodiments, clone CH3C.35.21.17.2 with the knob mutation and the mutations that increase serum stability has the sequence of SEQ ID NO:160.

In some embodiments, clone CH3C.35.21.17.2 may have a knob mutation (e.g., T139W as numbered with reference to SEQ ID NO:1), mutations that increase serum stability *(e.g.,* N207S with or without M201L as numbered with reference to SEQ ID NO:1), and at least 85% identity, at least 90% identity, or at least 95% identity to the sequence of SEQ ID NO:273. In some embodiments, clone CH3C.35.21.17.2 with the knob mutation and the mutations that increase serum stability has the sequence of SEQ ID NO:273.

In some embodiments, clone CH3C.35.21.17.2 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (*e*.*g*., M25Y, S27T, and T29E as numbered with reference to SEQ ID NO:1), and at least 85% identity, at least 90% identity, or at least 95% identity to the sequence of SEQ ID NO:161 or 162. In some embodiments, clone CH3C.35.21.17.2 with the knob mutation, the mutations that modulate effector function, and the mutations that increase serum stability has the sequence of SEQ ID NO:161 or 162.

In some embodiments, clone CH3C.35.21.17.2 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:274 or 275. In some embodiments, clone CH3C.35.21.17.2 with the knob mutation, the mutations that modulate effector function, and the mutations that increase serum stability has the sequence of SEQ ID NO:274 or 275.

In some embodiments, clone CH3C.35.21.17.2 may have hole mutations (e.g., T139S, L141A, and Y180V as numbered with reference to SEQ ID NO:1) and at least 85% identity, at least 90% identity, or at least 95% identity to the sequence of SEQ ID NO:163. In some embodiments, clone CH3C.35.21.17.2 with the hole mutations has the sequence of SEQ ID NO:163.

In some embodiments, clone CH3C.35.21.17.2 may have hole mutations (e.g., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), and at least 85% identity, at least 90% identity, or at least 95% identity to the sequence of SEQ ID NO:164 or 165. In some embodiments, clone CH3C.35.21.17.2 with the hole mutations and the mutations that modulate effector function has the sequence of SEQ ID NO:164 or 165.

In some embodiments, clone CH3C.35.21.17.2 may have hole mutations (e.g., T139S, L141A, and Y180V as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:166. In some embodiments, clone CH3C.35.21.17.2 with the hole mutations and the mutations that increase serum stability has the sequence of SEQ ID NO: 166.

In some embodiments, clone CH3C.35.21.17.2 may have hole mutations (e.g., T139S, L141A, and Y180V as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:276. In some embodiments, clone CH3C.35.21.17.2 with the hole mutations and the mutations that increase serum stability has the sequence of SEQ ID NO:276.

In some embodiments, clone CH3C.35.21.17.2 may have hole mutations (e.g., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:167 or 168. In some embodiments, clone CH3C.35.21.17.2 with the hole mutations, the mutations that modulate effector function, and the mutations that increase serum stability has the sequence of SEQ ID NO:167 or 168.

In some embodiments, clone CH3C.35.21.17.2 may have hole mutations (e.g., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:277 or 278. In some embodiments, clone CH3C.35.21.17.2 with the hole mutations, the mutations that modulate effector function, and the mutations that increase serum stability has the sequence of SEQ ID NO:277 or 278.

### Clone CH3C.35.23

In some embodiments, clone CH3C.35.23 may have a knob mutation (e.g., T139W as numbered with reference to SEQ ID NO:1) and at least 85% identity, at least 90% identity, or at least 95% identity to the sequence of SEQ ID NO:169. In some embodiments, clone CH3C.35.23 with the knob mutation has the sequence of SEQ ID NO:169.

In some embodiments, clone CH3C.35.23 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), and at least 85% identity, at least 90% identity, or at least 95% identity to the sequence of SEQ ID NO:170 or 171. In some embodiments, clone CH3C.35.23 with the knob mutation and the mutations that modulate effector function has the sequence of SEQ ID NO:170 or 171.

In some embodiments, clone CH3C.35.23 may have a knob mutation (e.g., T139W as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:172. In some embodiments, clone CH3C.35.23 with the knob mutation and the mutations that increase serum stability has the sequence of SEQ ID NO:172.

In some embodiments, clone CH3C.35.23 may have a knob mutation (e.g., T139W as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:280. In some embodiments, clone CH3C.35.23 with the knob mutation and the mutations that increase serum stability has the sequence of SEQ ID NO:280.

In some embodiments, clone CH3C.35.23 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:173 or 174. In some embodiments, clone CH3C.35.23 with the knob mutation, the mutations that modulate effector function, and the mutations that increase serum stability has the sequence of SEQ ID NO:173 or 174.

In some embodiments, clone CH3C.35.23 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:281 or 282. In some embodiments, clone CH3C.35.23 with the knob mutation, the mutations that modulate effector function, and the mutations that increase serum stability has the sequence of SEQ ID NO:281 or 282.

In some embodiments, clone CH3C.35.23 may have hole mutations (e.g., T139S, L141A, and Y180V as numbered with reference to SEQ ID NO: 1) and at least 85% identity, at least 90% identity, or at least 95% identity to the sequence of SEQ ID NO:175. In some embodiments, clone CH3C.35.23 with the hole mutations has the sequence of SEQ ID NO:175.

In some embodiments, clone CH3C.35.23 may have hole mutations (e.g., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), and at least 85% identity, at least 90% identity, or at least 95% identity to the sequence of SEQ ID NO:176 or 177. In some embodiments, clone CH3C.35.23 with the hole mutations and the mutations that modulate effector function has the sequence of SEQ ID NO:176 or 177.

In some embodiments, clone CH3C.35.23 may have hole mutations (e.g., T139S, L141A, and Y180V as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:178. In some embodiments, clone CH3C.35.23 with the hole mutations and the mutations that increase serum stability has the sequence of SEQ ID NO:178.

In some embodiments, clone CH3C.35.23 may have hole mutations (e.g., T139S, L141A, and Y180V as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:283. In some embodiments, clone CH3C.35.23 with the hole mutations and the mutations that increase serum stability has the sequence of SEQ ID NO:283.

In some embodiments, clone CH3C.35.23 may have hole mutations (e.g., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:179 or 180. In some embodiments, clone CH3C.35.23 with the hole mutations, the mutations that modulate effector function, and the mutations that increase serum stability has the sequence of SEQ ID NO:179 or 180.

In some embodiments, clone CH3C.35.23 may have hole mutations (e.g., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:284 or 285. In some embodiments, clone CH3C.35.23 with the hole mutations, the mutations that modulate effector function, and the mutations that increase serum stability has the sequence of SEQ ID NO:284 or 285.

### Clone CH3C.35.21

In some embodiments, clone CH3C.35.21 may have a knob mutation (e.g., T139W as numbered with reference to SEQ ID NO:1) and at least 85% identity, at least 90% identity, or at least 95% identity to the sequence of SEQ ID NO:196. In some embodiments, clone CH3C.35.21 with the knob mutation has the sequence of SEQ ID NO:196.

In some embodiments, clone CH3C.35.21 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), and at least 85% identity, at least 90% identity, or at least 95% identity to the sequence of SEQ ID NO:197 or 198. In some embodiments, clone CH3C.35.21 with the knob mutation and the mutations that modulate effector function has the sequence of SEQ ID NO:197 or 198.

In some embodiments, clone CH3C.35.21 may have a knob mutation (e.g., T139W as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:199. In some embodiments, clone CH3C.35.21 with the knob mutation and the mutations that increase serum stability has the sequence of SEQ ID NO:199.

In some embodiments, clone CH3C.35.21 may have a knob mutation (e.g., T139W as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:287. In some embodiments, clone CH3C.35.21 with the knob mutation and the mutations that increase serum stability has the sequence of SEQ ID NO:287.

In some embodiments, clone CH3C.35.21 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:200 or 201. In some embodiments, clone CH3C.35.21 with the knob mutation, the mutations that modulate effector function, and the mutations that increase serum stability has the sequence of SEQ ID NO:200 or 201.

In some embodiments, clone CH3C.35.21 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:288 or 289. In some embodiments, clone CH3C.35.21 with the knob mutation, the mutations that modulate effector function, and the mutations that increase serum stability has the sequence of SEQ ID NO:288 or 289.

In some embodiments, clone CH3C.35.21 may have hole mutations (e.g., T139S, L141A, and Y180V as numbered with reference to SEQ ID NO: 1) and at least 85% identity, at least 90% identity, or at least 95% identity to the sequence of SEQ ID NO:202. In some embodiments, clone CH3C.35.21 with the hole mutations has the sequence of SEQ ID NO:202.

In some embodiments, clone CH3C.35.21 may have hole mutations (e.g., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), and at least 85% identity, at least 90% identity, or at least 95% identity to the sequence of SEQ ID NO:203 or 204. In some embodiments, clone CH3C.35.21 with the hole mutations and the mutations that modulate effector function has the sequence of SEQ ID NO:203 or 204.

In some embodiments, clone CH3C.35.21 may have hole mutations (e.g., T139S, L141A, and Y180V as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:205. In some embodiments, clone CH3C.35.21 with the hole mutations and the mutations that increase serum stability has the sequence of SEQ ID NO:205.

In some embodiments, clone CH3C.35.21 may have hole mutations (e.g., T139S, L141A, and Y180V as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:290. In some embodiments, clone CH3C.35.21 with the hole mutations and the mutations that increase serum stability has the sequence of SEQ ID NO:290.

In some embodiments, clone CH3C.35.21 may have hole mutations (e.g., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:206 or 207. In some embodiments, clone CH3C.35.21 with the hole mutations, the mutations that modulate effector function, and the mutations that increase serum stability has the sequence of SEQ ID NO:206 or 207.

In some embodiments, clone CH3C.35.21 may have hole mutations (e.g., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:291 or 292. In some embodiments, clone CH3C.35.21 with the hole mutations, the mutations that modulate effector function, and the mutations that increase serum stability has the sequence of SEQ ID NO:291 or 292.

### Clone CH3C.35.20.1.1

In some embodiments, clone CH3C.35.20.1.1 may have a knob mutation (e.g., T139W as numbered with reference to SEQ ID NO:1) and at least 85% identity, at least 90% identity, or at least 95% identity to the sequence of SEQ ID NO:208. In some embodiments, clone CH3C.35.20.1.1 with the knob mutation has the sequence of SEQ ID NO:208.

In some embodiments, clone CH3C.35.20.1.1 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), and at least 85% identity, at least 90% identity, or at least 95% identity to the sequence of SEQ ID NO:209 or 210. In some embodiments, clone CH3C.35.20.1.1 with the knob mutation and the mutations that modulate effector function has the sequence of SEQ ID NO:209 or 210.

In some embodiments, clone CH3C.35.20.1.1 may have a knob mutation (e.g., T139W as numbered with reference to SEQ ID NO:1), mutations that increase serum stability *(e.g.,* M25Y, S27T, and T29E as numbered with reference to SEQ ID NO:1), and at least 85% identity, at least 90% identity, or at least 95% identity to the sequence of SEQ ID NO:211. In some embodiments, clone CH3C.35.20.1.1 with the knob mutation and the mutations that increase serum stability has the sequence of SEQ ID NO:211.

In some embodiments, clone CH3C.35.20.1.1 may have a knob mutation (e.g., T139W as numbered with reference to SEQ ID NO: 1), mutations that increase serum stability *(e.g.,* N207S with or without M201L as numbered with reference to SEQ ID NO:1), and at least 85% identity, at least 90% identity, or at least 95% identity to the sequence of SEQ ID NO:294. In some embodiments, clone CH3C.35.20.1.1 with the knob mutation and the mutations that increase serum stability has the sequence of SEQ ID NO:294.

In some embodiments, clone CH3C.35.20.1.1 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:212 or 213. In some embodiments, clone CH3C.35.20.1.1 with the knob mutation, the mutations that modulate effector function, and the mutations that increase serum stability has the sequence of SEQ ID NO:212 or 213.

In some embodiments, clone CH3C.35.20.1.1 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:295 or 296. In some embodiments, clone CH3C.35.20.1.1 with the knob mutation, the mutations that modulate effector function, and the mutations that increase serum stability has the sequence of SEQ ID NO:295 or 296.

In some embodiments, clone CH3C.35.20.1.1 may have hole mutations (e.g., T139S, L141A, and Y180V as numbered with reference to SEQ ID NO:1) and at least 85% identity, at least 90% identity, or at least 95% identity to the sequence of SEQ ID NO:214. In some embodiments, clone CH3C.35.20.1.1 with the hole mutations has the sequence of SEQ ID NO:214.

In some embodiments, clone CH3C.35.20.1.1 may have hole mutations (e.g., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), and at least 85% identity, at least 90% identity, or at least 95% identity to the sequence of SEQ ID NO:215 or 216. In some embodiments, clone CH3C.35.20.1.1 with the hole mutations and the mutations that modulate effector function has the sequence of SEQ ID NO:215 or 216.

In some embodiments, clone CH3C.35.20.1.1 may have hole mutations (e.g., T139S, L141A, and Y180V as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:217. In some embodiments, clone CH3C.35.20.1.1 with the hole mutations and the mutations that increase serum stability has the sequence of SEQ ID NO:217.

In some embodiments, clone CH3C.35.20.1.1 may have hole mutations (e.g., T139S, L141A, and Y180V as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:297. In some embodiments, clone CH3C.35.20.1.1 with the hole mutations and the mutations that increase serum stability has the sequence of SEQ ID NO:297.

In some embodiments, clone CH3C.35.20.1.1 may have hole mutations (e.g., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:218 or 219. In some embodiments, clone CH3C.35.20.1.1 with the hole mutations, the mutations that modulate effector function, and the mutations that increase serum stability has the sequence of SEQ ID NO:218 or 219.

In some embodiments, clone CH3C.35.20.1.1 may have hole mutations (e.g., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:298 or 299. In some embodiments, clone CH3C.35.20.1.1 with the hole mutations, the mutations that modulate effector function, and the mutations that increase serum stability has the sequence of SEQ ID NO:298 or 299.

### Clone CH3C.35.23.2.1

In some embodiments, clone CH3C.35.23.2.1 may have a knob mutation (e.g., T139W as numbered with reference to SEQ ID NO:1) and at least 85% identity, at least 90% identity, or at least 95% identity to the sequence of SEQ ID NO:220. In some embodiments, clone CH3C.35.23.2.1 with the knob mutation has the sequence of SEQ ID NO:220.

In some embodiments, clone CH3C.35.23.2.1 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), and at least 85% identity, at least 90% identity, or at least 95% identity to the sequence of SEQ ID NO:221 or 222. In some embodiments, clone CH3C.35.23.2.1 with the knob mutation and the mutations that modulate effector function has the sequence of SEQ ID NO:221 or 222.

In some embodiments, clone CH3C.35.23.2.1 may have a knob mutation (e.g., T139W as numbered with reference to SEQ ID NO: 1), mutations that increase serum stability *(e.g.,* M25Y, S27T, and T29E as numbered with reference to SEQ ID NO:1), and at least 85% identity, at least 90% identity, or at least 95% identity to the sequence of SEQ ID NO:223. In some embodiments, clone CH3C.35.23.2.1 with the knob mutation and the mutations that increase serum stability has the sequence of SEQ ID NO:223.

In some embodiments, clone CH3C.35.23.2.1 may have a knob mutation (e.g., T139W as numbered with reference to SEQ ID NO:1), mutations that increase serum stability *(e.g.,* N207S with or without M201L as numbered with reference to SEQ ID NO:1), and at least 85% identity, at least 90% identity, or at least 95% identity to the sequence of SEQ ID NO:301. In some embodiments, clone CH3C.35.23.2.1 with the knob mutation and the mutations that increase serum stability has the sequence of SEQ ID NO:301.

In some embodiments, clone CH3C.35.23.2.1 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (*e*.*g*., M25Y, S27T, and T29E as numbered with reference to SEQ ID NO:1), and at least 85% identity, at least 90% identity, or at least 95% identity to the sequence of SEQ ID NO:224 or 225. In some embodiments, clone CH3C.35.23.2.1 with the knob mutation, the mutations that modulate effector function, and the mutations that increase serum stability has the sequence of SEQ ID NO:224 or 225.

In some embodiments, clone CH3C.35.23.2.1 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:302 or 303. In some embodiments, clone CH3C.35.23.2.1 with the knob mutation, the mutations that modulate effector function, and the mutations that increase serum stability has the sequence of SEQ ID NO:302 or 303.

In some embodiments, clone CH3C.35.23.2.1 may have hole mutations (e.g., T139S, L141A, and Y180V as numbered with reference to SEQ ID NO:1) and at least 85% identity, at least 90% identity, or at least 95% identity to the sequence of SEQ ID NO:226. In some embodiments, clone CH3C.35.23.2.1 with the hole mutations has the sequence of SEQ ID NO:226.

In some embodiments, clone CH3C.35.23.2.1 may have hole mutations (e.g., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), and at least 85% identity, at least 90% identity, or at least 95% identity to the sequence of SEQ ID NO:227 or 228. In some embodiments, clone CH3C.35.23.2.1 with the hole mutations and the mutations that modulate effector function has the sequence of SEQ ID NO:227 or 228.

In some embodiments, clone CH3C.35.23.2.1 may have hole mutations (e.g., T139S, L141A, and Y180V as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:229. In some embodiments, clone CH3C.35.23.2.1 with the hole mutations and the mutations that increase serum stability has the sequence of SEQ ID NO:229.

In some embodiments, clone CH3C.35.23.2.1 may have hole mutations (e.g., T139S, L141A, and Y180V as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:304. In some embodiments, clone CH3C.35.23.2.1 with the hole mutations and the mutations that increase serum stability has the sequence of SEQ ID NO:304.

In some embodiments, clone CH3C.35.23.2.1 may have hole mutations (e.g., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:230 or 231. In some embodiments, clone CH3C.35.23.2.1 with the hole mutations, the mutations that modulate effector function, and the mutations that increase serum stability has the sequence of SEQ ID NO:230 or 231.

In some embodiments, clone CH3C.35.23.2.1 may have hole mutations (e.g., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:305 or 306. In some embodiments, clone CH3C.35.23.2.1 with the hole mutations, the mutations that modulate effector function, and the mutations that increase serum stability has the sequence of SEQ ID NO:305 or 306.

### Clone CH3C.35.23.1.1

In some embodiments, clone CH3C.35.23.1.1 may have a knob mutation (e.g., T139W as numbered with reference to SEQ ID NO:1) and at least 85% identity, at least 90% identity, or at least 95% identity to the sequence of SEQ ID NO:232. In some embodiments, clone CH3C.35.23.1.1 with the knob mutation has the sequence of SEQ ID NO:232.

In some embodiments, clone CH3C.35.23.1.1 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), and at least 85% identity, at least 90% identity, or at least 95% identity to the sequence of SEQ ID NO:233 or 234. In some embodiments, clone CH3C.35.23.1.1 with the knob mutation and the mutations that modulate effector function has the sequence of SEQ ID NO:233 or 234.

In some embodiments, clone CH3C.35.23.1.1 may have a knob mutation (e.g., T139W as numbered with reference to SEQ ID NO: 1), mutations that increase serum stability *(e.g.,* M25Y, S27T, and T29E as numbered with reference to SEQ ID NO:1), and at least 85% identity, at least 90% identity, or at least 95% identity to the sequence of SEQ ID NO:235. In some embodiments, clone CH3C.35.23.1.1 with the knob mutation and the mutations that increase serum stability has the sequence of SEQ ID NO:235.

In some embodiments, clone CH3C.35.23.1.1 may have a knob mutation (e.g., T139W as numbered with reference to SEQ ID NO: 1), mutations that increase serum stability *(e.g.,* N207S with or without M201L as numbered with reference to SEQ ID NO:1), and at least 85% identity, at least 90% identity, or at least 95% identity to the sequence of SEQ ID NO:308. In some embodiments, clone CH3C.35.23.1.1 with the knob mutation and the mutations that increase serum stability has the sequence of SEQ ID NO:308.

In some embodiments, clone CH3C.35.23.1.1 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:236 or 237. In some embodiments, clone CH3C.35.23.1.1 with the knob mutation, the mutations that modulate effector function, and the mutations that increase serum stability has the sequence of SEQ ID NO:236 or 237.

In some embodiments, clone CH3C.35.23.1.1 may have a knob mutation (e.g., T139W as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:309 or 310. In some embodiments, clone CH3C.35.23.1.1 with the knob mutation, the mutations that modulate effector function, and the mutations that increase serum stability has the sequence of SEQ ID NO:309 or 310.

In some embodiments, clone CH3C.35.23.1.1 may have hole mutations (e.g., T139S, L141A, and Y180V as numbered with reference to SEQ ID NO: 1) and at least 85% identity, at least 90% identity, or at least 95% identity to the sequence of SEQ ID NO:238. In some embodiments, clone CH3C.35.23.1.1 with the hole mutations has the sequence of SEQ ID NO:238.

In some embodiments, clone CH3C.35.23.1.1 may have hole mutations (e.g., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), and at least 85% identity, at least 90% identity, or at least 95% identity to the sequence of SEQ ID NO:239 or 240. In some embodiments, clone CH3C.35.23.1.1 with the hole mutations and the mutations that modulate effector function has the sequence of SEQ ID NO:239 or 240.

In some embodiments, clone CH3C.35.23.1.1 may have hole mutations (e.g., T139S, L141A, and Y180V as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:241. In some embodiments, clone CH3C.35.23.1.1 with the hole mutations and the mutations that increase serum stability has the sequence of SEQ ID NO:241.

In some embodiments, clone CH3C.35.23.1.1 may have hole mutations (e.g., T139S, L141A, and Y180V as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:311. In some embodiments, clone CH3C.35.23.1.1 with the hole mutations and the mutations that increase serum stability has the sequence of SEQ ID NO:311.

In some embodiments, clone CH3C.35.23.1.1 may have hole mutations (e.g., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., M25Y, S27T, and T29E as numbered with reference to SEQ ID NO:1), and at least 85% identity, at least 90% identity, or at least 95% identity to the sequence of SEQ ID NO:242 or 243. In some embodiments, clone CH3C.35.23.1.1 with the hole mutations, the mutations that modulate effector function, and the mutations that increase serum stability has the sequence of SEQ ID NO:242 or 243.

In some embodiments, clone CH3C.35.23.1.1 may have hole mutations (e.g., T139S, L141A, and Y180V as numbered with reference to SEQ ID NO:1), mutations that modulate effector function (*e.g.*, L7A, L8A, and/or P102G (*e.g.*, L7A and L8A) as numbered with reference to SEQ ID NO:1), mutations that increase serum stability (e.g., N207S with or without M201L as numbered with reference to SEQ ID NO:1), and at least 85% identity, at least 90% identity, or at least 95% identity to the sequence of SEQ ID NO:312 or 313. In some embodiments, clone CH3C.35.23.1.1 with the hole mutations, the mutations that modulate effector function, and the mutations that increase serum stability has the sequence of SEQ ID NO:312 or 313.

### IV. CONJUGATES

In some embodiments, a transferrin receptor-binding polypeptide comprising a modified CH3 domain in accordance with the invention is linked to an agent, *e.g.,* an agent that is to be internalized into a cell and/or for transcytosis across an endothelium, such as the blood-brain barrier, via a linker. The linker may be any linker suitable for joining an agent to the polypeptide. In some embodiments, the linkage is enzymatically cleavable. In certain embodiments, the linkage is cleavable by an enzyme present in the central nervous system.

In some embodiments, the linker is a peptide linker. The peptide linker may be configured such that it allows for the rotation of the agent and the transferrin receptor-binding polypeptide relative to each other; and/or is resistant to digestion by proteases. In some embodiments, the linker may be a flexible linker, e.g., containing amino acids such as Gly, Asn, Ser, Thr, Ala, and the like. Such linkers are designed using known parameters. For example, the linker may have repeats, such as Gly-Ser repeats.

In various embodiments, the conjugates can be generated using well-known chemical cross-linking reagents and protocols. For example, there are a large number of chemical cross-linking agents that are known to those skilled in the art and useful for cross-linking the polypeptide with an agent of interest. For example, the cross-linking agents are heterobifunctional cross-linkers, which can be used to link molecules in a stepwise manner. Heterobifunctional cross-linkers provide the ability to design more specific coupling methods for conjugating proteins, thereby reducing the occurrences of unwanted side reactions such as homo-protein polymers. A wide variety of heterobifunctional cross-linkers are known in the art, including N-hydroxysuccinimide (NHS) or its water soluble analog N-hydroxysulfosuccinimide (sulfo-NHS), succinimidyl 4-(N-maleimidomethyl)cyclohexane-1-carboxylate (SMCC), m-maleimidobenzoyl-N-hydroxysuccinimide ester (MBS); N-succinimidyl (4-iodoacetyl) aminobenzoate (SIAB), succinimidyl 4-(p-maleimidophenyl)butyrate (SMPB), 1-ethyl-3-(3-dimethylaminopropyl)carbodiimide hydrochloride (EDC); 4-succinimidyloxycarbonyl-a-methyl-a-(2-pyridyldithio)-toluene (SMPT), N-succinimidyl 3-(2-pyridyldithio)propionate (SPDP), and succinimidyl 6-[3-(2-pyridyldithio)propionate]hexanoate (LC-SPDP). Those cross-linking agents having N-hydroxysuccinimide moieties can be obtained as the N-hydroxysulfosuccinimide analogs, which generally have greater water solubility. In addition, those cross-linking agents having disulfide bridges within the linking chain can be synthesized instead as the alkyl derivatives so as to reduce the amount of linker cleavage *in vivo.* In addition to the heterobifunctional cross-linkers, there exist a number of other cross-linking agents including homobifunctional and photoreactive cross-linkers. Disuccinimidyl subcrate (DSS), bismaleimidohexane (BMH) and dimethylpimelimidate.2HCl (DMP) are examples of useful homobifunctional cross-linking agents, and bis-[B-(4-azidosalicylamido)ethyl]disulfide (BASED) and N-succinimidyl-6(4'-azido-2'-nitrophenylamino)hexanoate (SANPAH) are examples of useful photoreactive cross-linkers.

The agent of interest may be a therapeutic agent, including a cytotoxic agent, a DNA or RNA molecule, a chemical moiety, and the like. In some embodiments, the agent may be a peptide or small molecule therapeutic or imaging agent. In some embodiments, the small molecule is less than 1000 Da, less than 750 Da, or less than 500 Da.

The agent of interest may be linked to the N-terminal or C-terminal region of the transferrin receptor-binding polypeptide, or attached to any region of the polypeptide, so long as the agent does not interfere with binding of the transferrin receptor-binding polypeptide to the transferrin receptor.

### V. NUCLEIC ACIDS, VECTORS, AND HOST CELLS

Modified transferrin receptor-binding polypeptides as described herein are typically prepared using recombinant methods. Accordingly, in some aspects, the invention provides isolated nucleic acids comprising a nucleic acid sequence encoding any of the polypeptides comprising polypeptides as described herein, and host cells into which the nucleic acids are introduced that are used to replicate the polypeptide-encoding nucleic acids and/or to express the polypeptides. In some embodiments, the host cell is eukaryotic, e.g., a human cell.

In another aspect, polynucleotides are provided that comprise a nucleotide sequence that encodes the polypeptides described herein. The polynucleotides may be single-stranded or double-stranded. In some embodiments, the polynucleotide is DNA. In particular embodiments, the polynucleotide is cDNA. In some embodiments, the polynucleotide is RNA.

In some embodiments, the polynucleotide is included within a nucleic acid construct. In some embodiments, the construct is a replicable vector. In some embodiments, the vector is selected from a plasmid, a viral vector, a phagemid, a yeast chromosomal vector, and a non-episomal mammalian vector.

In some embodiments, the polynucleotide is operably linked to one or more regulatory nucleotide sequences in an expression construct. In one series of embodiments, the nucleic acid expression constructs are adapted for use as a surface expression library. In some embodiments, the library is adapted for surface expression in yeast. In some embodiments, the library is adapted for surface expression in phage. In another series of embodiments, the nucleic acid expression constructs are adapted for expression of the polypeptide in a system that permits isolation of the polypeptide in milligram or gram quantities. In some embodiments, the system is a mammalian cell expression system. In some embodiments, the system is a yeast cell expression system.

Expression vehicles for production of a recombinant polypeptide include plasmids and other vectors. For instance, suitable vectors include plasmids of the following types: pBR322-derived plasmids, pEMBL-derived plasmids, pEX-derived plasmids, pBTac-derived plasmids, and pUC-derived plasmids for expression in prokaryotic cells, such as *E. coli.* The pcDNAI/amp, pcDNAI/neo, pRc/CMV, pSV2gpt, pSV2neo, pSV2-dhfr, pTk2, pRSVneo, pMSG, pSVT7, pko-neo, and pHyg-derived vectors are examples of mammalian expression vectors suitable for transfection of eukaryotic cells. Alternatively, derivatives of viruses such as the bovine papilloma virus (BPV-1), or Epstein-Barr virus (pHEBo, pREP-derived, and p205) can be used for transient expression of polypeptides in eukaryotic cells. In some embodiments, it may be desirable to express the recombinant polypeptide by the use of a baculovirus expression system. Examples of such baculovirus expression systems include pVL-derived vectors (such as pVL1392, pVL1393, and pVL941), pAcUW-derived vectors (such as pAcUW1), and pBlueBac-derived vectors. Additional expression systems include adenoviral, adeno-associated virus, and other viral expression systems.

Vectors may be transformed into any suitable host cell. In some embodiments, the host cells, e.g., bacteria or yeast cells, may be adapted for use as a surface expression library. In some cells, the vectors are expressed in host cells to express relatively large quantities of the polypeptide. Such host cells include mammalian cells, yeast cells, insect cells, and prokaryotic cells. In some embodiments, the cells are mammalian cells, such as Chinese Hamster Ovary (CHO) cell, baby hamster kidney (BHK) cell, NS0 cell, Y0 cell, HEK293 cell, COS cell, Vero cell, or HeLa cell.

A host cell transfected with an expression vector encoding a transferrin receptor-binding polypeptide can be cultured under appropriate conditions to allow expression of the polypeptide to occur. The polypeptides may be secreted and isolated from a mixture of cells and medium containing the polypeptides. Alternatively, the polypeptide may be retained in the cytoplasm or in a membrane fraction and the cells harvested, lysed, and the polypeptide isolated using a desired method.

### VI. THERAPEUTIC INDICATIONS

A transferrin receptor-binding polypeptide in accordance with the invention may be used therapeutically in many indications. In some embodiments, the transferrin receptor-binding polypeptide is used to deliver a therapeutic agent to a target cell type that expresses the transferrin receptor. In some embodiments, a transferrin receptor-binding polypeptide may be used to transport a therapeutic moiety across an endothelium, e.g., the blood-brain barrier, to be taken up by the brain.

In some embodiments, a transferrin receptor-binding polypeptide of the present invention may be used, e.g., conjugated to a therapeutic agent, to deliver the therapeutic agent to treat a neurological disorder such as a disease of the brain or central nervous system (CNS). Illustrative diseases include Alzheimer's Disease, Parkinson's disease, amyotrophic lateral sclerosis, frontotemporal dementia, vascular dementia, Lewy body dementia, Pick's disease, primary age-related tauopathy, or progressive supranuclear palsy. In some embodiments, the disease may be a tauopathy, a prion disease (such as bovine spongiform encephalopathy, scrapie, Creutzfeldt-Jakob syndrome, kuru, Gerstmann-Straussler-Scheinker disease, chronic wasting disease, and fatal familial insomnia), bulbar palsy, motor neuron disease, or a nervous system heterodegenerative disorders (such as Canavan disease, Huntington's disease, neuronal ceroid-lipofuscinosis, Alexander's disease, Tourette's syndrome, Menkes kinky hair syndrome, Cockayne syndrome, Halervorden-Spatz syndrome, lafora disease, Rett syndrome, hepatolenticular degeneration, Lesch-Nyhan syndrome, Friedreich's ataxia, Spinal muscular atrophy, and Unverricht-Lundborg syndrome). In some embodiments, the disease is stroke or multiple sclerosis. In some embodiments, the patient may be asymptomatic, but has a marker that is associated with the disease of the brain or CNS. In some embodiments, the use of a transferrin receptor-binding polypeptide of the present invention in the manufacture of a medicament for treating a neurological disorder is provided.

In some embodiments, a transferrin receptor-binding polypeptide of the present invention is used for the treatment of cancer. In certain embodiments, the cancer is a primary cancer of the CNS, such as glioma, glioblastoma multiforme, meningioma, astrocytoma, acoustic neuroma, chondroma, oligodendroglioma, medulloblastomas, ganglioglioma, Schwannoma, neurofibroma, neuroblastoma, or extradural, intramedullary or intradural tumors. In some embodiments, the cancer is a solid tumor, or in other embodiments, the cancer is a non-solid tumor. Solid-tumor cancers include tumors of the central nervous system, breast cancer, prostate cancer, skin cancer (including basal cell carcinoma, cell carcinoma, squamous cell carcinoma and melanoma), cervical cancer, uterine cancer, lung cancer, ovarian cancer, testicular cancer, thyroid cancer, astrocytoma, glioma, pancreatic cancer, mesotheliomas, gastric cancer, liver cancer, colon cancer, rectal cancer, renal cancer including nephroblastoma, bladder cancer, oesophageal cancer, cancer of the larynx, cancer of the parotid, cancer of the biliary tract, endometrial cancer, adenocarcinomas, small cell carcinomas, neuroblastomas, adrenocortical carcinomas, epithelial carcinomas, desmoid tumors, desmoplastic small round cell tumors, endocrine tumors, Ewing sarcoma family tumors, germ cell tumors, hepatoblastomas, hepatocellular carcinomas, non-rhabdomyosarcome soft tissue sarcomas, osteosarcomas, peripheral primitive neuroectodermal tumors, retinoblastomas, and rhabdomyosarcomas. In some embodiments, the use of a transferrin receptor-binding polypeptide of the present invention in the manufacture of a medicament for treating cancer is provided.

In some embodiments, a transferrin receptor-binding polypeptide of the present invention may be used in the treatment of an autoimmune or inflammatory disease. Examples of such diseases include, but are not limited to, ankylosing spondylitis, arthritis, osteoarthritis, rheumatoid arthritis, psoriatic arthritis, asthma, scleroderma, stroke, atherosclerosis, Crohn's disease, colitis, ulcerative colitis, dermatitis, diverticulitis, fibrosis, idiopathic pulmonary fibrosis, fibromyalgia, hepatitis, irritable bowel syndrome (IBS), lupus, systemic lupus erythematous (SLE), nephritis, multiple sclerosis, and ulcerative colitis. In some embodiments, the use of a transferrin receptor-binding polypeptide of the present invention in the manufacture of a medicament for treating an autoimmune or inflammatory disease is provided.

In some embodiments, a transferrin receptor-binding polypeptide of the present invention may be used in the treatment of a cardiovascular disease, such as coronary artery disease, heart attack, abnormal heart rhythms or arrhythmias, heart failure, heart valve disease, congenital heart disease, heart muscle disease, cardiomyopathy, pericardial disease, aorta disease, marfan syndrome, vascular disease, and blood vessel disease. In some embodiments, the use of a transferrin receptor-binding polypeptide of the present invention in the manufacture of a medicament for treating a cardiovascular disease is provided.

The method may further comprise administering to the subject one or more additional therapeutic agents. For example, for treating a disease of the brain or central nervous system, the method may comprise administering to the subject a neuroprotective agent, *e.g.,* an anticholinergic agent, a dopaminergic agent, a glutamatergic agent, a histone deacetylase (HDAC) inhibitor, a cannabinoid, a caspase inhibitor, melatonin, an antiinflammatory agent, a hormone (e.g., estrogen or progesterone), or a vitamin. The method may comprise administering to the subject an agent for use in treating a cognitive or behavioral symptom of a neurological disorder *(e.g.,* an antidepressant, a dopamine agonist, or an anti-psychotic).

A transferrin receptor-binding polypeptide of the present invention is administered to a subject at a therapeutically effective amount or dose. Illustrative dosages include a daily dose range of about 0.01 mg/kg to about 500 mg/kg, or about 0.1 mg/kg to about 200 mg/kg, or about 1 mg/kg to about 100 mg/kg, or about 10 mg/kg to about 50 mg/kg, can be used. The dosages, however, may be varied according to several factors, including the chosen route of administration, the formulation of the composition, patient response, the severity of the condition, the subject's weight, and the judgment of the prescribing physician. The dosage can be increased or decreased over time, as required by an individual patient. In some embodiments, a patient initially is given a low dose, which is then increased to an efficacious dosage tolerable to the patient. Determination of an effective amount is well within the capability of those skilled in the art.

In various embodiments, a transferrin receptor-binding polypeptide of the present invention is administered parenterally. In some embodiments, the polypeptide is administered intravenously. Intravenous administration can be by infusion, e.g., over a period of from about 10 to about 30 minutes, or over a period of at least 1 hour, 2 hours, or 3 hours. In some embodiments, the polypeptide is administered as an intravenous bolus. Combinations of infusion and bolus administration may also be used.

In some parenteral embodiments, a transferrin receptor-binding polypeptide is administered intraperiotneally, subcutaneously, intradermally, or intramuscularly. In some embodiments, the polypeptide is administered intradermally or intramuscularly. In some embodiments, the polypeptide is administered intrathecally, such as by epidural administration, or intracerebroventricularly.

In other embodiments, a transferrin receptor-binding polypeptide may be administered orally, by pulmonary administration, intranasal administration, intraocular administration, or by topical administration. Pulmonary administration can also be employed, e.g., by use of an inhaler or nebulizer, and formulation with an aerosolizing agent.

### VII. PHARMACEUTICAL COMPOSITIONS AND KITS

In another aspect, pharmaceutical compositions and kits comprising a transferrin receptor-binding polypeptide are disclosed.

### Pharmaceutical compositions

Guidance for preparing formulations for use in the present invention can be found in any number of handbooks for pharmaceutical preparation and formulation that are known to those of skill in the art.

In some embodiments, a pharmaceutical composition comprises a transferrin receptor-binding polypeptide as described herein and further comprises one or more pharmaceutically acceptable carriers and/or excipients. A pharmaceutically acceptable carrier includes any solvents, dispersion media, or coatings that are physiologically compatible and that preferably does not interfere with or otherwise inhibit the activity of the active agent. Various pharmaceutically acceptable excipients are well-known.

In some embodiments, the carrier is suitable for intravenous, intrathecal, intracerebroventricular, intramuscular, oral, intraperitoneal, transdermal, topical, or subcutaneous administration. Pharmaceutically acceptable carriers can contain one or more physiologically acceptable compounds that act, for example, to stabilize the composition or to increase or decrease the absorption of the polypeptide. Physiologically acceptable compounds can include, for example, carbohydrates, such as glucose, sucrose, or dextrans, antioxidants, such as ascorbic acid or glutathione, chelating agents, low molecular weight proteins, compositions that reduce the clearance or hydrolysis of the active agents, or excipients or other stabilizers and/or buffers. Other pharmaceutically acceptable carriers and their formulations are also available in the art.

The pharmaceutical compositions described herein can be manufactured in a manner that is known to those of skill in the art, *e.g.,* by means of conventional mixing, dissolving, granulating, dragee-making, emulsifying, encapsulating, entrapping, or lyophilizing processes. The following methods and excipients are merely exemplary and are in no way limiting.

For oral administration, a transferrin receptor-binding polypeptide can be formulated by combining it with pharmaceutically acceptable carriers that are well-known in the art. Such carriers enable the compounds to be formulated as tablets, pills, dragees, capsules, emulsions, lipophilic and hydrophilic suspensions, liquids, gels, syrups, slurries, suspensions and the like, for oral ingestion by a patient to be treated. Pharmaceutical preparations for oral use can be obtained by mixing the polypeptides with a solid excipient, optionally grinding a resulting mixture, and processing the mixture of granules, after adding suitable auxiliaries, if desired, to obtain tablets or dragee cores. Suitable excipients include, for example, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone. If desired, disintegrating agents can be added, such as a cross-linked polyvinyl pyrrolidone, agar, or alginic acid or a salt thereof such as sodium alginate.

As disclosed above, a transferrin receptor-binding polypeptide as described herein can be formulated for parenteral administration by injection, e.g., by bolus injection or continuous infusion. For injection, the polypeptides can be formulated into preparations by dissolving, suspending, or emulsifying them in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers, and preservatives. In some embodiments, polypeptides can be formulated in aqueous solutions, preferably in physiologically compatible buffers such as Hanks's solution, Ringer's solution, or physiological saline buffer. Formulations for injection can be presented in unit dosage form, *e.g.,* in ampules or in multi-dose containers, with an added preservative. The compositions can take such forms as suspensions, solutions, or emulsions in oily or aqueous vehicles, and can contain formulatory agents such as suspending, stabilizing, and/or dispersing agents.

In some embodiments, a transferrin receptor-binding polypeptide is prepared for delivery in a sustained-release, controlled release, extended-release, timed-release, or delayed-release formulation, for example, in semi-permeable matrices of solid hydrophobic polymers containing the active agent. Various types of sustained-release materials have been established and are well-known by those skilled in the art. Extended-release formulations include film-coated tablets, multiparticulate or pellet systems, matrix technologies using hydrophilic or lipophilic materials and wax-based tablets with pore-forming excipients. Sustained-release delivery systems can, depending on their design, release the compounds over the course of hours or days, for instance, over 4, 6, 8, 10, 12, 16, 20, 24 hours or more. Usually, sustained release formulations can be prepared using naturally occurring or synthetic polymers, for instance, polymeric vinyl pyrrolidones, such as polyvinyl pyrrolidone; carboxyvinyl hydrophilic polymers; hydrophobic and/or hydrophilic hydrocolloids, such as methylcellulose, ethylcellulose, hydroxypropylcellulose, and hydroxypropylmethylcellulose; and carboxypolymethylene.

Typically, a pharmaceutical composition for use in *in vivo* administration is sterile. Sterilization can be accomplished according to methods known in the art, *e.g.,* heat sterilization, steam sterilization, sterile filtration, or irradiation.

Dosages and desired drug concentration of pharmaceutical compositions of the invention may vary depending on the particular use envisioned. The determination of the appropriate dosage or route of administration is well within the skill of one in the art. Suitable dosages are also described in Section VII above.

### Kits

In some aspects, kits comprising a transferrin receptor-binding polypeptide as described herein are provided. In some aspects, the kits are for use in preventing or treating a neurological disorder such as a disease of the brain or central nervous system (CNS).

In some aspects, the kit further comprises one or more additional therapeutic agents. For example, in some aspects, the kit comprises a transferrin receptor-binding polypeptide as described herein and further comprises one or more additional therapeutic agents for use in the treatment of a neurological disorder. In some aspects, the kit further comprises instructional materials containing directions *(i.e.,* protocols) for the practice of the methods described herein (e.g., instructions for using the kit for administering a composition across the blood-brain barrier). While the instructional materials typically comprise written or printed materials, they are not limited to such. Any medium capable of storing such instructions and communicating them to an end user is contemplated by this aspect of the disclosure. Such media include, but are not limited to, electronic storage media (*e.g.*, magnetic discs, tapes, cartridges, chips), optical media (e.g., CD-ROM), and the like. Such media may include addresses to internet sites that provide such instructional materials.

### VIII. EXAMPLES

The present invention will be described in greater detail by way of specific examples. The following examples are offered for illustrative purposes only, and are not intended to limit the invention in any manner. Those of skill in the art will readily recognize a variety of noncritical parameters which can be changed or modified to yield essentially the same results. Efforts have been made to ensure accuracy with respect to numbers used (e.g., amounts, temperatures, *etc.*), but some experimental error and deviation may be present. The practice of the present invention will employ, unless otherwise indicated, conventional methods of protein chemistry, biochemistry, recombinant DNA techniques and pharmacology, within the skill of the art. Such techniques are explained fully in the literature. Additionally, it should be apparent to one of skill in the art that the methods for engineering as applied to certain libraries can also be applied to other libraries described herein.

### Example 1. Generation of TfR Target

DNA encoding the transferrin receptor (TfR) ectodomain (ECD) (residues 121-760 of the human (SEQ ID NO:38) or cyno (SEQ ID NO: 103) TfR) was cloned into a mammalian expression vector with C-terminal cleavable His- and Avi-tags. The plasmid was transfected and expressed in HEK293 cells. The ectodomain was purified from the harvested supernatant using Ni-NTA chromatography followed by size-exclusion chromatography to remove any aggregated protein. The yield was about 5 mg per liter of culture. The protein was stored in 10 mM K₃PO₄ (pH 6.7), 100 mM KCl, 100 mM NaCl, and 20% glycerol and frozen at -20 °C.

DNA encoding the permutated TfR apical domain (SEQ ID NO: 104) (residues 326-379 and 194-296 of the human or cyno TfR) was cloned into a pET28 vector with an N-terminal His-tag for purification and an Avi-tag for *in vivo* biotinylation. The plasmid was co-transformed with a BirA expression vector into BL21 (DE3) cells. Cells were grown in LB media at 37 °C until log phase, and then induced with 1 mM isopropyl 1-thio-β-D-galactopyranoside (IPTG) followed by culture overnight at 18 °C. The cells were lysed and the soluble fraction was applied to an Ni-NTA column for affinity purification followed by size-exclusion chromatography to remove any aggregated protein. The yield was about 10 mg per liter of culture. The protein was stored in 50 mM HEPES (pH 7.5), 150 mM NaCl, and 1 mM DTT and frozen at -20 °C.

The purified TfR ECDs were biotinylated using an EZ-link sulfo-NHS-LC-Biotin kit (obtained from Thermo Scientific). Five-fold molar excess of biotin was used for the reaction. The excess biotin was removed by extensively dialyzing against PBS.

The Avi-tagged TfR ECDs and apical domains was biotinylated using BirA-500 (BirA biotin-protein ligase standard reaction kit from Avidity, LLC). After reaction, the labeled proteins were further purified by size-exclusion chromatography to remove excess BirA enzyme. The final material was stored in 10 mM K₃PO₄ (pH 6.7), 100 mM KCl, 100 mM NaCl, and 20% glycerol and frozen at -20 °C.

### Example 2. Design and Characterization of Engineered Transferrin Receptor Binding Polypeptides

This example describes the design, generation, and characterization of polypeptides of the present invention. For the purposes of this example and comparing the amino acids that are the same in clone sequences, a "conserved" mutation is considered to be one that occurred in all of the identified clones (not a conservative amino acid substitution), while a "semi-conserved" mutation is one that occurs in >50% of clones.

Unless otherwise indicated, the positions of amino acid residues in this section are numbered based on SEQ ID NO:1, a human IgG1 wild-type Fc region having three residues from the hinge, PCP, at the amino-terminal end.

### Design of polypeptide Fc region domain libraries

New molecular recognition was engineered into polypeptide Fc regions by selecting certain solvent exposed surface patches for modification, constructing surface display libraries in which the amino acid composition of the selected patch was altered by randomization and then screening the surface-displayed sequence variants for desired functionality using standard expression display techniques. As used herein, the term "randomization" includes partial randomization as well as sequence changes with pre-defined nucleotide or amino acid mixing ratios. Typical surface-exposed patches selected for randomization had areas between about 600 to 1500 Å², and comprised about 7 to 15 amino acids.

### Clone register

The following register was designed and generated according to the methods described herein. As used herein, the term "register" refers to a series of surface-exposed amino acid residues that form a contiguous surface that can be altered *(e.g.,* by the introduction of mutations into the polypeptide coding gene sequences to produce amino acid substitutions, insertions, and/or deletions at the positions listed in the register).

### CH3 register C

The CH3C register (Table 1) included amino acid positions 157, 159, 160, 161, 162, 163, 186, 189, and 194 as numbered with reference to the human IgG1 Fc region amino acid sequence set forth in SEQ ID NO:1. The CH3C register positions form a contiguous surface by including surface-exposed residues from two loops, both distant from the FcyR and FcRn binding sites.

### Generation of phase-display libraries

A DNA template coding for the wild-type human Fc sequence (SEQ ID NO:1) was synthesized and incorporated into a phagemid vector. The phagemid vector contained an ompA or pelB leader sequence, the Fc insert fused to c-Myc and 6xHis epitope tags, and an amber stop codon followed by M13 coat protein pill.

Primers containing "NNK" tricodons at the corresponding positions for randomization were generated, where N is any DNA base *(i.e.,* A, C, G, or T) and K is either G or T. Alternatively, primers for "soft" randomization were used, where a mix of bases corresponding to 70% wild-type base and 10% of each of the other three bases was used for each randomization position. Libraries were generated by performing PCR amplification of fragments of the Fc region corresponding to regions of randomization and then assembled using end primers containing *Sfi*I restriction sites, then digested with *Sfi*I and ligated into the phagemid vectors. Alternatively, the primers were used to conduct Kunkel mutagenesis. Methods of performing Kunkel mutagenesis will be known to one of skill in the art. The ligated products or Kunkel products were transformed into electrocompetent *E. coli* cells of strain TG1 (obtained from Lucigen^{®}). The *E. coli* cells were infected with M13K07 helper phage after recovery and grown overnight, after which library phage were precipitated with 5% PEG/NaCl, resuspended in 15% glycerol in PBS, and frozen until use. Typical library sizes ranged from about 10⁹ to about 10¹¹ transformants. Fc-dimers were displayed on phage via pairing between pIII-fused Fc and soluble Fc not attached to pIII (the latter being generated due to the amber stop codon before pill).

### Generation of yeast-display libraries

A DNA template coding for the wild-type human Fc sequence was synthesized and incorporated into a yeast display vector. For CH3 libraries, the Fc polypeptides were displayed on the Aga2p cell wall protein. Both vectors contained prepro leader peptides with a Kex2 cleavage sequence, and a c-Myc epitope tag fused to the terminus of the Fc.

Yeast display libraries were assembled using methods similar to those described for the phage libraries, except that amplification of fragments was performed with primers containing homologous ends for the vector. Freshly prepared electrocompetent yeast (*i*.*e*., strain EBY100) were electroporated with linearized vector and assembled library inserts. Electroporation methods will be known to one of skill in the art. After recovery in selective SD-CAA media, the yeast were grown to confluence and split twice, then induced for protein expression by transferring to SG-CAA media. Typical library sizes ranged from about 10⁷ to about 10⁹ transformants. Fc-dimers were formed by pairing of adjacently displayed Fc monomers.

### General methods for phage selection

Phage methods were adapted from Phage Display: A Laboratory Manual (Barbas, 2001). Additional protocol details can be obtained from this reference.

### Plate sorting methods

Human TfR target was coated on MaxiSorp^{®} microtiter plates (typically 200 µL at 1-10 µg/mL in PBS) overnight at 4 °C. All binding was done at room temperature unless otherwise specified. The phage libraries were added into each well and incubated overnight for binding. Microtiter wells were washed extensively with PBS containing 0.05 % Tween^{®} 20 (PBST) and bound phage were eluted by incubating the wells with acid (typically 50 mM HCl with 500 mM KCl, or 100 mM glycine, pH 2.7) for 30 minutes. Eluted phage were neutralized with 1 M Tris (pH 8) and amplified using TG1 cells and M13/KO7 helper phage and grown overnight at 37 °C in 2YT media containing 50 µg/mL carbenacillin and 50 ug/mL Kanamycin. The titers of phage eluted from a target-containing well were compared to titers of phage recovered from a non-target-containing well to assess enrichment. Selection stringency was increased by subsequently decreasing the incubation time during binding and increasing washing time and number of washes.

### Bead sorting methods

Human TfR target was biotinylated through free amines using NHS-PEG4-Biotin (obtained from Pierce^{™}). For biotinylation reactions, a 3- to 5-fold molar excess of biotin reagent was used in PBS. Reactions were quenched with Tris followed by extensive dialysis in PBS. The biotinylated target was immobilized on streptavidin-coated magnetic beads, *(i.e.,* M280-streptavidin beads obtained Thermo Fisher). The phage display libraries were incubated with the target-coated beads at room temperature for 1 hour. The unbound phage were then removed and beads were washed with PBST. The bound phage were eluted by incubating with 50 mM HCl containing 500 mM KCl (or 0.1 M glycine, pH 2.7) for 30 minutes, and then neutralized and propagated as described above for plate sorting.

After three to five rounds of panning, single clones were screened by either expressing Fc on phage or solubly in the *E. coli* periplasm. Such expression methods will be known to one of skill in the art. Individual phage supernatants or periplasmic extracts were exposed to blocked ELISA plates coated with target or a negative control and were subsequently detected using HRP-conjugated goat anti-Fc (obtained from Jackson Immunoresearch) for periplasmic extracts or anti-M13 (GE Healthcare) for phage, and then developed with TMB reagent (obtained from Thermo Fisher). Wells with OD₄₅₀ values greater than around 5-fold over background were considered positive clones and sequenced, after which some clones were expressed either as a soluble Fc fragment or fused to Fab fragments.

### General methods for yeast selection

### Bead sorting (Magnetic-assisted cell sorting (MACS)) methods

MACS and FACS selections were performed similarly to as described in Ackerman, et al. 2009 Biotechnol. Prog. 25(3), 774. Streptavidin magnetic beads (*e.g.*, M-280 streptavidin beads from ThermoFisher) were labeled with biotinylated target and incubated with yeast (typically 5-10x library diversity). Unbound yeast were removed, the beads were washed, and bound yeast were grown in selective media and induced for subsequent rounds of selection.

### Fluorescence-activated cell sorting (FACS) methods

Yeast were labeled with anti-c-Myc antibody to monitor expression and biotinylated target (concentration varied depending on the sorting round). In some experiments, the target was pre-mixed with streptavidin-Alexa Fluor^{®} 647 in order to enhance the avidity of the interaction. In other experiments, the biotinylated target was detected after binding and washing with streptavidin-Alexa Fluor^{®} 647. Singlet yeast with binding were sorted using a FACS Aria III cell sorter. The sorted yeast were grown in selective media then induced for subsequent selection rounds.

After an enriched yeast population was achieved, yeast were plated on SD-CAA agar plates and single colonies were grown and induced for expression, then labeled as described above to determine their propensity to bind to the target. Positive single clones were subsequently sequenced for binding target, after which some clones were expressed either as a soluble Fc fragment or as fused to Fab fragments.

### General methods for screening

### Screening by ELISA

Clones were selected from panning outputs and grown in individual wells of 96-well deep-well plates. The clones were either induced for periplasmic expression using autoinduction media (obtained from EMD Millipore) or infected with helper phage for phage-display of the individual Fc variants on phage. The cultures were grown overnight and spun to pellet *E. coli.* For phage ELISA, phage containing supernatant was used directly. For periplasmic expression, pellets were resuspended in 20% sucrose, followed by dilution at 4:1 with water, and shaken at 4 °C for 1 hour. Plates were spun to pellet the solids and supernatant was used in the ELISA.

ELISA plates were coated with target, typically at 0.5 mg/mL overnight, then blocked with 1% BSA before addition of phage or periplasmic extracts. After a 1-hour incubation and washing off unbound protein, HRP-conjugated secondary antibody was added *(i.e.,* anti-Fc or anti-M13 for soluble Fc or phage-displayed Fc, respectively) and incubated for 30 minutes. The plates were washed again, and then developed with TMB reagent and quenched with 2N sulfuric acid. Absorbance at 450 nm was quantified using a plate reader (BioTek^{®}) and binding curves were polotted using Prism software where applicable. Absorbance signal for tested clones was compared to negative control (phage or paraplasmic extract lacking Fc). In some assays, soluble holo-transferrin was added during the binding step, typically at significant molar excess (greater than 10-fold excess).

### Screening by flow cytometry

Fc variant polypeptides (expressed either on phage, in periplasmic extracts, or solubly as fusions to Fab fragments) were added to cells in 96-well V-bottom plates (about 100,000 cells per well in PBS+1%BSA (PBSA)), and incubated at 4 °C for 1 hour. The plates were subsequently spun and the media was removed, and then the cells were washed once with PBSA. The cells were resuspended in PBSA containing secondary antibody (goat anti-human-IgG-Alexa Fluor^{®} 647 (obtained from Thermo Fisher)). After 30 minutes, the plates were spun and the media was removed, the cells were washed 1-2 times with PBSA, and then the plates were read on a flow cytometer (*i.e.*, a FACSCanto^{™} II flow cytometer). Median fluorescence values were calculated for each condition using FlowJo software and binding curves were plotted with Prism software.

### CH3C clone generation and characterization

### Selections with CH3C library against transferrin receptor (TfR)

Yeast libraries against CH3C were panned and sorted against TfR as described above. Population enrichment FACS plots for the first three sort rounds are shown in FIG. 1. After an additional two rounds of sorting, single clones were sequenced and four unique sequences *(i.e.,* clones CH3C.1 (SEQ ID NO:4), CH3C.2 (SEQ ID NO:5), CH3C.3 (SEQ ID NO:6), and CH3C.4 (SEQ ID NO:7)) were identified. These sequences had a conserved Trp at position 161, and all had an aromatic residue *(i.e.,* Trp, Tyr, or His) at position 194. There was a great deal of diversity at other positions.

### Characterization of first generation CH3C clones

The four clones selected from the CH3C library were expressed as Fc fusions to Fab fragments in CHO or 293 cells, and purified by Protein A and size-exclusion chromatography, and then screened for binding to cyno and human TfR in the presence or absence of holo-Tf by ELISA. As shown in FIG. 2, the clones all bound to human TfR and the binding was not affected by the addition of excess (5 µM) holo-Tf. However, the clones did not bind appreciably to cyno TfR. Clones were also tested for binding to 293F cells, which endogenously express human TfR. FIG. 3 shows that while the clones bound to 293F cells, the overall binding was substantially weaker than the high-affinity positive control.

Next it was tested whether clone CH3C.3 could internalize in TfR-expressing cells. Adherent HEK293 cells were grown in 96-well plates to about 80% confluence, media was removed, and samples were added at 1 µM concentrations: CH3C.3 anti-TfR benchmark positive control antibody (Ab204), anti-BACE1 benchmark negative control antibody (Ab107), and human IgG isotype control (obtained from Jackson Immunoresearch). The cells were incubated at 37 °C and 8% CO₂ concentration for 30 minutes, then washed, permeabilized with 0.1% Triton^{™} X-100, and stained with anti-human-IgG-Alexa Fluor^{®} 488 secondary antibody. After additional washing, the cells were imaged under a high content fluorescence microscope (*i*.*e*., an Opera Phenix^{™} system), and the number of puncta per cell was quantified, as shown in FIG. 4. At 1 µM, clone CH3C.3 showed a similar propensity for internalization to the positive anti-TfR control, while the negative controls showed no internalization.

### Secondary engineering of CH3C clones

Additional libraries were generated to improve the affinity of the initial CH3C hits against human TfR, and to attempt to introduce binding to cyno TfR. A soft randomization approach was used, wherein DNA oligos were generated to introduce soft mutagenesis based on each of the original four hits. The first portion of the register (WESXGXXXXXYK; SEQ ID NO:34) and the second portion of the register (TVXKSXWQQGXV; SEQ ID NO:35) were built via separate fragments, so the soft randomized registers were shuffled during PCR amplification (e.g., the first portion of the register from clone CH3C.1 was mixed with the second portion of the register from clones CH3C.1, CH3C.2, CH3C.3, and CH3C.4, and so forth). The fragments were all mixed and then introduced into yeast for surface expression and selection.

The selection scheme is shown in FIG. 5. After one round of MACS and three rounds of FACS, individual clones were sequenced (clones CH3C.17 (SEQ ID NO:8), CH3C.18 (SEQ ID NO:9), CH3C.21 (SEQ ID NO:10), CH3C.25 (SEQ ID NO:11), CH3C.34 (SEQ ID NO:12), CH3C.35 (SEQ ID NO:13), CH3C.44 (SEQ ID NO: 14), and CH3C.51 (SEQ ID NO:15)). The selected clones fell into two general sequence groups. Group 1 clones *(i.e.,* clones CH3C.18, CH3C.21, CH3C.25, and CH3C.34) had a semi-conserved Leu at position 157, a Leu or His at position 159, a conserved and a semi-conserved Val at positions 160 and 162, respectively, and a semi-conserved P-T-W motif at positions 186, 189, and 194, respectively. Group 2 clones had a conserved Tyr at position 157, the motif TXWSX (SEQ ID NO:341) at positions 159-163, and the conserved motif S/T-E-F at positions 186, 189, and 194, respectively. Clones CH3C.18 and CH3.35 were used in additional studies as representative members of each sequence group. It was noted that clone CH3C.51 had the first portion of its register from group 1 and the second portion of its register from group 2.

### Binding characterization of CH3C clones from the soft mutagenesis library

Clones from the soft mutagenesis library were reformatted as Fc-Fab fusion polypeptides and expressed and purified as described above. As shown in FIG. 7, these variants had improved ELISA binding to human TfR as compared to the top clone from the initial library selections (CH3C.3), and also did not compete with holo-Tf. The EC₅₀ values, as shown below in Table 2, were not appreciably affected beyond the margin of error of the experiment by the presence or absence of holo-Tf.

**Table 2. EC₅₀ values (nM) for ELISA binding of CH3C variants to TfR in the presence or absence of holo-Tf**

| **Clone** | **-Tf** | +**Tf** |
|---|---|---|
| CH3C.3 | 8.1 | 6.3 |
| CH3C.17 | 5.3 | 17 |
| CH3C.18 | 6.9 | 3.5 |
| CH3C.25 | 51 | 48 |
| CH3C.35 | 0.49 | 0.61 |
| CH3C.51 | 160 | 36 |
| Ab204 | 1.6 | 0.24 |

Notably, clone CH3C.35 bound to human TfR about as well as the high affinity anti-TfR control antibody Ab204. The clones selected from the soft randomization library also had improved cell binding to 293F cells, as shown in FIG. 8. In a similar cell binding assay, these clones were tested for binding to CHO-K1 cells that stably express high levels of human or cyno TfR on their surface. The clones selected from the soft randomization library bound to cells expressing human TfR (FIG. 9A) as well as cyno TfR (FIG. 9B) and did not bind to the parental CHO-K1 cells (FIG. 9C). The magnitude and binding EC₅₀ values were substantially lower for cyno TfR as compared to human TfR. Data is summarized in Table 3 below.

**Table 3. EC₅₀ and max. MFI (Median Fluorescence Intensity) values for CH3C clones binding to cells**

| **Clone** | **293F EC50 (nM)** | **293F MFI at 200 nM** | **CHO-huTfR EC₅₀ (nM)** | **CHO-huTf MFI at 200 nM** | **CHO-cyTfR EC₅₀ (nM)** | **CHO-cyTfR MFI at 200 nM** |
|---|---|---|---|---|---|---|
| **CH3C.3** | n.d. | 1385 | 6.5 | 10296 | n.d. | 941 |
| **CH3C.17** | n.d. | 1556 | 4.2 | 13933 | > 50 | 8205 |
| **CH3C.18** | 22 | 2100 | 2.3 | 22997 | 6.6 | 9614 |
| **CH3C.25** | n.d. | 314 | 17 | 11434 | > 50 | 12515 |
| **CH3C.35** | 0.67 | 1481 | 2.6 | 22059 | 11 | 8292 |
| **CH3C.51** | n.d. | 784 | 27 | 11892 | > 50 | 14455 |
| **Ab204** | 0.25 | 3404 | 1.8 | 35744 | 2.4 | 41041 |

### Epitope mapping

To determine whether the engineered CH3C Fc regions bound to the apical domain of TfR, TfR apical domain (SEQ ID NOS:30 and 31 for human and cyno, respectively) was expressed on the surface of phage. To properly fold and display the apical domain, one of the loops had to be truncated and the sequence needed to be circularly permuted; the sequences expressed on phage are identified as SEQ ID NOS:32 and 33 for human and cyno, respectively. Clones CH3C.18 and CH3C.35 were coated on ELISA plates and the previously described phage ELISA protocol was followed. Briefly, after washing and blocking with 1% PBSA, dilutions of phage displaying were added and incubated at room temperature for 1 hour. The plates were subsequently washed and anti-M13-HRP was added, and after additional washing the plates were developed with TMB substrate and quenched with 2N H₂SO₄. Both CH3C.18 and CH3C.35 bound to the apical domain in this assay.

Since binding to cyno TfR was known to be much weaker than binding to human TfR, it was hypothesized that one or more of the amino acid differences between cyno and human apical domains was likely responsible for the binding difference. Therefore, a series of six point mutations was made in the human TfR apical domain where the human residue was replaced with the corresponding cyno residue. These mutants were displayed on phage and the phage concentrations were normalized by OD₂₆₈ and binding to CH3C.18 and CH3C.35 was tested by phage ELISA titration (FIGS. 11B and 11C). Capture on anti-Myc antibody 9E10 showed that display levels for all mutants were similar (FIG. 11A). Binding to the human TfR mutations clearly showed a strong effect of the R208G mutation, which suggested that this residue is a key part of the epitope and is negatively impacted by the cyno residue at this position. The G208R mutation was made on phage-displayed cyno apical domain and it was shown that this mutation dramatically improved binding to cyno apical domain (FIGS. 11D and 11E). These results show that the CH3C clones bound to the apical domain of TfR and that position 208 was important for binding, while positions 247, 292, 364, 370, and 372 were significantly less important.

### Paratope mapping

To understand which residues in the Fc domain were most critical for TfR binding, a series of mutant CH3C.18 and CH3C.35 clones was created in which each mutant had a single position in the TfR-binding register mutated back to wild-type. The resulting variants were expressed recombinantly as CH3C Fc-Fab fusions and tested for binding to human or cyno TfR (FIG. 12). For CH3C.35, positions 161 and 194 were absolutely critical for binding; reversion of either of these to wild-type completely ablated binding to human TfR. Surprisingly, reverting position 163 to wild-type provided a dramatic boost to cyno TfR binding, while having little effect on human binding. Conversely, the reversion of residue 163 to wild-type had little effect in CH3C.18, but in this variant reversion of positions 189 and 194 completely abolished binding to human TfR. In both variants, other single reversions had modest (detrimental) impact on human TfR binding, while in many cases binding to cyno TfR was abolished.

### Additional engineering to improve binding to cyno TfR

Additional libraries were prepared to further increase the affinity of the CH3C variants for cyno TfR. These libraries were designed to be of less than about 10⁷ clones in terms of theoretical diversity, so that the full diversity space could be explored using yeast surface display. The design of these libraries is shown in FIGS. 13A-13D. Four library designs were used; all libraries were generated using degenerate oligos with NNK or other degenerate codon positions, and amplified by overlap PCR, as described above.

The first library was based on the consensus of CH3C.35-like sequences (FIG. 13A). Here, positions 157-161 were held constant as YGTEW (SEQ ID NO:36), while positions 162, 163, 186, 189, and 194 were mutated using saturation mutagenesis.

The second library was based on the consensus of CH3C.18-like sequences (FIG. 13B). Here, position 157 was restricted to Leu and Met, position 159 was restricted to Leu and His, position 160 was held constant as Val, position 161 was restricted to Trp and Gly, position 162 was restricted to Val and Ala, position 163 was fully randomized, position 164 was added to the register and fully randomized, position 186 was soft randomized, position 189 was fully randomized, and position 194 was restricted to aromatic amino acids and Leu.

The third library added new randomized positions to the library (FIG. 13C). Two versions were generated, one each with CH3C.18 and CH3C.35 as the starting register, and then additional positions were randomized by saturation mutagenesis: E153, E155, Y164, S188, and Q192.

The fourth library held certain positions constant for CH3C.18 but allowed variation at other positions, with less bias than the consensus library (FIG. 13D). Positions 160, 161, and 186 were fixed, and positions 157, 159, 162, 163, and 189 were randomized by saturating mutagenesis; position 194 was mutated but restricted to aromatic residues and Leu.

The libraries were selected in yeast for four to five rounds against cynoTfR and single clones were sequenced and converted to polypeptide-Fab fusions, as described above. The greatest enrichment in cynoTfR binding was observed from the second library (*i.e.*, derivatives of the CH3.18 parent), though there was also some loss in huTfR binding.

### Binding characterization of CH3C maturation clones

Binding ELISAs were conducted with purified CH3C Fc-Fab fusion variants with human or cyno TfR coated on the plate, as described above. The variants from the CH3C.18 maturation library, CH3C3.2-1, CH3C.3.2-5, and CH3C.3.2-19, bound human and cyno TfR with approximately equivalent EC₅₀ values, whereas the parent clone CH3C.18, and CH3C.35, had greater than 10-fold better binding to human versus cyno TfR (FIG. 14).

Next, it was tested whether the new polypeptides internalized in human and monkey cells. Using the protocol previously described above in the section titled "Characterization of first generation CH3C clones," internalization in human HEK293 cells and rhesus LLC-MK2 cells was tested. As shown in FIG. 15, the variants that similarly bound human and cyno TfR, CH3C.3.2-5 and CH3C.3.2-19, had significantly improved internalization in LLC-MK2 cells as compared with CH3C.35.

### Additional engineering of CH3C clones

Additional engineering to further affinity mature clones CH3C.18 and CH3C.35 involved adding additional mutations to the backbone *(i.e.,* non-register) positions that enhanced binding through direct interactions, second-shell interactions, or structure stabilization. This was achieved via generation and selection from an "NNK walk" or "NNK patch" library. The NNK walk library involved making one-by-one NNK mutations of residues that are near to the paratope. By looking at the structure of Fc bound to FcgRI (PDB ID: 4W4O), 44 residues near the original library register, as shown in FIG. 16, were identified as candidates for interrogation. Specifically, the following residues were targeted for NNK mutagenesis: K21, R28, Q115, R117, E118, Q120, T132, K133, N134, Q135, S137, K143, E153, E155, S156, G158, Y164, K165, T166, D172, S173, D174, S176, K182, L183, T184, V185, K187, S188, Q191, Q192, G193, V195, F196, S197, S199, Q211, S213, S215, L216, S217, P218, G219, and K220. The 44 single point NNK libraries were generated using Kunkel mutagenesis, and the products were pooled and introduced to yeast via electroporation, as described above for other yeast libraries.

The combination of these mini-libraries (each of which had one position mutated, resulting in 20 variants) generated a small library that was selected using yeast surface display for any positions that lead to higher affinity binding. Selections were performed as described above, using TfR apical domain proteins (FIG. 17). After three rounds of sorting, clones from the enriched yeast library were sequenced, and several "hot-spot" positions were identified where certain point mutations significantly improved the binding to apical domain proteins. For CH3C.35, these mutations included E153 (mutated to Trp, Tyr, Leu, or Gln) and S188 (mutated to Glu). The sequences of the CH3C.35 single and combination mutants are set forth in SEQ ID NOS:21-23, 39-44, and 100-102. For CH3C.18, these mutations included E153 (mutated to Trp, Tyr, or Leu) and K165 (mutated to Gln, Phe, or His). The sequences of the CH3C.18 single mutants are set forth in SEQ ID NOS:45-50.

The "NNK patch" approach was similar to that described above for the CH3B library, but with patches directly adjacent to the CH3C register. Clone CH3C.35 was used as a starting point and the following libraries were generated:
CH3C-patch1: amino acid positions: K21, R28, Y164, K165, and T166;
CH3C-patch2: amino acid positions: Q115, R117, E118, Q120, and K143;
CH3C-patch3: amino acid positions: T132, K133, N134, Q135, and S137;
CH3C-patch4: amino acid positions: E153, E155, S156, and G158;
CH3C-patch5: amino acid positions: D172, S173, D174, S176, and K182;
CH3C-patch6: amino acid positions: L183, T184, V185, K187, and S188;
CH3C-patch7: amino acid positions: Q191, Q192, G193, V195, and F196;
CH3C-patch8: amino acid positions: S197, S199, Q211, S213, and S215; and
CH3C-patch9: amino acid positions: L216, S217, P218, G219, and K220.

Selections were performed as described above, using TfR apical domain proteins. However, no clones with enhanced binding were identified.

### Additional maturation libraries to improve CH3C.35 affinity

An additional library to identify combinations of mutations from the NNK walk library, while adding several additional positions on the periphery of these, was generated as described for previous yeast libraries. In this library, the YxTEWSS and TxxExxxxF motifs were kept constant, and six positions were completely randomized: E153, K165, K187, S188, S197, and S199. Positions E153 and S188 were included because they were "hot spots" in the NNK walk library. Positions K165, S197, and S199 were included because they make up part of the core that may position the binding region, while K187 was selected due to its adjacency to position 188.

This library was sorted, as previously described, with the cyno TfR apical domain only. The enriched pool was sequenced after five rounds, and the sequences of the CH3 regions of the identified unique clones are set forth in SEQ ID NOS:51-68.

### Exploration of acceptable diversity within the original register and hot spots for CH3C.35.21

The next libraries were designed to explore the totality of acceptable diversity in the main binding paratope. The approach taken was similar to the NNK walk libraries. Each of the original register positions (157, 159, 160, 161, 162, 163, 186, 189, and 194) plus the two hot spots (153 and 188) were individually randomized with NNK codons to generate a series of single-position saturation mutagenesis libraries on yeast. In addition, each position was individually reverted to the wild-type residue, and these individual clones were displayed on yeast. FIG. 18 shows binding of the parental clone CH3C.35.21 as compared to the wild-type reversions and single-position NNK libraries. It was noted that positions 153, 162, 163, and 188 were the only positions that retained substantial binding to TfR upon reversion to the wild-type residue (some residual but greatly diminished binding was observed for reversion of 186 to wild-type).

The single-position NNK libraries were sorted for three rounds against the human TfR apical domain to collect the top ~5% of binders, and then at least 16 clones were sequenced from each library. The results indicate what amino acids at each position can be tolerated without significantly reducing binding to human TfR, in the context of the CH3C.35 clone. A summary is below:
Position 153: Trp, Leu, or Glu;
Position 157: Tyr or Phe;
Position 159: Thr only;
Position 160: Glu only;
Position 161: Trp only;
Position 162: Ser, Ala, or Val (note that although the wild type Asn residue seems to retain some binding, it did not appear following library sorting);
Position 163: Ser or Asn;
Position 186: Thr or Ser;
Position 188: Glu or Ser;
Position 189: Glu only; and
Position 194: Phe only.

The above residues, when substituted into clone CH3C.35 as single changes or in combinations, represent paratope diversity that retains binding to TfR apical domain. Clones having mutations at these positions are shown in Table 4, and the sequences of the CH3 domains of these clones are set forth in SEQ ID NOS:40-44, 67, and 69-99.

### Monovalent polypeptide-Fab fusions

### Generation of monovalent TfR-binding polypeptide-Fab fusions

Although Fc domains naturally form homodimers, a series of asymmetric mutations known as "knobs-in-holes" can lead to preferential heterodimerization of two Fc fragments, where one Fc unit has the T139W knob mutation (which corresponds to position 366 using EU numbering scheme) and the other Fc unit has the T139S, L141A, and Y180V hole mutations (positions 366, 368, and 407, respectively using EU numbering scheme). In some embodiments, a modified CH3 domain of the invention comprises a Trp at position 139. In some embodiments, a modified CH3 domain of the invention comprises a Ser at position 139, an Ala at position 141 and a Val at position 180. Heterodimeric TfR-binding polypeptides were expressed in 293 or CHO cells by transient co-transfection of two plasmids *(i.e.,* a knob-Fc and a hole-Fc), while polypeptide-Fab fusions were expressed by transient co-transfection of three plasmids *(i.e.,* a knob-Fc-Fab heavy chain, a hole-Fc-Fab heavy chain, and a common light chain). Purification of secreted heterodimeric polypeptides or polypeptide-Fab fusions was performed identically to that for homodimers *(i.e.,* a two-column purification using Protein A followed by size-exclusion, and then concentration and buffer exchange if required). Mass-spectrometry or hydrophobic interaction chromatography was used to determine the amount of heterodimer versus homodimer (e.g., knob-knob or hole-hole paired Fc's) formed. From typical preps, greater than 95% of polypeptides, and often greater than 98%, were heterodimers. For clarity, all monovalent TfR binders (Fc homodimers) generated in this fashion were named "ZZ.mono" where ZZ was the name of the polypeptide and ".mono" indicated monovalent TfR binding. For heterodimeric polypeptides and polypeptide-Fab fusions, the mutations that conferred TfR binding included the "knob" mutation, whereas a non-TfR-binding Fc region was used with the "hole" region, unless otherwise indicated. In some cases, additional mutations that alter Fc properties were also included in these constructs, such as L7A/L8A, M25Y/S27T/T29E, N207S, or N207S/M201L for modified FcyR or FcRn binding, respectively.

### Binding characterization of CH3C. mono Fc polypeptides

Binding of monovalent CH3C polypeptides was measured in an ELISA using a modification of the procedure described above. Streptavidin was coated on 96-well ELISA plates overnight at 1 µg/mL in PBS. After washing, the plates were blocked with 1% BSA in PBS, then biotinylated human or cyno TfR was added at 1 µg/mL and incubated for 30 minutes. After additional washing, polypeptides were added to the plates at serial dilutions, and incubated for 1 hour. The plates were washed and secondary antibody (*i*.*e*., anti-kappa-HRP, 1:5,000) was added for 30 minutes and the plates were washed again. The plates were developed with TMB substrate and quenched with 2N H₂SO₄ and then absorbance at 450 nm was read on a BioTek^{®} plate reader. Results are shown in FIG. 19, which directly compares standard (*i*.*e*., bivalent TfR-binding) and monovalent TfR-binding polypeptides. Ab204 is a high affinity anti-TfR control antibody.

Additional testing was performed for binding to 293F cells, which endogenously express human TfR, as well as CHO-K1 cells that were stably transfected with human TfR or cyno TfR (FIG. 20).

In general, substantially reduced binding to human TfR for monovalent polypeptides was observed as compared to bivalent polypeptides, and cyno binding was too weak to be detected in these assays for the monovalent polypeptides.

Next it was tested whether monovalent versions of CH3C polypeptides could internalize in human-TfR expressing HEK293 cells. Methods described above for internalization assays were used. As shown in FIG. 21, which compares bivalent and monovalent polypeptides, the monovalent peptides could also internalize, but the overall signal was weaker than for the respective bivalent versions, presumably due to the loss of binding affinity/avidity.

### Kinetics of binding for CH3C polypeptides measured by biolayer inferometry

Binding kinetics were determined for several monovalent and bivalent CH3C polypeptide variants, fused to anti-BACE1 Fabs, and compared to their bivalent equivalents using biolayer inferometry (*i.e.,* using an Octet^{®} RED system). TfR was captured on a streptavidin sensor, then CH3C polypeptides were bound and washed off. Sensograms were fitted to a 1:1 binding model; the K_{D} (app) value for bivalent polypeptides represented avid binding to the TfR dimer. The results are shown in Table 5 and FIGS. 22 and 23.

**Table 5. Kinetics for CH3C polypeptides using Octet^{®} Red**

| **Polypeptide** | **K_{D} (app) (nM) [human TfR]** | **K_{D} (app) (nM) [cyno TfR]** |
|---|---|---|
| CH3C.35.N163 | 67 | 374 |
| CH3C.35.N163.mono | 251 | n.d. |
| CH3C.35 | 59 | 934 |
| CH3C.35.mono | 483 | n.d. |
| CH3C.3.2-1 | 337 | 367 |
| CH3C.3.2-5 | 270 | 385 |
| CH3C.3.2-19 | 367 | 454 |

| | | |
|---|---|---|
| n.d. = not determined due to too low binding signal | | |

The polypeptides that were converted to monovalent format had significantly weaker K_{D} (app) values, due to loss of avidity. Clones CH3C.3.2-1, CH3C.3.2-5, and CH3C.3.2-19, which were previously shown to have similar human and cyno TfR binding by ELISA, also had very similar K_{D} (app) values between human and cyno TfR. An attempt was made to test the monovalent forms of these polypeptides, but the binding in this assay was too weak to calculate kinetic parameters.

### Example 3. Binding Characterization of Additional CH3C Variants Using Biacore^{™}

The affinity of clone variants for recombinant TfR apical domain was determined by surface plasmon resonance using a Biacore^{™} T200 instrument. Biacore^{™} Series S CM5 sensor chips were immobilized with anti-human Fab (human Fab capture kit from GE Healthcare). 5 µg/mL of polypeptide-Fab fusion was captured for 1 minute on each flow cell and serial 3-fold dilutions of human or cyno apical domain were injected at a flow rate of 30 µL/min at room temperature. Each sample was analyzed with a 45-second association and a 3-minute dissociation. After each injection, the chip was regenerated using 10 mM glycine-HCl (pH 2.1). Binding response was corrected by subtracting the RU from a flow cell capturing an irrelevant IgG at similar density. Steady-state affinities were obtained by fitting the response at equilibrium against the concentration using Biacore^{™} T200 Evaluation Software v3.1.

To determine the affinity of clone variants for recombinant TfR ectodomain (ECD), Biacore^{™} Series S CM5 sensor chips were immobilized with streptavidin. Biotinylated human or cyno TfR ECD was captured for 1 minute on each flow cell and serial 3-fold dilutions of clone variants were injected at a flow rate of 30 µL/min at room temperature. Each sample was analyzed with a 45-second association and a 3-minute dissociation. The binding response was corrected by subtracting the RU from a flow cell without TfR ECD at a similar density. Steady-state affinities were obtained by fitting the response at equilibrium against the concentration using Biacore^{™} T200 Evaluation Software v3.1.

The binding affinities are summarized in Table 6. Affinities were obtained by steady-state fitting.

**Table 6. Binding affinities for additional CH3C variants**

| **Clone** | **Human TfR (µM)** | **Cyno TfR (µM)** | **Human apical TfR (µM)** | **Cyno apical TfR (µM)** |
|---|---|---|---|---|
| CH3C.35.19.mono | 0.4 | 5.9 | 0.37 | 5.6 |
| CH3C.35.20.mono | 0.25 | 6.7 | 0.17 | 8 |
| CH3C.35.21.mono | 0.1 | 2.1 | 0.12 | 2.2 |
| CH3C.35.24.mono | 0.29 | 3.3 | 0.23 | 3 |
| CH3C.35.21.11.mono | 0.24 | 4 | 0.13 | 2.2 |
| CH3C.35.21.16.mono | 0.18 | 1.8 | 0.12 | 1.9 |
| CH3C.35.21.17.mono | 0.3 | 2.9 | 0.13 | 2.6 |
| CH3C.35.mono | 0.61 | >10 | 0.61 | >10 |
| CH3C.35.N153.mono | 0.42 | >10 | 0.95 | >10 |
| CH3C.35.bi | 0.22 | >2 | not tested | not tested |
| CH3C.35.N153.bi | 0.37 | 3.3 | not tested | not tested |
| CH3C.3.2-19.bi | 5.2 | 5.6 | not tested | not tested |
| CH3C.35.19.bi | 0.074 | 1.5 | not tested | not tested |
| CH3C.35.20.bi | 0.054 | 1.7 | not tested | not tested |
| CH3C.35.21.bi | 0.049 | 0.7 | not tested | not tested |
| CH3C.35.24.bi | 0.061 | 0.65 | not tested | not tested |

Additional CH3C variants CH3C.35.20.1.1, CH3C.35.23.2.1, CH3C.35.23.1.1, CH3C.35.S413, CH3C.35.23.3.1, CH3C.35.N390.1, and CH3C.35.23.6.1 were created and their binding affinities to human TfR were measured following the same protocol as previously described. The binding affinities of CH3C.35.20.1.1, CH3C.35.23.2.1, CH3C.35.23.1.1, CH3C.35.S413, CH3C.35.23.3.1, CH3C.35.N390.1, and CH3C.35.23.6.1 are 620 nM, 690 nM, 750 nM, 1700 nM, 1900 nM, 2000 nM, and 2100 nM, respectively.

### Example 4. Binding Characterization of CH3C Variants to FcRn

FcRn binding assays were performed using a FortèBio^{®} Octet^{®} RED384 instrument using FortéBio^{®} Streptavidin biosensors. Biotinylated recombinant BACE1 was diluted to a concentration of 10 µg/mL in kinetic buffer (obtained from FortéBio^{®}) and captured onto individual biosensors for 1 minute. A baseline was then established for 1 minute in kinetic buffer. 10 µg/mL of the polypeptide-Fab fusions (comprising anti-BACE1 Fab arms) were bound to the sensor tips in the presence or absence of 1 uM human TfR ECD. Recombinant human FcRn (pH5.5) binding to immobilized polypeptide-Fab fusion was analyzed with a 3-minute association and a 3-minute dissociation.

The sensograms obtained from these experiments (FIG. 24), indicate that polypeptide-Fab fusions variants bound to FcRn at acidic pH (pH 5.5) and that TfR binding did not appreciably interfere with FcRn binding.

### Example 5. Pharmacokinetic/Pharmacodynamic Characterization of CH3C Variants

This example describes pharmacokinetic/pharmacodynamic (PK/PD) characterization of CH3C variant polypeptides of the present invention in mouse plasma and brain tissue.

### Pharmacokinetics of CH3C variants in wild-type mouse plasma

Pharmacokinetics (PK) were tested for several CH3C variants in wild-type mice to demonstrate *in vivo* stability in a model lacking TfR-mediated clearance, as the polypeptide-Fab fusions bind only human TfR and not murine TfR. The study design is shown in Table 7 below. 6-8 week-old C57Bl6 mice were intravenously dosed and in-life bleeds were taken via submandibular-bleeds, at time points as indicated in Table 7. Blood was collected in EDTA plasma tubes, spun at 14,000 rpm for 5 minutes, and then plasma was isolated for subsequent analysis.

**Table 7. PK study design**

| **Group** | **Polypeptide** | **Time points** | **N** | **Dose (IV)** |
|---|---|---|---|---|
| 1A/1B | Ab 122 | A = 30min, 24h, 4d | A=2 | 12.3 mg/kg |
| | | B = 4h, 2d, 7d | B=3 | |
| 2A/2B | Ab 153 | A = 30min, 24h, 4d | A=2 | 11.4 mg/kg |
| | | B = 4h, 2d, 7d | B=3 | |
| 3A/3B | CH3C.35.163 mono (Ab153 fusion) | A = 30min, 24h, 4d | A=2 | 11.4 mg/kg |
| | | B = 4h, 2d, 7d | B=3 | |
| 4A/4B | CH3C.3.2-19 (Ab153 fusion) | A = 30min, 24h, 4d | A=2 | 11.0 mg/kg |
| | | B = 4h, 2d, 7d | B=3 | |
| 5A/5B | CH3C.3.2-5 (Ab153 fusion) | A = 30min, 24h, 4d | A=2 | 10.5 mg/kg |
| | | B = 4h, 2d, 7d | B=3 | |
| 6A/6B | CH3C.3.2-1 (Ab153 fusion) | A = 30min, 24h, 4d | A=2 | 10.0 mg/kg |
| | | B = 4h, 2d, 7d | B=3 | |

Ab122 served as an anti-RSV control that has normal PK in mice. Ab153 served as an anti-BACE1 control that has normal PK in mice. The Fab arms of Ab153 were fused to the polypeptides in this study.

Polypeptide concentrations in mouse plasma were quantified using a generic human IgG assay (MSD^{®} human IgG kit #K150JLD-4) following the manufacturer's instructions. Briefly, precoated plates were blocked for 30 minutes with MSD^{®} Blocker A. Plasma samples were diluted 1:2,500 using a Hamilton^{®} NIMBUS liquid handler and added in duplicate to the blocked plates. Dosing solutions were also analyzed on the same plate to confirm the correct dosage. The standard curve, 0.78-200 ng/mL IgG, was fit using a four-parameter logistic regression. FIG. 25 and Table 8 show the analysis of these data. All of the CH3C polypeptide variants had clearance and half-life values comparable to the standard Ab122, except for CH3C.3.2-5, which had substantially faster clearance and a shorter half-life. Interestingly, this variant was a point mutant of CH3C.3.2-19 (N163D), the latter of which had a normal PK profile.

**Table 8. PK parameters for CH3C polypeptide-Fab fusions**

| **Polypeptide** | **Clearance (mg/day/kg)** | **Half-life (days)** |
|---|---|---|
| Ab122 | 6.12 | 9.12 |
| Ab153 | 9.11 | 4.74 |
| CH3C.35.N163 mono (Ab153 fusion) | 8.44 | 5.35 |
| CH3C.3.2-19 (Ab153 fusion) | 10.3 | 5.42 |
| CH3C.3.2-5 (Ab153 fusion | 21.0 | 1.90 |
| CH3C.3.2-1 (Ab153 fusion) | 9.25 | 4.65 |

### Additional PK study in wild-type mouse

A second PK study was conducted in wild-type mice according to the study design in Table 9 below (all polypeptide-Fab fusions to Ab153 Fab):

**Table 9**

| **Polypeptide** | **Dose (mg/kg)** | **Timepoint** | **n/group** |
|---|---|---|---|
| Ab153 | 10 | 0.5h, 1d, 4d, 7d | 3 |
| CH3C.35.21.mono | 10 | 0.5h, 1d, 4d, 7d | 3 |
| CH3C.35.24.mono | 10 | 0.5h, 1d, 4d, 7d | 3 |
| CH3 C.35.21.16.mono | 10 | 0.5h, 1d, 4d, 7d | 3 |
| CH3 C.35.21.17.mono | 10 | 0.5h, 1d, 4d, 7d | 3 |
| CH3C.35.20.bi | 10 | 0.5h, 1d, 4d, 7d | 3 |
| CH3C.35.21.bi | 10 | 0.5h, 1d, 4d, 7d | 3 |

Mice and samples were processed as described in the previous study. Data is provided in Table 10.

**Table 10. Clearance values for CH3C.35 polypeptide-Fab fusions**

| **Test polypeptide** | **Clearance (mL/day/kg)** |
|---|---|
| Ab 153 | 9.53 |
| CH3C.35.21.mono | 8.99 |
| CH3C.35.24.mono | 9.00 |
| CH3C.35.21.16.mono | 11.6 |
| CH3C.35.21.17.mono | 10.9 |
| CH3C.35.20.bi | 7.13 |
| CH3C.35.21.bi | 11.6 |

As is apparent from the clearance values, these polypeptide-Fab fusions exhibited similar clearance in wild-type mice as compared with a standard control antibody.

### PK/PD evaluation of monovalent CH3C.35.N163 in wild-type mouse brain tissue

Transgenic mice expressing human *Tfrc* apical domain within the murine *Tfrc* gene were generated using CRISPR/Cas9 technology. The resulting chimeric TfR was expressed *in vivo* under the control of the endogenous promoter.

Chimeric huTfR^{apical} heterozygous mice (n=4/group) were intravenously dosed with 42 mg/kg of either Ab153 or monovalent CH3C.35.N163, and wild-type mice (n=3) were dosed intravenously with 50 mg/kg of control human IgG1. Ab153 served as a control that has normal PK in mice. All mice were perfused with PBS 24 hours post-dose. Prior to perfusion, blood was collected in EDTA plasma tubes via cardiac puncture and spun at 14,000 rpm for 5 minutes. Plasma was then isolated for subsequent PK and PD analysis. Brains were extracted after perfusion and hemi-brains were isolated for homogenization in 10x by tissue weight of 1% NP-40 in PBS (for PK) or 5 M GuHCl (for PD).

FIG. 26 shows the results of the brain PK study. Uptake was greater in the monovalent CH3C.35.N163 group than the Ab153 and control human IgG1 groups.

### Brain and plasma PKPD of polypeptide-Fab fusions in hTfR^{apical+/+} mice: CH3C.35.21 and CH3C.35.N153

Homozygous hTfR^{apical+/+} mice were intravenously injected with 50 mg/kg of either anti-BACE1 antibody Ab153, anti-TfR/BACE1 bispecific antibody Ab116, CH3C.35.21.mono fused to Ab153 Fab, or CH3C.35.N153.bi fused to Ab153 Fab, as indicated in the study design in Table 11. In this study, all Fc's had LALAPG mutations to remove effector functions.

**Table 11. Study design for single point brain and plasma PKPD study**

| **Polypeptide** | **hTfR affinity (nM)** | **Dose (mg/kg)** | **Timepoint (day)** | **n/group** |
|---|---|---|---|---|
| Ab 153 | n/a | 50 | 1 | 8 |
| Ab116 | 330 | 50 | 1 | 8 |
| CH3C.35.21.mono | 160 | 50 | 1 | 8 |
| CH3C.35.N153.bi | 370 | 50 | 1 | 8 |

After 24 hours, blood was collected via cardiac puncture and the mice were perfused with PBS. Brain tissue was homogenized in 10x tissue weight of lysis buffer containing 1% NP-40 in PBS. Blood was collected in EDTA tubes to prevent clotting and spun at 14,000 rpm for 7 minutes to isolate plasma. Polypeptide concentrations in mouse plasma and brain lysates were quantified using a generic human IgG assay (MSD human IgG kit #K150JLD) following the manufacturer's instructions. Briefly, pre-coated plates were blocked for 30 minutes with MSD Blocker A. Plasma samples were diluted 1:10,000 using a Hamilton Nimbus liquid handler and added in duplicate to the blocked plates. Brain samples were homogenized in 1% NP40 lysis buffer and lysates diluted 1:10 for PK analysis. Dosing solutions were also analyzed on the same plate to confirm the correct dosage. The standard curve, 0.78 - 200 ng/mL IgG, was fit using a four-parameter logistic regression.

After 24 hours, the plasma levels of TfR-binding polypeptides were lower than the levels for anti-BACE1, likely due to clearance of this antibody via binding to peripherally-expressed hTfR^{apical} (FIG. 27A). In brain, there was a signficant increase in the concentration of anti-TfR/BACE1 compared to anti-BACE1 (FIG. 27B). The greatest increase was observed for CH3C.35.21.mono, but brain uptake was also significantly improved as compared to anti-BACE with CH3C35.N153.bi. The significant accumulation of the engineered TfR-binding polypeptides was due to TfR-mediated transcytosis at the blood-brain barrier, thus validating the utility of engineering TfR binding into the Fc region.

BACE1 inhibition of amyloid precursor protein APP cleavage was used as a pharmacodynamic readout of antibody activity in plasma and brain. Brain tissue was homogenized in 10x tissue weight of 5 M guanidine-HCl and then diluted 1:10 in 0.25% casein buffer in PBS. Mouse Aβ40 levels in plasma and brain lysate were measured using a sandwich ELISA. A 384-well MaxiSorp plate was coated overnight with a polyclonal capture antibody specific for the C-terminus of the Aβ40 peptide (Millipore #ABN240). Casein-diluted guanidine brain lysates were further diluted 1:2 on the ELISA plate and added concurrently with the detection antibody, biotinylated M3.2. Plasma was analyzed at a 1:5 dilution. Samples were incubated overnight at 4 °C prior to addition of streptavidin-HRP followed by TMB substrate. The standard curve, 0.78 - 50 pg/mL msAβ40, was fit using a four-parameter logistic regression.

Plasma amyloid beta-protein (Abeta) was reduced to a similar extent for all polypeptides, as compared to untreated wild-type mice (FIG. 28A), due to the presence of anti-BACE1 Fab arms on all polypeptides. Compared to anti-BACE1, treatment with TfR-binding polypeptides resulted in an increased reduction of Abeta in hTfR^{apical+/+} mice, indicating BACE1 target engagement in the brain was achieved (FIG. 28B). The level of target engagement in brain was similar for the engineering polypeptide fusions and the anti-TfR/BACE1 bispecific antibody.

### Brain and plasma PKPD of polypeptide-Fab fusions in hTfR^{apical+/+} mice: CH3C.35.21, CH3C.35.20, CH3C.35, CH3C.35.23, CH3C.35.23.3

To evaluate the impact of TfR binding affinity for PK and brain uptake, anti-BACE1 Ab153 and TfR-binding polypeptide fusions (CH3C.35.21:Ab153, CH3C.35.20:Ab153, CH3C.35:Ab153 fusions) were generated that differed in their binding affinity to apical human TfR as measured by Biacore. The binding affinities of CH3C.35.21:Ab153, CH3C.35.20:Ab153, CH3C.35:Ab153 fusions to human TfR are 100 nM, 170 nM and 620 nM, respectively. hTfR^{apical+/+} knock-in mice were systemically administered either Ab153 or the polypeptide-Fab fusions at 50 mg/kg, and plasma PK and brain PKPD was evaluated at 1, 3, and 7 days post-dose. Brain and plasma PKPD analysis was conducted as described in the previous section. Due to expression of TfR on peripheral tissues, CH3C.35.21:Ab153, CH3C.35.20:Ab153, and CH3C.35:Ab153 fusions exhibited faster clearance in plasma as compared to Ab153 alone, consistent with target-mediated clearance and indicative of *in vivo* TfR binding (FIG. 39A). Impressively, brain concentrations of CH3C.35.21:Ab153, CH3C.35.20:Ab153, and CH3C.35:Ab153 fusions were significantly increased compared to Ab153, achieving a maximum brain concentration of more than 30 nM at 1 day post-dose, compared to only about 3 nM for Ab153 at this same time point (FIG. 39B). The increase in brain exposure of CH3C.35.21:Ab153, CH3C.35.20:Ab153, and CH3C.35:Ab153 fusions resulted in about 55-60% lower endogenous mouse Aβ levels in brains of mice compared to Aβ levels in mice dosed with Ab153 (FIG. 39C). The lower brain Aβ levels were sustained while concentrations of CH3C.35.21:Ab153, CH3C.35.20:Ab153, and CH3C.35:Ab153 fusions remained elevated in brain, and returned to levels similar to Ab153 treated mice at when exposure was reduced by day 7. The reduction in brain exposure over time correlated with a reduction in peripheral exposure of CH3C.35.21:Ab153, CH3C.35.20:Ab153, and CH3C.35:Ab153 fusions, providing a clear PK/PD relationship *in vivo* (compare FIGS. 39A and 39C). Additionally, total brain TfR levels were comparable for Ab153-treated and polypeptide-Fab fusion-treated mice after this single high dose, indicating no significant impact of increased brain exposure of the polypeptide-Fab fusions to TfR expression in brain (FIG. 39D).

To further evaluate the relationship between PK and brain uptake with a wider affinity range of TfR-binding polypeptide-Fab fusions, additional fusions with a wider affinity range for hTfR binding was generated. The binding affinities of CH3C.35.23:Ab153 and CH3C.35.23.3:Ab153 fusions to human TfR are 420 nM and 1440 nM, respectively. hTfR^{apical+/+} knock-in mice were dosed as described above. Plasma PK and brain PKPD were evaluated at 1, 4, 7, and 10 days post-dose. Peripheral PK of the polypeptide-Fab fusions were hTfR affinity-dependent, where the higher affinity CH3C.35.23:Ab153 fusion exhibited faster clearance compared to the much lower affinity CH3C.35.23.3:Ab153 fusion (FIG. 40A). Both CH3C.35.23:Ab153 and CH3C.35.23.3:Ab153 fusions had significantly greater brain exposure than compared to Ab153 alone, with CH3C.35.23:Ab153 achieving about 36 nM in brain at 1 day post-dose (FIG. 40B). Despite similar plasma concentrations, this maximum brain uptake of CH3C.35.23.3:Ab153 fusion was lower than that of CH3.35.23:Ab153 fusion, likely due to the about 3.5-fold lower affinity of the latter fusion for hTfR. Interestingly, because the lower affinity fusion provided a more sustained peripheral exposure by day 10, its brain exposure was also higher than that of the higher affinity CH3C.35.23 :Ab 153 fusion. This illustrates a trade-off of lower brain Cₘₐₓ but more sustained PK over time for lower affinity TfR-binding polypeptide-Fab Fusions. Significantly lower concentrations of Aβ40 was observed in brains of mice dosed with the anti-BACE1 polypeptide fusions compared to anti-BACE1 alone (FIG. 40C). This duration of Aβ40 reduction was consistent with levels of huIgG1 exposure in brain over time (FIG. 40B). Impressively, mice dosed with CH3C.35:Ab153 fusion exhibited a prolonged brain Aβ40 reduction out to 7-10 days after a single dose. Total brain TfR levels were comparable between mice dosed with Ab153 versus CH3C.35:Ab153 fusion at 1 day post-dose (FIG. 40D). Together these data demonstrate that TfR-binding polypeptide fusion can increase brain exposure of anti-BACE1 to significantly reduce brain Aβ40 after a single dose.

### Example 6. CH3C.18 Fc and Transferrin Receptor Apical Domain Crystallization

This example describes the crystallization and analysis of the binding interface between CH3C.18 and the apical domain of the transferrin receptor (TfR-AD).

### Expression

The apical domain of human transferrin receptor (TfR-AD) and an engineered human Fc (CH3C.18 Fc) were expressed (SEQ ID NOS:104 and 105, respectively) in Expi293 cells at the initial cell density of 2.5 × 10⁶ cells/mL. Expressions were performed in volumes of 200 mL or more, as necessary. Kifunensine, a glycosylation inhibitor, was added 20 hours post transfection at a final concentration of 25 µM. Expression cultures were collected 3 to 4 days post transfection, when cell viability had significantly decreased.

### Purification

Expressed TfR-AD and CH3C.18 Fc were purified with protein A and Ni-NTA resins, respectively, followed by size-exclusion chromatography on a Superdex200 26/60 gel filtration column. The following buffers were used:
Protein A wash buffer: 20 mM Hepes pH 7.4, 100 mM NaCl;
Protein A elution buffer: 30mM glycine pH 2.5 (the eluate was collected into a tube containing 1M Tris, pH 9.0 to immediately neutralize the eluate);
Ni-NTA wash buffer: 30 mM Tris pH 8.0, 10 mM imidazole, and 200 mM NaCl;
Ni-NTA elution buffer: 30mM Tris pH 8.0, 200 mM NaCl, and 250mM imidazole; and
Size-exclusion buffer (SEC): 30 mM HEPES pH 7.5, 200 mM NaCl, and 3% glycerol.

### Complex formation and purification

Purified TfR-AD and CH3C.18 Fc were mixed with an excess of apical domain, incubated at room temperature for 1 hour, and the complex was purified using size-exclusion chromatography on a Superdex200 26/60 gel-filtration column using the previously mentioned SEC buffer. The sizing gave two major peaks as expected; one corresponded to the complex (retention volume = 180 ml) and the other one corresponded to the excess apical domain (retention volume = 240 ml). The peak fractions were analyzed by Coomassie stained SDS-PAGE gel (FIG. 29).

### Crystallization

Initial crystallization screening of the complex was performed by the sitting drop vapor diffusion method at 15 °C and room temperature (RT) at 8.5 mg/mlL protein concentration. Showers of thin needles of crystals were observed in the condition that contained 25% PEG 3350, 0.1M Tris pH 8.5 and 0.2M MgCh. These crystals were used to seed in the same condition but at 20% PEG 3350 to produce single thin needles of mountable size.

### X-ray data collection

Crystals were flash-cooled by direct immersion in liquid nitrogen using the crystallization mother liquor supplemented with 20% (v/v) ethylene glycol. X-ray intensity data were collected at the SER-CAT beam line of the Advanced Photon Source (APS) using a Rayonix 300 high speed detector. Crystals were diffracted to 3.6 Å, and belonged to the hexagonal space group *P6*₄ with two complex molecules in the asymmetric unit (Table 12). Data were indexed, integrated, and scaled using the program HKL2000. Data collected from two crystals were merged to produce 3.6 Å data.

**Table 12, Crystal data for CH3C.18 Fc-TfR-AD complex structure**

| **Name/code** | | **CH3C.18 Fc-TfR-AD complex** |
|---|---|---|
| **Cell dimensions** | a (Å) | 124.3 |
| | b | 124.3 |
| | c | 113.1 |
| | α (°) | **90.0** |
| | β | **90.0** |
| | γ | **120.0** |
| **Space group** | | P6₄ |
| **Resolution range (Å)** | Overall | 50-3.6 |
| | Last shell | 3.71-3.6 |
| **Number of unique reflections** | | 11,259 |
| **Completeness (%)** | (Overall/Last shell) | 95.9/74.1 |
| **R_{merge}¹** | (Overall/Last shell) | 20/93 |
| **Refinement Statistics** | Resolution (Å) | 50-3.6 |
| | R factor²/Rfree (%) | 30/39 |

| | | |
|---|---|---|
| ¹R_{merge} = ∑ⱼ(\|Iₕ-<I>ₕ\|)/ ∑Iₕ, where <Iₕ> is the average intensity over symmetry equivalents ²R-factor =∑\|F_{obs}-F_{calc}\|/ Σ\|F_{obs}\| | | |

### Structure determination and refinement

The crystal structure of the complex was determined by molecular replacement with PHASER using the CH3C.18 Fc dimer and TFR-AD monomer as the initial search models. The model was refined by rigid-body refinement followed by restrained refinement using REFMAC. All crystallographic calculations were performed with the CCP4 suite of programs (www.ccp4.ac.uk/). Model building of the complex into the electron density was done using the graphics program COOT. The electron density for the complex molecule was good, especially at the CH3C.18 Fc-TfF-AD interface (2Fo-Fc map contoured to 1.2 sigma level). After iterative model building and refinement, high R and freeR (R/freeR=0.30/0.39) were noticed due to the low resolution of the data and disordered CH2 domain. The disorder of the CH2, as found in other available Fc structures, was due to the flexible elbow angle between the CH2 and CH3 domains.

### Binding interface interactions

The binding interface between CH3C.18 Fc and TfR-AD is depicted in FIGS. 30A-30B and FIGS. 31A-31B. As shown in FIGS. 32A-32B, interactions were observed between:
Trp154 of CH3C.18 and Arg208 of TfR-AD;
Glu155 of CH3C.18 and Arg208 of TfR-AD;
Ser156 of CH3C.18 and Arg208 and Leu212 of TfR-AD;
Leu157 of CH3C.18 and Ser 199 and Asn215 of TfR-AD;
His159 of CH3C.18 and Lys188, Ser199, and Arg208 of TfR-AD;
Val160 of CH3C.18 and Gly207 and Arg208 of TfR-AD;
Trp161 of CH3C.18 and Arg208, Val210, and Leu212 of TfR-AD;
Ala162 of CH3C.18 and Arg208 of TfR-AD;
Val163 of CH3C.18 and Leu209 of TfR-AD;
Ser188 of CH3C.18 and Tyr211 of TfR-AD;
Thr189 of CH3C.18 and Tyr211 and Leu212 of TfR-AD;
Gln192 of CH3C.18 and Lys158 and Glu294 of TfR-AD;
Trp194 of CH3C.18 and Leu212, Val213, Glu214, and Asn215 of TfR-AD; and
Phe196 of CH3C.18 and Arg208 of TfR-AD.

Furthermore, as described in the section titled "Paratope Mapping" of Example 2 and as shown in FIGS. 32A-32B, several positions outside of the CH3C register also participate in binding to TfR.

### Example 7. CH3C.35 Fc and Transferrin Receptor Apical Domain Crystallization

This example describes the crystallization and analysis of the binding interface between CH3C.35 and the apical domain of the transferrin receptor (TfR-AD).

### Expression

The apical domain of human transferrin receptor (TfR-AD) and an engineered human Fc (CH3C.35 Fc) were expressed (SEQ ID NOS: 104 and 181, respectively) in CHO cells at an initial cell density of 2.5 × 10⁶ cells/mL. Expressions were performed in volumes of 500 mL or more, as necessary. Expression cultures were collected 3 to 4 days post transfection, when cell viability had significantly decreased.

### Purification

Expressed TfR-AD and CH3C.35 Fc were purified with protein A (Genescript) and Ni-NTA (Sigma) resins, respectively, followed by size-exclusion chromatography on a Superdex200 26/60 gel filtration column. The following buffers were used:
Protein A elution buffer: 30 mM glycine pH 2.5 (the eluate was collected into a tube containing 1 M Tris, pH 9.0 to immediately neutralize the eluate);
Ni-NTA elution buffer: 30 mM Tris pH 8.0, 200 mM NaCl, and 250 mM imidazole; and
Size-exclusion buffer (SEC): 30 mM HEPES pH 7.5, 150 mM NaCl, 50 mM KCl, 3% glycerol, and 0.01% sodium azide.

### Complex formation and purification

Purified TfR-AD and CH3C.35 Fc were mixed with an excess of apical domain, incubated at room temperature for 1 hour, and the complex was purified using size-exclusion chromatography on a Superdex200 26/60 gel filtration column using the previously mentioned SEC buffer.

### Crystallization

Initial crystallization screening of the complex was performed by the sitting drop vapor diffusion method at 4 °C, 15 °C, and room temperature (RT). Showers of thin needles of crystals were observed in the condition that contained 25% PEG 3350, 0.1 M Bis-Tris pH 6.5, and 0.2 M LiSO₄. These crystals were used to seed in the same condition but at 20% PEG 3350 to produce single thin needles and the seeding was repeated sequentially four times to produce crystals of mountable size.

### X-ray data collection

Crystals were flash-cooled by direct immersion in liquid nitrogen using the crystallization mother liquor supplemented with 20% (*v*/*v*) ethylene glycol. X-ray intensity data were collected at IO4 beam line of the Diamond Light Source (DLS) using PILATUS detector. Micro focus beam of size 5 micron was used for the data collection. Crystals were diffracted to 3.38 Å, and belonged to the hexagonal space group *P6*₄ with two complex molecules in the asymmetric unit (Table 13). Data were indexed, integrated, and scaled using the CCP4 suite programs (Xia2- XDS and XSCALE).

**Table 13. Crystal data for CH3C.35 Fc-TfR-AD complex structure**

| **Name/code** | | **CH3C.35 Fc-TfR-AD complex** |
|---|---|---|
| **Cell dimensions** | a (Å) | 126.4 |
| | b | 126.4 |
| | c | 113.8 |
| | α (°) | **90.0** |
| | β | **90.0** |
| | γ | **120.0** |
| **Space group** | | P6₄ |
| **Resolution range (Å)** | Overall | 50-3.38 |
| | Last shell | 3.44-3.38 |
| **Number of unique reflections** | | 14,541 |
| **Completeness (%)** | (Overall/Last shell) | 100/99.7 |
| **R_{merge}¹** | (Overall/Last shell) | 31/152 |
| **Refinement Statistics** | Resolution (Å) | 50-3.38 |
| | R factor²/Rfree (%) | 27/35 |

| | | |
|---|---|---|
| ¹R_{merge} = ∑ⱼ(\|Iₕ-<I>ₕ\|)/ ∑Iₕ, where <Iₕ> is the average intensity over symmetry equivalents ²R-factor =∑\|F_{obs}-F_{calc}\|/ ∑\|F_{obs}\| | | |

### Structure determination and refinement

The crystal structure of the complex was determined by molecular replacement with PHASER using the CH3C.35 Fc-AD TfR complex as the search model. The model was refined by rigid-body refinement followed by restrained refinement using REFMAC. All crystallographic calculations were performed with the CCP4 suite of programs. Model building of the complex into the electron density was done using the graphics program COOT. The electron density for the complex molecule was good, especially at the CH3C.35 Fc-TfF-AD interface.

### Binding interface interactions

The binding interface between CH3C.35 Fc and TfR-AD is depicted in FIGS. 34A-34C. FIG. 34A shows the complex of CH3C.35 Fc and TfR-AD at 3.4 Å. FIG. 34B shows residue W161 in CH3C.35 Fc is stabilized by residues L209, L212, and Y211 in TfR-AD. FIG. 34C shows a salt bridge between residue E160 in CH3C.35 Fc and residue R208 in TfR-AD as a central binding interaction, which may partially account for the difference in binding affinity of the Fc polypeptide to human TfR (Arg at position 208) and to cynomolgus TfR (Gly at position 208). FIG. 35A shows an overlaid structure between the CH3C.35 Fc and TfR-AD complex and the CH3C.18 Fc and TfR-AD complex (described in Example 6), demonstrating that there is no significant Fc backbone conformational change between CH3C.35 and CH3C.18. FIG. 35B shows an enlarged view of the overlaid structure in FIG. 35A. Residues 206-212 in TfR-AD of the CH3C.35 Fc/TfR-AD complex adopted different conformations from the residues in the TfR-AD of the CH3C.18 Fc/TfR-AD complex. Residue R208 in TfR-AD appeared buried in surface of the CH3C.18 Fc/TfR-AD complex, but appeared solvent exposed in the CH3C.35 Fc/TfR-AD complex. Further, residue L209 in TfR-AD of the CH3C.35 Fc/TfR-AD complex appeared rotated 180° and bound to the surface, but appeared away from the surface in the in the CH3C.18 Fc/TfR-AD complex.

As shown in FIGS. 36A and 36B, interactions were observed between:
Thr159 of CH3C.35 and Gly207, Arg208, Lys188, and Leu209 of TfR-AD;
Glu160 of CH3C.35 and Arg208 and Leu209 of TfR-AD;
Ser162 of CH3C.35 and Arg208 and Leu209 of TfR-AD;
Ser156 of CH3C.35 and Leu209 of TfR-AD;
Trp161 of CH3 C.3 5 and Leu209, Tyr211, and Leu212 of TfR-AD;
Glu189 of CH3C.35 and Tyr211 and Leu212 of TfR-AD;
Phe194 of CH3C.35 and Leu212, Asn215, and Val213 of TfR-AD;
Tyr157 of CH3C.35 and Leu212, Asn215, and Ser199 of TfR-AD;
Gln192 of CH3C.35 and Val213 and Lys158 of TfR-AD; and
Phe196 of CH3 C.3 5 and Val213 and Leu212 of TfR-AD.

Furthermore, as described in the section titled "Paratope Mapping" of Example 2 and as shown in FIGS. 36A and 36B, several positions outside of the CH3C register also participate in binding to TfR.

### Example 8. Pharmacokinetic/Pharmacodynamic Studies of Fc-Fab Fusion Polypeptides Comprising CH3C Variants in Cynomolgus Monkeys

This example describes pharmacokinetic/pharmacodynamic (PK/PD) characterization of Fc-Fab fusions comprising CH3C variant polypeptides of the present invention in cynomolgus monkeys.

### Study design

A single 30 mg/kg dose of Ab122 (an anti-RSV antibody as control IgG), Ab153 (an anti-BACE1 antibody), Ab210 (anti-TfR/BACE1 bispecific antibody), or Fc-Fab fusion polypeptides comprising CH3C variant polypeptides fused to the Fab domain of Ab153 were intravenously administered in male cynomolgus monkeys 2-4 years old to evaluate plasma PK, plasma PD (Aβ40), and cerebrospinal fluid (CSF) PD (Aβ40) over the course of 29 days (n=4/group). To establish baseline, pre-dose CSF and blood samples were taken from each animal 7 days prior to dosing. After dosing, CSF was collected via an IT-L catheter at 12, 24, 48, 72, and 96 hours post-dose, and on study days 8, 11, 15, 18, 22, 25, and 29 for PD analysis. Blood samples were collected for plasma and serum PK at 0.25, 1, 6, 12, 24, 72 hours post-dose, and on study days 8, 11, 15, 18, 22, 25, and 29.

Table 14 shows an outline of the study design. "CH3C.35.21.16:Ab153" is a monovalent Fc-Fab fusion polypeptide comprising clone CH3C.35.21.16 fused to the Ab153 Fab domain. "CH3C.35.21:Ab153" is a monovalent Fc-Fab fusion polypeptide comprising clone CH3C.35.21 fused to the Ab153 Fab domain. "CH3C.35.9:Ab153" is a bivalent Fc-Fab fusion polypeptide comprising clone CH3C.35.21 fused to the Ab153 Fab domain. "CH3C.35.8:Ab153" is a bivalent Fc-Fab fusion polypeptide comprising clone CH3C.35.20 fused to the Ab153 Fab domain. "LALAPG" indicates that the antibody or Fc-Fab fusion polypeptide contains the mutations L7A, L8A, and P102G in the Fc sequence (as numbered with reference to SEQ ID NO:1). "LALAPG.YTE" indicates that the Fc-Fab fusion polypeptide contains the mutations L7A, L8A, P102G, M25Y, S27T, and T29E in the Fc sequence (as numbered with reference to SEQ ID NO:1).

**Table 14**

| **Treatment** | **Isotype** | **Cyno TfR full-length affinity (nM)** | **Cyno TfR apical affinity (nM)** | **Dose** | **N** | **Material (mg)** |
|---|---|---|---|---|---|---|
| Ab122 (control IgG) | huIgG1.LALAPG | -- | | 30 | 4 | 750 |
| Ab153 | huIgG1.LALAPG | -- | | 30 | 4 | 750 |
| Ab210 | huIgG1.LALAPG | 52 | 140 | 30 | 4 | 750 |
| CH3C.35.21.16:Ab153 (monovalent) | huIgG1.LALAPG | 1800 | 1900 | 30 | 4 | 750 |
| CH3C.35.21.16:Ab153 (monovalent) | huIgG1.LALAPG.YTE | 1800 | 1900 | 30 | 4 | 750 |
| CH3C.35.21:Ab153 (monovalent) | huIgG1.LALAPG.YTE | 2100 | 2200 | 30 | 4 | 750 |
| CH3C.35.9:Ab153 (bivalent) | huIgG1.LALAPG.YTE | 700 | | 30 | 4 | 750 |
| CH3C.35.8:Ab153 (bivalent) | huIgG1.LALAPG.YTE | 1700 | | 30 | 4 | 750 |

### Methods

### Human IgG PK assay

Antibody or Fc-Fab fusion polypeptide concentrations in cyno serum were quantified using a human IgG-specific sandwich ELISA. A 384-well MaxiSorp plate was coated overnight with an antibody specific for the Fc of human IgG. Serum samples were diluted 1:100, 1:1,000, 1:10,000, and 1:100,000 and added to the blocked plates. The detection antibody was a polyclonal anti-human IgG monkey-absorbed antibody. The standard curves were prepared for each antibody or Fc-Fab fusion polypeptide individually (48-200,000 pg/mL IgG) and the assay has a lower limit of quantification (LLOQ) in serum of 20 ng/mL.

### PD assays

Soluble APPα/β levels in cyno CSF were measured using a MesoScale Discovery (MSD) multiplex kit (MSD #K15120E). Two different antibodies specifically captured either sAPPα or sAPPβ, and then both analytes were detected with a SULFO-tag labeled anti-APP mouse monoclonal antibody. Cyno Aβ40 levels were measured using a MSD ultra-sensitive kit (MSD #K151FTE). This assay used the huAβ-specific 6E10 antibody as the capture and an anti-Aβ40 antibody specific for the C-terminus of the peptide as the detection molecule. Both assays were run according to the manufacturer's instructions. Briefly, precoated plates were blocked for 1 hour with MSD Blocker A. CSF samples were diluted 1:5 and added in duplicate to the blocked plates followed by an overnight incubation at 4 °C. Next, the respective detection antibodies were added and the plates read on a Sector S600 instrument. The standard curves, 0.92-3750 pg/mL huAβ40 and 0.1-100 ng/mL for both sAPPα/β, were fit using a four-parameter logistic regression. The assays had a LLOQ of 73 pg/mL for Aβ40 and 0.5 ng/mL for sAPPα/β.

### Results

Interim serum PK from the first 7 days post-dose showed the expected target-mediated clearance for Ab210 and CH3C.35.9:Ab153 due to their binding to TfR in the periphery (FIG. 37A). Both Ab153 and Ab210 antibodies, as well as CH3C.35.9:Ab153, resulted in a significant and sustained reduction in plasma Aβ40 compared to control IgG (FIG. 37B), confirming all three molecules were able to inhibit BACE1 activity *in vivo* to a similar extent. In the CSF, both Ab210 and CH3C.35.9:Ab153 were able to reduce CSF Aβ40 and sAPβ/sAPPα ratio to about 70% and about 75%, respectively, compared to control IgG (FIGS. 38A and 38B). Ab153, an anti-BACE1 antibody that does not bind TfR, showed minimal impact on CSF Aβ40 and sAPPβ/sAPPα ratio compared to control IgG. These results demonstrate that binding to TfR with a CH3C variant polypeptide (*e*.*g*., clone CH3C.35.9) enhances CNS penetration of an Fc-Fab fusion comprising the CH3C variant polypeptide fused to the Fab domain of an anti-BACE1 antibody (e.g., CH3C.35.9:Ab153) to inhibit CSF Aβ40 and sAPPβ/sAPPα production.

Serum PK, plasma Aβ, and CSF Aβ levels were also evaluated for four weeks following a single dose. Similar to what was observed in mouse, peripheral serum PK of TfR-binding Fc-Fab fusions (CH3C.35.21.16:Ab153 LALAPG, CH3C.35.21.16:Ab153 LALAPGYTE, and CH3C.35.21:Ab153 LALAPGYTE) exhibited faster clearance compared to Ab122 and Ab153 due to binding to TfR on peripheral tissues (FIG. 41A). Both Ab153 and CH3C:Ab153 fusion reduced plasma Aβ levels by greater than about 50% compared to control IgG_Ab122 (FIG. 41B). The maximum Aβ was similar between Ab153 and CH3C:Ab153 fusion, indicating that the Fc modifications did not affect ability of anti-BACE1 Fab to inhibit APP cleavage in vivo (FIG. 41B). The duration of plasma Aβ correlated with the exposure of Ab153 and CH3C:Ab153 over time. In the CSF, all three Fc-Fab fusions were able to significantly reduce both Aβ40 and sAPPβ/sAPPα ratio to about 70% compared to control IgG_Ab122, whereas no significant reduction was observed in animals dosed with Ab153 (FIGS. 41C and 41D). These results demonstrate that binding to TfR with a CH3C variant polypeptide (*e*.*g*., clone CH3C.35.21.16 and CH3C.35.21) enhances CNS penetration of an Fc-Fab fusion comprising the CH3C variant polypeptide fused to the Fab domain of an anti-BACE1 antibody (*e*.*g*., CH3C.35.21.16:Ab153 and CH3C.35.21:Ab153) to inhibit CSF Aβ40 and sAPPβ/sAPPα production.

Because of the high level of TfR expression on immature red blood cells, peripheral blood clinical pathology was evaluated throughout the course of the study to evaluate reticulocyte number, serum iron, and red blood cell count. The assessment of serum iron levels utilized a variation of the method using TPTZ [2,4,6-Tri-(2-pyridyl)-5-triazine] as the chromogen. In an acidic medium, transferrin-bound iron dissociated into free ferric ions and apo transferrin. Hydrochloric acid and sodium ascorbate reduced the ferric ions to the ferrous state. The ferrous ions then reacted with TPTZ to form a blue colored complex that was measured bichromatically at 600/800 nm. The increase in absorbance was directly proportional to the amount of transferrin bound iron present. This is performed on the Beckman/Olympus AU640e chemistry analyzer. Absolute reticulocytes and RBC morphology were analyzed by the Siemens Advia 120 automated hematology system. Fc-Fab fusions had no impact on reticulocyte number, as compared to their pre-dose values (FIG. 42A). Additionally, serum iron as well as red blood cell number were also not impacted (FIGS. 42B and 42C). Together these data indicate that modified TfR-binding Fc polypeptide-Fab fusions can safely and effectively increase brain exposure of antibodies in non-human primates to produce a robust pharmacodynamic response (*i*.*e*., CSF Aβ reduction).

### Example 9. Pharmacokinetic Analysis of CH3C.35 Containing M201L and N207S Mutations

This example describes that mutations M201L and N207S are compatible with CH3C.35.

In order to evaluate whether mutations that increase serum stability, M201L and N207S as numbered with reference to SEQ ID NO: 1 (M428L/N434S according to EU numbering; also referred to as "LS" mutations), are compatible with TfR-binding Fc modifications, human FcRn knock-in mice were dosed with Ab153_LALAPG, Ab153_LALA.LS, CH3C.35.21:Ab153_LALA.LS, or Ab153_LALAPG.YTE at 10 mg/kg. Plasma PK evaluation over 14 days showed a similar about 2-fold improvement for Ab153_LALA.LS, CH3C.35.21:Ab153_LALA.LS, and Ab153_LALAPG.YTE compared to Ab153_LALAPG without any serum stability mutations (FIGS. 43A and 43B). This indicates that the additional Fc mutations for TfR binding do not impact the ability of the LS mutations to improve huIgG1 half-life *in vivo.*

### Example 10. Single Amino Acid Substitution of CH3C.35.21

This example describes the construction of a library of CH3C.35.21 single amino acid mutants.

### Methods

A library of CH3C.35.21 mutants each containing a single amino acid substitution of CH3C.35.21 was constructed using Kunkel mutagenesis (Kunkel, Proc Natl Acad Sci U S A. 82(2):488-92, 1985). For each position of CH3C.35.21, W153, Y157, T159, E160, W161, S162, S163, K165, T186, K187, E188, E189, F194, S197, and S199, as numbered according to SEQ ID NO:1 (W380, Y384, T386, E387, W388, S389, S390, K392, T413, K414, E415, E416, F421, S424, and S426, as numbered according to the EU numbering scheme) were mutated individually to the codon NNK using degenerate mutagenic oligos. To avoid obtaining the original CH3C.35.21 clone in the library, the single-stranded DNA (ssDNA) Kunkel template encoded a wild-type IgG1 Fc was used. Two mutagenic oligos (one with an NNK and the other encoding the other CH3C.35.21 region) were used in combination so that when both oligos were incorporated it yielded the CH3C.35.21 amino acid sequence, but with an NNK codon at the desired library positon. Because the template is a wild-type Fc, a single oligo insertion or no oligo insertion will not bind TfR, therefore, these constructs were easily eliminated from any analysis. Similarly, stop codons arising from the NNK positon were excluded. Libraries were transfected into EBY100 yeast. Eight colonies were sequenced from each library to ensure the naïve library contains the desired position randomization.

The top approximately 10% of the circularly permuted TfR apical domain bound population measured by yeast display and flow cytometry, were collected at a TfR concentration providing the best range for distinguishing affinities. Sequences were obtained for 12 clones for each positon. For libraries with distinct populations, the same experiment was done with better defined high, medium, low gates. There were 36 clones sequenced for each collected population. Further, in order to compare the binding of a mutant to the binding of the corresponding mutant having the wild-type residue at the corresponding amino acid position, the amino acid at the same position was reverted back to the wild-type IgG1 residue using a mutagenic oligo in similar methods.

Table 15 shows the library of CH3C.35.21 mutants. Each mutant contained a single amino acid substitution of CH3C.35.21. For example, one mutant may contain W380E and the amino acids at the rest of the positions are the same as those in CH3C.35.21. The positions shown in Table 15 are numbered according to the EU numbering scheme.

**Table 15. CH3C.35.21 single amino acid mutants**

| **Position** | **380** | **384** | **386** | **387** | **388** | **389** | **390** | **413** | **415** | **416** | **421** | **424** | **426** |
|---|---|---|---|---|---|---|---|---|---|---|---|---|---|
| Wild-type Fc | E | N | Q | P | E | N | N | D | S | R | N | S | S |
| CH3C.35.21 | W | Y | T | E | W | S | S | T | E | E | F | S | S |
| Residues found to have affinity in the range: <190nM to about ∼500 nM | E | Y | T | E | W | S | S | T | S | E | F | S | S |
| | L | F | N | I | | A | N | H | D | R | H | T | C |
| | S | M | V | P | | I | R | S | G | | K | W | P |
| | V | P | | V | | T | T | | T | | Y | | M |
| | W | V | | | | V | | | P | | | | W |
| | Y | W | | | | | | | Q | | | | |
| | | | | | | | | | R | | | | |

### Example 11. Construction of CH3C.18 Variants

This example describes the construction of a library of CH3C.18 variants.

Single clones were isolated, and grown overnight in SG-CAA media supplemented with 0.2% glucose overnight to induce surface expression of CH3C.18 variants. For each clone, two million cells were washed three times in PBS+0.5% BSA at pH 7.4. Cells were stained with biotinylated target, 250 nM human TfR, 250 nM cyno TfR, or 250 nM of an unrelated biotinylated protein for 1 hour at 4 °C with shaking, then washed twice with the same buffer. Cells were stained with nuetravidin-Alexafluor647 (AF647) for 30 minutes at 4 °C, then washed twice again. Expression was measured using anti-c-myc antibody with anti-chicken- Alexfluor488 (AF488) secondary antibody. Cells were resuspended, and median fluorescence intensity (MFI) of AF647 and AF488 was measured on a BD FACS CantoII. MFI was calculated for the TfR-binding population for each population and plotted with human TfR, cyno TfR, or control binding (FIG. 44).

Table 16 shows the library of CH3C.18 variants. Each row represents a variant that contains the indicated amino acid substitutions at each position and the amino acids at the rest of the positions are the same as those in CH3C.18. The positions shown in Table 16 are numbered according to the EU numbering scheme.

**Table 16. CH3C.18 variants**

| **Position** | **384** | **386** | **387** | **389** | **390** | **391** | **413** | **416** | **421** |
|---|---|---|---|---|---|---|---|---|---|
| Wild-type Fc | N | Q | P | N | N | Y | D | R | N |
| CH3C.4 (CH3C.18.1) | V | T | P | A | L | Y | L | E | W |
| CH3C.2 (CH3C.18.2) | Y | T | V | S | H | Y | S | E | Y |
| CH3C.3 (CH3C.18.3) | Y | T | E | S | Q | Y | E | D | H |
| CH3C.1 (CH3C.18.4) | L | L | V | V | G | Y | A | T | W |
| CH3C.18 (CH3C.18.1.18) | L | H | V | A | V | Y | P | T | W |
| CH3C.3.1-3 (CH3C.18.3.1-3) | L | H | V | V | A | T | P | T | W |
| CH3C.3.1-9 (CH3C. 18.3.1-9) | L | P | V | V | H | T | P | T | W |
| CH3C.3.2-1 (CH3C.18.3.2-1) | L | H | V | V | N | F | P | T | W |
| CH3C.3.2-5 (CH3C.18.3.2-5) | L | H | V | V | D | Q | P | T | W |
| CH3C.3.2-19 (CH3C.18.3.2-19) | L | H | V | V | N | Q | P | T | W |
| CH3C.3.4-1 (CH3C.18.3.4-1) | W | F | V | S | T | T | P | N | F |
| CH3C.3.4-19 (CH3C.18.3.4-19) | W | H | V | S | T | T | P | N | Y |
| CH3C.3.2-3 (CH3C.18.3.2-3) | L | H | V | V | E | Q | P | T | W |
| CH3C.3.2-14 (CH3C.18.3.2-14) | L | H | V | V | G | V | P | T | W |
| CH3C.3.2-24 (CH3C.18.3.2-24) | L | H | V | V | H | T | P | T | W |
| CH3C.3.4-26 (CH3C.18.3.4-26) | W | T | V | G | T | Y | P | N | Y |
| CH3C.3.2-17 (CH3C.18.3.2-17) | L | H | V | V | G | T | P | T | W |

The amino acid substitutions for each clone described in the Tables (*e*.*g*., Table 4) dictate the amino acid substitutions at the register positions of that clone over the amino acids found in the sequence set forth in the Sequence Listing, in case of discrepancy.

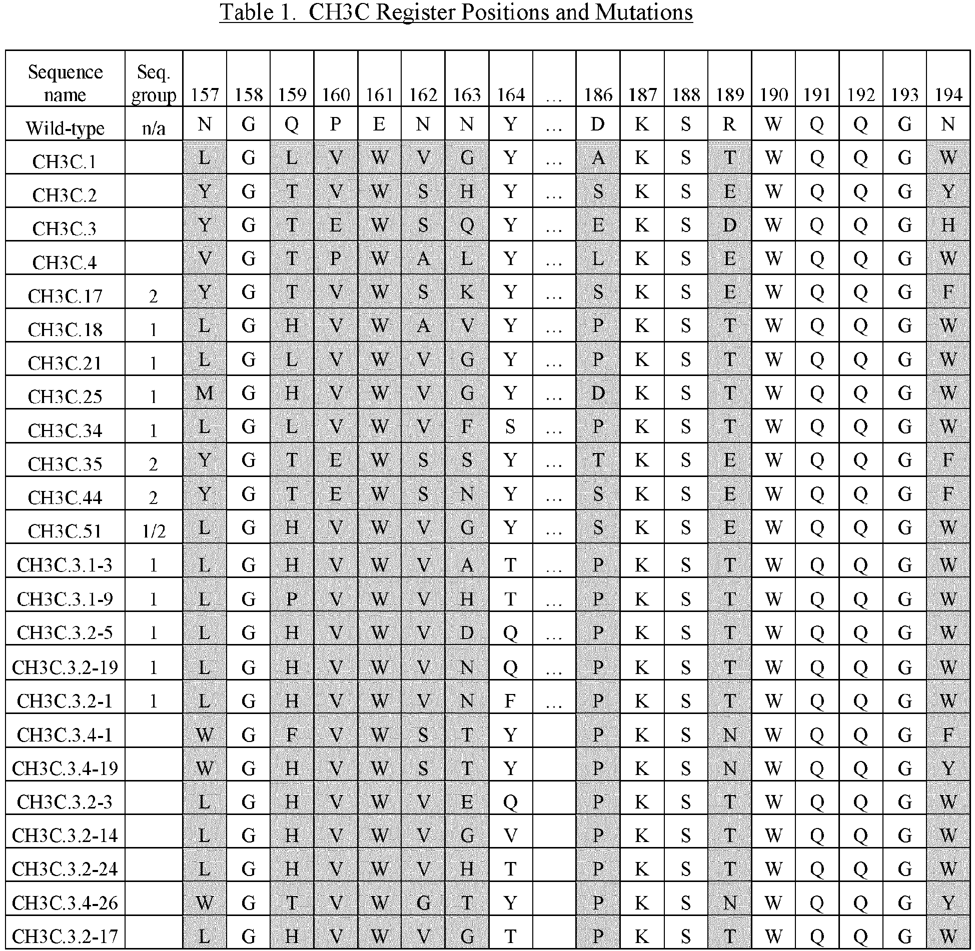

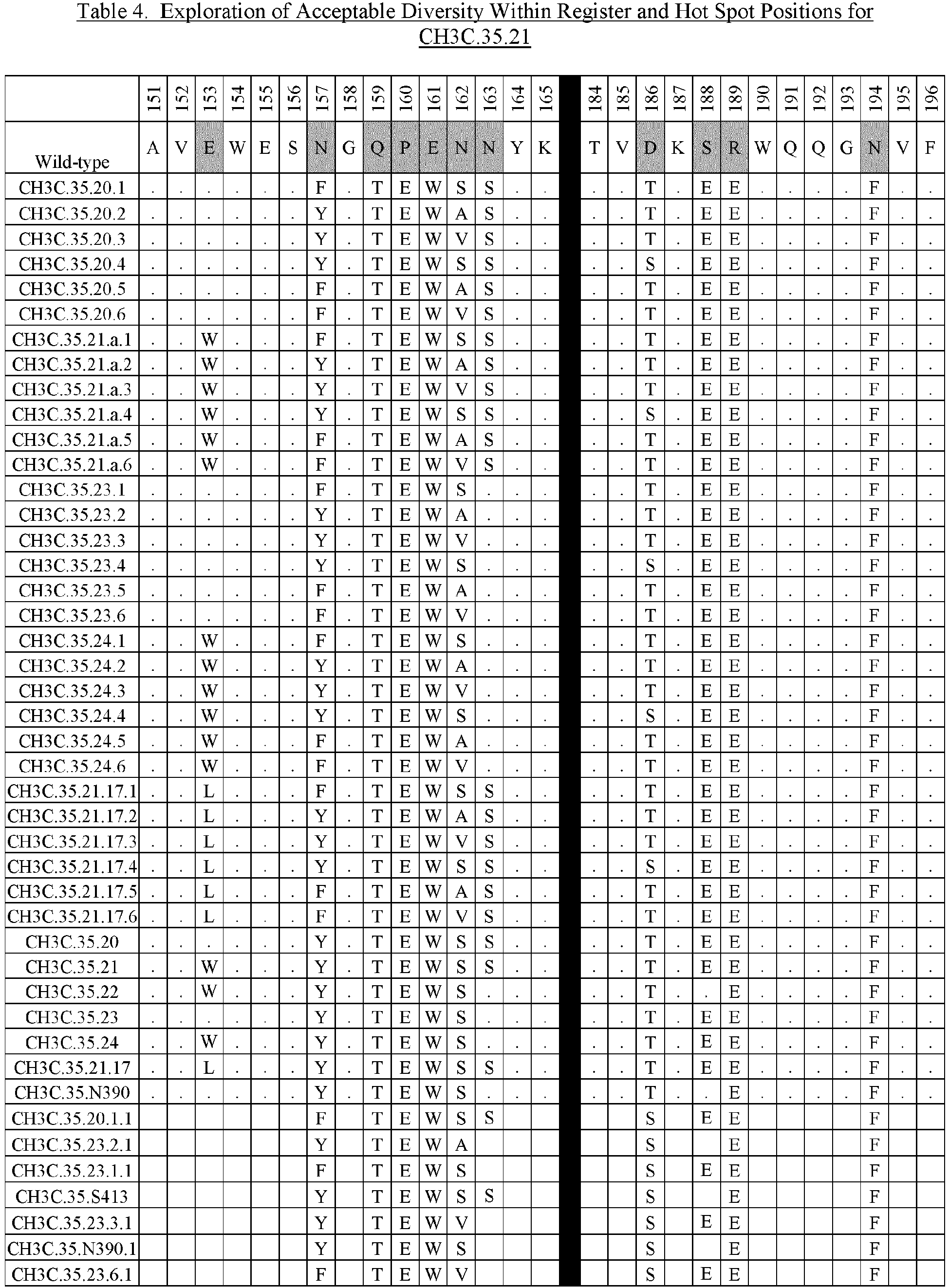

### Informal Sequence Listing

| **SEQ ID NO:** | **Sequence** | **Description** |
|---|---|---|
| 1 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESNGQPENNYKTTPPVLDSDGSFFLYSKLTVD KSRWQQGNVFSCSVMHEALHNHYTQKSLSLSPGK | Wild-type human Fc sequence amino acids 1-3 (PCP) are from a hinge region |
| 2 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAK | CH2 domain sequence, including three amino acids (PCP) at the N-terminus from the hinge region |
| 3 | GQPREPQVYTLPPSRDELTKNQVSLTCLVKGFYPSDIAVEWESNGQP ENNYKTTPPVLDSDGSFFLYSKLTVDKSRWQQGNVFSCSVMHEALH NHYTQKSLSLSPGK | CH3 domain sequence |
| 4 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESLGLVWVGYKTTPPVLDSDGSFFLYSKLTV AKSTWQQGWVFSCSVMHEALHNHYTQKSLSLSPGK | Clone CH3C.1 (Clone CH3C.18.4) |
| 5 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTVWSHYKTTPPVLDSDGSFFLYSKLTVS KSEWQQGYVFSCSVMHEALHNHYTQKSLSLSPGK | Clone CH3C.2 (Clone CH3C.18.2) |
| 6 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWSQYKTTPPVLDSDGSFFLYSKLTVE KSDWQQGHVFSCSVMHEALHNHYTQKSLSLSPGK | Clone CH3C.3 (Clone CH3C.18.3) |
| 7 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESVGTPWALYKTTPPVLDSDGSFFLYSKLTVL KSEWQQGWVFSCSVMHEALHNHYTQKSLSLSPGK | Clone CH3C.4 (Clone CH3C.18.1) |
| 8 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTVWSKYKTTPPVLDSDGSFFLYSKLTVS KSEWQQGFVFSCSVMHEALHNHYTQKSLSLSPGK | Clone CH3C.17 |
| 9 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESLGHVWAVYKTTPPVLDSDGSFFLYSKLTV PKSTWQQGWVFSCSVMHEALHNHYTQKSLSLSPGK | Clone CH3C.18 (Clone CH3C.18.1.18) |
| 10 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESLGLVWVGYKTTPPVLDSDGSFFLYSKLTV PKSTWQQGWVFSCSVMHEALHNHYTQKSLSLSPGK | Clone CH3C.21 |
| 11 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESMGHVWVGYKTTPPVLDSDGSFFLYSKLTV DKSTWQQGWVFSCSVMHEALHNHYTQKSLSLSPGK | Clone CH3C.25 |
| 12 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESLGLVWVFSKTTPPVLDSDGSFFLYSKLTVP KSTWQQGWVFSCSVMHEALHNHYTQKSLSLSPGK | Clone CH3C.34 |
| 13 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWSSYKTTPPVLDSDGSFFLYSKLTVT KSEWQQGFVFSCSVMHEALHNHYTQKSLSLSPGK | Clone CH3C.35 |
| 14 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWSNYKTTPPVLDSDGSFFLYSKLTVS KSEWQQGFVFSCSVMHEALHNHYTQKSLSLSPGK | Clone CH3C.44 |
| 15 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESLGHVWVGYKTTPPVLDSDGSFFLYSKLTV SKSEWQQGWVFSCSVMHEALHNHYTQKSLSLSPGK | Clone CH3C.51 |
| 16 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESLGHVWVATKTTPPVLDSDGSFFLYSKLTV PKSTWQQGWVFSCSVMHEALHNHYTQKSLSLSPGK | Clone CH3C.3.1-3 (Clone CH3C.18.3.1-3) |
| 17 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESLGPVWVHTKTTPPVLDSDGSFFLYSKLTVP KSTWQQGWVFSCSVMHEALHNHYTQKSLSLSPGK | Clone CH3C.3.1-9 (Clone CH3C.18.3.1-9) |
| 18 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESLGHVWVDQKTTPPVLDSDGSFFLYSKLTV PKSTWQQGWVFSCSVMHEALHNHYTQKSLSLSPGK | Clone CH3C.3.2-5 (Clone CH3C.18.3.2-5) |
| 19 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESLGHVWVNQKTTPPVLDSDGSFFLYSKLTV PKSTWQQGWVFSCSVMHEALHNHYTQKSLSLSPGK | Clone CH3C.3.2-19 (Clone CH3C.18.3.2-19) |
| 20 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESLGHVWVNFKTTPPVLDSDGSFFLYSKLTVP KSTWQQGWVFSCSVMHEALHNHYTQKSLSLSPGK | Clone CH3C.3.2-1 (Clone CH3C.18.3.2-1) |
| 21 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESLGHVWAVYKTTPPVLDSDGSFFLYSKLTV PKSTWQQGWVFSCSVMHEALHNHYTQKSLSLSPGK | Clone CH3C.18.E153W (CH3C.35.13) |
| 22 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESLGHVWAVYQTTPPVLDSDGSFFLYSKLTV PKSTWQQGWVFSCSVMHEALHNHYTQKSLSLSPGK | Clone CH3C.18.K165Q (CH3C.35.14) |
| 23 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESLGHVWAVYQTTPPVLDSDGSFFLYSKLTV PKSTWQQGWVFSCSVMHEALHNHYTQKSLSLSPGK | Clone CH3C.18.E153W. K165Q (CH3C.35.15) |
| 24 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESYGTEWSSYKTTPPVLDSDGSFFLYSKLTV TKSEWQQGFVFSCSVMHEALHNHYTQKSLSLSPGK | Clone CH3C.35.E153W (CH3C.35.19) |
| 25 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWSSYKTTPPVLDSDGSFFLYSKLTVT KEEWQQGFVFSCSVMHEALHNHYTQKSLSLSPGK | Clone CH3C.35.S188E (CH3C.35.20) |
| 26 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESYGTEWSSYKTTPPVLDSDGSFFLYSKLTV TKEEWQQGFVFSCSVMHEALHNHYTQKSLSLSPGK | Clone CH3C.35.E153W. S188E (CH3C.35.21) |
| 27 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWSNYKTTPPVLDSDGSFFLYSKLTVT KSEWQQGFVFSCSVMHEALHNHYTQKSLSLSPGK | Clone CH3C.35.N163 |
| 28 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWSSYQTTPPVLDSDGSFFLYSKLTVT KSEWQQGFVFSCSVMHEALHNHYTQKSLSLSPGK | Clone CH3C.35.K165Q |
| 29 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWSNYQTTPPVLDSDGSFFLYSKLTVT KSEWQQGFVFSCSVMHEALHNHYTQKSLSLSPGK | Clone CH3C.35.N163. K165Q |
| 30 | NSVIIVDKNGRLVYLVENPGGYVAYSKAATVTGKLVHANFGTKKD FEDLYTPVNGSIVIVRAGKITFAEKVANAESLNAIGVLIYMDQTKFPI VNAELSFFGHAHLGTGDPYTPGFPSFNHTQFPPSRSSGLPNIPVQTISR AAAEKLFGNMEGDCPSDWKTDSTCRMVTSESKNVKLTVS | Human TfR apical domain |
| 31 | NSVIIVDKNGGLVYLVENPGGYVAYSKAATVTGKLVHANFGTKKD FEDLDSPVNGSIVIVRAGKITFAEKVANAESLNAIGVLIYMDQTKFPI VKADLSFFGHAHLGTGDPYTPGFPSFNHTQFPPSQSSGLPNIPVQTIS RAAAEKLFGNMEGDCPSDWKTDSTCKMVTSENKSVKLTVS | Cynomolgus TfR apical domain |
| 32 | SSGLPNIPVQTISRAAAEKLFGNMEGDCPSDWKTDSTCRMVTSESKN VKLTVSNDSAQNSVIIVDKNGRLVYLVENPGGYVAYSKAATVTGKL VHANFGTKKDFEDLYTPVNGSIVIVRAGKITFAEKVANAESLNAIGV LIYMDQTKFPIVNAELSGP | Loop-truncated human TfR apical domain displayed on phage |
| 33 | SSGLPNIPVQTISRAAAEKLFGNMEGDCPSDWKTDSTCKMVTSENK SVKLTVSNDSAQNSVIIVDKNGGLVYLVENPGGYVAYSKAATVTGK LVHANFGTKKDFEDLDSPVNGSIVIVRAGKITFAEKVANAESLNAIG VLIYMDQTKFPIVKADLSGP | Loop-truncated cynomolgus TfR apical domain displayed on phage |
| 34 | WESXGXXXXXYK | First portion CH3C register |
| 35 | TVXKSXWQQGXV | Second portion CH3C register |
| 36 | YGTEW | CH3C conserved sequence |
| 37 | EPKSCDKTHTCPPCP | Human IgG1 hinge amino acid sequence |
| 38 | MMDQARSAFSNLFGGEPLSYTRFSLARQVDGDNSHVEMKLAVDEE ENADNNTKANVTKPKRCSGSICYGTIAVIVFFLIGFMIGYLGYCKGV EPKTECERLAGTESPVREEPGEDFPAARRLYWDDLKRKLSEKLDST DFTGTIKLLNENSYVPREAGSQKDENLALYVENQFREFKLSKVWRD QHFVKIQVKDSAQNSVIIVDKNGRLVYLVENPGGYVAYSKAATVTG KLVHANFGTKKDFEDLYTPVNGSIVIVRAGKITFAEKVANAESLNAI GVLIYMDQTKFPIVNAELSFFGHAHLGTGDPYTPGFPSFNHTQFPPSR SSGLPNIPVQTISRAAAEKLFGNMEGDCPSDWKTDSTCRMVTSESKN VKLTVSNVLKEIKILNIFGVIKGFVEPDHYVVVGAQRDAWGPGAAK SGVGTALLLKLAQMFSDMVLKDGFQPSRSIIFASWSAGDFGSVGAT EWLEGYLSSLHLKAFTYINLDKAVLGTSNFKVSASPLLYTLIEKTMQ NVKHPVTGQFLYQDSNWASKVEKLTLDNAAFPFLAYSGIPAVSFCF CEDTDYPYLGTTMDTYKELIERIPELNKVARAAAEVAGQFVIKLTH DVELNLDYERYNSQLLSFVRDLNQYRADIKEMGLSLQWLYSARGD FFRATSRLTTDFGNAEKTDRFVMKKLNDRVMRVEYHFLSPYVSPKE SPFRHVFWGSGSHTLPALLENLKLRKQNNGAFNETLFRNQLALATW TIQGAANALSGDVWDIDNEF | Human transferrin receptor protein 1 (TFR1) |
| 39 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESYGTEWSSYKTTPPVLDSDGSFFLYSKLTV TKSEWQQGFVFSCSVMHEALHNHYTQKSLSLSPGK | Clone CH3C.35.19 |
| 40 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWSSYKTTPPVLDSDGSFFLYSKLTVT KEEWQQGFVFSCSVMHEALHNHYTQKSLSLSPGK | Clone CH3C.35.20 |
| 41 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESYGTEWSSYKTTPPVLDSDGSFFLYSKLTV TKEEWQQGFVFSCSVMHEALHNHYTQKSLSLSPGK | Clone CH3C.35.21 |
| 42 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESYGTEWSNYKTTPPVLDSDGSFFLYSKLTV TKSEWQQGFVFSCSVMHEALHNHYTQKSLSLSPGK | Clone CH3C.35.22 |
| 43 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWSNYKTTPPVLDSDGSFFLYSKLTVT KEEWQQGFVFSCSVMHEALHNHYTQKSLSLSPGK | Clone CH3C.35.23 |
| 44 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESYGTEWSNYKTTPPVLDSDGSFFLYSKLTV TKEEWQQGFVFSCSVMHEALHNHYTQKSLSLSPGK | Clone CH3C.35.24 |
| 45 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESLGHVWAVYKTTPPVLDSDGSFFLYSKLTV PKSTWQQGWVFSCSVMHEALHNHYTQKSLSLSPGK | CH3C.18 variant |
| 46 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESLGHVWAVYKTTPPVLDSDGSFFLYSKLTV PKSTWQQGWVFSCSVMHEALHNHYTQKSLSLSPGK | CH3C.18 variant |
| 47 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVYWESLGHVWAVYKTTPPVLDSDGSFFLYSKLTV PKSTWQQGWVFSCSVMHEALHNHYTQKSLSLSPGK | CH3C.18 variant |
| 48 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESLGHVWAVYQTTPPVLDSDGSFFLYSKLTV PKSTWQQGWVFSCSVMHEALHNHYTQKSLSLSPGK | CH3C.18 variant |
| 49 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESLGHVWAVYFTTPPVLDSDGSFFLYSKLTVP KSTWQQGWVFSCSVMHEALHNHYTQKSLSLSPGK | CH3C.18 variant |
| 50 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESLGHVWAVYHTTPPVLDSDGSFFLYSKLTV PKSTWQQGWVFSCSVMHEALHNHYTQKSLSLSPGK | CH3C.18 variant |
| 51 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESYGTEWSSYKTTPPVLDSDGSFFLYSKLTVT KSEWQQGFVFSCSVMHEALHNHYTQKSLSLSPGK | Clone CH3C.35.21.1 |
| 52 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESYGTEWSSYRTTPPVLDSDGSFFLYSKLTVT KSEWQQGFVFSCSVMHEALHNHYTQKSLSLSPGK | Clone CH3C.35.21.2 |
| 53 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESYGTEWSSYRTTPPVLDSDGSFFLYSKLTVT REEWQQGFVFSCSVMHEALHNHYTQKSLSLSPGK | Clone CH3C.35.21.3 |
| 54 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESYGTEWSSYRTTPPVLDSDGSFFLYSKLTVT GEEWQQGFVFSCSVMHEALHNHYTQKSLSLSPGK | Clone CH3C.35.21.4 |
| 55 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESYGTEWSSYRTTPPVLDSDGSFFLYSKLTVT REEWQQGFVFSCWVMHEALHNHYTQKSLSLSPGK | Clone CH3C.35.21.5 |
| 56 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESYGTEWSSYRTTPPVLDSDGSFFLYSKLTVT KEEWQQGFVFSCWVMHEALHNHYTQKSLSLSPGK | Clone CH3C.35.21.6 |
| 57 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESYGTEWSSYRTTPPVLDSDGSFFLYSKLTVT REEWQQGFVFTCWVMHEALHNHYTQKSLSLSPGK | Clone CH3C.35.21.7 |
| 58 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESYGTEWSSYRTTPPVLDSDGSFFLYSKLTVT REEWQQGFVFTCGVMHEALHNHYTQKSLSLSPGK | Clone CH3C.35.21.8 |
| 59 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESYGTEWSSYRTTPPVLDSDGSFFLYSKLTVT REEWQQGFVFECWVMHEALHNHYTQKSLSLSPGK | Clone CH3C.35.21.9 |
| 60 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESYGTEWSSYRTTPPVLDSDGSFFLYSKLTVT REEWQQGFVFKCWVMHEALHNHYTQKSLSLSPGK | Clone CH3C.35.21.10 |
| 61 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESYGTEWSSYRTTPPVLDSDGSFFLYSKLTVT PEEWQQGFVFKCWVMHEALHNHYTQKSLSLSPGK | Clone CH3C.35.21.11 |
| 62 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESYGTEWSSYRTTPPVLDSDGSFFLYSKLTV TREEWQQGFVFSCSVMHEALHNHYTQKSLSLSPGK | Clone CH3C.35.21.12 |
| 63 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESYGTEWSSYRTTPPVLDSDGSFFLYSKLTV TGEEWQQGFVFSCSVMHEALHNHYTQKSLSLSPGK | Clone CH3C.35.21.13 |
| 64 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESYGTEWSSYRTTPPVLDSDGSFFLYSKLTV TREEWQQGFVFTCWVMHEALHNHYTQKSLSLSPGK | Clone CH3C.35.21.14 |
| 65 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESYGTEWSSYRTTPPVLDSDGSFFLYSKLTV TGEEWQQGFVFTCWVMHEALHNHYTQKSLSLSPGK | Clone CH3C.35.21.15 |
| 66 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESYGTEWSSYRTTPPVLDSDGSFFLYSKLTV TREEWQQGFVFTCGVMHEALHNHYTQKSLSLSPGK | Clone CH3C.35.21.16 |
| 67 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESYGTEWSSYKTTPPVLDSDGSFFLYSKLTVT KEEWQQGFVFSCSVMHEALHNHYTQKSLSLSPGK | Clone CH3C.35.21.17 |
| 68 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESYGTEWSSYRTTPPVLDSDGSFFLYSKLTVT KEEWQQGFVFSCSVMHEALHNHYTQKSLSLSPGK | Clone CH3C.35.21.18 |
| 69 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESFGTEWSSYKTTPPVLDSDGSFFLYSKLTVT KEEWQQGFVFSCSVMHEALHNHYTQKSLSLSPGK | Clone CH3C.35.20.1 |
| 70 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWASYKTTPPVLDSDGSFFLYSKLTVT KEEWQQGFVFSCSVMHEALHNHYTQKSLSLSPGK | Clone CH3C.35.20.2 |
| 71 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWVSYKTTPPVLDSDGSFFLYSKLTVT KEEWQQGFVFSCSVMHEALHNHYTQKSLSLSPGK | Clone CH3C.35.20.3 |
| 72 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWSSYKTTPPVLDSDGSFFLYSKLTVS KEEWQQGFVFSCSVMHEALHNHYTQKSLSLSPGK | Clone CH3C.35.20.4 |
| 73 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESFGTEWASYKTTPPVLDSDGSFFLYSKLTVT KEEWQQGFVFSCSVMHEALHNHYTQKSLSLSPGK | Clone CH3C.35.20.5 |
| 74 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESFGTEWVSYKTTPPVLDSDGSFFLYSKLTVT KEEWQQGFVFSCSVMHEALHNHYTQKSLSLSPGK | Clone CH3C.35.20.6 |
| 75 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESFGTEWSSYKTTPPVLDSDGSFFLYSKLTVT KEEWQQGFVFSCSVMHEALHNHYTQKSLSLSPGK | Clone CH3C35.21.a.1 |
| 76 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESYGTEWASYKTTPPVLDSDGSFFLYSKLTV TKEEWQQGFVFSCSVMHEALHNHYTQKSLSLSPGK | Clone CH3C.35.21.a.2 |
| 77 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESYGTEWVSYKTTPPVLDSDGSFFLYSKLTV TKEEWQQGFVFSCSVMHEALHNHYTQKSLSLSPGK | Clone CH3C.35.21.a.3 |
| 78 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESYGTEWSSYKTTPPVLDSDGSFFLYSKLTV SKEEWQQGFVFSCSVMHEALHNHYTQKSLSLSPGK | Clone CH3C.35.21.a.4 |
| 79 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESFGTEWASYKTTPPVLDSDGSFFLYSKLTV TKEEWQQGFVFSCSVMHEALHNHYTQKSLSLSPGK | Clone CH3C.35.21.a.5 |
| 80 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESFGTEWVSYKTTPPVLDSDGSFFLYSKLTV TKEEWQQGFVFSCSVMHEALHNHYTQKSLSLSPGK | Clone CH3C.35.21.a.6 |
| 81 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESFGTEWSNYKTTPPVLDSDGSFFLYSKLTVT KEEWQQGFVFSCSVMHEALHNHYTQKSLSLSPGK | Clone CH3C.35.23.1 |
| 82 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWANYKTTPPVLDSDGSFFLYSKLTV TKEEWQQGFVFSCSVMHEALHNHYTQKSLSLSPGK | Clone CH3C.35.23.2 |
| 83 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWVNYKTTPPVLDSDGSFFLYSKLTV TKEEWQQGFVFSCSVMHEALHNHYTQKSLSLSPGK | Clone CH3C.35.23.3 |
| 84 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWSNYKTTPPVLDSDGSFFLYSKLTVS KEEWQQGFVFSCSVMHEALHNHYTQKSLSLSPGK | Clone CH3C.35.23.4 |
| 85 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESFGTEWANYKTTPPVLDSDGSFFLYSKLTVT KEEWQQGFVFSCSVMHEALHNHYTQKSLSLSPGK | Clone CH3C.35.23.5 |
| 86 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESFGTEWVNYKTTPPVLDSDGSFFLYSKLTVT KEEWQQGFVFSCSVMHEALHNHYTQKSLSLSPGK | Clone CH3C.35.23.6 |
| 87 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESFGTEWSNYKTTPPVLDSDGSFFLYSKLTV TKEEWQQGFVFSCSVMHEALHNHYTQKSLSLSPGK | Clone CH3C.35.24.1 |
| 88 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESYGTEWANYKTTPPVLDSDGSFFLYSKLTV TKEEWQQGFVFSCSVMHEALHNHYTQKSLSLSPGK | Clone CH3C.35.24.2 |
| 89 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESYGTEWVNYKTTPPVLDSDGSFFLYSKLTV TKEEWQQGFVFSCSVMHEALHNHYTQKSLSLSPGK | Clone CH3C.35.24.3 |
| 90 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESYGTEWSNYKTTPPVLDSDGSFFLYSKLTV SKEEWQQGFVFSCSVMHEALHNHYTQKSLSLSPGK | Clone CH3C.35.24.4 |
| 91 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESFGTEWANYKTTPPVLDSDGSFFLYSKLTV TKEEWQQGFVFSCSVMHEALHNHYTQKSLSLSPGK | Clone CH3C.35.24.5 |
| 92 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESFGTEWVNYKTTPPVLDSDGSFFLYSKLTV TKEEWQQGFVFSCSVMHEALHNHYTQKSLSLSPGK | Clone CH3C.35.24.6 |
| 93 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESFGTEWSSYKTTPPVLDSDGSFFLYSKLTVT KEEWQQGFVFSCSVMHEALHNHYTQKSLSLSPGK | Clone CH3C.35.21.17.1 |
| 94 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESYGTEWASYKTTPPVLDSDGSFFLYSKLTVT KEEWQQGFVFSCSVMHEALHNHYTQKSLSLSPGK | Clone CH3C.35.21.17.2 |
| 95 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESYGTEWVSYKTTPPVLDSDGSFFLYSKLTVT KEEWQQGFVFSCSVMHEALHNHYTQKSLSLSPGK | Clone CH3C.35.21.17.3 |
| 96 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESYGTEWSSYKTTPPVLDSDGSFFLYSKLTVS KEEWQQGFVFSCSVMHEALHNHYTQKSLSLSPGK | Clone CH3C.35.21.17.4 |
| 97 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESFGTEWASYKTTPPVLDSDGSFFLYSKLTVT KEEWQQGFVFSCSVMHEALHNHYTQKSLSLSPGK | Clone CH3C.35.21.17.5 |
| 98 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESFGTEWVSYKTTPPVLDSDGSFFLYSKLTVT KEEWQQGFVFSCSVMHEALHNHYTQKSLSLSPGK | Clone CH3C.35.21.17.6 |
| 99 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWSNYKTTPPVLDSDGSFFLYSKLTVT KSEWQQGFVFSCSVMHEALHNHYTQKSLSLSPGK | Clone CH3C.35.N390 |
| 100 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESLGHVWVNQKTTPPVLDSDGSFFLYSKLTV PKSTWQQGWVFSCSVMHEALHNHYTQKSLSLSPGK | Clone CH3C.35.16 |
| 101 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESLGHVWVNQQTTPPVLDSDGSFFLYSKLTV PKSTWQQGWVFSCSVMHEALHNHYTQKSLSLSPGK | Clone CH3C.35.17 |
| 102 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESLGHVWVNQQTTPPVLDSDGSFFLYSKLTV PKSTWQQGWVFSCSVMHEALHNHYTQKSLSLSPGK | Clone CH3C.35.18 |
| 103 | MMDQARSAFSNLFGGEPLSYTRFSLARQVDGDNSHVEMKLGVDEE ENTDNNTKANGTKPKRCGGNICYGTIAVIIFFLIGFMIGYLGYCKGV EPKTECERLAGTESPAREEPEEDFPAAPRLYWDDLKRKLSEKLDTTD FTSTIKLLNENLYVPREAGSQKDENLALYIENQFREFKLSKVWRDQH FVKIQVKDSAQNSVIIVDKNGGLVYLVENPGGYVAYSKAATVTGKL VHANFGTKKDFEDLDSPVNGSIVIVRAGKITFAEKVANAESLNAIGV LIYMDQTKFPIVKADLSFFGHAHLGTGDPYTPGFPSFNHTQFPPSQSS GLPNIPVQTISRAAAEKLFGNMEGDCPSDWKTDSTCKMVTSENKSV KLTVSNVLKETKILNIFGVIKGFVEPDHYVVVGAQRDAWGPGAAKS SVGTALLLKLAQMFSDMVLKDGFQPSRSIIFASWSAGDFGSVGATE WLEGYLSSLHLKAFTYINLDKAVLGTSNFKVSASPLLYTLIEKTMQD VKHPVTGRSLYQDSNWASKVEKLTLDNAAFPFLAYSGIPAVSFCFC EDTDYPYLGTTMDTYKELVERIPELNKVARAAAEVAGQFVIKLTHD TELNLDYERYNSQLLLFLRDLNQYRADVKEMGLSLQWLYSARGDF FRATSRLTTDFRNAEKRDKFVMKKLNDRVMRVEYYFLSPYVSPKES PFRHVFWGSGSHTLSALLESLKLRRQNNSAFNETLFRNQLALATWTI QGAANALSGDVWDIDNEF | Cyno TfR |
| 104 | MGWSCIILFLVATATGAYAGTSSGLPNIPVQTISRAAAEKLFGNMEG DCPSDWKTDSTCRMVTSESKNVKLTVSNDSAQNSVIIVDKNGRLVY LVENPGGYVAYSKAATVTGKLVHANFGTKKDFEDLYTPVNGSIVIV RAGKITFAEKVANAESLNAIGVLIYMDQTKFPIVNAELSASHHHHHH | His-tagged permutated TfR apical domain |
| 105 | METDTLLLWVLLLWVPGSTGDKTHTCPPCPAPEAAGGPSVFLFPPK PKDTLYITREPEVTCVVVDVSHEDPEVKFNWYVDGVEVHNAKTKP REEQYNSTYRVVSVLTVLHQDWLNGKEYKCKVSNKALGAPIEKTIS KAKGQPREPQVYTLPPSRDELTKNQVSLTCLVKGFYPSDIAVEWESL GHVWAVYKTTPPVLDSDGSFFLYSKLTVPKSTWQQGWVFSCSVMH EALHNHYTQKSLSLSPGK | Expressed CH3C.18 Fc sequence |
| 106 | TVXKXXWQQGXV | Second portion CH3C register |
| 107 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLT CLVKGFYPSDIAVEWESYGTEWSSYKTTPPVLDSDGSFFLYSKLTVT KEEWQQGFVFSCSVMHEALHNHYTQKSLSLSPGK | Clone CH3C.35.8 (Clone CH3C.35.20 with YTE and LALAPG mutations) |
| 108 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLT CLVKGFYPSDIAVEWESFGTEWSSYKTTPPVLDSDGSFFLYSKLTVT KEEWQQGFVFSCSVMHEALHNHYTQKSLSLSPGK | Clone CH3C.35.9 (Clone CH3C.35.21 with YTE and LALAPG mutations) |
| 109 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESFGTEWSSYKTTPPVLDSDGSFFLYSKLTVT KEEWQQGFVFSCSVMHEALHNHYTQKSLSLSPGK | Clone CH3C.35.20.1 with knob mutation |
| 110 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVEWESFGTEWSSYKTTPPVLDSDGSFFLYSKLTV TKEEWQQGFVFSCSVMHEALHNHYTQKSLSLSPGK | Clone CH3C.35.20.1 with knob and LALA mutations |
| 111 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVEWESFGTEWSSYKTTPPVLDSDGSFFLYSKLTV TKEEWQQGFVFSCSVMHEALHNHYTQKSLSLSPGK | Clone CH3C.35.20.1 with knob and LALAPG mutations |
| 112 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESFGTEWSSYKTTPPVLDSDGSFFLYSKLTVT KEEWQQGFVFSCSVMHEALHNHYTQKSLSLSPGK | Clone CH3C.35.20.1 with knob and YTE mutations |
| 113 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESFGTEWSSYKTTPPVLDSDGSFFLYSKLTVT KEEWQQGFVFSCSVMHEALHNHYTQKSLSLSPGK | Clone CH3C.35.20.1 with knob, LALA, and YTE mutations |
| 114 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLW CLVKGFYPSDIAVEWESFGTEWSSYKTTPPVLDSDGSFFLYSKLTVT KEEWQQGFVFSCSVMHEALHNHYTQKSLSLSPGK | Clone CH3C.35.20.1 with knob, LALAPG, and YTE mutations |
| 115 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESFGTEWSSYKTTPPVLDSDGSFFLVSKLTVT KEEWQQGFVFSCSVMHEALHNHYTQKSLSLSPGK | Clone CH3C.35.20.1 with hole mutations |
| 116 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVEWESFGTEWSSYKTTPPVLDSDGSFFLVSKLTV TKEEWQQGFVFSCSVMHEALHNHYTQKSLSLSPGK | Clone CH3C.35.20.1 with hole and LALA mutations |
| 117 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVEWESFGTEWSSYKTTPPVLDSDGSFFLVSKLTV TKEEWQQGFVFSCSVMHEALHNHYTQKSLSLSPGK | Clone CH3C.35.20.1 with hole and LALAPG mutations |
| 118 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESFGTEWSSYKTTPPVLDSDGSFFLVSKLTVT KEEWQQGFVFSCSVMHEALHNHYTQKSLSLSPGK | Clone CH3C.35.20.1 with hole and YTE mutations |
| 119 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESFGTEWSSYKTTPPVLDSDGSFFLVSKLTVT KEEWQQGFVFSCSVMHEALHNHYTQKSLSLSPGK | Clone CH3C.35.20.1 with hole, LALA, and YTE mutations |
| 120 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLS CAVKGFYPSDIAVEWESFGTEWSSYKTTPPVLDSDGSFFLVSKLTVT KEEWQQGFVFSCSVMHEALHNHYTQKSLSLSPGK | Clone CH3C.35.20.1 with hole, LALAPG, and YTE mutations |
| 121 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWANYKTTPPVLDSDGSFFLYSKLTV TKEEWQQGFVFSCSVMHEALHNHYTQKSLSLSPGK | Clone CH3C.35.23.2 with knob mutation |
| 122 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVEWESYGTEWANYKTTPPVLDSDGSFFLYSKLT VTKEEWQQGFVFSCSVMHEALHNHYTQKSLSLSPGK | Clone CH3C.35.23.2 with knob and LALA mutations |
| 123 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVEWESYGTEWANYKTTPPVLDSDGSFFLYSKLT VTKEEWQQGFVFSCSVMHEALHNHYTQKSLSLSPGK | Clone CH3C.35.23.2 with knob and LALAPG mutations |
| 124 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWANYKTTPPVLDSDGSFFLYSKLTV TKEEWQQGFVFSCSVMHEALHNHYTQKSLSLSPGK | Clone CH3C.35.23.2 with knob and YTE mutations |
| 125 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWANYKTTPPVLDSDGSFFLYSKLTV TKEEWQQGFVFSCSVMHEALHNHYTQKSLSLSPGK | Clone CH3C.35.23.2 with knob, LALA, and YTE mutations |
| 126 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLW CLVKGFYPSDIAVEWESYGTEWANYKTTPPVLDSDGSFFLYSKLTV TKEEWQQGFVFSCSVMHEALHNHYTQKSLSLSPGK | Clone CH3C.35.23.2 with knob, LALAPG, and YTE mutations |
| 127 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWANYKTTPPVLDSDGSFFLVSKLTV TKEEWQQGFVFSCSVMHEALHNHYTQKSLSLSPGK | Clone CH3C.35.23.2 with hole mutations |
| 128 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVEWESYGTEWANYKTTPPVLDSDGSFFLVSKLT VTKEEWQQGFVFSCSVMHEALHNHYTQKSLSLSPGK | Clone CH3C.35.23.2 with hole and LALA mutations |
| 129 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVEWESYGTEWANYKTTPPVLDSDGSFFLVSKLT VTKEEWQQGFVFSCSVMHEALHNHYTQKSLSLSPGK | Clone CH3C.35.23.2 with hole and LALAPG mutations |
| 130 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWANYKTTPPVLDSDGSFFLVSKLTV TKEEWQQGFVFSCSVMHEALHNHYTQKSLSLSPGK | Clone CH3C.35.23.2 with hole and YTE mutations |
| 131 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWANYKTTPPVLDSDGSFFLVSKLTV TKEEWQQGFVFSCSVMHEALHNHYTQKSLSLSPGK | Clone CH3C.35.23.2 with hole, LALA, and YTE mutations |
| 132 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLS CAVKGFYPSDIAVEWESYGTEWANYKTTPPVLDSDGSFFLVSKLTV TKEEWQQGFVFSCSVMHEALHNHYTQKSLSLSPGK | Clone CH3C.35.23.2 with hole, LALAPG, and YTE mutations |
| 133 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWVNYKTTPPVLDSDGSFFLYSKLTV TKEEWQQGFVFSCSVMHEALHNHYTQKSLSLSPGK | Clone CH3C.35.23.3 with knob mutation |
| 134 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVEWESYGTEWVNYKTTPPVLDSDGSFFLYSKLT VTKEEWQQGFVFSCSVMHEALHNHYTQKSLSLSPGK | Clone CH3C.35.23.3 with knob and LALA mutations |
| 135 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVEWESYGTEWVNYKTTPPVLDSDGSFFLYSKLT VTKEEWQQGFVFSCSVMHEALHNHYTQKSLSLSPGK | Clone CH3C.35.23.3 with knob and LALAPG mutations |
| 136 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWVNYKTTPPVLDSDGSFFLYSKLTV TKEEWQQGFVFSCSVMHEALHNHYTQKSLSLSPGK | Clone CH3C.35.23.3 with knob and YTE mutations |
| 137 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWVNYKTTPPVLDSDGSFFLYSKLTV TKEEWQQGFVFSCSVMHEALHNHYTQKSLSLSPGK | Clone CH3C.35.23.3 with knob, LALA, and YTE mutations |
| 138 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLW CLVKGFYPSDIAVEWESYGTEWVNYKTTPPVLDSDGSFFLYSKLTV TKEEWQQGFVFSCSVMHEALHNHYTQKSLSLSPGK | Clone CH3C.35.23.3 with knob, LALAPG, and YTE mutations |
| 139 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWVNYKTTPPVLDSDGSFFLVSKLTV TKEEWQQGFVFSCSVMHEALHNHYTQKSLSLSPGK | Clone CH3C.35.23.3 with hole mutations |
| 140 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVEWESYGTEWVNYKTTPPVLDSDGSFFLVSKLT VTKEEWQQGFVFSCSVMHEALHNHYTQKSLSLSPGK | Clone CH3C.35.23.3 with hole and LALA mutations |
| 141 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVEWESYGTEWVNYKTTPPVLDSDGSFFLVSKLT VTKEEWQQGFVFSCSVMHEALHNHYTQKSLSLSPGK | Clone CH3C.35.23.3 with hole and LALAPG mutations |
| 142 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWVNYKTTPPVLDSDGSFFLVSKLTV TKEEWQQGFVFSCSVMHEALHNHYTQKSLSLSPGK | Clone CH3C.35.23.3 with hole and YTE mutations |
| 143 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWVNYKTTPPVLDSDGSFFLVSKLTV TKEEWQQGFVFSCSVMHEALHNHYTQKSLSLSPGK | Clone CH3C.35.23.3 with hole, LALA, and YTE mutations |
| 144 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLS CAVKGFYPSDIAVEWESYGTEWVNYKTTPPVLDSDGSFFLVSKLTV TKEEWQQGFVFSCSVMHEALHNHYTQKSLSLSPGK | Clone CH3C.35.23.3 with hole, LALAPG, and YTE mutations |
| 145 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWSNYKTTPPVLDSDGSFFLYSKLTVS KEEWQQGFVFSCSVMHEALHNHYTQKSLSLSPGK | Clone CH3C.35.23.4 with knob mutation |
| 146 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVEWESYGTEWSNYKTTPPVLDSDGSFFLYSKLT VSKEEWQQGFVFSCSVMHEALHNHYTQKSLSLSPGK | Clone CH3C.35.23.4 with knob and LALA mutations |
| 147 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVEWESYGTEWSNYKTTPPVLDSDGSFFLYSKLT VSKEEWQQGFVFSCSVMHEALHNHYTQKSLSLSPGK | Clone CH3C.35.23.4 with knob and LALAPG mutations |
| 148 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWSNYKTTPPVLDSDGSFFLYSKLTVS KEEWQQGFVFSCSVMHEALHNHYTQKSLSLSPGK | Clone CH3C.35.23.4 with knob and YTE mutations |
| 149 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWSNYKTTPPVLDSDGSFFLYSKLTVS KEEWQQGFVFSCSVMHEALHNHYTQKSLSLSPGK | Clone CH3C.35.23.4 with knob, LALA, and YTE mutations |
| 150 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLW CLVKGFYPSDIAVEWESYGTEWSNYKTTPPVLDSDGSFFLYSKLTVS KEEWQQGFVFSCSVMHEALHNHYTQKSLSLSPGK | Clone CH3C.35.23.4 with knob, LALAPG, and YTE mutations |
| 151 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWSNYKTTPPVLDSDGSFFLVSKLTVS KEEWQQGFVFSCSVMHEALHNHYTQKSLSLSPGK | Clone CH3C.35.23.4 with hole mutations |
| 152 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVEWESYGTEWSNYKTTPPVLDSDGSFFLVSKLTV SKEEWQQGFVFSCSVMHEALHNHYTQKSLSLSPGK | Clone CH3C.35.23.4 with hole and LALA mutations |
| 153 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVEWESYGTEWSNYKTTPPVLDSDGSFFLVSKLTV SKEEWQQGFVFSCSVMHEALHNHYTQKSLSLSPGK | Clone CH3C.35.23.4 with hole and LALAPG mutations |
| 154 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWSNYKTTPPVLDSDGSFFLVSKLTVS KEEWQQGFVFSCSVMHEALHNHYTQKSLSLSPGK | Clone CH3C.35.23.4 with hole and YTE mutations |
| 155 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWSNYKTTPPVLDSDGSFFLVSKLTVS KEEWQQGFVFSCSVMHEALHNHYTQKSLSLSPGK | Clone CH3C.35.23.4 with hole, LALA, and YTE mutations |
| 156 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLS CAVKGFYPSDIAVEWESYGTEWSNYKTTPPVLDSDGSFFLVSKLTVS KEEWQQGFVFSCSVMHEALHNHYTQKSLSLSPGK | Clone CH3C.35.23.4 with hole, LALAPG, and YTE mutations |
| 157 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVLWESYGTEWASYKTTPPVLDSDGSFFLYSKLTVT KEEWQQGFVFSCSVMHEALHNHYTQKSLSLSPGK | Clone CH3C.35.21.17.2 with knob mutation |
| 158 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVLWESYGTEWASYKTTPPVLDSDGSFFLYSKLT VTKEEWQQGFVFSCSVMHEALHNHYTQKSLSLSPGK | Clone CH3C.35.21.17.2 with knob and LALA mutations |
| 159 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVLWESYGTEWASYKTTPPVLDSDGSFFLYSKLT VTKEEWQQGFVFSCSVMHEALHNHYTQKSLSLSPGK | Clone CH3C.35.21.17.2 with knob and LALAPG mutations |
| 160 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVLWESYGTEWASYKTTPPVLDSDGSFFLYSKLTVT KEEWQQGFVFSCSVMHEALHNHYTQKSLSLSPGK | Clone CH3C.35.21.17.2 with knob and YTE mutations |
| 161 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVLWESYGTEWASYKTTPPVLDSDGSFFLYSKLTVT KEEWQQGFVFSCSVMHEALHNHYTQKSLSLSPGK | Clone CH3C.35.21.17.2 with knob, LALA, and YTE mutations |
| 162 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLW CLVKGFYPSDIAVLWESYGTEWASYKTTPPVLDSDGSFFLYSKLTVT KEEWQQGFVFSCSVMHEALHNHYTQKSLSLSPGK | Clone CH3C.35.21.17.2 with knob, LALAPG, and YTE mutations |
| 163 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVLWESYGTEWASYKTTPPVLDSDGSFFLVSKLTV TKEEWQQGFVFSCSVMHEALHNHYTQKSLSLSPGK | Clone CH3C.35.21.17.2 with hole mutations |
| 164 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVLWESYGTEWASYKTTPPVLDSDGSFFLVSKLTV TKEEWQQGFVFSCSVMHEALHNHYTQKSLSLSPGK | Clone CH3C.35.21.17.2 with hole and LALA mutations |
| 165 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVLWESYGTEWASYKTTPPVLDSDGSFFLVSKLTV TKEEWQQGFVFSCSVMHEALHNHYTQKSLSLSPGK | Clone CH3C.35.21.17.2 with hole and LALAPG mutations |
| 166 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVLWESYGTEWASYKTTPPVLDSDGSFFLVSKLTV TKEEWQQGFVFSCSVMHEALHNHYTQKSLSLSPGK | Clone CH3C.35.21.17.2 with hole and YTE mutations |
| 167 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVLWESYGTEWASYKTTPPVLDSDGSFFLVSKLTV TKEEWQQGFVFSCSVMHEALHNHYTQKSLSLSPGK | Clone CH3C.35.21.17.2 with hole, LALA, and YTE mutations |
| 168 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLS CAVKGFYPSDIAVLWESYGTEWASYKTTPPVLDSDGSFFLVSKLTV TKEEWQQGFVFSCSVMHEALHNHYTQKSLSLSPGK | Clone CH3C.35.21.17.2 with hole, LALAPG, and YTE mutations |
| 169 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWSNYKTTPPVLDSDGSFFLYSKLTVT KEEWQQGFVFSCSVMHEALHNHYTQKSLSLSPGK | Clone CH3C.35.23 with knob mutation |
| 170 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVEWESYGTEWSNYKTTPPVLDSDGSFFLYSKLT VTKEEWQQGFVFSCSVMHEALHNHYTQKSLSLSPGK | Clone CH3C.35.23 with knob and LALA mutations |
| 171 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVEWESYGTEWSNYKTTPPVLDSDGSFFLYSKLT VTKEEWQQGFVFSCSVMHEALHNHYTQKSLSLSPGK | Clone CH3C.35.23 with knob and LALAPG mutations |
| 172 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWSNYKTTPPVLDSDGSFFLYSKLTVT KEEWQQGFVFSCSVMHEALHNHYTQKSLSLSPGK | Clone CH3C.35.23 with knob and YTE mutations |
| 173 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWSNYKTTPPVLDSDGSFFLYSKLTVT KEEWQQGFVFSCSVMHEALHNHYTQKSLSLSPGK | Clone CH3C.35.23 with knob, LALA, and YTE mutations |
| 174 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLW CLVKGFYPSDIAVEWESYGTEWSNYKTTPPVLDSDGSFFLYSKLTVT KEEWQQGFVFSCSVMHEALHNHYTQKSLSLSPGK | Clone CH3C.35.23 with knob, LALAPG, and YTE mutations |
| 175 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWSNYKTTPPVLDSDGSFFLVSKLTV TKEEWQQGFVFSCSVMHEALHNHYTQKSLSLSPGK | Clone CH3C.35.23 with hole mutations |
| 176 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVEWESYGTEWSNYKTTPPVLDSDGSFFLVSKLTV TKEEWQQGFVFSCSVMHEALHNHYTQKSLSLSPGK | Clone CH3C.35.23 with hole and LALA mutations |
| 177 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVEWESYGTEWSNYKTTPPVLDSDGSFFLVSKLTV TKEEWQQGFVFSCSVMHEALHNHYTQKSLSLSPGK | Clone CH3C.35.23 with hole and LALAPG mutations |
| 178 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWSNYKTTPPVLDSDGSFFLVSKLTV TKEEWQQGFVFSCSVMHEALHNHYTQKSLSLSPGK | Clone CH3C.35.23 with hole and YTE mutations |
| 179 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWSNYKTTPPVLDSDGSFFLVSKLTV TKEEWQQGFVFSCSVMHEALHNHYTQKSLSLSPGK | Clone CH3C.35.23 with hole, LALA, and YTE mutations |
| 180 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLS CAVKGFYPSDIAVEWESYGTEWSNYKTTPPVLDSDGSFFLVSKLTV TKEEWQQGFVFSCSVMHEALHNHYTQKSLSLSPGK | Clone CH3C.35.23 with hole, LALAPG, and YTE mutations |
| 181 | METDTLLLWVLLLWVPGSTGDKTHTCPPCPAPEAAGGPSVFLFPPK PKDTLYITREPEVTCVVVDVSHEDPEVKFNWYVDGVEVHNAKTKP REEQYNSTYRVVSVLTVLHQDWLNGKEYKCKVSNKALGAPIEKTIS KAKGQPREPQVYTLPPSRDELTKNQVSLTCLVKGFYPSDIAVEWES YGTEWSSYKTTPPVLDSDGSFFLYSKLTVTKSEWQQGFVFSCSVMH EALHNHYTQKSLSLSPGK | Expressed CH3C.35 Fc sequence |
| 182 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESWGFVWSTYKTTPPVLDSDGSFFLYSKLTV PKSNWQQGFVFSCSVMHEALHNHYTQKSLSLSPGK | Clone CH3C.18.3.4-1 (CH3C.3.4-1) |
| 183 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESWGHVWSTYKTTPPVLDSDGSFFLYSKLTV PKSNWQQGYVFSCSVMHEALHNHYTQKSLSLSPGK | Clone CH3C.18.3.4-19 (CH3C.3.4-19) |
| 184 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESLGHVWVEQKTTPPVLDSDGSFFLYSKLTV PKSTWQQGWVFSCSVMHEALHNHYTQKSLSLSPGK | Clone CH3C.18.3.2-3 (CH3C.3.2-3) |
| 185 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESLGHVWVGVKTTPPVLDSDGSFFLYSKLTV PKSTWQQGWVFSCSVMHEALHNHYTQKSLSLSPGK | Clone CH3C.18.3.2-14 (CH3C.3.2-14) |
| 186 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESLGHVWVHTKTTPPVLDSDGSFFLYSKLTV PKSTWQQGWVFSCSVMHEALHNHYTQKSLSLSPGK | Clone CH3C.18.3.2-24 (CH3C.3.2-24) |
| 187 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESWGTVWGTYKTTPPVLDSDGSFFLYSKLTV PKSNWQQGYVFSCSVMHEALHNHYTQKSLSLSPGK | Clone CH3C.18.3.4-26 (CH3C.3.4-26) |
| 188 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESLGHVWVGTKTTPPVLDSDGSFFLYSKLTV PKSTWQQGWVFSCSVMHEALHNHYTQKSLSLSPGK | Clone CH3C.18.3.2-17 (CH3C.3.2-17) |
| 189 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESFGTEWSSYKTTPPVLDSDGSFFLYSKLTVS KEEWQQGFVFSCSVMHEALHNHYTQKSLSLSPGK | Clone CH3C.35.20.1.1 |
| 190 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWANYKTTPPVLDSDGSFFLYSKLTV SKSEWQQGFVFSCSVMHEALHNHYTQKSLSLSPGK | Clone CH3C.35.23.2.1 |
| 191 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESFGTEWSNYKTTPPVLDSDGSFFLYSKLTVS KEEWQQGFVFSCSVMHEALHNHYTQKSLSLSPGK | Clone CH3C.35.23.1.1 |
| 192 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWSSYKTTPPVLDSDGSFFLYSKLTVS KSEWQQGFVFSCSVMHEALHNHYTQKSLSLSPGK | Clone CH3C.35.S413 |
| 193 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWVNYKTTPPVLDSDGSFFLYSKLTV SKEEWQQGFVFSCSVMHEALHNHYTQKSLSLSPGK | Clone CH3C.35.23.3.1 |
| 194 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWSNYKTTPPVLDSDGSFFLYSKLTVS KSEWQQGFVFSCSVMHEALHNHYTQKSLSLSPGK | Clone CH3C.35.N390.1 |
| 195 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESFGTEWVNYKTTPPVLDSDGSFFLYSKLTVS KEEWQQGFVFSCSVMHEALHNHYTQKSLSLSPGK | Clone CH3C.35.23.6.1 |
| 196 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVWWESYGTEWSSYKTTPPVLDSDGSFFLYSKLTV TKEEWQQGFVFSCSVMHEALHNHYTQKSLSLSPGK | Clone CH3C.35.21 with knob mutation |
| 197 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVWWESYGTEWSSYKTTPPVLDSDGSFFLYSKLT VTKEEWQQGFVFSCSVMHEALHNHYTQKSLSLSPGK | Clone CH3C.35.21 with knob and LALA mutations |
| 198 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVWWESYGTEWSSYKTTPPVLDSDGSFFLYSKLT VTKEEWQQGFVFSCSVMHEALHNHYTQKSLSLSPGK | Clone CH3C.35.21 with knob and LALAPG mutations |
| 199 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVWWESYGTEWSSYKTTPPVLDSDGSFFLYSKLTV TKEEWQQGFVFSCSVMHEALHNHYTQKSLSLSPGK | Clone CH3C.35.21 with knob and YTE mutations |
| 200 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVWWESYGTEWSSYKTTPPVLDSDGSFFLYSKLTV TKEEWQQGFVFSCSVMHEALHNHYTQKSLSLSPGK | Clone CH3C.35.21 with knob, LALA, and YTE mutations |
| 201 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLW CLVKGFYPSDIAVWWESYGTEWSSYKTTPPVLDSDGSFFLYSKLTV TKEEWQQGFVFSCSVMHEALHNHYTQKSLSLSPGK | Clone CH3C.35.21 with knob, LALAPG, and YTE mutations |
| 202 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVWWESYGTEWSSYKTTPPVLDSDGSFFLVSKLTV TKEEWQQGFVFSCSVMHEALHNHYTQKSLSLSPGK | Clone CH3C.35.21 with hole mutations |
| 203 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVWWESYGTEWSSYKTTPPVLDSDGSFFLVSKLT VTKEEWQQGFVFSCSVMHEALHNHYTQKSLSLSPGK | Clone CH3C.35.21 with hole and LALA mutations |
| 204 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVWWESYGTEWSSYKTTPPVLDSDGSFFLVSKLT VTKEEWQQGFVFSCSVMHEALHNHYTQKSLSLSPGK | Clone CH3C.35.21 with hole and LALAPG mutations |
| 205 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVWWESYGTEWSSYKTTPPVLDSDGSFFLVSKLTV TKEEWQQGFVFSCSVMHEALHNHYTQKSLSLSPGK | Clone CH3C.35.21 with hole and YTE mutations |
| 206 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVWWESYGTEWSSYKTTPPVLDSDGSFFLVSKLTV TKEEWQQGFVFSCSVMHEALHNHYTQKSLSLSPGK | Clone CH3C.35.21 with hole, LALA, and YTE mutations |
| 207 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLS CAVKGFYPSDIAVWWESYGTEWSSYKTTPPVLDSDGSFFLVSKLTV TKEEWQQGFVFSCSVMHEALHNHYTQKSLSLSPGK | Clone CH3C.35.21 with hole, LALAPG, and YTE mutations |
| 208 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESFGTEWSSYKTTPPVLDSDGSFFLYSKLTVS KEEWQQGFVFSCSVMHEALHNHYTQKSLSLSPGK | Clone CH3C.35.20.1.1 with knob mutation |
| 209 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVEWESFGTEWSSYKTTPPVLDSDGSFFLYSKLTV SKEEWQQGFVFSCSVMHEALHNHYTQKSLSLSPGK | Clone CH3C.35.20.1.1 with knob and LALA mutations |
| 210 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVEWESFGTEWSSYKTTPPVLDSDGSFFLYSKLTV SKEEWQQGFVFSCSVMHEALHNHYTQKSLSLSPGK | Clone CH3C.35.20.1.1 with knob and LALAPG mutations |
| 211 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESFGTEWSSYKTTPPVLDSDGSFFLYSKLTVS KEEWQQGFVFSCSVMHEALHNHYTQKSLSLSPGK | Clone CH3C.35.20.1.1 with knob and YTE mutations |
| 212 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESFGTEWSSYKTTPPVLDSDGSFFLYSKLTVS KEEWQQGFVFSCSVMHEALHNHYTQKSLSLSPGK | Clone CH3C.35.20.1.1 with knob, LALA, and YTE mutations |
| 213 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLW CLVKGFYPSDIAVEWESFGTEWSSYKTTPPVLDSDGSFFLYSKLTVS KEEWQQGFVFSCSVMHEALHNHYTQKSLSLSPGK | Clone CH3C.35.20.1.1 with knob, LALAPG, and YTE mutations |
| 214 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESFGTEWSSYKTTPPVLDSDGSFFLVSKLTVS KEEWQQGFVFSCSVMHEALHNHYTQKSLSLSPGK | Clone CH3C.35.20.1.1 with hole mutations |
| 215 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVEWESFGTEWSSYKTTPPVLDSDGSFFLVSKLTV SKEEWQQGFVFSCSVMHEALHNHYTQKSLSLSPGK | Clone CH3C.35.20.1.1 with hole and LALA mutations |
| 216 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVEWESFGTEWSSYKTTPPVLDSDGSFFLVSKLTV SKEEWQQGFVFSCSVMHEALHNHYTQKSLSLSPGK | Clone CH3C.35.20.1.1 with hole and LALAPG mutations |
| 217 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESFGTEWSSYKTTPPVLDSDGSFFLVSKLTVS KEEWQQGFVFSCSVMHEALHNHYTQKSLSLSPGK | Clone CH3C.35.20.1.1 with hole and YTE mutations |
| 218 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESFGTEWSSYKTTPPVLDSDGSFFLVSKLTVS KEEWQQGFVFSCSVMHEALHNHYTQKSLSLSPGK | Clone CH3C.35.20.1.1 with hole, LALA, and YTE mutations |
| 219 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLS CAVKGFYPSDIAVEWESFGTEWSSYKTTPPVLDSDGSFFLVSKLTVS KEEWQQGFVFSCSVMHEALHNHYTQKSLSLSPGK | Clone CH3C.35.20.1.1 with hole, LALAPG, and YTE mutations |
| 220 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWANYKTTPPVLDSDGSFFLYSKLTV SKSEWQQGFVFSCSVMHEALHNHYTQKSLSLSPGK | Clone CH3C.35.23.2.1 with knob mutation |
| 221 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVEWESYGTEWANYKTTPPVLDSDGSFFLYSKLT VSKSEWQQGFVFSCSVMHEALHNHYTQKSLSLSPGK | Clone CH3C.35.23.2.1 with knob and LALA mutations |
| 222 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVEWESYGTEWANYKTTPPVLDSDGSFFLYSKLT VSKSEWQQGFVFSCSVMHEALHNHYTQKSLSLSPGK | Clone CH3C.35.23.2.1 with knob and LALAPG mutations |
| 223 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWANYKTTPPVLDSDGSFFLYSKLTV SKSEWQQGFVFSCSVMHEALHNHYTQKSLSLSPGK | Clone CH3C.35.23.2.1 with knob and YTE mutations |
| 224 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWANYKTTPPVLDSDGSFFLYSKLTV SKSEWQQGFVFSCSVMHEALHNHYTQKSLSLSPGK | Clone CH3C.35.23.2.1 with knob, LALA, and YTE mutations |
| 225 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLW CLVKGFYPSDIAVEWESYGTEWANYKTTPPVLDSDGSFFLYSKLTV SKSEWQQGFVFSCSVMHEALHNHYTQKSLSLSPGK | Clone CH3C.35.23.2.1 with knob, LALAPG, and YTE mutations |
| 226 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWANYKTTPPVLDSDGSFFLVSKLTV SKSEWQQGFVFSCSVMHEALHNHYTQKSLSLSPGK | Clone CH3C.35.23.2.1 with hole mutations |
| 227 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVEWESYGTEWANYKTTPPVLDSDGSFFLVSKLT VSKSEWQQGFVFSCSVMHEALHNHYTQKSLSLSPGK | Clone CH3C.35.23.2.1 with hole and LALA mutations |
| 228 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVEWESYGTEWANYKTTPPVLDSDGSFFLVSKLT VSKSEWQQGFVFSCSVMHEALHNHYTQKSLSLSPGK | Clone CH3C.35.23.2.1 with hole and LALAPG mutations |
| 229 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWANYKTTPPVLDSDGSFFLVSKLTV SKSEWQQGFVFSCSVMHEALHNHYTQKSLSLSPGK | Clone CH3C.35.23.2.1 with hole and YTE mutations |
| 230 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWANYKTTPPVLDSDGSFFLVSKLTV SKSEWQQGFVFSCSVMHEALHNHYTQKSLSLSPGK | Clone CH3C.35.23.2.1 with hole, LALA, and YTE mutations |
| 231 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLS CAVKGFYPSDIAVEWESYGTEWANYKTTPPVLDSDGSFFLVSKLTV SKSEWQQGFVFSCSVMHEALHNHYTQKSLSLSPGK | Clone CH3C.35.23.2.1 with hole, LALAPG, and YTE mutations |
| 232 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESFGTEWSNYKTTPPVLDSDGSFFLYSKLTVS KEEWQQGFVFSCSVMHEALHNHYTQKSLSLSPGK | Clone CH3C.35.23.1.1 with knob mutation |
| 233 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVEWESFGTEWSNYKTTPPVLDSDGSFFLYSKLT VSKEEWQQGFVFSCSVMHEALHNHYTQKSLSLSPGK | Clone CH3C.35.23.1.1 with knob and LALA mutations |
| 234 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVEWESFGTEWSNYKTTPPVLDSDGSFFLYSKLT VSKEEWQQGFVFSCSVMHEALHNHYTQKSLSLSPGK | Clone CH3C.35.23.1.1 with knob and LALAPG mutations |
| 235 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESFGTEWSNYKTTPPVLDSDGSFFLYSKLTVS KEEWQQGFVFSCSVMHEALHNHYTQKSLSLSPGK | Clone CH3C.35.23.1.1 with knob and YTE mutations |
| 236 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESFGTEWSNYKTTPPVLDSDGSFFLYSKLTVS KEEWQQGFVFSCSVMHEALHNHYTQKSLSLSPGK | Clone CH3C.35.23.1.1 with knob, LALA, and YTE mutations |
| 237 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLW CLVKGFYPSDIAVEWESFGTEWSNYKTTPPVLDSDGSFFLYSKLTVS KEEWQQGFVFSCSVMHEALHNHYTQKSLSLSPGK | Clone CH3C.35.23.1.1 with knob, LALAPG, and YTE mutations |
| 238 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESFGTEWSNYKTTPPVLDSDGSFFLVSKLTVS KEEWQQGFVFSCSVMHEALHNHYTQKSLSLSPGK | Clone CH3C.35.23.1.1 with hole mutations |
| 239 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVEWESFGTEWSNYKTTPPVLDSDGSFFLVSKLTV SKEEWQQGFVFSCSVMHEALHNHYTQKSLSLSPGK | Clone CH3C.35.23.1.1 with hole and LALA mutations |
| 240 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVEWESFGTEWSNYKTTPPVLDSDGSFFLVSKLTV SKEEWQQGFVFSCSVMHEALHNHYTQKSLSLSPGK | Clone CH3C.35.23.1.1 with hole and LALAPG mutations |
| 241 | PCPAPELLGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESFGTEWSNYKTTPPVLDSDGSFFLVSKLTVS KEEWQQGFVFSCSVMHEALHNHYTQKSLSLSPGK | Clone CH3C.35.23.1.1 with hole and YTE mutations |
| 242 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESFGTEWSNYKTTPPVLDSDGSFFLVSKLTVS KEEWQQGFVFSCSVMHEALHNHYTQKSLSLSPGK | Clone CH3C.35.23.1.1 with hole, LALA, and YTE mutations |
| 243 | PCPAPEAAGGPSVFLFPPKPKDTLYITREPEVTCVVVDVSHEDPEVKF NWYVDGVEVHNAKTKPREEQYNSTYRVVSVLTVLHQDWLNGKEY KCKVSNKALGAPIEKTISKAKGQPREPQVYTLPPSRDELTKNQVSLS CAVKGFYPSDIAVEWESFGTEWSNYKTTPPVLDSDGSFFLVSKLTVS KEEWQQGFVFSCSVMHEALHNHYTQKSLSLSPGK | Clone CH3C.35.23.1.1 with hole, LALAPG, and YTE mutations |
| 244 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESFGTEWSSYKTTPPVLDSDGSFFLYSKLTVT KEEWQQGFVFSCSVLHEALHSHYTQKSLSLSPGK | Clone CH3C.35.20.1 M201L and N207S mutations |
| 245 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESFGTEWSSYKTTPPVLDSDGSFFLYSKLTVT KEEWQQGFVFSCSVLHEALHSHYTQKSLSLSPGK | Clone CH3C.35.20.1 with knob and M201L and N207S mutations |
| 246 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVEWESFGTEWSSYKTTPPVLDSDGSFFLYSKLTV TKEEWQQGFVFSCSVLHEALHSHYTQKSLSLSPGK | Clone CH3C.35.20.1 with knob, LALA, and M201L and N207S mutations |
| 247 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVEWESFGTEWSSYKTTPPVLDSDGSFFLYSKLTV TKEEWQQGFVFSCSVLHEALHSHYTQKSLSLSPGK | Clone CH3C.35.20.1 with knob, LALAPG, and M201L and N207S mutations |
| 248 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESFGTEWSSYKTTPPVLDSDGSFFLVSKLTVT KEEWQQGFVFSCSVLHEALHSHYTQKSLSLSPGK | Clone CH3C.35.20.1 with hole and M201L and N207S mutations |
| 249 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVEWESFGTEWSSYKTTPPVLDSDGSFFLVSKLTV TKEEWQQGFVFSCSVLHEALHSHYTQKSLSLSPGK | Clone CH3C.35.20.1 with hole, LALA, and M201L and N207S mutations |
| 250 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVEWESFGTEWSSYKTTPPVLDSDGSFFLVSKLTV TKEEWQQGFVFSCSVLHEALHSHYTQKSLSLSPGK | Clone CH3C.35.20.1 with hole, LALAPG, and M201L and N207S mutations |
| 251 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWANYKTTPPVLDSDGSFFLYSKLTV TKEEWQQGFVFSCSVLHEALHSHYTQKSLSLSPGK | Clone CH3C.35.23.2 with M201L and N207S mutations |
| 252 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWANYKTTPPVLDSDGSFFLYSKLTV TKEEWQQGFVFSCSVLHEALHSHYTQKSLSLSPGK | Clone CH3C.35.23.2 with knob and M201L and N207S mutations |
| 253 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVEWESYGTEWANYKTTPPVLDSDGSFFLYSKLT VTKEEWQQGFVFSCSVLHEALHSHYTQKSLSLSPGK | Clone CH3C.35.23.2 with knob, LALA, and M201L and N207S mutations |
| 254 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVEWESYGTEWANYKTTPPVLDSDGSFFLYSKLT VTKEEWQQGFVFSCSVLHEALHSHYTQKSLSLSPGK | Clone CH3C.35.23.2 with knob, LALAPG, and M201L and N207S mutations |
| 255 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWANYKTTPPVLDSDGSFFLVSKLTV TKEEWQQGFVFSCSVLHEALHSHYTQKSLSLSPGK | Clone CH3C.35.23.2 with hole and M201L and N207S mutations |
| 256 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVEWESYGTEWANYKTTPPVLDSDGSFFLVSKLT VTKEEWQQGFVFSCSVLHEALHSHYTQKSLSLSPGK | Clone CH3C.35.23.2 with hole, LALA, and M201L and N207S mutations |
| 257 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVEWESYGTEWANYKTTPPVLDSDGSFFLVSKLT VTKEEWQQGFVFSCSVLHEALHSHYTQKSLSLSPGK | Clone CH3C.35.23.2 with hole, LALAPG, and M201L and N207S mutations |
| 258 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWVNYKTTPPVLDSDGSFFLYSKLTV TKEEWQQGFVFSCSVLHEALHSHYTQKSLSLSPGK | Clone CH3C.35.23.3 with M201L and N207S mutations |
| 259 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWVNYKTTPPVLDSDGSFFLYSKLTV TKEEWQQGFVFSCSVLHEALHSHYTQKSLSLSPGK | Clone CH3C.35.23.3 with knob and M201L and N207S mutations |
| 260 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVEWESYGTEWVNYKTTPPVLDSDGSFFLYSKLT VTKEEWQQGFVFSCSVLHEALHSHYTQKSLSLSPGK | Clone CH3C.35.23.3 with knob, LALA, and M201L and N207S mutations |
| 261 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVEWESYGTEWVNYKTTPPVLDSDGSFFLYSKLT VTKEEWQQGFVFSCSVLHEALHSHYTQKSLSLSPGK | Clone CH3C.35.23.3 with knob, LALAPG, and M201L and N207S mutations |
| 262 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWVNYKTTPPVLDSDGSFFLVSKLTV TKEEWQQGFVFSCSVLHEALHSHYTQKSLSLSPGK | Clone CH3C.35.23.3 with hole and M201L and N207S mutations |
| 263 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVEWESYGTEWVNYKTTPPVLDSDGSFFLVSKLT VTKEEWQQGFVFSCSVLHEALHSHYTQKSLSLSPGK | Clone CH3C.35.23.3 with hole, LALA, and M201L and N207S mutations |
| 264 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVEWESYGTEWVNYKTTPPVLDSDGSFFLVSKLT VTKEEWQQGFVFSCSVLHEALHSHYTQKSLSLSPGK | Clone CH3C.35.23.3 with hole, LALAPG, and M201L and N207S mutations |
| 265 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWSNYKTTPPVLDSDGSFFLYSKLTVS KEEWQQGFVFSCSVLHEALHSHYTQKSLSLSPGK | Clone CH3C.35.23.4 with M201L and N207S mutations |
| 266 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWSNYKTTPPVLDSDGSFFLYSKLTVS KEEWQQGFVFSCSVLHEALHSHYTQKSLSLSPGK | Clone CH3C.35.23.4 with knob and M201L and N207S mutations |
| 267 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVEWESYGTEWSNYKTTPPVLDSDGSFFLYSKLT VSKEEWQQGFVFSCSVLHEALHSHYTQKSLSLSPGK | Clone CH3C.35.23.4 with knob, LALA, and M201L and N207S mutations |
| 268 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVEWESYGTEWSNYKTTPPVLDSDGSFFLYSKLT VSKEEWQQGFVFSCSVLHEALHSHYTQKSLSLSPGK | Clone CH3C.35.23.4 with knob, LALAPG, and M201L and N207S mutations |
| 269 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWSNYKTTPPVLDSDGSFFLVSKLTVS KEEWQQGFVFSCSVLHEALHSHYTQKSLSLSPGK | Clone CH3C.35.23.4 with hole and M201L and N207S mutations |
| 270 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVEWESYGTEWSNYKTTPPVLDSDGSFFLVSKLTV SKEEWQQGFVFSCSVLHEALHSHYTQKSLSLSPGK | Clone CH3C.35.23.4 with hole, LALA, and M201L and N207S mutations |
| 271 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVEWESYGTEWSNYKTTPPVLDSDGSFFLVSKLTV SKEEWQQGFVFSCSVLHEALHSHYTQKSLSLSPGK | Clone CH3C.35.23.4 with hole, LALAPG, and M201L and N207S mutations |
| 272 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVLWESYGTEWASYKTTPPVLDSDGSFFLYSKLTVT KEEWQQGFVFSCSVLHEALHSHYTQKSLSLSPGK | Clone CH3C.35.21.17.2 with M201L and N207S mutations |
| 273 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVLWESYGTEWASYKTTPPVLDSDGSFFLYSKLTVT KEEWQQGFVFSCSVLHEALHSHYTQKSLSLSPGK | Clone CH3C.35.21.17.2 with knob and M201L and N207S mutations |
| 274 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVLWESYGTEWASYKTTPPVLDSDGSFFLYSKLT VTKEEWQQGFVFSCSVLHEALHSHYTQKSLSLSPGK | Clone CH3C.35.21.17.2 with knob, LALA, and M201L and N207S mutations |
| 275 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVLWESYGTEWASYKTTPPVLDSDGSFFLYSKLT VTKEEWQQGFVFSCSVLHEALHSHYTQKSLSLSPGK | Clone CH3C.35.21.17.2 with knob, LALAPG, and M201L and N207S mutations |
| 276 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVLWESYGTEWASYKTTPPVLDSDGSFFLVSKLTV TKEEWQQGFVFSCSVLHEALHSHYTQKSLSLSPGK | Clone CH3C.35.21.17.2 with hole and M201L and N207S mutations |
| 277 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVLWESYGTEWASYKTTPPVLDSDGSFFLVSKLTV TKEEWQQGFVFSCSVLHEALHSHYTQKSLSLSPGK | Clone CH3C.35.21.17.2 with hole, LALA, and M201L and N207S mutations |
| 278 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVLWESYGTEWASYKTTPPVLDSDGSFFLVSKLTV TKEEWQQGFVFSCSVLHEALHSHYTQKSLSLSPGK | Clone CH3C.35.21.17.2 with hole, LALAPG, and M201L and N207S mutations |
| 279 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWSNYKTTPPVLDSDGSFFLYSKLTVT KEEWQQGFVFSCSVLHEALHSHYTQKSLSLSPGK | Clone CH3C.35.23 with M201L and N207S mutations |
| 280 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWSNYKTTPPVLDSDGSFFLYSKLTVT KEEWQQGFVFSCSVLHEALHSHYTQKSLSLSPGK | Clone CH3C.35.23 with knob and M201L and N207S mutations |
| 281 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVEWESYGTEWSNYKTTPPVLDSDGSFFLYSKLT VTKEEWQQGFVFSCSVLHEALHSHYTQKSLSLSPGK | Clone CH3C.35.23 with knob, LALA, and M201L and N207S mutations |
| 282 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVEWESYGTEWSNYKTTPPVLDSDGSFFLYSKLT VTKEEWQQGFVFSCSVLHEALHSHYTQKSLSLSPGK | Clone CH3C.35.23 with knob, LALAPG, and M201L and N207S mutations |
| 283 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWSNYKTTPPVLDSDGSFFLVSKLTV TKEEWQQGFVFSCSVLHEALHSHYTQKSLSLSPGK | Clone CH3C.35.23 with hole and M201L and N207S mutations |
| 284 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVEWESYGTEWSNYKTTPPVLDSDGSFFLVSKLTV TKEEWQQGFVFSCSVLHEALHSHYTQKSLSLSPGK | Clone CH3C.35.23 with hole, LALA, and M201L and N207S mutations |
| 285 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVEWESYGTEWSNYKTTPPVLDSDGSFFLVSKLTV TKEEWQQGFVFSCSVLHEALHSHYTQKSLSLSPGK | Clone CH3C.35.23 with hole, LALAPG, and M201L and N207S mutations |
| 286 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVWWESYGTEWSSYKTTPPVLDSDGSFFLYSKLTV TKEEWQQGFVFSCSVLHEALHSHYTQKSLSLSPGK | Clone CH3C.35.21 with M201L and N207S mutations |
| 287 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVWWESYGTEWSSYKTTPPVLDSDGSFFLYSKLTV TKEEWQQGFVFSCSVLHEALHSHYTQKSLSLSPGK | Clone CH3C.35.21 with knob and M201L and N207S mutations |
| 288 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVWWESYGTEWSSYKTTPPVLDSDGSFFLYSKLT VTKEEWQQGFVFSCSVLHEALHSHYTQKSLSLSPGK | Clone CH3C.35.21 with knob, LALA, and M201L and N207S mutations |
| 289 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVWWESYGTEWSSYKTTPPVLDSDGSFFLYSKLT VTKEEWQQGFVFSCSVLHEALHSHYTQKSLSLSPGK | Clone CH3C.35.21 with knob, LALAPG, and M201L and N207S mutations |
| 290 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVWWESYGTEWSSYKTTPPVLDSDGSFFLVSKLTV TKEEWQQGFVFSCSVLHEALHSHYTQKSLSLSPGK | Clone CH3C.35.21 with hole and M201L and N207S mutations |
| 291 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVWWESYGTEWSSYKTTPPVLDSDGSFFLVSKLT VTKEEWQQGFVFSCSVLHEALHSHYTQKSLSLSPGK | Clone CH3C.35.21 with hole, LALA, and M201L and N207S mutations |
| 292 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVWWESYGTEWSSYKTTPPVLDSDGSFFLVSKLT VTKEEWQQGFVFSCSVLHEALHSHYTQKSLSLSPGK | Clone CH3C.35.21 with hole, LALAPG, and M201L and N207S mutations |
| 293 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESFGTEWSSYKTTPPVLDSDGSFFLYSKLTVS KEEWQQGFVFSCSVLHEALHSHYTQKSLSLSPGK | Clone CH3C.35.20.1.1 with M201L and N207S mutations |
| 294 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESFGTEWSSYKTTPPVLDSDGSFFLYSKLTVS KEEWQQGFVFSCSVLHEALHSHYTQKSLSLSPGK | Clone CH3C.35.20.1.1 with knob and M201L and N207S mutations |
| 295 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVEWESFGTEWSSYKTTPPVLDSDGSFFLYSKLTV SKEEWQQGFVFSCSVLHEALHSHYTQKSLSLSPGK | Clone CH3C.35.20.1.1 with knob, LALA, and M201L and N207S mutations |
| 296 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVEWESFGTEWSSYKTTPPVLDSDGSFFLYSKLTV SKEEWQQGFVFSCSVLHEALHSHYTQKSLSLSPGK | Clone CH3C.35.20.1.1 with knob, LALAPG, and M201L and N207S mutations |
| 297 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESFGTEWSSYKTTPPVLDSDGSFFLVSKLTVS KEEWQQGFVFSCSVLHEALHSHYTQKSLSLSPGK | Clone CH3C.35.20.1.1 with hole and M201L and N207S mutations |
| 298 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVEWESFGTEWSSYKTTPPVLDSDGSFFLVSKLTV SKEEWQQGFVFSCSVLHEALHSHYTQKSLSLSPGK | Clone CH3C.35.20.1.1 with hole, LALA, and M201L and N207S mutations |
| 299 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVEWESFGTEWSSYKTTPPVLDSDGSFFLVSKLTV SKEEWQQGFVFSCSVLHEALHSHYTQKSLSLSPGK | Clone CH3C.35.20.1.1 with hole, LALAPG, and M201L and N207S mutations |
| 300 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESYGTEWANYKTTPPVLDSDGSFFLYSKLTV SKSEWQQGFVFSCSVLHEALHSHYTQKSLSLSPGK | Clone CH3C.35.23.2.1 with M201L and N207S mutations |
| 301 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESYGTEWANYKTTPPVLDSDGSFFLYSKLTV SKSEWQQGFVFSCSVLHEALHSHYTQKSLSLSPGK | Clone CH3C.35.23.2.1 with knob and M201L and N207S mutations |
| 302 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVEWESYGTEWANYKTTPPVLDSDGSFFLYSKLT VSKSEWQQGFVFSCSVLHEALHSHYTQKSLSLSPGK | Clone CH3C.35.23.2.1 with knob, LALA, and M201L and N207S mutations |
| 303 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVEWESYGTEWANYKTTPPVLDSDGSFFLYSKLT VSKSEWQQGFVFSCSVLHEALHSHYTQKSLSLSPGK | Clone CH3C.35.23.2.1 with knob, LALAPG, and M201L and N207S mutations |
| 304 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESYGTEWANYKTTPPVLDSDGSFFLVSKLTV SKSEWQQGFVFSCSVLHEALHSHYTQKSLSLSPGK | Clone CH3C.35.23.2.1 with hole and M201L and N207S mutations |
| 305 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVEWESYGTEWANYKTTPPVLDSDGSFFLVSKLT VSKSEWQQGFVFSCSVLHEALHSHYTQKSLSLSPGK | Clone CH3C.35.23.2.1 with hole, LALA, and M201L and N207S mutations |
| 306 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVEWESYGTEWANYKTTPPVLDSDGSFFLVSKLT VSKSEWQQGFVFSCSVLHEALHSHYTQKSLSLSPGK | Clone CH3C.35.23.2.1 with hole, LALAPG, and M201L and N207S mutations |
| 307 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLT CLVKGFYPSDIAVEWESFGTEWSNYKTTPPVLDSDGSFFLYSKLTVS KEEWQQGFVFSCSVLHEALHSHYTQKSLSLSPGK | Clone CH3C.35.23.1.1 with M201L and N207S mutations |
| 308 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLW CLVKGFYPSDIAVEWESFGTEWSNYKTTPPVLDSDGSFFLYSKLTVS KEEWQQGFVFSCSVLHEALHSHYTQKSLSLSPGK | Clone CH3C.35.23.1.1 with knob and M201L and N207S mutations |
| 309 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL WCLVKGFYPSDIAVEWESFGTEWSNYKTTPPVLDSDGSFFLYSKLT VSKEEWQQGFVFSCSVLHEALHSHYTQKSLSLSPGK | Clone CH3C.35.23.1.1 with knob, LALA, and M201L and N207S mutations |
| 310 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL WCLVKGFYPSDIAVEWESFGTEWSNYKTTPPVLDSDGSFFLYSKLT VSKEEWQQGFVFSCSVLHEALHSHYTQKSLSLSPGK | Clone CH3C.35.23.1.1 with knob, LALAPG, and M201L and N207S mutations |
| 311 | PCPAPELLGGPSVFLFPPKPKDTLMISRTPEVTCVVVDVSHEDPEVKF NWYVDGVEVHNAKTKPREEQYNSTYRVVSVLTVLHQDWLNGKEY KCKVSNKALPAPIEKTISKAKGQPREPQVYTLPPSRDELTKNQVSLS CAVKGFYPSDIAVEWESFGTEWSNYKTTPPVLDSDGSFFLVSKLTVS KEEWQQGFVFSCSVLHEALHSHYTQKSLSLSPGK | Clone CH3C.35.23.1.1 with hole and M201L and N207S mutations |
| 312 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALPAPIEKTISKAKGQPREPQVYTLPPSRDELTKNQVSL SCAVKGFYPSDIAVEWESFGTEWSNYKTTPPVLDSDGSFFLVSKLTV SKEEWQQGFVFSCSVLHEALHSHYTQKSLSLSPGK | Clone CH3C.35.23.1.1 with hole, LALA, and M201L and N207S mutations |
| 313 | PCPAPEAAGGPSVFLFPPKPKDTLMISRTPEVTCVVVDVSHEDPEVK FNWYVDGVEVHNAKTKPREEQYNSTYRVVSVLTVLHQDWLNGKE YKCKVSNKALGAPIEKTISKAKGQPREPQVYTLPPSRDELTKNQVSL SCAVKGFYPSDIAVEWESFGTEWSNYKTTPPVLDSDGSFFLVSKLTV SKEEWQQGFVFSCSVLHEALHSHYTQKSLSLSPGK | Clone CH3C.35.23.1.1 with hole, LALAPG, and M201L and N207S mutations |
| 314 | X₁WESX₂GX₃X₄WX₅X₆ | CH3C.35_consensus sequence_1 |
| | X₁ is E, L, S, V, W, or Y; X₂ is an aromatic amino acid (*e.g.,* Y, F, or W), M, P, or V; X₃ is T, N, or V; X₄ is E, I, P, or V; X₅ is an aliphatic amino acid (*e.g.*, A, I, or V), S, or T; and X₆ is S, N, R, or T | |
| 315 | X₁KX₂X₃WQQGX₄VFX₅CX₆ | CH3C.35_consensus sequence_2 |
| | X₁ is T, H, or S; X₂ is E, S, D, G, T, P, Q, or R; X₃ is E or R; X₄ is F, H, K, Y, or W; X₅ is S, T, or W; and X₆ is S, C, P, M, or W | |
| 316 | GQPREPQVYTLPPSRDELTKNQVSLTCLVKGFYPSDIAVX1WESX2G X3X4WX5X6YKTTPPVLDSDGSFFLYSKLTVX7KX8X9WQQGX10VFX11 CX12VMHEALHNHYTQKSLSLSPGK X₁ is E, L, S, V, W, or Y; X₂ is an aromatic amino acid (*e.g.,* Y, F, or W), M, P, or V; X₃ is T, N, or V; X₄ is E, I, P, or V; X₅ is an aliphatic amino acid (*e.g.,* A, I, or V), S, or T; X₆ is S, N, R, or T; X₇ is T, H, or S; X₈ is E, S, D, G, T, P, Q, or R; X₉ is E or R; X₁₀ is F, H, K, Y, or W; X₁₁ is S, T, or W; and X₁₂ is S, C, P, M, or W | CH3C.35_consensus sequence_3 |
| 317 | X₁WESX₂GX₃X₄WX₅X₆ | CH3C.35_consensus sequence_4 |
| | X₁ is E, L, or W; X₂ is an aromatic amino acid (*e.g.,* Y or F); X₃ is T; X₄ is E; X₅ is an aliphatic amino acid (*e.g.,* A or V) or S; and X₆ is S or N | |
| 318 | X₁KX₂X₃WQQGX₄VFX₅CX₆ | CH3C.35_consensus sequence_5 |
| | X₁ is T or S; X₂ is E or S; X₃ is E; X₄ is F, H, Y, or W; X₅ is S; and X₆ is S | |
| 319 | GQPREPQVYTLPPSRDELTKNQVSLTCLVKGFYPSDIAVX1WESX2G X3X4WX5X6YKTTPPVLDSDGSFFLYSKLTVX7KX8X9WQQGX10VFX11 CX12VMHEALHNHYTQKSLSLSPGK X₁ is E, L, or W; X₂ is an aromatic amino acid (*e*.*g*., Y or F); X₃ is T; X₄ is E; X₅ is an aliphatic amino acid (*e*.*g*., A or V) or S; X₆ is S or N; X₇ is T or S; X₈ is E or S; X₉ is E; X₁₀ is F, H, Y, or W; X₁₁ is S; and X₁₂ is S | CH3C.35_consensus sequence_6 |
| 320 | X₁WESX₂GX₃X₄WX₅X₆ | CH3C.35_consensus sequence_7 |
| | X₁ is E, L, or W; X₂ is Y or F; X₃ is T; X₄ is E; X₅ is S, A or V; and X₆ is S or N | |
| 321 | X₁KX₂X₃WQQGX₄VFX₅CX₆ | CH3C.35_consensus sequence_8 |
| | X₁ is T or S; X₂ is E or S; X₃ is E; X₄ is F; X₅ is S; and X₆ is S | |
| 322 | GQPREPQVYTLPPSRDELTKNQVSLTCLVKGFYPSDIAVX1WESX2G X3X4WX5X6YKTTPPVLDSDGSFFLYSKLTVX7KX8X9WQQGX10VFX11 CX12VMHEALHNHYTQKSLSLSPGK | CH3C.35_consensus sequence_9 |
| | X₁ is E, L, or W; X₂ is Y or F; X₃ is T; X₄ is E; X₅ is S, A or V; X₆ is S or N; X₇ is T or S; X₈ is E or S; X₉ is E; X₁₀ is F; X₁₁ is S; and X₁₂ is S | |
| 323 | GQPREPQVYTLPPSRDELTKNQVSLTCLVKGFYPSDIAVXWESYGTE WSSYKTTPPVLDSDGSFFLYSKLTVTKEEWQQGFVFSCSVMHEALH NHYTQKSLSLSPGK | CH3C.35_consensus sequence_10 |
| | X is E, L, S, V, W, or Y | |
| 324 | GQPREPQVYTLPPSRDELTKNQVSLTCLVKGFYPSDIAVWWESXGT EWSSYKTTPPVLDSDGSFFLYSKLTVTKEEWQQGFVFSCSVMHEAL HNHYTQKSLSLSPGK | CH3C.35_consensus sequence_11 |
| | X is Y, F, M, P, V, or W | |
| 325 | GQPREPQVYTLPPSRDELTKNQVSLTCLVKGFYPSDIAVWWESYGX EWSSYKTTPPVLDSDGSFFLYSKLTVTKEEWQQGFVFSCSVMHEAL HNHYTQKSLSLSPGK | CH3C.35_consensus sequence_12 |
| | X is T, N, or V | |
| 326 | GQPREPQVYTLPPSRDELTKNQVSLTCLVKGFYPSDIAVWWESYGT XWSSYKTTPPVLDSDGSFFLYSKLTVTKEEWQQGFVFSCSVMHEAL HNHYTQKSLSLSPGK | CH3C.35_consensus sequence_13 |
| | X is E, I, P, or V | |
| 327 | GQPREPQVYTLPPSRDELTKNQVSLTCLVKGFYPSDIAVWWESYGT EWXSYKTTPPVLDSDGSFFLYSKLTVTKEEWQQGFVFSCSVMHEAL HNHYTQKSLSLSPGK | CH3C.35_consensus sequence_14 |
| | X is S, A, I, T, or V | |
| 328 | GQPREPQVYTLPPSRDELTKNQVSLTCLVKGFYPSDIAVWWESYGT EWSXYKTTPPVLDSDGSFFLYSKLTVTKEEWQQGFVFSCSVMHEAL HNHYTQKSLSLSPGK | CH3C.35_consensus sequence_15 |
| | X is S, N, R, or T | |
| 329 | GQPREPQVYTLPPSRDELTKNQVSLTCLVKGFYPSDIAVWWESYGT EWSSYKTTPPVLDSDGSFFLYSKLTVXKEEWQQGFVFSCSVMHEAL HNHYTQKSLSLSPGK | CH3C.35_consensus sequence_16 |
| | X is T, H, or S | |
| 330 | GQPREPQVYTLPPSRDELTKNQVSLTCLVKGFYPSDIAVWWESYGT EWSSYKTTPPVLDSDGSFFLYSKLTVTKXEWQQGFVFSCSVMHEAL HNHYTQKSLSLSPGK | CH3C.35_consensus sequence_17 |
| | X is E, S, D, G, T, P, Q, or R | |
| 331 | GQPREPQVYTLPPSRDELTKNQVSLTCLVKGFYPSDIAVWWESYGT EWSSYKTTPPVLDSDGSFFLYSKLTVTKEXWQQGFVFSCSVMHEAL HNHYTQKSLSLSPGK | CH3C.35_consensus sequence_18 |
| | X is E or R | |
| 332 | GQPREPQVYTLPPSRDELTKNQVSLTCLVKGFYPSDIAVWWESYGT EWSSYKTTPPVLDSDGSFFLYSKLTVTKEEWQQGXVFSCSVMHEAL HNHYTQKSLSLSPGK | CH3C.35_consensus sequence_19 |
| | X is F, H, K, or Y | |
| 333 | GQPREPQVYTLPPSRDELTKNQVSLTCLVKGFYPSDIAVWWESYGT EWSSYKTTPPVLDSDGSFFLYSKLTVTKEEWQQGFVFXCSVMHEAL HNHYTQKSLSLSPGK | CH3C.35_consensus sequence_20 |
| | X is S, T, or W | |
| 334 | GQPREPQVYTLPPSRDELTKNQVSLTCLVKGFYPSDIAVWWESYGT EWSSYKTTPPVLDSDGSFFLYSKLTVTKEEWQQGFVFSCXVMHEAL HNHYTQKSLSLSPGK | CH3C.35_consensus sequence_21 |
| | X is S, C, P, M, or W | |
| 335 | X₁WESX₂GX₃X₄WX₅X₆X₇ | CH3C.18_consensus sequence_1 |
| | X₁ is E or W; X₂ is V, W, L, or Y; X₃ is L, P, F, T, or H; X₄ is P, V, or E; X₅ is A, S, V, or G; X₆ is L, H, Q, G, V, A N, D, T, or E; and X₇ is T, F, Q, V, or Y | |
| 336 | X₁KSX₂WQQGX₃ | CH3C.18_consensus sequence_2 |
| | X₁ is L, S, E, A, or P; X₂ is E, D, T, or N; and X₂ is W, Y, H, or F | |
| 337 | GQPREPQVYTLPPSRDELTKNQVSLTCLVKGFYPSDIAVX1WESX2G X3X4WX5X6X7KTTPPVLDSDGSFFLYSKLTVX8KSX9WQQGX10VFSCS VMHEALHNHYTQKSLSLSPGK | CH3C.18_consensus sequence_3 |
| | X₁ is E or W; X₂ is V, W, L, or Y; X₃ is L, P, F, T, or H; X₄ is P, V, or E; X₅ is A, S, V, or G; X₆ is L, H, Q, G, V, A N, D, T, or E; X₇ is T, F, Q, V, or Y; X₈ is L, S, E, A, or P; X₉ is E, D, T, or N; and X₁₀ is W, Y, H, or F | |
| 338 | X₁WESX₂GX₃X₄WX₅X₆X₇ | CH3C.18_consensus sequence_4 |
| | X₁ is E or W; X₂ is W, L, or Y; X₃ is T or H; X₄ is V; X₅ is A, S, or V; X₆ is V, T, or N; and X₇ is Y or Q | |
| 339 | X₁KSX₂WQQGX₃ | CH3C.18_consensus sequence_5 |
| | X₁ is P; X₂ is T or N; and X₃ is W, Y, H, or F | |
| 340 | GQPREPQVYTLPPSRDELTKNQVSLTCLVKGFYPSDIAVX1WESX2G X3X4WX5X6X7KTTPPVLDSDGSFFLYSKLTVX8KSX9WQQGX10VFSCS VMHEALHNHYTQKSLSLSPGK | CH3C.18_consensus sequence_6 |
| | X₁ is E or W; X₂ is W, L, or Y; X₃ is T or H; X₄ is V; X₅ is A, S, or V; X₆ is V, T, or N; X₇ is Y or Q; X₈ is P; X₉ is T or N; and X₁₀ is W, Y, H, or F | |
| 341 | TXWSX | Clone motif |
| 342 | NSVIIVDKNGRLVYLVENPGGYVAYSKAATVTGKLVHANFGTKKD FEDLDSPVNGSIVIVRAGKITFAEKVANAESLNAIGVLIYMDQTKFPI VNAXLSFFGHAHLGTGDPYTPGFPSFNHTQFPPSRSSGLPNIPVQTIS RAAAEKLFGNMEGDCPSDWKTDSTCRMVTSENKNVKLTVS | Consensus sequence between human and cyno TfR |
| | X is D or E | |
| 343 | | IGHG1_P01857 |
| 344 | ASTKGPSVFPLAPCSRSTSESTAALGCLVKDYFPEPVTVSWNSGALT SGVHTFPAVLQSSGLYSLSSVVTVPSSNFGTQTYTCNVDHKPSNTKV DKTVERKCCVECPPCPAPPVAGPSVFLFPPKPKDTLMISRTPEVTCV VVDVSHEDPEVQFNWYVDGVEVHNAKTKPREEQFNSTFRVVSVLT VVHQDWLNGKEYKCKVSNKGLPAPIEKTISKTKGQPREPQVYTLPP SREEMTKNQVSLTCLVKGFYPSDISVEWESNGQPENNYKTTPPMLD SDGSFFLYSKLTVDKSRWQQGNVFSCSVMHEALHNHYTQKSLSLSP GK | IGHG2_P01859 |
| 345 | ASTKGPSVFPLAPCSRSTSGGTAALGCLVKDYFPEPVTVSWNSGALT SGVHTFPAVLQSSGLYSLSSVVTVPSSSLGTQTYTCNVNHKPSNTKV DKRVELKTPLGDTTHTCPRCPEPKSCDTPPPCPRCPEPKSCDTPPPCP RCPEPKSCDTPPPCPRCPAPELLGGPSVFLFPPKPKDTLMISRTPEVTC VVVDVSHEDPEVQFKWYVDGVEVHNAKTKPREEQYNSTFRVVSVL TVLHQDWLNGKEYKCKVSNKALPAPIEKTISKTKGQPREPQVYTLP PSREEMTKNQVSLTCLVKGFYPSDIAVEWESSGQPENNYNTTPPML DSDGSFFLYSKLTVDKSRWQQGNIFSCSVMHEALHNRFTQKSLSLSP GK | IGHG3_P01860 |
| 346 | ASTKGPSVFPLAPCSRSTSESTAALGCLVKDYFPEPVTVSWNSGALT SGVHTFPAVLQSSGLYSLSSVVTVPSSSLGTKTYTCNVDHKPSNTKV DKRVESKYGPPCPSCPAPEFLGGPSVFLFPPKPKDTLMISRTPEVTCV VVDVSQEDPEVQFNWYVDGVEVHNAKTKPREEQFNSTYRVVSVLT VLHQDWLNGKEYKCKVSNKGLPSSIEKTISKAKGQPREPQVYTLPPS QEEMTKNQVSLTCLVKGFYPSDIAVEWESNGQPENNYKTTPPVLDS DGSFFLYSRLTVDKSRWQEGNVFSCSVMHEALHNHYTQKSLSLSLG K | IGHG4_P01861 |
| 347 | APEAAGGPSVFLFPPKPKDTLMISRTPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSRDELTKNQVSLWCL VKGFYPSDIAVEWESX₁GTEWX₂NYKTTPPVLDSDGSFFLYSKLTVX₃ KEEWQQGFVFSCSVX₄HEALHX₅HYTQKSLSLSPGK, wherein X₁ is F or Y; X₂ is S, A, or V; X₃ is S or T; X₄ is M or L; and X₅ is N or S | Consensus sequence of clones CH3C.35.23.1.1, CH3C.35.23.3, CH3C.35.23.4, CH3C.35.23, and CH3C.35.23.2 with knob, LALA, and optionally M428L and N434S mutations (EU numbering) |
| 348 | APEAAGGPSVFLFPPKPKDTLMISRTPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSRDELTKNQVSLSCA VKGFYPSDIAVEWESX₁GTEWX₂NYKTTPPVLDSDGSFFLVSKLTVX₃ KEEWQQGFVFSCSVX₄HEALHX₅HYTQKSLSLSPGK, wherein X₁ is F or Y; X₂ is S, A, or V; X₃ is S or T; X₄ is M or L; and X₅ is N or S | Consensus sequence of clones CH3C.35.23.1.1, CH3C.35.23.3, CH3C.35.23.4, CH3C.35.23, and CH3C.35.23.2 with hole, LALA, and optionally M428L and N434S mutations (EU numbering) |
| 349 | APEAAGGPSVFLFPPKPKDTLMISRTPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSRDELTKNQVSLWCL VKGFYPSDIAVEWESNGQPENNYKTTPPVLDSDGSFFLYSKLTVDKS RWQQGNVFSCSVX₁HEALHX₂HYTQKSLSLSPGK, wherein X₁ is M or L; and X₂ is N or S | Fc sequence with knob, LALA, and optionally M428L and N434S mutations (EU numbering) |
| 350 | APEAAGGPSVFLFPPKPKDTLMISRTPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSRDELTKNQVSLSCA VKGFYPSDIAVEWESNGQPENNYKTTPPVLDSDGSFFLVSKLTVDKS RWQQGNVFSCSVX₁HEALHX₂HYTQKSLSLSPGK, wherein X₁ is M or L; and X₂ is N or S | Fc sequence with hole, LALA, and optionally M428L and N434S mutations (EU numbering) |
| 351 | APELLGGPSVFLFPPKPKDTLMISRTPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSRDELTKNQVSLWCL VKGFYPSDIAVEWESNGQPENNYKTTPPVLDSDGSFFLYSKLTVDKS RWQQGNVFSCSVMHEALHNHYTQKSLSLSPGK | Fc sequence with knob mutation |
| 352 | APEAAGGPSVFLFPPKPKDTLMISRTPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSRDELTKNQVSLWCL VKGFYPSDIAVEWESNGQPENNYKTTPPVLDSDGSFFLYSKLTVDKS RWQQGNVFSCSVMHEALHNHYTQKSLSLSPGK | Fc sequence with knob and LALA mutations |
| 353 | APEAAGGPSVFLFPPKPKDTLMISRTPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSRDELTKNQVSLWCL VKGFYPSDIAVEWESNGQPENNYKTTPPVLDSDGSFFLYSKLTVDKS RWQQGNVFSCSVLHEALHSHYTQKSLSLSPGK | Fc sequence with knob, LALA, and M428L and N434S mutations (EU numbering) |
| 354 | APELLGGPSVFLFPPKPKDTLMISRTPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSRDELTKNQVSLSCA VKGFYPSDIAVEWESNGQPENNYKTTPPVLDSDGSFFLVSKLTVDKS RWQQGNVFSCSVMHEALHNHYTQKSLSLSPGK | Fc sequence with hole mutations |
| 355 | APEAAGGPSVFLFPPKPKDTLMISRTPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSRDELTKNQVSLSCA VKGFYPSDIAVEWESNGQPENNYKTTPPVLDSDGSFFLVSKLTVDKS RWQQGNVFSCSVMHEALHNHYTQKSLSLSPGK | Fc sequence with hole and LALA mutations |
| 356 | APEAAGGPSVFLFPPKPKDTLMISRTPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSRDELTKNQVSLSCA VKGFYPSDIAVEWESNGQPENNYKTTPPVLDSDGSFFLVSKLTVDKS RWQQGNVFSCSVLHEALHSHYTQKSLSLSPGK | Fc sequence with hole, LALA, and M428L and N434S mutations (EU numbering) |

## Claims

1. A polypeptide that specifically binds to a transferrin receptor, comprising a sequence having at least 85%, 90%, or 95% sequence identity to the sequence of any one of SEQ ID NOS:83, 84, 191, 43, and 82, wherein the polypeptide comprises Glu at position 153, Tyr or Phe at position 157, Thr at position 159, Glu at position 160, Trp at position 161, Val, Ser, or Ala at position 162, Asn at position 163, Thr or Ser at position 186, Glu at position 188, Glu at position 189, and Phe at position 194, as numbered with reference to SEQ ID NO: 1.

2. The polypeptide of claim 1, further comprising:
(a) Trp at position 139, as numbered with reference to SEQ ID NO: 1;
(b) Ala at position 7, Ala at position 8, and Trp at position 139, as numbered with reference to SEQ ID NO: 1;
(c) Ala at position 7, Ala at position 8, Gly at position 102, and Trp at position 139, as numbered with reference to SEQ ID NO: 1;
(d) Ala at position 7, Ala at position 8, Trp at position 139, Leu at position 201, and Ser at position 207, as numbered with reference to SEQ ID NO: 1;
(e) Ala at position 7, Ala at position 8, Gly at position 102, Trp at position 139, Leu at position 201, and Ser at position 207, as numbered with reference to SEQ ID NO:1;
(f) Ser at position 139, Ala at position 141, and Val at position 180, as numbered with reference to SEQ ID NO: 1;
(g) Ala at position 7, Ala at position 8, Ser at position 139, Ala at position 141, and Val at position 180, as numbered with reference to SEQ ID NO: 1;
(h) Ala at position 7, Ala at position 8, Gly at position 102, Ser at position 139, Ala at position 141, and Val at position 180, as numbered with reference to SEQ ID NO:1;
(i) Ala at position 7, Ala at position 8, Ser at position 139, Ala at position 141, Val at position 180, Leu at position 201, and Ser at position 207, as numbered with reference to SEQ ID NO:1; or
(j) Ala at position 7, Ala at position 8, Gly at position 102, Ser at position 139, Ala at position 141, Val at position 180, Leu at position 201, and Ser at position 207, as numbered with reference to SEQ ID NO:1.

3. The polypeptide of claim 1, comprising:
A. a sequence having at least 85%, 90%, or 95% sequence identity to the sequence of SEQ ID NO:83, wherein the polypeptide comprises Glu at position 153, Tyr at position 157, Thr at position 159, Glu at position 160, Trp at position 161, Val at position 162, Asn at position 163, Thr at position 186, Glu at position 188, Glu at position 189, and Phe at position 194, as numbered with reference to SEQ ID NO:1, optionally further comprising:
(a) Trp at position 139, as numbered with reference to SEQ ID NO:1, optionally wherein the polypeptide comprises the sequence of SEQ ID NO:133;
(b) Ala at position 7, Ala at position 8, and Trp at position 139, as numbered with reference to SEQ ID NO:1, optionally wherein the polypeptide comprises the sequence of SEQ ID NO:134;
(c) Ala at position 7, Ala at position 8, Gly at position 102, and Trp at position 139, as numbered with reference to SEQ ID NO:1, optionally wherein the polypeptide comprises the sequence of SEQ ID NO:135;
(d) Ala at position 7, Ala at position 8, Trp at position 139, Leu at position 201, and Ser at position 207, as numbered with reference to SEQ ID NO:1, optionally wherein the polypeptide comprises the sequence of SEQ ID NO:260;
(e) Ala at position 7, Ala at position 8, Gly at position 102, Trp at position 139, Leu at position 201, and Ser at position 207, as numbered with reference to SEQ ID NO:1, optionally wherein the polypeptide comprises the sequence of SEQ ID NO:261;
(f) Ser at position 139, Ala at position 141, and Val at position 180, as numbered with reference to SEQ ID NO:1, optionally wherein the polypeptide comprises the sequence of SEQ ID NO:139;
(g) Ala at position 7, Ala at position 8, Ser at position 139, Ala at position 141, and Val at position 180, as numbered with reference to SEQ ID NO:1, optionally wherein the polypeptide comprises the sequence of SEQ ID NO:140;
(h) Ala at position 7, Ala at position 8, Gly at position 102, Ser at position 139, Ala at position 141, and Val at position 180, as numbered with reference to SEQ ID NO:1, optionally wherein the polypeptide comprises the sequence of SEQ ID NO:141;
(i) Ala at position 7, Ala at position 8, Ser at position 139, Ala at position 141, Val at position 180, Leu at position 201, and Ser at position 207, as numbered with reference to SEQ ID NO:1, optionally wherein the polypeptide comprises the sequence of SEQ ID NO:263; or
(j) Ala at position 7, Ala at position 8, Gly at position 102, Ser at position 139, Ala at position 141, Val at position 180, Leu at position 201, and Ser at position 207, as numbered with reference to SEQ ID NO:1, optionally wherein the polypeptide comprises the sequence of SEQ ID NO:264; or
B. a sequence having at least 85%, 90%, or 95% sequence identity to the sequence of SEQ ID NO:84, wherein the polypeptide comprises Glu at position 153, Tyr at position 157, Thr at position 159, Glu at position 160, Trp at position 161, Ser at position 162, Asn at position 163, Ser at position 186, Glu at position 188, Glu at position 189, and Phe at position 194, as numbered with reference to SEQ ID NO:1, optionally further comprising:
(a) Trp at position 139, as numbered with reference to SEQ ID NO:1, optionally wherein the polypeptide comprises the sequence of SEQ ID NO:145;
(b) Ala at position 7, Ala at position 8, and Trp at position 139, as numbered with reference to SEQ ID NO:1, optionally wherein the polypeptide comprises the sequence of SEQ ID NO:146;
(c) Ala at position 7, Ala at position 8, Gly at position 102, and Trp at position 139, as numbered with reference to SEQ ID NO:1, optionally wherein the polypeptide comprises the sequence of SEQ ID NO: 147;
(d) Ala at position 7, Ala at position 8, Trp at position 139, Leu at position 201, and Ser at position 207, as numbered with reference to SEQ ID NO:1, optionally wherein the polypeptide comprises the sequence of SEQ ID NO:267;
(e) Ala at position 7, Ala at position 8, Gly at position 102, Trp at position 139, Leu at position 201, and Ser at position 207, as numbered with reference to SEQ ID NO:1, optionally wherein the polypeptide comprises the sequence of SEQ ID NO:268;
(f) Ser at position 139, Ala at position 141, and Val at position 180, as numbered with reference to SEQ ID NO:1, optionally wherein the polypeptide comprises the sequence of SEQ ID NO:151;
(g) Ala at position 7, Ala at position 8, Ser at position 139, Ala at position 141, and Val at position 180, as numbered with reference to SEQ ID NO:1, optionally wherein the polypeptide comprises the sequence of SEQ ID NO:152;
(h) Ala at position 7, Ala at position 8, Gly at position 102, Ser at position 139, Ala at position 141, and Val at position 180, as numbered with reference to SEQ ID NO: 1, optionally wherein the polypeptide comprises the sequence of SEQ ID NO: 153;
(i) Ala at position 7, Ala at position 8, Ser at position 139, Ala at position 141, Val at position 180, Leu at position 201, and Ser at position 207, as numbered with reference to SEQ ID NO:1, optionally wherein the polypeptide comprises the sequence of SEQ ID NO:270; or
(j) Ala at position 7, Ala at position 8, Gly at position 102, Ser at position 139, Ala at position 141, Val at position 180, Leu at position 201, and Ser at position 207, as numbered with reference to SEQ ID NO:1, optionally wherein the polypeptide comprises the sequence of SEQ ID NO:271; or
C. a sequence having at least 85%, 90%, or 95% sequence identity to the sequence of SEQ ID NO:191, wherein the polypeptide comprises Glu at position 153, Phe at position 157, Thr at position 159, Glu at position 160, Trp at position 161, Ser at position 162, Asn at position 163, Ser at position 186, Glu at position 188, Glu at position 189, and Phe at position 194, as numbered with reference to SEQ ID NO:1, optionally further comprising:
(a) Trp at position 139, as numbered with reference to SEQ ID NO:1, optionally wherein the polypeptide comprises the sequence of SEQ ID NO:232;
(b) Ala at position 7, Ala at position 8, and Trp at position 139, as numbered with reference to SEQ ID NO:1, optionally wherein the polypeptide comprises the sequence of SEQ ID NO:233;
(c) Ala at position 7, Ala at position 8, Gly at position 102, and Trp at position 139, as numbered with reference to SEQ ID NO:1, optionally wherein the polypeptide comprises the sequence of SEQ ID NO:234;
(d) Ala at position 7, Ala at position 8, Trp at position 139, Leu at position 201, and Ser at position 207, as numbered with reference to SEQ ID NO:1, optionally wherein the polypeptide comprises the sequence of SEQ ID NO:309;
(e) Ala at position 7, Ala at position 8, Gly at position 102, Trp at position 139, Leu at position 201, and Ser at position 207, as numbered with reference to SEQ ID NO:1, optionally wherein the polypeptide comprises the sequence of SEQ ID NO:310;
(f) Ser at position 139, Ala at position 141, and Val at position 180, as numbered with reference to SEQ ID NO:1, optionally wherein the polypeptide comprises the sequence of SEQ ID NO:238;
(g) Ala at position 7, Ala at position 8, Ser at position 139, Ala at position 141, and Val at position 180, as numbered with reference to SEQ ID NO:1, optionally wherein the polypeptide comprises the sequence of SEQ ID NO:239;
(h) Ala at position 7, Ala at position 8, Gly at position 102, Ser at position 139, Ala at position 141, and Val at position 180, as numbered with reference to SEQ ID NO:1, optionally wherein the polypeptide comprises the sequence of SEQ ID NO:240;
(i) Ala at position 7, Ala at position 8, Ser at position 139, Ala at position 141, Val at position 180, Leu at position 201, and Ser at position 207, as numbered with reference to SEQ ID NO:1, optionally wherein the polypeptide comprises the sequence of SEQ ID NO:312; or
(j) Ala at position 7, Ala at position 8, Gly at position 102, Ser at position 139, Ala at position 141, Val at position 180, Leu at position 201, and Ser at position 207, as numbered with reference to SEQ ID NO:1, optionally wherein the polypeptide comprises the sequence of SEQ ID NO:313; or
D. a sequence having at least 85%, 90%, or 95% sequence identity to the sequence of SEQ ID NO:43, wherein the polypeptide comprises Glu at position 153, Tyr at position 157, Thr at position 159, Glu at position 160, Trp at position 161, Ser at position 162, Asn at position 163, Thr at position 186, Glu at position 188, Glu at position 189, and Phe at position 194, as numbered with reference to SEQ ID NO:1, optionally further comprising:
(a) Trp at position 139, as numbered with reference to SEQ ID NO:1, optionally wherein the polypeptide comprises the sequence of SEQ ID NO:169;
(b) Ala at position 7, Ala at position 8, and Trp at position 139, as numbered with reference to SEQ ID NO:1, optionally wherein the polypeptide comprises the sequence of SEQ ID NO:170;
(c) Ala at position 7, Ala at position 8, Gly at position 102, and Trp at position 139, as numbered with reference to SEQ ID NO:1, optionally wherein the polypeptide comprises the sequence of SEQ ID NO:171;
(d) Ala at position 7, Ala at position 8, Trp at position 139, Leu at position 201, and Ser at position 207, as numbered with reference to SEQ ID NO:1, optionally wherein the polypeptide comprises the sequence of SEQ ID NO:281;
(e) Ala at position 7, Ala at position 8, Gly at position 102, Trp at position 139, Leu at position 201, and Ser at position 207, as numbered with reference to SEQ ID NO:1, optionally wherein the polypeptide comprises the sequence of SEQ ID NO:282;
(f) Ser at position 139, Ala at position 141, and Val at position 180, as numbered with reference to SEQ ID NO:1, optionally wherein the polypeptide comprises the sequence of SEQ ID NO:175;
(g) Ala at position 7, Ala at position 8, Ser at position 139, Ala at position 141, and Val at position 180, as numbered with reference to SEQ ID NO:1, optionally wherein the polypeptide comprises the sequence of SEQ ID NO:176;
(h) Ala at position 7, Ala at position 8, Gly at position 102, Ser at position 139, Ala at position 141, and Val at position 180, as numbered with reference to SEQ ID NO: 1, optionally wherein the polypeptide comprises the sequence of SEQ ID NO: 177;
(i) Ala at position 7, Ala at position 8, Ser at position 139, Ala at position 141, Val at position 180, Leu at position 201, and Ser at position 207, as numbered with reference to SEQ ID NO:1, optionally wherein the polypeptide comprises the sequence of SEQ ID NO:284 ; or
(j) Ala at position 7, Ala at position 8, Gly at position 102, Ser at position 139, Ala at position 141, Val at position 180, Leu at position 201, and Ser at position 207, as numbered with reference to SEQ ID NO:1, optionally wherein the polypeptide comprises the sequence of SEQ ID NO:285; or
E. a sequence having at least 85%, 90%, or 95% sequence identity to the sequence of SEQ ID NO:82, wherein the polypeptide comprises Glu at position 153, Tyr at position 157, Thr at position 159, Glu at position 160, Trp at position 161, Ala at position 162, Asn at position 163, Thr at position 186, Glu at position 188, Glu at position 189, and Phe at position 194, as numbered with reference to SEQ ID NO:1, optionally further comprising:
(a) Trp at position 139, as numbered with reference to SEQ ID NO:1, optionally wherein the polypeptide comprises the sequence of SEQ ID NO:121;
(b) Ala at position 7, Ala at position 8, and Trp at position 139, as numbered with reference to SEQ ID NO:1, optionally wherein the polypeptide comprises the sequence of SEQ ID NO:122;
(c) Ala at position 7, Ala at position 8, Gly at position 102, and Trp at position 139, as numbered with reference to SEQ ID NO:1, optionally wherein the polypeptide comprises the sequence of SEQ ID NO:123;
(d) Ala at position 7, Ala at position 8, Trp at position 139, Leu at position 201, and Ser at position 207, as numbered with reference to SEQ ID NO:1, optionally wherein the polypeptide comprises the sequence of SEQ ID NO:253;
(e) Ala at position 7, Ala at position 8, Gly at position 102, Trp at position 139, Leu at position 201, and Ser at position 207, as numbered with reference to SEQ ID NO:1, optionally wherein the polypeptide comprises the sequence of SEQ ID NO:254;
(f) Ser at position 139, Ala at position 141, and Val at position 180, as numbered with reference to SEQ ID NO:1, optionally wherein the polypeptide comprises the sequence of SEQ ID NO:127;
(g) Ala at position 7, Ala at position 8, Ser at position 139, Ala at position 141, and Val at position 180, as numbered with reference to SEQ ID NO:1, optionally wherein the polypeptide comprises the sequence of SEQ ID NO:128; or
(h) Ala at position 7, Ala at position 8, Gly at position 102, Ser at position 139, Ala at position 141, and Val at position 180, as numbered with reference to SEQ ID NO: 1, optionally wherein the polypeptide comprises the sequence of SEQ ID NO: 129; or
(i) Ala at position 7, Ala at position 8, Ser at position 139, Ala at position 141, Val at position 180, Leu at position 201, and Ser at position 207, as numbered with reference to SEQ ID NO:1, optionally wherein the polypeptide comprises the sequence of SEQ ID NO:256; or
(j) Ala at position 7, Ala at position 8, Gly at position 102, Ser at position 139, Ala at position 141, Val at position 180, Leu at position 201, and Ser at position 207, as numbered with reference to SEQ ID NO:1, optionally wherein the polypeptide comprises the sequence of SEQ ID NO:257.

4. The polypeptide of any one of claims 1 to 3, wherein the polypeptide does not comprise the first three amino acids "PCP" at the amino-terminal end of the sequence, optionally further comprising an antibody heavy chain variable region.

5. A polypeptide that specifically binds to a transferrin receptor,
(a) comprising the sequence of SEQ ID NO:347; or
(b) comprising the sequence of SEQ ID NO:348;
optionally further comprising an antibody heavy chain variable region.

6. A polypeptide comprising from N- to C- terminus: an antibody heavy chain variable region, a CH1 domain, a hinge region, and the polypeptide of any one of claims 1 to 5.

7. An Fc polypeptide dimer, or a dimeric fragment thereof, comprising:
(a) a first Fc polypeptide comprising the polypeptide of any one of claims 1 to 6; and
(b) a second Fc polypeptide capable of dimerizing with the first Fc polypeptide of (a).

8. The Fc polypeptide dimer of claim 7, wherein:
(a) the dimer is monovalent for TfR binding; and/or
(b) the second Fc polypeptide comprises the sequence of any one of SEQ ID NOS:351-356.

9. The Fc polypeptide dimer of claim 7 or 8:
(a) wherein the first Fc polypeptide comprises the sequence of any one of SEQ ID NOS: 133, 134, and 260; and the second Fc polypeptide comprises the sequence of any one of SEQ ID NOS:354-356;
(b) wherein the first Fc polypeptide comprises the sequence of any one of SEQ ID NOS: 145, 146, and 267; and the second Fc polypeptide comprises the sequence of any one of SEQ ID NOS:354-356;
(c) wherein the first Fc polypeptide comprises the sequence of any one of SEQ ID NOS:232, 233, and 309; and the second Fc polypeptide comprises the sequence of any one of SEQ ID NOS:354-356;
(d) wherein the first Fc polypeptide comprises the sequence of any one of SEQ ID NOS: 169, 170, and 281; and the second Fc polypeptide comprises the sequence of any one of SEQ ID NOS:354-356;
(e) wherein the first Fc polypeptide comprises the sequence of any one of SEQ ID NOS:121, 122, and 253; and the second Fc polypeptide comprises the sequence of any one of SEQ ID NOS:354-356;
(f) wherein the first Fc polypeptide comprises the sequence of any one of SEQ ID NOS:139, 140, and 263; and the second Fc polypeptide comprises the sequence of any one of SEQ ID NOS:351-353;
(g) wherein the first Fc polypeptide comprises the sequence of any one of SEQ ID NOS:151, 152, and 270; and the second Fc polypeptide comprises the sequence of any one of SEQ ID NOS:351-353;
(h) wherein the first Fc polypeptide comprises the sequence of any one of SEQ ID NOS:238, 239, and 312; and the second Fc polypeptide comprises the sequence of any one of SEQ ID NOS:351-353;
(i) wherein the first Fc polypeptide comprises the sequence of any one of SEQ ID NOS:175, 176, and 284; and the second Fc polypeptide comprises the sequence of any one of SEQ ID NOS:351-353;
(j) wherein the first Fc polypeptide comprises the sequence of any one of SEQ ID NOS:127, 128, and 256; and the second Fc polypeptide comprises the sequence of any one of SEQ ID NOS:351-353;
(k) wherein:
(i) the first Fc polypeptide comprises the sequence of SEQ ID NO:134 and the second Fc polypeptide comprises the sequence of SEQ ID NO:355; or
(ii) the first Fc polypeptide comprises the sequence of SEQ ID NO:134 and the second Fc polypeptide comprises the sequence of SEQ ID NO:356; or
(iii) the first Fc polypeptide comprises the sequence of SEQ ID NO:260 and the second Fc polypeptide comprises the sequence of SEQ ID NO:355; or
(iv) the first Fc polypeptide comprises the sequence of SEQ ID NO:260 and the second Fc polypeptide comprises the sequence of SEQ ID NO:356; or
(v) the first Fc polypeptide comprises the sequence of SEQ ID NO:140 and the second Fc polypeptide comprises the sequence of SEQ ID NO:352; or
(vi) the first Fc polypeptide comprises the sequence of SEQ ID NO:140 and the second Fc polypeptide comprises the sequence of SEQ ID NO:353; or
(vii) the first Fc polypeptide comprises the sequence of SEQ ID NO:263 and the second Fc polypeptide comprises the sequence of SEQ ID NO:352; or
(viii) the first Fc polypeptide comprises the sequence of SEQ ID NO:263 and the second Fc polypeptide comprises the sequence of SEQ ID NO:353;
(l) wherein:
(i) the first Fc polypeptide comprises the sequence of SEQ ID NO:146 and the second Fc polypeptide comprises the sequence of SEQ ID NO:355; or
(ii) the first Fc polypeptide comprises the sequence of SEQ ID NO:146 and the second Fc polypeptide comprises the sequence of SEQ ID NO:356; or
(iii) the first Fc polypeptide comprises the sequence of SEQ ID NO:267 and the second Fc polypeptide comprises the sequence of SEQ ID NO:355; or
(iv) the first Fc polypeptide comprises the sequence of SEQ ID NO:267 and the second Fc polypeptide comprises the sequence of SEQ ID NO:356; or
(v) the first Fc polypeptide comprises the sequence of SEQ ID NO:152 and the second Fc polypeptide comprises the sequence of SEQ ID NO:352; or
(vi) the first Fc polypeptide comprises the sequence of SEQ ID NO:152 and the second Fc polypeptide comprises the sequence of SEQ ID NO:353; or
(vii) the first Fc polypeptide comprises the sequence of SEQ ID NO:270 and the second Fc polypeptide comprises the sequence of SEQ ID NO:352; or
(viii) the first Fc polypeptide comprises the sequence of SEQ ID NO:270 and the second Fc polypeptide comprises the sequence of SEQ ID NO:353;
(m) wherein:
(i) the first Fc polypeptide comprises the sequence of SEQ ID NO:233 and the second Fc polypeptide comprises the sequence of SEQ ID NO:355; or
(ii) the first Fc polypeptide comprises the sequence of SEQ ID NO:233 and the second Fc polypeptide comprises the sequence of SEQ ID NO:356; or
(iii) the first Fc polypeptide comprises the sequence of SEQ ID NO:309 and the second Fc polypeptide comprises the sequence of SEQ ID NO:355; or
(iv) the first Fc polypeptide comprises the sequence of SEQ ID NO:309 and the second Fc polypeptide comprises the sequence of SEQ ID NO:356; or
(v) the first Fc polypeptide comprises the sequence of SEQ ID NO:239 and the second Fc polypeptide comprises the sequence of SEQ ID NO:352; or
(vi) the first Fc polypeptide comprises the sequence of SEQ ID NO:239 and the second Fc polypeptide comprises the sequence of SEQ ID NO:353; or
(vii) the first Fc polypeptide comprises the sequence of SEQ ID NO:312 and the second Fc polypeptide comprises the sequence of SEQ ID NO:352; or
(viii) the first Fc polypeptide comprises the sequence of SEQ ID NO:312 and the second Fc polypeptide comprises the sequence of SEQ ID NO:353;
(n) wherein:
(i) the first Fc polypeptide comprises the sequence of SEQ ID NO:170 and the second Fc polypeptide comprises the sequence of SEQ ID NO:355; or
(ii) the first Fc polypeptide comprises the sequence of SEQ ID NO:170 and the second Fc polypeptide comprises the sequence of SEQ ID NO:356; or
(iii) the first Fc polypeptide comprises the sequence of SEQ ID NO:281 and the second Fc polypeptide comprises the sequence of SEQ ID NO:355; or
(iv) the first Fc polypeptide comprises the sequence of SEQ ID NO:281 and the second Fc polypeptide comprises the sequence of SEQ ID NO:356; or
(v) the first Fc polypeptide comprises the sequence of SEQ ID NO:176 and the second Fc polypeptide comprises the sequence of SEQ ID NO:352; or
(vi) the first Fc polypeptide comprises the sequence of SEQ ID NO:176 and the second Fc polypeptide comprises the sequence of SEQ ID NO:353; or
(vii) the first Fc polypeptide comprises the sequence of SEQ ID NO:284 and the second Fc polypeptide comprises the sequence of SEQ ID NO:352; or
(viii) the first Fc polypeptide comprises the sequence of SEQ ID NO:284 and the second Fc polypeptide comprises the sequence of SEQ ID NO:353;
(o) wherein:
(i) the first Fc polypeptide comprises the sequence of SEQ ID NO:122 and the second Fc polypeptide comprises the sequence of SEQ ID NO:355; or
(ii) the first Fc polypeptide comprises the sequence of SEQ ID NO:122 and the second Fc polypeptide comprises the sequence of SEQ ID NO:356; or
(iii) the first Fc polypeptide comprises the sequence of SEQ ID NO:253 and the second Fc polypeptide comprises the sequence of SEQ ID NO:355; or
(iv) the first Fc polypeptide comprises the sequence of SEQ ID NO:253 and the second Fc polypeptide comprises the sequence of SEQ ID NO:356; or
(v) the first Fc polypeptide comprises the sequence of SEQ ID NO:128 and the second Fc polypeptide comprises the sequence of SEQ ID NO:352; or
(vi) the first Fc polypeptide comprises the sequence of SEQ ID NO:128 and the second Fc polypeptide comprises the sequence of SEQ ID NO:353; or
(vii) the first Fc polypeptide comprises the sequence of SEQ ID NO:256 and the second Fc polypeptide comprises the sequence of SEQ ID NO:352; or
(viii) the first Fc polypeptide comprises the sequence of SEQ ID NO:256 and the second Fc polypeptide comprises the sequence of SEQ ID NO:353;
(p) wherein the first Fc polypeptide comprises the sequence of SEQ ID NO:347 and the second Fc polypeptide comprises the sequence of SEQ ID NO:350; or
(q) wherein the first Fc polypeptide comprises the sequence of SEQ ID NO:348 and the second Fc polypeptide comprises the sequence of SEQ ID NO:349.

10. The Fc polypeptide dimer of claim 7 or 8, wherein the first Fc polypeptide comprises the sequence of SEQ ID NO:260, and optionally the second Fc polypeptide comprises the sequence of SEQ ID NO:356.

11. The Fc polypeptide dimer of claim 7 or 8, wherein the first Fc polypeptide comprises the sequence of SEQ ID NO:134, and optionally the second Fc polypeptide comprises the sequence of SEQ ID NO:355.

12. The Fc polypeptide dimer of claim 7 or 8, wherein the first Fc polypeptide comprises the sequence of SEQ ID NO:122, and optionally the second Fc polypeptide comprises the sequence of SEQ ID NO:355.

13. The Fc polypeptide dimer of claim 7 or 8, wherein the first Fc polypeptide comprises the sequence of SEQ ID NO:253, and optionally the second Fc polypeptide comprises the sequence of SEQ ID NO:356.

14. The Fc polypeptide dimer of claim 7 or 8, wherein the first Fc polypeptide comprises the sequence of SEQ ID NO:146, and optionally the second Fc polypeptide comprises the sequence of SEQ ID NO:355.

15. The Fc polypeptide dimer of claim 7 or 8, wherein the first Fc polypeptide comprises the sequence of SEQ ID NO:267, and optionally the second Fc polypeptide comprises the sequence of SEQ ID NO:356.

16. The Fc polypeptide dimer of claim 7, wherein the dimer is bivalent for TfR. binding; optionally wherein:
(a) the second Fc polypeptide comprises the sequence of SEQ ID NO:347 or 348;
(b) the first Fc polypeptide comprises the sequence of SEQ ID NO:347 and the second Fc polypeptide comprises the sequence of SEQ ID NO:348; and/or
(c) the first and second Fc polypeptides comprise the same TfR binding site.

17. An Fc polypeptide dimer-Fab fusion protein comprising:
(a) an antibody variable region that is capable of binding an antigen, or antigen-binding fragment thereof; and
(b) an Fc polypeptide dimer of any one of claims 7 to 16;
optionally wherein:
(a) the antibody variable region forms part of a Fab domain; and/or
(b) the antibody variable region comprises two antibody heavy chain variable regions and two antibody light chain variable regions, or respective fragments thereof.

18. A polynucleotide comprising a nucleic acid sequence encoding the polypeptide of any one of claims 1 to 6; or a vector or a host cell comprising said polynucleotide.

19. A method for producing a polypeptide that specifically binds to a transferrin receptor, comprising culturing a host cell under conditions in which the polypeptide encoded by the polynucleotide of claim 18 is expressed.

20. A pharmaceutical composition comprising: the polypeptide of any one of claims 1 to 6, the Fc polypeptide dimer of any one of claims 7 to 16, or the Fc polypeptide dimer-Fab fusion protein of claim 17; and a pharmaceutically acceptable carrier.

21. A composition comprising the polypeptide of any one of claims 1 to 6, the Fc polypeptide dimer of any one of claims 7 to 16, or the Fc polypeptide dimer-Fab fusion protein of claim 17, for use in a method for transcytosis of a composition across an endothelium, the method comprising contacting the endothelium with said composition;
optionally wherein the endothelium is the blood-brain barrier (BBB).

## Patentansprüche

1. Polypeptid, das spezifisch an einen Transferrinrezeptor bindet, umfassend eine Sequenz mit mindestens 85 %, 90 % oder 95 % Sequenzidentität mit der Sequenz einer der SEQ ID NOS: 83, 84, 191, 43 und 82, wobei das Polypeptid Glu an Position 153, Tyr oder Phe an Position 157, Thr an Position 159, Glu an Position 160, Trp an Position 161, Val, Ser oder Ala an Position 162, Asn an Position 163, Thr oder Ser an Position 186, Glu an Position 188, Glu an Position 189 und Phe an Position 194 umfasst, wie unter Bezugnahme auf SEQ ID NO: 1 nummeriert.

2. Polypeptid nach Anspruch 1, das ferner Folgendes umfasst:
(a) Trp an Position 139, wie unter Bezugnahme auf SEQ ID NO: 1 nummeriert;
(b) Ala an Position 7, Ala an Position 8 und Trp an Position 139, wie unter Bezugnahme auf SEQ ID NO: 1 nummeriert;
(c) Ala an Position 7, Ala an Position 8, Gly an Position 102 und Trp an Position 139, wie unter Bezugnahme auf SEQ ID NO: 1 nummeriert;
(d) Ala an Position 7, Ala an Position 8, Trp an Position 139, Leu an Position 201 und Ser an Position 207, wie unter Bezugnahme auf SEQ ID NO: 1 nummeriert;
(e) Ala an Position 7, Ala an Position 8, Gly an Position 102, Trp an Position 139, Leu an Position 201 und Ser an Position 207, wie unter Bezugnahme auf SEQ ID NO: 1 nummeriert;
(f) Ser an Position 139, Ala an Position 141 und Val an Position 180, wie unter Bezugnahme auf SEQ ID NO: 1 nummeriert;
(g) Ala an Position 7, Ala an Position 8, Ser an Position 139, Ala an Position 141 und Val an Position 180, wie unter Bezugnahme auf SEQ ID NO: 1 nummeriert;
(h) Ala an Position 7, Ala an Position 8, Gly an Position 102, Ser an Position 139, Ala an Position 141 und Val an Position 180, wie unter Bezugnahme auf SEQ ID NO: 1 nummeriert;
(i) Ala an Position 7, Ala an Position 8, Ser an Position 139, Ala an Position 141, Val an Position 180, Leu an Position 201 und Ser an Position 207, wie unter Bezugnahme auf SEQ ID NO: 1 nummeriert; oder
(j) Ala an Position 7, Ala an Position 8, Gly an Position 102, Ser an Position 139, Ala an Position 141, Val an Position 180, Leu an Position 201 und Ser an Position 207, wie unter Bezugnahme auf SEQ ID NO: 1 nummeriert.

3. Polypeptid nach Anspruch 1, das Folgendes umfasst:
A. eine Sequenz, die mindestens 85 %, 90 % oder 95 % Sequenzidentität mit der Sequenz von SEQ ID NO: 83 aufweist, wobei das Polypeptid Glu an Position 153, Tyr an Position 157, Thr an Position 159, Glu an Position 160, Trp an Position 161, Val an Position 162, Asn an Position 163, Thr an Position 186, Glu an Position 188, Glu an Position 189 und Phe an Position 194 umfasst, wie unter Bezugnahme auf SEQ ID NO: 1 nummeriert, optional ferner umfassend:
(a) Trp an Position 139, wie unter Bezugnahme auf SEQ ID NO: 1 nummeriert, wobei das Polypeptid optional die Sequenz von SEQ ID NO: 133 umfasst;
(b) Ala an Position 7, Ala an Position 8 und Trp an Position 139, wie unter Bezugnahme auf SEQ ID NO: 1 nummeriert, wobei das Polypeptid optional die Sequenz von SEQ ID NO: 134 umfasst;
(c) Ala an Position 7, Ala an Position 8, Gly an Position 102 und Trp an Position 139, wie unter Bezugnahme auf SEQ ID NO: 1 nummeriert, wobei das Polypeptid optional die Sequenz von SEQ ID NO: 135 umfasst;
(d) Ala an Position 7, Ala an Position 8, Trp an Position 139, Leu an Position 201 und Ser an Position 207, wie unter Bezugnahme auf SEQ ID NO: 1 nummeriert, wobei das Polypeptid optional die Sequenz von SEQ ID NO: 260 umfasst;
(e) Ala an Position 7, Ala an Position 8, Gly an Position 102, Trp an Position 139, Leu an Position 201 und Ser an Position 207, wie unter Bezugnahme auf SEQ ID NO: 1 nummeriert, wobei das Polypeptid optional die Sequenz von SEQ ID NO: 261 umfasst;
(f) Ser an Position 139, Ala an Position 141 und Val an Position 180, wie unter Bezugnahme auf SEQ ID NO: 1 nummeriert, wobei das Polypeptid optional die Sequenz von SEQ ID NO: 139 umfasst;
(g) Ala an Position 7, Ala an Position 8, Ser an Position 139, Ala an Position 141 und Val an Position 180, wie unter Bezugnahme auf SEQ ID NO: 1 nummeriert, wobei das Polypeptid optional die Sequenz von SEQ ID NO: 140 umfasst;
(h) Ala an Position 7, Ala an Position 8, Gly an Position 102, Ser an Position 139, Ala an Position 141 und Val an Position 180, wie unter Bezugnahme auf SEQ ID NO: 1 nummeriert, wobei das Polypeptid optional die Sequenz von SEQ ID NO: 141 umfasst;
(i) Ala an Position 7, Ala an Position 8, Ser an Position 139, Ala an Position 141, Val an Position 180, Leu an Position 201 und Ser an Position 207, wie unter Bezugnahme auf SEQ ID NO: 1 nummeriert, wobei das Polypeptid optional die Sequenz von SEQ ID NO: 263 umfasst; oder
(j) Ala an Position 7, Ala an Position 8, Gly an Position 102, Ser an Position 139, Ala an Position 141, Val an Position 180, Leu an Position 201 und Ser an Position 207, wie unter Bezugnahme auf SEQ ID NO: 1 nummeriert, wobei das Polypeptid optional die Sequenz von SEQ ID NO: 264 umfasst; oder
B. eine Sequenz, die mindestens 85 %, 90 % oder 95 % Sequenzidentität mit der Sequenz von SEQ ID NO: 84 aufweist, wobei das Polypeptid Glu an Position 153, Tyr an Position 157, Thr an Position 159, Glu an Position 160, Trp an Position 161, Ser an Position 162, Asn an Position 163, Ser an Position 186, Glu an Position 188, Glu an Position 189 und Phe an Position 194 umfasst, wie unter Bezugnahme auf SEQ ID NO: 1 nummeriert, optional ferner umfassend:
(a) Trp an Position 139, wie unter Bezugnahme auf SEQ ID NO: 1 nummeriert, wobei das Polypeptid optional die Sequenz von SEQ ID NO: 145 umfasst;
(b) Ala an Position 7, Ala an Position 8 und Trp an Position 139, wie unter Bezugnahme auf SEQ ID NO: 1 nummeriert, wobei das Polypeptid optional die Sequenz von SEQ ID NO: 146 umfasst;
(c) Ala an Position 7, Ala an Position 8, Gly an Position 102 und Trp an Position 139, wie unter Bezugnahme auf SEQ ID NO: 1 nummeriert, wobei das Polypeptid optional die Sequenz von SEQ ID NO: 147 umfasst;
(d) Ala an Position 7, Ala an Position 8, Trp an Position 139, Leu an Position 201 und Ser an Position 207, wie unter Bezugnahme auf SEQ ID NO: 1 nummeriert, wobei das Polypeptid optional die Sequenz von SEQ ID NO: 267 umfasst;
(e) Ala an Position 7, Ala an Position 8, Gly an Position 102, Trp an Position 139, Leu an Position 201 und Ser an Position 207, wie unter Bezugnahme auf SEQ ID NO: 1 nummeriert, wobei das Polypeptid optional die Sequenz von SEQ ID NO: 268 umfasst;
(f) Ser an Position 139, Ala an Position 141 und Val an Position 180, wie unter Bezugnahme auf SEQ ID NO: 1 nummeriert, wobei das Polypeptid optional die Sequenz von SEQ ID NO: 151 umfasst;
(g) Ala an Position 7, Ala an Position 8, Ser an Position 139, Ala an Position 141 und Val an Position 180, wie unter Bezugnahme auf SEQ ID NO: 1 nummeriert, wobei das Polypeptid optional die Sequenz von SEQ ID NO: 152 umfasst;
(h) Ala an Position 7, Ala an Position 8, Gly an Position 102, Ser an Position 139, Ala an Position 141 und Val an Position 180, wie unter Bezugnahme auf SEQ ID NO: 1 nummeriert, wobei das Polypeptid optional die Sequenz von SEQ ID NO: 153 umfasst;
(i) Ala an Position 7, Ala an Position 8, Ser an Position 139, Ala an Position 141, Val an Position 180, Leu an Position 201 und Ser an Position 207, wie unter Bezugnahme auf SEQ ID NO: 1 nummeriert, wobei das Polypeptid optional die Sequenz von SEQ ID NO: 270 umfasst; oder
(j) Ala an Position 7, Ala an Position 8, Gly an Position 102, Ser an Position 139, Ala an Position 141, Val an Position 180, Leu an Position 201 und Ser an Position 207, wie unter Bezugnahme auf SEQ ID NO: 1 nummeriert, wobei das Polypeptid optional die Sequenz von SEQ ID NO: 271 umfasst; oder
C. eine Sequenz, die mindestens 85 %, 90 % oder 95 % Sequenzidentität mit der Sequenz von SEQ ID NO: 191 aufweist, wobei das Polypeptid Glu an Position 153, Phe an Position 157, Thr an Position 159, Glu an Position 160, Trp an Position 161, Ser an Position 162, Asn an Position 163, Ser an Position 186, Glu an Position 188, Glu an Position 189 und Phe an Position 194 umfasst, wie unter Bezugnahme auf SEQ ID NO: 1 nummeriert, optional ferner umfassend:
(a) Trp an Position 139, wie unter Bezugnahme auf SEQ ID NO: 1 nummeriert, wobei das Polypeptid optional die Sequenz von SEQ ID NO: 232 umfasst;
(b) Ala an Position 7, Ala an Position 8 und Trp an Position 139, wie unter Bezugnahme auf SEQ ID NO: 1 nummeriert, wobei das Polypeptid optional die Sequenz von SEQ ID NO: 233 umfasst;
(c) Ala an Position 7, Ala an Position 8, Gly an Position 102 und Trp an Position 139, wie unter Bezugnahme auf SEQ ID NO: 1 nummeriert, wobei das Polypeptid optional die Sequenz von SEQ ID NO: 234 umfasst;
(d) Ala an Position 7, Ala an Position 8, Trp an Position 139, Leu an Position 201 und Ser an Position 207, wie unter Bezugnahme auf SEQ ID NO: 1 nummeriert, wobei das Polypeptid optional die Sequenz von SEQ ID NO: 309 umfasst;
(e) Ala an Position 7, Ala an Position 8, Gly an Position 102, Trp an Position 139, Leu an Position 201 und Ser an Position 207, wie unter Bezugnahme auf SEQ ID NO: 1 nummeriert, wobei das Polypeptid optional die Sequenz von SEQ ID NO: 310 umfasst;
(f) Ser an Position 139, Ala an Position 141 und Val an Position 180, wie unter Bezugnahme auf SEQ ID NO: 1 nummeriert, wobei das Polypeptid optional die Sequenz von SEQ ID NO: 238 umfasst;
(g) Ala an Position 7, Ala an Position 8, Ser an Position 139, Ala an Position 141 und Val an Position 180, wie unter Bezugnahme auf SEQ ID NO: 1 nummeriert, wobei das Polypeptid optional die Sequenz von SEQ ID NO: 239 umfasst;
(h) Ala an Position 7, Ala an Position 8, Gly an Position 102, Ser an Position 139, Ala an Position 141 und Val an Position 180, wie unter Bezugnahme auf SEQ ID NO: 1 nummeriert, wobei das Polypeptid optional die Sequenz von SEQ ID NO: 240 umfasst;
(i) Ala an Position 7, Ala an Position 8, Ser an Position 139, Ala an Position 141, Val an Position 180, Leu an Position 201 und Ser an Position 207, wie unter Bezugnahme auf SEQ ID NO: 1 nummeriert, wobei das Polypeptid optional die Sequenz von SEQ ID NO: 312 umfasst; oder
(j) Ala an Position 7, Ala an Position 8, Gly an Position 102, Ser an Position 139, Ala an Position 141, Val an Position 180, Leu an Position 201 und Ser an Position 207, wie unter Bezugnahme auf SEQ ID NO: 1 nummeriert, wobei das Polypeptid optional die Sequenz von SEQ ID NO: 313 umfasst; oder
D. eine Sequenz, die mindestens 85 %, 90 % oder 95 % Sequenzidentität mit der Sequenz von SEQ ID NO: 43 aufweist, wobei das Polypeptid Glu an Position 153, Tyr an Position 157, Thr an Position 159, Glu an Position 160, Trp an Position 161, Ser an Position 162, Asn an Position 163, Thr an Position 186, Glu an Position 188, Glu an Position 189 und Phe an Position 194 umfasst, wie unter Bezugnahme auf SEQ ID NO: 1 nummeriert, optional ferner umfassend:
(a) Trp an Position 139, wie unter Bezugnahme auf SEQ ID NO: 1 nummeriert, wobei das Polypeptid optional die Sequenz von SEQ ID NO: 169 umfasst;
(b) Ala an Position 7, Ala an Position 8 und Trp an Position 139, wie unter Bezugnahme auf SEQ ID NO: 1 nummeriert, wobei das Polypeptid optional die Sequenz von SEQ ID NO: 170 umfasst;
(c) Ala an Position 7, Ala an Position 8, Gly an Position 102 und Trp an Position 139, wie unter Bezugnahme auf SEQ ID NO: 1 nummeriert, wobei das Polypeptid optional die Sequenz von SEQ ID NO: 171 umfasst;
(d) Ala an Position 7, Ala an Position 8, Trp an Position 139, Leu an Position 201 und Ser an Position 207, wie unter Bezugnahme auf SEQ ID NO: 1 nummeriert, wobei das Polypeptid optional die Sequenz von SEQ ID NO: 281 umfasst;
(e) Ala an Position 7, Ala an Position 8, Gly an Position 102, Trp an Position 139, Leu an Position 201 und Ser an Position 207, wie unter Bezugnahme auf SEQ ID NO: 1 nummeriert, wobei das Polypeptid optional die Sequenz von SEQ ID NO: 282 umfasst;
(f) Ser an Position 139, Ala an Position 141 und Val an Position 180, wie unter Bezugnahme auf SEQ ID NO: 1 nummeriert, wobei das Polypeptid optional die Sequenz von SEQ ID NO: 175 umfasst;
(g) Ala an Position 7, Ala an Position 8, Ser an Position 139, Ala an Position 141 und Val an Position 180, wie unter Bezugnahme auf SEQ ID NO: 1 nummeriert, wobei das Polypeptid optional die Sequenz von SEQ ID NO: 176 umfasst;
(h) Ala an Position 7, Ala an Position 8, Gly an Position 102, Ser an Position 139, Ala an Position 141 und Val an Position 180, wie unter Bezugnahme auf SEQ ID NO: 1 nummeriert, wobei das Polypeptid optional die Sequenz von SEQ ID NO: 177 umfasst;
(i) Ala an Position 7, Ala an Position 8, Ser an Position 139, Ala an Position 141, Val an Position 180, Leu an Position 201 und Ser an Position 207, wie unter Bezugnahme auf SEQ ID NO: 1 nummeriert, wobei das Polypeptid optional die Sequenz von SEQ ID NO: 284 umfasst; oder
(j) Ala an Position 7, Ala an Position 8, Gly an Position 102, Ser an Position 139, Ala an Position 141, Val an Position 180, Leu an Position 201 und Ser an Position 207, wie unter Bezugnahme auf SEQ ID NO: 1 nummeriert, wobei das Polypeptid optional die Sequenz von SEQ ID NO: 285 umfasst; oder
E. eine Sequenz, die mindestens 85 %, 90 % oder 95 % Sequenzidentität mit der Sequenz von SEQ ID NO: 82 aufweist, wobei das Polypeptid Glu an Position 153, Tyr an Position 157, Thr an Position 159, Glu an Position 160, Trp an Position 161, Ala an Position 162, Asn an Position 163, Thr an Position 186, Glu an Position 188, Glu an Position 189 und Phe an Position 194 umfasst, wie unter Bezugnahme auf SEQ ID NO: 1 nummeriert, optional ferner umfassend:
(a) Trp an Position 139, wie unter Bezugnahme auf SEQ ID NO: 1 nummeriert, wobei das Polypeptid optional die Sequenz von SEQ ID NO: 121 umfasst;
(b) Ala an Position 7, Ala an Position 8 und Trp an Position 139, wie unter Bezugnahme auf SEQ ID NO: 1 nummeriert, wobei das Polypeptid optional die Sequenz von SEQ ID NO: 122 umfasst;
(c) Ala an Position 7, Ala an Position 8, Gly an Position 102 und Trp an Position 139, wie unter Bezugnahme auf SEQ ID NO: 1 nummeriert, wobei das Polypeptid optional die Sequenz von SEQ ID NO: 123 umfasst;
(d) Ala an Position 7, Ala an Position 8, Trp an Position 139, Leu an Position 201 und Ser an Position 207, wie unter Bezugnahme auf SEQ ID NO: 1 nummeriert, wobei das Polypeptid optional die Sequenz von SEQ ID NO: 253 umfasst;
(e) Ala an Position 7, Ala an Position 8, Gly an Position 102, Trp an Position 139, Leu an Position 201 und Ser an Position 207, wie unter Bezugnahme auf SEQ ID NO: 1 nummeriert, wobei das Polypeptid optional die Sequenz von SEQ ID NO: 254 umfasst;
(f) Ser an Position 139, Ala an Position 141 und Val an Position 180, wie unter Bezugnahme auf SEQ ID NO: 1 nummeriert, wobei das Polypeptid optional die Sequenz von SEQ ID NO: 127 umfasst;
(g) Ala an Position 7, Ala an Position 8, Ser an Position 139, Ala an Position 141 und Val an Position 180, wie unter Bezugnahme auf SEQ ID NO: 1 nummeriert, wobei das Polypeptid optional die Sequenz von SEQ ID NO: 128 umfasst; oder
(h) Ala an Position 7, Ala an Position 8, Gly an Position 102, Ser an Position 139, Ala an Position 141 und Val an Position 180, wie unter Bezugnahme auf SEQ ID NO: 1 nummeriert, wobei das Polypeptid optional die Sequenz von SEQ ID NO: 129 umfasst; oder
(i) Ala an Position 7, Ala an Position 8, Ser an Position 139, Ala an Position 141, Val an Position 180, Leu an Position 201 und Ser an Position 207, wie unter Bezugnahme auf SEQ ID NO: 1 nummeriert, wobei das Polypeptid optional die Sequenz von SEQ ID NO: 256 umfasst; oder
(j) Ala an Position 7, Ala an Position 8, Gly an Position 102, Ser an Position 139, Ala an Position 141, Val an Position 180, Leu an Position 201 und Ser an Position 207, wie unter Bezugnahme auf SEQ ID NO: 1 nummeriert, wobei das Polypeptid optional die Sequenz von SEQ ID NO: 257 umfasst.

4. Polypeptid nach einem der Ansprüche 1 bis 3, wobei das Polypeptid die ersten drei Aminosäuren "PCP" am aminoterminalen Ende der Sequenz nicht umfasst und optional ferner eine variable Region der schweren Kette eines Antikörpers umfasst.

5. Polypeptid, das spezifisch an einen Transferrinrezeptor bindet,
(a) die Sequenz von SEQ ID NO: 347 umfasst; oder
(b) die Sequenz von SEQ ID NO: 348 umfasst;
optional ferner umfassend eine variable Region der schweren Kette des Antikörpers.

6. Polypeptid, umfassend vom N- zum C-Terminus: eine variable Region der schweren Antikörperkette, eine CH1-Domäne, eine Scharnierregion und das Polypeptid nach einem der Ansprüche 1 bis 5.

7. Fc-Polypeptiddimer oder dimeres Fragment davon, umfassend:
(a) ein erstes Fc-Polypeptid, das das Polypeptid nach einem der Ansprüche 1 bis 6 umfasst; und
(b) ein zweites Fc-Polypeptid, das zur Dimerisierung mit dem ersten Fc-Polypeptid aus (a) fähig ist.

8. Fc-Polypeptiddimer nach Anspruch 7, wobei:
(a) das Dimer monovalent für die TfR-Bindung ist; und/oder
(b) das zweite Fc-Polypeptid die Sequenz aus einer der SEQ ID NOS: 351 bis 356 umfasst.

9. Fc-Polypeptiddimer nach Anspruch 7 oder 8:
(a) wobei das erste Fc-Polypeptid die Sequenz einer beliebigen der SEQ ID NOS: 133, 134 und 260 umfasst; und das zweite Fc-Polypeptid die Sequenz einer beliebigen der SEQ ID NOS: 354 bis 356 umfasst;
(b) wobei das erste Fc-Polypeptid die Sequenz einer beliebigen der SEQ ID NOS: 145, 146 und 267 umfasst; und das zweite Fc-Polypeptid die Sequenz einer beliebigen der SEQ ID NOS: 354 bis 356 umfasst;
(c) wobei das erste Fc-Polypeptid die Sequenz einer beliebigen der SEQ ID NOS: 232, 233 und 309 umfasst; und das zweite Fc-Polypeptid die Sequenz einer beliebigen der SEQ ID NOS: 354 bis 356 umfasst;
(d) wobei das erste Fc-Polypeptid die Sequenz einer beliebigen der SEQ ID NOS: 169, 170 und 281 umfasst; und das zweite Fc-Polypeptid die Sequenz einer beliebigen der SEQ ID NOS: 354 bis 356 umfasst;
(e) wobei das erste Fc-Polypeptid die Sequenz einer beliebigen der SEQ ID NOS: 121, 122 und 253 umfasst; und das zweite Fc-Polypeptid die Sequenz einer beliebigen der SEQ ID NOS: 354 bis 356 umfasst;
(f) wobei das erste Fc-Polypeptid die Sequenz einer beliebigen der SEQ ID NOS: 139, 140 und 263 umfasst; und das zweite Fc-Polypeptid die Sequenz einer beliebigen der SEQ ID NOS: 351 bis 353 umfasst;
(g) wobei das erste Fc-Polypeptid die Sequenz einer beliebigen der SEQ ID NOS: 151, 152 und 270 umfasst; und das zweite Fc-Polypeptid die Sequenz einer beliebigen der SEQ ID NOS: 351 bis 353 umfasst;
(h) wobei das erste Fc-Polypeptid die Sequenz einer beliebigen der SEQ ID NOS: 238, 239 und 312 umfasst; und das zweite Fc-Polypeptid die Sequenz einer beliebigen der SEQ ID NOS: 351 bis 353 umfasst;
(i) wobei das erste Fc-Polypeptid die Sequenz einer beliebigen der SEQ ID NOS: 175, 176 und 284 umfasst; und das zweite Fc-Polypeptid die Sequenz einer beliebigen der SEQ ID NOS: 351 bis 353 umfasst;
(j) wobei das erste Fc-Polypeptid die Sequenz einer beliebigen der SEQ ID NOS: 127, 128 und 256 umfasst; und das zweite Fc-Polypeptid die Sequenz einer beliebigen der SEQ ID NOS: 351 bis 353 umfasst;
(k) wobei:
(i) das erste Fc-Polypeptid die Sequenz von SEQ ID NO: 134 umfasst und das zweite Fc-Polypeptid die Sequenz von SEQ ID NO: 355 umfasst; oder
(ii) das erste Fc-Polypeptid die Sequenz von SEQ ID NO: 134 umfasst und das zweite Fc-Polypeptid die Sequenz von SEQ ID NO: 356 umfasst; oder
(iii) das erste Fc-Polypeptid die Sequenz von SEQ ID NO: 260 umfasst und das zweite Fc-Polypeptid die Sequenz von SEQ ID NO: 355 umfasst; oder
(iv) das erste Fc-Polypeptid die Sequenz von SEQ ID NO: 260 umfasst und das zweite Fc-Polypeptid die Sequenz von SEQ ID NO: 356 umfasst; oder
(v) das erste Fc-Polypeptid die Sequenz von SEQ ID NO: 140 umfasst und das zweite Fc-Polypeptid die Sequenz von SEQ ID NO: 352 umfasst; oder
(vi) das erste Fc-Polypeptid die Sequenz von SEQ ID NO: 140 umfasst und das zweite Fc-Polypeptid die Sequenz von SEQ ID NO: 353 umfasst; oder
(vii) das erste Fc-Polypeptid die Sequenz von SEQ ID NO: 263 umfasst und das zweite Fc-Polypeptid die Sequenz von SEQ ID NO: 352 umfasst; oder
(viii) das erste Fc-Polypeptid die Sequenz von SEQ ID NO: 263 umfasst und das zweite Fc-Polypeptid die Sequenz von SEQ ID NO: 353 umfasst;
(l) wobei:
(i) das erste Fc-Polypeptid die Sequenz von SEQ ID NO: 146 umfasst und das zweite Fc-Polypeptid die Sequenz von SEQ ID NO: 355 umfasst; oder
(ii) das erste Fc-Polypeptid die Sequenz von SEQ ID NO: 146 umfasst und das zweite Fc-Polypeptid die Sequenz von SEQ ID NO: 356 umfasst; oder
(iii) das erste Fc-Polypeptid die Sequenz von SEQ ID NO: 267 umfasst und das zweite Fc-Polypeptid die Sequenz von SEQ ID NO: 355 umfasst; oder
(iv) das erste Fc-Polypeptid die Sequenz von SEQ ID NO: 267 umfasst und das zweite Fc-Polypeptid die Sequenz von SEQ ID NO: 356 umfasst; oder
(v) das erste Fc-Polypeptid die Sequenz von SEQ ID NO: 152 umfasst und das zweite Fc-Polypeptid die Sequenz von SEQ ID NO: 352 umfasst; oder
(vi) das erste Fc-Polypeptid die Sequenz von SEQ ID NO: 152 umfasst und das zweite Fc-Polypeptid die Sequenz von SEQ ID NO: 353 umfasst; oder
(vii) das erste Fc-Polypeptid die Sequenz von SEQ ID NO: 270 umfasst und das zweite Fc-Polypeptid die Sequenz von SEQ ID NO: 352 umfasst; oder
(viii) das erste Fc-Polypeptid die Sequenz von SEQ ID NO: 270 umfasst und das zweite Fc-Polypeptid die Sequenz von SEQ ID NO: 353 umfasst;
(m) wobei:
(i) das erste Fc-Polypeptid die Sequenz von SEQ ID NO: 233 umfasst und das zweite Fc-Polypeptid die Sequenz von SEQ ID NO: 355 umfasst; oder
(ii) das erste Fc-Polypeptid die Sequenz von SEQ ID NO: 233 umfasst und das zweite Fc-Polypeptid die Sequenz von SEQ ID NO: 356 umfasst; oder
(iii) das erste Fc-Polypeptid die Sequenz von SEQ ID NO: 309 umfasst und das zweite Fc-Polypeptid die Sequenz von SEQ ID NO: 355 umfasst; oder
(iv) das erste Fc-Polypeptid die Sequenz von SEQ ID NO: 309 umfasst und das zweite Fc-Polypeptid die Sequenz von SEQ ID NO: 356 umfasst; oder
(v) das erste Fc-Polypeptid die Sequenz von SEQ ID NO: 239 umfasst und das zweite Fc-Polypeptid die Sequenz von SEQ ID NO: 352 umfasst; oder
(vi) das erste Fc-Polypeptid die Sequenz von SEQ ID NO: 239 umfasst und das zweite Fc-Polypeptid die Sequenz von SEQ ID NO: 353 umfasst; oder
(vii) das erste Fc-Polypeptid die Sequenz von SEQ ID NO: 312 umfasst und das zweite Fc-Polypeptid die Sequenz von SEQ ID NO: 352 umfasst; oder
(viii) das erste Fc-Polypeptid die Sequenz von SEQ ID NO: 312 umfasst und das zweite Fc-Polypeptid die Sequenz von SEQ ID NO: 353 umfasst;
(n) wobei:
(i) das erste Fc-Polypeptid die Sequenz von SEQ ID NO: 170 umfasst und das zweite Fc-Polypeptid die Sequenz von SEQ ID NO: 355 umfasst; oder
(ii) das erste Fc-Polypeptid die Sequenz von SEQ ID NO: 170 umfasst und das zweite Fc-Polypeptid die Sequenz von SEQ ID NO: 356 umfasst; oder
(iii) das erste Fc-Polypeptid die Sequenz von SEQ ID NO: 281 umfasst und das zweite Fc-Polypeptid die Sequenz von SEQ ID NO: 355 umfasst; oder
(iv) das erste Fc-Polypeptid die Sequenz von SEQ ID NO: 281 umfasst und das zweite Fc-Polypeptid die Sequenz von SEQ ID NO: 356 umfasst; oder
(v) das erste Fc-Polypeptid die Sequenz von SEQ ID NO: 176 umfasst und das zweite Fc-Polypeptid die Sequenz von SEQ ID NO: 352 umfasst; oder
(vi) das erste Fc-Polypeptid die Sequenz von SEQ ID NO: 176 umfasst und das zweite Fc-Polypeptid die Sequenz von SEQ ID NO: 353 umfasst; oder
(vii) das erste Fc-Polypeptid die Sequenz von SEQ ID NO: 284 umfasst und das zweite Fc-Polypeptid die Sequenz von SEQ ID NO: 352 umfasst; oder
(viii) das erste Fc-Polypeptid die Sequenz von SEQ ID NO: 284 umfasst und das zweite Fc-Polypeptid die Sequenz von SEQ ID NO: 353 umfasst;
(o) wobei:
(i) das erste Fc-Polypeptid die Sequenz von SEQ ID NO: 122 umfasst und das zweite Fc-Polypeptid die Sequenz von SEQ ID NO: 355 umfasst; oder
(ii) das erste Fc-Polypeptid die Sequenz von SEQ ID NO: 122 umfasst und das zweite Fc-Polypeptid die Sequenz von SEQ ID NO: 356 umfasst; oder
(iii) das erste Fc-Polypeptid die Sequenz von SEQ ID NO: 253 umfasst und das zweite Fc-Polypeptid die Sequenz von SEQ ID NO: 355 umfasst; oder
(iv) das erste Fc-Polypeptid die Sequenz von SEQ ID NO: 253 umfasst und das zweite Fc-Polypeptid die Sequenz von SEQ ID NO: 356 umfasst; oder
(v) das erste Fc-Polypeptid die Sequenz von SEQ ID NO: 128 umfasst und das zweite Fc-Polypeptid die Sequenz von SEQ ID NO: 352 umfasst; oder
(vi) das erste Fc-Polypeptid die Sequenz von SEQ ID NO: 128 umfasst und das zweite Fc-Polypeptid die Sequenz von SEQ ID NO: 353 umfasst; oder
(vii) das erste Fc-Polypeptid die Sequenz von SEQ ID NO: 256 umfasst und das zweite Fc-Polypeptid die Sequenz von SEQ ID NO: 352 umfasst; oder
(viii) das erste Fc-Polypeptid die Sequenz von SEQ ID NO: 256 umfasst und das zweite Fc-Polypeptid die Sequenz von SEQ ID NO: 353 umfasst;
(p) wobei das erste Fc-Polypeptid die Sequenz von SEQ ID NO: 347 umfasst und das zweite Fc-Polypeptid die Sequenz von SEQ ID NO: 350 umfasst; oder
(q) wobei das erste Fc-Polypeptid die Sequenz von SEQ ID NO: 348 umfasst und das zweite Fc-Polypeptid die Sequenz von SEQ ID NO: 349 umfasst.

10. Fc-Polypeptiddimer nach Anspruch 7 oder 8, wobei das erste Fc-Polypeptid die Sequenz von SEQ ID NO: 260 umfasst und optional das zweite Fc-Polypeptid die Sequenz von SEQ ID NO: 356 umfasst.

11. Fc-Polypeptiddimer nach Anspruch 7 oder 8, wobei das erste Fc-Polypeptid die Sequenz von SEQ ID NO: 134 umfasst und optional das zweite Fc-Polypeptid die Sequenz von SEQ ID NO: 355 umfasst.

12. Fc-Polypeptiddimer nach Anspruch 7 oder 8, wobei das erste Fc-Polypeptid die Sequenz von SEQ ID NO: 122 umfasst und optional das zweite Fc-Polypeptid die Sequenz von SEQ ID NO: 355 umfasst.

13. Fc-Polypeptiddimer nach Anspruch 7 oder 8, wobei das erste Fc-Polypeptid die Sequenz von SEQ ID NO: 253 umfasst und optional das zweite Fc-Polypeptid die Sequenz von SEQ ID NO: 356 umfasst.

14. Fc-Polypeptiddimer nach Anspruch 7 oder 8, wobei das erste Fc-Polypeptid die Sequenz von SEQ ID NO: 146 umfasst und optional das zweite Fc-Polypeptid die Sequenz von SEQ ID NO: 355 umfasst.

15. Fc-Polypeptiddimer nach Anspruch 7 oder 8, wobei das erste Fc-Polypeptid die Sequenz von SEQ ID NO: 267 umfasst und optional das zweite Fc-Polypeptid die Sequenz von SEQ ID NO: 356 umfasst.

16. Fc-Polypeptiddimer nach Anspruch 7, wobei das Dimer für die TfR-Bindung bivalent ist; optional wobei:
(a) das zweite Fc-Polypeptid die Sequenz von SEQ ID NO: 347 oder 348 umfasst;
(b) das erste Fc-Polypeptid die Sequenz von SEQ ID NO: 347 umfasst und das zweite Fc-Polypeptid die Sequenz von SEQ ID NO: 348 umfasst; und/oder
(c) das erste und das zweite Fc-Polypeptid die gleiche TfR-Bindungsstelle umfassen.

17. Fc-Polypeptid-Dimer-Fab-Fusionsprotein, das Folgendes umfasst:
(a) eine variable Antikörperregion, die in der Lage ist, ein Antigen oder ein antigenbindendes Fragment davon zu binden; und
(b) ein Fc-Polypeptiddimer nach einem der Ansprüche 7 bis 16;
wobei optional:
(a) die variable Region des Antikörpers einen Teil einer Fab-Domäne bildet; und/oder
(b) die variable Region des Antikörpers zwei variable Regionen der schweren Kette des Antikörpers und zwei variable Regionen der leichten Kette des Antikörpers oder entsprechende Fragmente davon umfasst.

18. Polynukleotid, das Folgendes umfasst: eine Nukleinsäuresequenz, die für das Polypeptid nach einem der Ansprüche 1 bis 6 codiert; oder einen Vektor oder eine Wirtszelle, der/die das Polynukleotid umfasst.

19. Verfahren zur Herstellung eines Polypeptids, das spezifisch an einen Transferrinrezeptor bindet, umfassend die Kultivierung einer Wirtszelle unter Bedingungen, unter denen das durch das Polynukleotid nach Anspruch 18 codierte Polypeptid exprimiert wird.

20. Pharmazeutische Zusammensetzung, die Folgendes umfasst: das Polypeptid nach einem der Ansprüche 1 bis 6, das Fc-Polypeptiddimer nach einem der Ansprüche 7 bis 16 oder das Fc-Polypeptiddimer-Fab-Fusionsprotein nach Anspruch 17; und einen pharmazeutisch annehmbaren Träger.

21. Zusammensetzung, die Folgendes umfasst: das Polypeptid nach einem der Ansprüche 1 bis 6, das Fc-Polypeptiddimer nach einem der Ansprüche 7 bis 16 oder das Fc-Polypeptiddimer-Fab-Fusionsprotein nach Anspruch 17, zur Verwendung in einem Verfahren zur Transzytose einer Zusammensetzung durch ein Endothel, wobei das Verfahren das Inkontaktbringen des Endothels mit der Zusammensetzung umfasst;
wobei es sich bei dem Endothel optional um die Blut-Hirn-Schranke (Blood-Brain Barrier, BBB) handelt.

## Revendications

1. Polypeptide qui se lie spécifiquement à un récepteur de la transferrine, comprenant une séquence ayant au moins 85 %, 90 % ou 95 % d'identité de séquence avec la séquence de l'une quelconque des SEQ ID NO : 83, 84, 191, 43 et 82, dans lequel le polypeptide comprend Glu en position 153, Tyr ou Phe en position 157, Thr en position 159, Glu en position 160, Trp en position 161, Val, Ser ou Ala en position 162, Asn en position 163, Thr ou Ser en position 186, Glu en position 188, Glu en position 189 et Phe en position 194, tels que numérotés en référence à SEQ ID NO:1.

2. Polypeptide selon la revendication 1, comprenant en outre :
(a) Trp en position 139, tel que numéroté en référence à SEQ ID NO : 1 ;
(b) Ala en position 7, Ala en position 8 et Trp en position 139, tels que numérotés en référence à SEQ ID NO : 1;
(c) Ala en position 7, Ala en position 8, Gly en position 102 et Trp en position 139, tels que numérotés en référence à SEQ ID NO : 1;
(d) Ala en position 7, Ala en position 8, Trp en position 139, Leu en position 201 et Ser en position 207, tels que numérotés en référence à SEQ ID NO : 1;
(e) Ala en position 7, Ala en position 8, Gly en position 102, Trp en position 139, Leu en position 201 et Ser en position 207, tels que numérotés en référence à SEQ ID NO : 1;
(f) Ser en position 139, Ala en position 141 et Val en position 180, tels que numérotés en référence à SEQ ID NO : 1;
(g) Ala en position 7, Ala en position 8, Ser en position 139, Ala en position 141 et Val en position 180, tels que numérotés en référence à SEQ ID NO : 1;
(h) Ala en position 7, Ala en position 8, Gly en position 102, Ser en position 139, Ala en position 141 et Val en position 180, tels que numérotés en référence à SEQ ID NO : 1;
(i) Ala en position 7, Ala en position 8, Ser en position 139, Ala en position 141, Val en position 180, Leu en position 201 et Ser en position 207, tels que numérotés en référence à SEQ ID NO : 1 ; ou
(j) Ala en position 7, Ala en position 8, Gly en position 102, Ser en position 139, Ala en position 141, Val en position 180, Leu en position 201 et Ser en position 207, tels que numérotés en référence à SEQ ID NO : 1.

3. Polypeptide selon la revendication 1, comprenant :
A. une séquence ayant au moins 85 %, 90 % ou 95 % d'identité de séquence avec la séquence de SEQ ID NO : 83, dans laquelle le polypeptide comprend Glu en position 153, Tyr en position 157, Thr en position 159, Glu en position 160, Trp en position 161, Val en position 162, Asn en position 163, Thr en position 186, Glu en position 188, Glu en position 189 et Phe en position 194, tels que numérotés en référence à SEQ ID NO : 1, éventuellement comprenant en outre :
(a) Trp en position 139, tel que numéroté en référence à SEQ ID NO : 1, éventuellement dans lequel le polypeptide comprend la séquence de SEQ ID NO : 133 ;
(b) Ala en position 7, Ala en position 8 et Trp en position 139, tels que numérotés en référence à SEQ ID NO : 1, éventuellement dans lequel le polypeptide comprend la séquence de SEQ ID NO : 134 ;
(c) Ala en position 7, Ala en position 8, Gly en position 102 et Trp en position 139, tels que numérotés en référence à SEQ ID NO : 1, éventuellement dans lequel le polypeptide comprend la séquence de SEQ ID NO : 135 ;
(d) Ala en position 7, Ala en position 8, Trp en position 139, Leu en position 201 et Ser en position 207, tels que numérotés en référence à SEQ ID NO : 1, éventuellement dans lequel le polypeptide comprend la séquence de SEQ ID NO : 260 ;
(e) Ala en position 7, Ala en position 8, Gly en position 102, Trp en position 139, Leu en position 201 et Ser en position 207, tels que numérotés en référence à SEQ ID NO : 1, éventuellement dans lequel le polypeptide comprend la séquence de SEQ ID NO : 261 ;
(f) Ser en position 139, Ala en position 141 et Val en position 180, tels que numérotés en référence à SEQ ID NO : 1, éventuellement dans lequel le polypeptide comprend la séquence de SEQ ID NO : 139 ;
(g) Ala en position 7, Ala en position 8, Ser en position 139, Ala en position 141 et Val en position 180, tels que numérotés en référence à SEQ ID NO : 1, éventuellement dans lequel le polypeptide comprend la séquence de SEQ ID NO : 140 ;
(h) Ala en position 7, Ala en position 8, Gly en position 102, Ser en position 139, Ala en position 141 et Val en position 180, tels que numérotés en référence à SEQ ID NO : 1, éventuellement dans lequel le polypeptide comprend la séquence de SEQ ID NO : 141 ;
(i) Ala en position 7, Ala en position 8, Ser en position 139, Ala en position 141, Val en position 180, Leu en position 201 et Ser en position 207, tels que numérotés en référence à SEQ ID NO : 1, éventuellement dans laquelle le polypeptide comprend la séquence de SEQ ID NO : 263 ; ou
(j) Ala en position 7, Ala en position 8, Gly en position 102, Ser en position 139, Ala en position 141, Val en position 180, Leu en position 201 et Ser en position 207, numérotés en référence à SEQ ID NO : 1, éventuellement dans lequel le polypeptide comprend la séquence de SEQ ID NO : 264 ; ou
B. une séquence ayant au moins 85 %, 90 % ou 95 % d'identité de séquence avec la séquence de SEQ ID NO : 84, dans lequel le polypeptide comprend Glu en position 153, Tyr en position 157, Thr en position 159, Glu en position 160, Trp en position 161, Ser en position 162, Asn en position 163, Ser en position 186, Glu en position 188, Glu en position 189 et Phe en position 194, tels que numérotés en référence à SEQ ID NO : 1, éventuellement comprenant en outre :
(a) Trp en position 139, tel que numéroté en référence à SEQ ID NO : 1, éventuellement dans lequel le polypeptide comprend la séquence de SEQ ID NO : 145 ;
(b) Ala en position 7, Ala en position 8 et Trp en position 139, tels que numérotés en référence à SEQ ID NO : 1, éventuellement dans lequel le polypeptide comprend la séquence de SEQ ID NO : 146 ;
(c) Ala en position 7, Ala en position 8, Gly en position 102 et Trp en position 139, tels que numérotés en référence à SEQ ID NO : 1, éventuellement dans lequel le polypeptide comprend la séquence de SEQ ID NO : 147 ;
(d) Ala en position 7, Ala en position 8, Trp en position 139, Leu en position 201 et Ser en position 207, tels que numérotés en référence à SEQ ID NO : 1, éventuellement dans lequel le polypeptide comprend la séquence de SEQ ID NO : 267 ;
(e) Ala en position 7, Ala en position 8, Gly en position 102, Trp en position 139, Leu en position 201 et Ser en position 207, tels que numérotés en référence à SEQ ID NO : 1, éventuellement dans lequel le polypeptide comprend la séquence de SEQ ID NO : 268 ;
(f) Ser en position 139, Ala en position 141 et Val en position 180, tels que numérotés en référence à SEQ ID NO : 1, éventuellement dans lequel le polypeptide comprend la séquence de SEQ ID NO : 151 ;
(g) Ala en position 7, Ala en position 8, Ser en position 139, Ala en position 141 et Val en position 180, tels que numérotés en référence à SEQ ID NO : 1, éventuellement dans lequel le polypeptide comprend la séquence de SEQ ID NO : 152 ;
(h) Ala en position 7, Ala en position 8, Gly en position 102, Ser en position 139, Ala en position 141 et Val en position 180, tels que numérotés en référence à SEQ ID NO : 1, éventuellement dans lequel le polypeptide comprend la séquence de SEQ ID NO : 153 ;
(i) Ala en position 7, Ala en position 8, Ser en position 139, Ala en position 141, Val en position 180, Leu en position 201 et Ser en position 207, tels que numérotés en référence à SEQ ID NO : 1, éventuellement dans lequel le polypeptide comprend la séquence de SEQ ID NO : 270 ; ou
(j) Ala en position 7, Ala en position 8, Gly en position 102, Ser en position 139, Ala en position 141, Val en position 180, Leu en position 201 et Ser en position 207, tel que numérotés en référence à SEQ ID NO : 1, éventuellement dans lequel le polypeptide comprend la séquence de SEQ ID NO : 271 ; ou
C. une séquence ayant au moins 85 %, 90 % ou 95 % d'identité de séquence avec la séquence de SEQ ID NO : 191, dans lequel le polypeptide comprend Glu en position 153, Phe en position 157, Thr en position 159, Glu en position 160, Trp en position 161, Ser en position 162, Asn en position 163, Ser en position 186, Glu en position 188, Glu en position 189 et Phe en position 194, tels que numérotés en référence à SEQ ID NO : 1, éventuellement comprenant en outre :
(a) Trp en position 139, tel que numéroté en référence à SEQ ID NO : 1, éventuellement dans lequel le polypeptide comprend la séquence de SEQ ID NO : 232 ;
(b) Ala en position 7, Ala en position 8 et Trp en position 139, tels que numérotés en référence à SEQ ID NO : 1, éventuellement dans lequel le polypeptide comprend la séquence de SEQ ID NO : 233 ;
(c) Ala en position 7, Ala en position 8, Gly en position 102 et Trp en position 139, tels que numérotés en référence à SEQ ID NO : 1, éventuellement dans lequel le polypeptide comprend la séquence de SEQ ID NO : 234 ;
(d) Ala en position 7, Ala en position 8, Trp en position 139, Leu en position 201 et Ser en position 207, tels que numérotés en référence à SEQ ID NO : 1, éventuellement dans lequel le polypeptide comprend la séquence de SEQ ID NO : 309 ;
(e) Ala en position 7, Ala en position 8, Gly en position 102, Trp en position 139, Leu en position 201 et Ser en position 207, tels que numérotés en référence à SEQ ID NO : 1, éventuellement dans lequel le polypeptide comprend la séquence de SEQ ID NO : 310 ;
(f) Ser en position 139, Ala en position 141 et Val en position 180, tels que numérotés en référence à SEQ ID NO : 1, éventuellement dans lequel le polypeptide comprend la séquence de SEQ ID NO : 238 ;
(g) Ala en position 7, Ala en position 8, Ser en position 139, Ala en position 141 et Val en position 180, tels que numérotés en référence à SEQ ID NO : 1, éventuellement dans lequel le polypeptide comprend la séquence de SEQ ID NO : 239 ;
(h) Ala en position 7, Ala en position 8, Gly en position 102, Ser en position 139, Ala en position 141 et Val en position 180, tels que numérotés en référence à SEQ ID NO : 1, éventuellement dans lequel le polypeptide comprend la séquence de SEQ ID NO : 240 ;
(i) Ala en position 7, Ala en position 8, Ser en position 139, Ala en position 141, Val en position 180, Leu en position 201 et Ser en position 207, tels que numérotés en référence à SEQ ID NO : 1, éventuellement dans lequel le polypeptide comprend la séquence de SEQ ID NO : 312 ; ou
(j) Ala en position 7, Ala en position 8, Gly en position 102, Ser en position 139, Ala en position 141, Val en position 180, Leu en position 201 et Ser en position 207, tels que numérotés en référence à SEQ ID NO : 1, éventuellement dans lequel le polypeptide comprend la séquence de SEQ ID NO : 313 ; ou
D. une séquence ayant au moins 85 %, 90 % ou 95 % d'identité de séquence avec la séquence de SEQ ID NO : 43, dans lequel le polypeptide comprend Glu en position 153, Tyr en position 157, Thr en position 159, Glu en position 160, Trp en position 161, Ser en position 162, Asn en position 163, Thr en position 186, Glu en position 188, Glu en position 189 et Phe en position 194, tels que numérotés en référence à SEQ ID NO : 1, éventuellement comprenant en outre :
(a) Trp en position 139, tel que numéroté en référence à SEQ ID NO : 1, éventuellement dans lequel le polypeptide comprend la séquence de SEQ ID NO : 169 ;
(b) Ala en position 7, Ala en position 8 et Trp en position 139, tels que numérotés en référence à SEQ ID NO : 1, éventuellement dans lequel le polypeptide comprend la séquence de SEQ ID NO : 170 ;
(c) Ala en position 7, Ala en position 8, Gly en position 102 et Trp en position 139, tels que numérotés en référence à SEQ ID NO : 1, éventuellement dans lequel le polypeptide comprend la séquence de SEQ ID NO : 171 ;
(d) Ala en position 7, Ala en position 8, Trp en position 139, Leu en position 201 et Ser en position 207, tels que numérotés en référence à SEQ ID NO : 1, éventuellement dans lequel le polypeptide comprend la séquence de SEQ ID NO : 281 ;
(e) Ala en position 7, Ala en position 8, Gly en position 102, Trp en position 139, Leu en position 201 et Ser en position 207, tels que numérotés en référence à SEQ ID NO : 1, éventuellement dans lequel le polypeptide comprend la séquence de SEQ ID NO : 282 ;
(f) Ser en position 139, Ala en position 141 et Val en position 180, tels que numérotés en référence à SEQ ID NO : 1, éventuellement dans lequel le polypeptide comprend la séquence de SEQ ID NO : 175 ;
(g) Ala en position 7, Ala en position 8, Ser en position 139, Ala en position 141 et Val en position 180, tels que numérotés en référence à SEQ ID NO : 1, éventuellement dans lequel le polypeptide comprend la séquence de SEQ ID NO : 176 ;
(h) Ala en position 7, Ala en position 8, Gly en position 102, Ser en position 139, Ala en position 141 et Val en position 180, tels que numérotés en référence à SEQ ID NO : 1, éventuellement dans lequel le polypeptide comprend la séquence de SEQ ID NO : 177 ;
(i) Ala en position 7, Ala en position 8, Ser en position 139, Ala en position 141, Val en position 180, Leu en position 201 et Ser en position 207, tels que numérotés en référence à SEQ ID NO : 1, éventuellement dans lequel le polypeptide comprend la séquence de SEQ ID NO : 284 ; ou
(j) Ala en position 7, Ala en position 8, Gly en position 102, Ser en position 139, Ala en position 141, Val en position 180, Leu en position 201 et Ser en position 207, tels que numérotés en référence à SEQ ID NO : 1, éventuellement dans lequel le polypeptide comprend la séquence de SEQ ID NO : 285 ; ou
E. une séquence ayant au moins 85 %, 90 % ou 95 % d'identité de séquence avec la séquence de SEQ ID NO : 82, dans lequel le polypeptide comprend Glu en position 153, Tyr en position 157, Thr en position 159, Glu en position 160, Trp en position 161, Ala en position 162, Asn en position 163, Thr en position 186, Glu en position 188, Glu en position 189 et Phe en position 194, tels que numérotés en référence à SEQ ID NO : 1, éventuellement comprenant en outre :
(a) Trp en position 139, tel que numéroté en référence à SEQ ID NO : 1, éventuellement dans lequel le polypeptide comprend la séquence de SEQ ID NO : 121 ;
(b) Ala en position 7, Ala en position 8 et Trp en position 139, tels que numérotés en référence à SEQ ID NO : 1, éventuellement dans lequel le polypeptide comprend la séquence de SEQ ID NO : 122 ;
(c) Ala en position 7, Ala en position 8, Gly en position 102 et Trp en position 139, tels que numérotés en référence à SEQ ID NO : 1, éventuellement dans lequel le polypeptide comprend la séquence de SEQ ID NO : 123 ;
(d) Ala en position 7, Ala en position 8, Trp en position 139, Leu en position 201 et Ser en position 207, tels que numérotés en référence à SEQ ID NO : 1, éventuellement dans lequel le polypeptide comprend la séquence de SEQ ID NO : 253 ;
(e) Ala en position 7, Ala en position 8, Gly en position 102, Trp en position 139, Leu en position 201 et Ser en position 207, tels que numérotés en référence à SEQ ID NO : 1, éventuellement dans lequel le polypeptide comprend la séquence de SEQ ID NO : 254 ;
(f) Ser en position 139, Ala en position 141 et Val en position 180, tels que numérotés en référence à SEQ ID NO : 1, éventuellement dans lequel le polypeptide comprend la séquence de SEQ ID NO : 127 ;
(g) Ala en position 7, Ala en position 8, Ser en position 139, Ala en position 141 et Val en position 180, tels que numérotés en référence à SEQ ID NO : 1, éventuellement dans lequel le polypeptide comprend la séquence de SEQ ID NO : 128 ; ou
(h) Ala en position 7, Ala en position 8, Gly en position 102, Ser en position 139, Ala en position 141 et Val en position 180, tels que numérotés en référence à SEQ ID NO : 1, éventuellement dans lequel le polypeptide comprend la séquence de SEQ ID NO : 129 ; ou
(i) Ala en position 7, Ala en position 8, Ser en position 139, Ala en position 141, Val en position 180, Leu en position 201 et Ser en position 207, tels que numérotés en référence à SEQ ID NO : 1, éventuellement dans lequel le polypeptide comprend la séquence de SEQ ID NO : 256 ; ou
(j) Ala en position 7, Ala en position 8, Gly en position 102, Ser en position 139, Ala en position 141, Val en position 180, Leu en position 201 et Ser en position 207, tels que numérotés en référence à SEQ ID NO : 1, éventuellement dans lequel le polypeptide comprend la séquence de SEQ ID NO : 257.

4. Polypeptide selon l'une quelconque des revendications 1 à 3, dans lequel le polypeptide ne comprend pas les trois premiers acides aminés « PCP » à l'extrémité amino-terminale de la séquence, comprenant éventuellement en outre une région variable de chaîne lourde d'anticorps.

5. Polypeptide qui se lie spécifiquement à un récepteur de la transferrine,
(a) comprenant la séquence de SEQ ID NO : 347 ; ou
(b) comprenant la séquence de SEQ ID NO : 348 ;
comprenant éventuellement en outre une région variable de chaîne lourde d'anticorps.

6. Polypeptide comprenant de l'extrémité N à C : une région variable de chaîne lourde d'anticorps, un domaine CH1, une région charnière et le polypeptide selon l'une quelconque des revendications 1 à 5.

7. Dimère de polypeptide Fc, ou un fragment dimère de celui-ci, comprenant :
(a) un premier polypeptide Fc comprenant le polypeptide selon l'une quelconque des revendications 1 à 6 ; et
(b) un second polypeptide Fc capable de se dimériser avec le premier polypeptide Fc de (a).

8. Dimère de polypeptide Fc selon la revendication 7, dans lequel :
(a) le dimère est monovalent pour la liaison de TfR ; et/ou
(b) le second polypeptide Fc comprend la séquence de l'une quelconque des SEQ ID NO : 351 à 356.

9. Dimère de polypeptide Fc selon la revendication 7 ou 8 :
(a) dans lequel le premier polypeptide Fc comprend la séquence de l'une quelconque des SEQ ID NO : 133, 134 et 260 ; et le second polypeptide Fc comprend la séquence de l'une quelconque des SEQ ID NO : 354 à 356 ;
(b) dans lequel le premier polypeptide Fc comprend la séquence de l'une quelconque des SEQ ID NO : 145, 146 et 267 ; et le second polypeptide Fc comprend la séquence de l'une quelconque des SEQ ID NO : 354 à 356 ;
(c) dans lequel le premier polypeptide Fc comprend la séquence de l'une quelconque des SEQ ID NO : 232, 233 et 309 ; et le second polypeptide Fc comprend la séquence de l'une quelconque des SEQ ID NO : 354 à 356 ;
(d) dans lequel le premier polypeptide Fc comprend la séquence de l'une quelconque des SEQ ID NO : 169, 170 et 281 ; et le second polypeptide Fc comprend la séquence de l'une quelconque des SEQ ID NO : 354 à 356 ;
(e) dans lequel le premier polypeptide Fc comprend la séquence de l'une quelconque des SEQ ID NO : 121, 122 et 253 ; et le second polypeptide Fc comprend la séquence de l'une quelconque des SEQ ID NO : 354 à 356 ;
(f) dans lequel le premier polypeptide Fc comprend la séquence de l'une quelconque des SEQ ID NO : 139, 140 et 263 ; et le second polypeptide Fc comprend la séquence de l'une quelconque des SEQ ID NO : 351 à 353 ;
(g) dans lequel le premier polypeptide Fc comprend la séquence de l'une quelconque des SEQ ID NO : 151, 152 et 270 ; et le second polypeptide Fc comprend la séquence de l'une quelconque des SEQ ID NO : 351 à 353 ;
(h) dans lequel le premier polypeptide Fc comprend la séquence de l'une quelconque des SEQ ID NOS : 238, 239 et 312 ; et le second polypeptide Fc comprend la séquence de l'une quelconque des SEQ ID NO : 351 à 353 ;
(i) dans lequel le premier polypeptide Fc comprend la séquence de l'une quelconque des SEQ ID NO : 175, 176 et 284 ; et le second polypeptide Fc comprend la séquence de l'une quelconque des SEQ ID NO : 351 à 353 ;
(j) dans lequel le premier polypeptide Fc comprend la séquence de l'une quelconque des SEQ ID NO : 127, 128 et 256 ; et le second polypeptide Fc comprend la séquence de l'une quelconque des SEQ ID NO : 351 à 353 ;
(k) dans lequel :
(i) le premier polypeptide Fc comprend la séquence de SEQ ID NO : 134 et le second polypeptide Fc comprend la séquence de SEQ ID NO : 355 ; ou
(ii) le premier polypeptide Fc comprend la séquence de SEQ ID NO : 134 et le second polypeptide Fc comprend la séquence de SEQ ID NO : 356 ; ou
(iii) le premier polypeptide Fc comprend la séquence de SEQ ID NO : 260 et le second polypeptide Fc comprend la séquence de SEQ ID NO : 355 ; ou
(iv) le premier polypeptide Fc comprend la séquence de SEQ ID NO : 260 et le second polypeptide Fc comprend la séquence de SEQ ID NO : 356 ; ou
(v) le premier polypeptide Fc comprend la séquence de SEQ ID NO : 140 et le second polypeptide Fc comprend la séquence de SEQ ID NO : 352 ; ou
(vi) le premier polypeptide Fc comprend la séquence de SEQ ID NO : 140 et le second polypeptide Fc comprend la séquence de SEQ ID NO : 353 ; ou
(vii) le premier polypeptide Fc comprend la séquence de SEQ ID NO : 263 et le second polypeptide Fc comprend la séquence de SEQ ID NO : 352 ; ou
(viii) le premier polypeptide Fc comprend la séquence de SEQ ID NO : 263 et le second polypeptide Fc comprend la séquence de SEQ ID NO : 353 ;
(l) dans lequel :
(i) le premier polypeptide Fc comprend la séquence de SEQ ID NO : 146 et le second polypeptide Fc comprend la séquence de SEQ ID NO : 355 ; ou
(ii) le premier polypeptide Fc comprend la séquence de SEQ ID NO : 146 et le second polypeptide Fc comprend la séquence de SEQ ID NO : 356 ; ou
(iii) le premier polypeptide Fc comprend la séquence de SEQ ID NO : 267 et le second polypeptide Fc comprend la séquence de SEQ ID NO : 355 ; ou
(iv) le premier polypeptide Fc comprend la séquence de SEQ ID NO : 267 et le second polypeptide Fc comprend la séquence de SEQ ID NO : 356 ; ou
(v) le premier polypeptide Fc comprend la séquence de SEQ ID NO : 152 et le second polypeptide Fc comprend la séquence de SEQ ID NO : 352 ; ou
(vi) le premier polypeptide Fc comprend la séquence de SEQ ID NO : 152 et le second polypeptide Fc comprend la séquence de SEQ ID NO : 353 ; ou
(vii) le premier polypeptide Fc comprend la séquence de SEQ ID NO : 270 et le second polypeptide Fc comprend la séquence de SEQ ID NO : 352 ; ou
(viii) le premier polypeptide Fc comprend la séquence de SEQ ID NO : 270 et le second polypeptide Fc comprend la séquence de SEQ ID NO : 353 ;
(m) dans lequel :
(i) le premier polypeptide Fc comprend la séquence de SEQ ID NO : 233 et le second polypeptide Fc comprend la séquence de SEQ ID NO : 355 ; ou
(ii) le premier polypeptide Fc comprend la séquence de SEQ ID NO : 233 et le second polypeptide Fc comprend la séquence de SEQ ID NO : 356 ; ou
(iii) le premier polypeptide Fc comprend la séquence de SEQ ID NO : 309 et le second polypeptide Fc comprend la séquence de SEQ ID NO : 355 ; ou
(iv) le premier polypeptide Fc comprend la séquence de SEQ ID NO : 309 et le second polypeptide Fc comprend la séquence de SEQ ID NO : 356 ; ou
(v) le premier polypeptide Fc comprend la séquence de SEQ ID NO : 239 et le second polypeptide Fc comprend la séquence de SEQ ID NO : 352 ; ou
(vi) le premier polypeptide Fc comprend la séquence de SEQ ID NO : 239 et le second polypeptide Fc comprend la séquence de SEQ ID NO : 353 ; ou
(vii) le premier polypeptide Fc comprend la séquence de SEQ ID NO : 312 et le second polypeptide Fc comprend la séquence de SEQ ID NO : 352 ; ou
(viii) le premier polypeptide Fc comprend la séquence de SEQ ID NO : 312 et le second polypeptide Fc comprend la séquence de SEQ ID NO : 353 ;
(n) dans lequel :
(i) le premier polypeptide Fc comprend la séquence de SEQ ID NO : 170 et le second polypeptide Fc comprend la séquence de SEQ ID NO : 355 ; ou
(ii) le premier polypeptide Fc comprend la séquence de SEQ ID NO : 170 et le second polypeptide Fc comprend la séquence de SEQ ID NO : 356 ; ou
(iii) le premier polypeptide Fc comprend la séquence de SEQ ID NO : 281 et le second polypeptide Fc comprend la séquence de SEQ ID NO : 355 ; ou
(iv) le premier polypeptide Fc comprend la séquence de SEQ ID NO : 281 et le second polypeptide Fc comprend la séquence de SEQ ID NO : 356 ; ou
(v) le premier polypeptide Fc comprend la séquence de SEQ ID NO : 176 et le second polypeptide Fc comprend la séquence de SEQ ID NO : 352 ; ou
(vi) le premier polypeptide Fc comprend la séquence de SEQ ID NO : 176 et le second polypeptide Fc comprend la séquence de SEQ ID NO : 353 ; ou
(vii) le premier polypeptide Fc comprend la séquence de SEQ ID NO : 284 et le second polypeptide Fc comprend la séquence de SEQ ID NO : 352 ; ou
(viii) le premier polypeptide Fc comprend la séquence de SEQ ID NO : 284 et le second polypeptide Fc comprend la séquence de SEQ ID NO : 353 ;
(o) dans lequel :
(i) le premier polypeptide Fc comprend la séquence de SEQ ID NO : 122 et le second polypeptide Fc comprend la séquence de SEQ ID NO : 355 ; ou
(ii) le premier polypeptide Fc comprend la séquence de SEQ ID NO : 122 et le second polypeptide Fc comprend la séquence de SEQ ID NO : 356 ; ou
(iii) le premier polypeptide Fc comprend la séquence de SEQ ID NO : 253 et le second polypeptide Fc comprend la séquence de SEQ ID NO : 355 ; ou
(iv) le premier polypeptide Fc comprend la séquence de SEQ ID NO : 253 et le second polypeptide Fc comprend la séquence de SEQ ID NO : 356 ; ou
(v) le premier polypeptide Fc comprend la séquence de SEQ ID NO : 128 et le second polypeptide Fc comprend la séquence de SEQ ID NO : 352 ; ou
(vi) le premier polypeptide Fc comprend la séquence de SEQ ID NO : 128 et le second polypeptide Fc comprend la séquence de SEQ ID NO : 353 ; ou
(vii) le premier polypeptide Fc comprend la séquence de SEQ ID NO : 256 et le second polypeptide Fc comprend la séquence de SEQ ID NO : 352 ; ou
(viii) le premier polypeptide Fc comprend la séquence de SEQ ID NO : 256 et le second polypeptide Fc comprend la séquence de SEQ ID NO : 353 ;
(p) dans lequel le premier polypeptide Fc comprend la séquence de SEQ ID NO : 347 et le second polypeptide Fc comprend la séquence de SEQ ID NO : 350 ; ou
(q) dans lequel le premier polypeptide Fc comprend la séquence de SEQ ID NO : 348 et le second polypeptide Fc comprend la séquence de SEQ ID NO : 349.

10. Dimère de polypeptide Fc selon la revendication 7 ou 8, dans lequel le premier polypeptide Fc comprend la séquence de SEQ ID NO : 260, et éventuellement le second polypeptide Fc comprend la séquence de SEQ ID NO : 356.

11. Dimère de polypeptide Fc selon la revendication 7 ou 8, dans lequel le premier polypeptide Fc comprend la séquence de SEQ ID NO : 134, et éventuellement le second polypeptide Fc comprend la séquence de SEQ ID NO : 355.

12. Dimère de polypeptide Fc selon la revendication 7 ou 8, dans lequel le premier polypeptide Fc comprend la séquence de SEQ ID NO : 122, et éventuellement le second polypeptide Fc comprend la séquence de SEQ ID NO : 355.

13. Dimère de polypeptide Fc selon la revendication 7 ou 8, dans lequel le premier polypeptide Fe comprend la séquence de SEQ ID NO : 253, et éventuellement le second polypeptide Fc comprend la séquence de SEQ ID NO : 356.

14. Dimère de polypeptide Fc selon la revendication 7 ou 8, dans lequel le premier polypeptide Fc comprend la séquence de SEQ ID NO : 146, et éventuellement le second polypeptide Fc comprend la séquence de SEQ ID NO : 355.

15. Dimère de polypeptide Fc selon la revendication 7 ou 8, dans lequel le premier polypeptide Fc comprend la séquence de SEQ ID NO : 267, et éventuellement le second polypeptide Fc comprend la séquence de SEQ ID NO : 356.

16. Dimère de polypeptide Fc selon la revendication 7, dans lequel le dimère est bivalent pour la liaison au TfR ; éventuellement dans lequel :
(a) le second polypeptide Fc comprend la séquence de SEQ ID NO : 347 ou 348 ;
(b) le premier polypeptide Fc comprend la séquence de SEQ ID NO : 347 et le second polypeptide Fc comprend la séquence de SEQ ID NO : 348 ; et/ou
(c) les premier et second polypeptides Fc comprennent le même site de liaison TfR.

17. Protéine de fusion dimère de polypeptide Fc-Fab comprenant :
(a) une région variable d'anticorps qui est capable de se lier à un antigène, ou un fragment de liaison à l'antigène de celui-ci ; et
(b) un dimère de polypeptide Fc selon l'une quelconque des revendications 7 à 16 ; éventuellement dans lequel :
(a) la région variable de l'anticorps fait partie d'un domaine Fab ; et/ou
(b) la région variable d'anticorps comprend deux régions variables de chaîne lourde d'anticorps et deux régions variables de chaîne légère d'anticorps, ou des fragments respectifs de celles-ci.

18. Polynucléotide comprenant une séquence d'acide nucléique codant pour le polypeptide selon l'une quelconque des revendications 1 à 6 ; ou un vecteur ou une cellule hôte comprenant ledit polynucléotide.

19. Procédé de production d'un polypeptide qui se lie spécifiquement à un récepteur de la transferrine, comprenant la culture d'une cellule hôte dans des conditions dans lesquelles le polypeptide codé par le polynucléotide de la revendication 18 est exprimé.

20. Composition pharmaceutique comprenant : le polypeptide selon l'une quelconque des revendications 1 à 6, le dimère de polypeptide Fc selon l'une quelconque des revendications 7 à 16, ou la protéine de fusion dimère de polypeptide Fc-Fab selon la revendication 17 ; et un support pharmaceutiquement acceptable.

21. Composition comprenant le polypeptide selon l'une quelconque des revendications 1 à 6, le dimère de polypeptide Fc selon l'une quelconque des revendications 7 à 16, ou la protéine de fusion dimère de polypeptide Fc-Fab selon la revendication 17, pour une utilisation dans un procédé de transcytose d'une composition à travers un endothélium, le procédé comprenant la mise en contact de l'endothélium avec ladite composition ;
éventuellement dans lequel l'endothélium est la barrière hémato-encéphalique (BBB).
